# EUROPEAN PATENT APPLICATION

(11) **EP 3 831 940 A1**
(43) Date of publication of application: **09.06.2021**
(21) Application number: 19213724.8
(22) Date of filing: 05.12.2019
(51) Int. Cl.: C12N 15/10, C12N 15/11, A61K 31/415, A61K 31/445, A61K 31/454, A61K 31/7105, A61K 45/06, A61P 31/00, C12N 9/22, C12Q 1/68

(54) **SMALL MOLECULE INHIBITORS OF CRISPR-CAS ASSOCIATED ACTIVITY**

(71) Applicant: Danmarks Tekniske Universitet, 2800 Kgs. Lyngby (DK); University of Copenhagen, 1165 Copenhagen K (DK)
(72) Inventor: TRAN, Kim Tai, 2800 Kgs. Lyngby (DK); LEE, Sang-Woo, 2800 Kgs. Lyngby (DK); SOMMER, Morten, 2800 Kgs. Lyngby (DK); BACH, Anders, 2100 Copenhagen (DK); VAZQUEZ-URIBE, Ruben, 2800 Kgs. Lyngby (DK); VAN DER HELM, Eric, 2200 København N (DK)
(74) Representative: Guardian IP Consulting I/S

(57) **Abstract**

The invention relates to small-molecules having a biphenyl or pyrrolylthiazole core structure that are capable of regulating CRISPR-Cas associated activity, and their use in regulating CRISPR-Cas associated activity, in particular in the treatment of diseases, control of gene drives, gene editing, and other biotechnological applications.

## Description

### Technical field of the invention

The invention relates to small molecules having a biphenyl or pyrrolylthiazole core structure that are capable of regulating CRISPR-Cas associated activity, and their use in methods for regulating CRISPR-Cas associated activity, in particular in the treatment of diseases, control of gene drives, gene editing, and other biotechnological applications.

### Background of the invention

CRISPR-Cas systems originate from bacteria where they function as adaptive immune systems that cleave foreign DNA or RNA in a sequence specific manner. Components of type II CRISPR-Cas systems, such as Cas9 from *Streptoccocus pyogenes* (spCas9), have been exploited for precise and programmable gene editing with a substantial impact on life sciences. CRISPR-Cas technologies provide effective biotechnological tools that broadly impact life sciences. Applications range from treating human genetic diseases to eliminating pathogens using gene-drives. Beyond genome editing, CRISPR-Cas systems have several other applications, including programmable transcriptional regulators, and ultra-sensitive detection devices. However, there is increasing concern about the safety and precision of CRISPR-Cas. Indeed, the 2016 worldwide threat assessment of U.S. intelligence listed genome editing technologies as a potential weapon of mass destruction and proliferation. Accordingly, more tools are needed to control the CRISPR-Cas technology in order to reduce potential risks of genome editing, and to contain their spread and potential harm to other organisms.

A common challenge in employing CRISPR-Cas systems is precise control over their activity. Current inhibitors are small proteins found in nature that bind to the CRISPR-Cas machinery and thereby prevent its activity. These naturally occurring inhibitors termed anti-CRISPRs (ACRs) were initially discovered for type I CRISPR-Cas systems (Bondy et al., 2013). Subsequent bioinformatic mining identified ACRs against the type II systems, where Cas9 is the effector (Pawluk et al., 2016; Rauch et al., 2017; Hynes et al., 2017; and Hynes et al., 2018). This enabled new applications, such as specifically blocking SpCas9 binding to double-stranded DNA or limiting the amount of time that SpCas9 is active in the nucleus to reduce off-target gene editing (Rauch et al., 2017). The importance and potential of SpCas9 inhibitors have awaken an interest in identifying different ways of controlling both CRISPR-Cas and CRISPR-dCas activity in different manners. Accordingly, the present invention addresses the need for identifying new inhibitors, that can be used to regulate CRISPR-cas in its diverse applications.

### Summary of the invention

A first embodiment of the invention provides a method for regulating CRISPR-Cas associated activity and/or CRISPR-dCas associated activity, the method comprising contacting an RNA guided endonuclease-guide RNA complex or an RNA guided DNA binding-guide RNA complex, respectively with a small molecule, wherein said molecule is a compound having formula I: 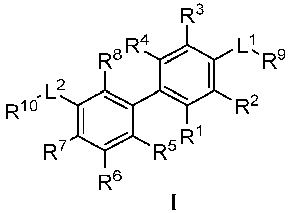 wherein
**R¹, R², R³, R⁴, R⁵, R⁶, R⁷,** and **R⁸** are each independently selected from R¹¹;
**R⁹** is R¹¹ or R¹²;
**R¹⁰** is selected from among: R¹¹, R¹², and -NR¹³R¹⁴;
**L¹** is selected from among: absent, -S(=O)₂N(R¹⁵)-, -C(=O)-, -C(=O)N(R¹⁶)-, - CH₂N(R¹⁷)-, -N(R¹⁷)CH₂-, -CH₂O-, -OCH₂-, C₁₋₃ alkylene, C₂₋₃ alkenylene having one double bond, and C₂₋₃ alkynylene having one triple bond;
**L²** is selected from among: absent, -C(=O)-, -S(=O)₂-, and C₁₋₃ alkylene;
**R¹¹** is selected from among: H, halo, -NO₂, -CN, -OR¹⁸, -NR¹⁹R²¹, -C(=O)OR²¹, - C(=O)NR²²R²³, Ph, -OPh,
C₁₋₈ alkyl optionally substituted with one to three halo and/or -OH groups,
C₃₋₈ cycloalkyl optionally substituted with one to three halo and/or -OH groups,
4-8 membered heterocycloalkyl comprising one to three heteroatoms independently selected from among N, O, and S, and
5-6 membered heteroaryl comprising one to three heteroatoms independently selected from among N, O, and S;
**R¹²** is selected from among:
   - C₁₋₈ alkyl optionally substituted with one to three groups independently selected from among:
      halo, -OR²⁴, -C(=O)OR²⁵, -C(=O)NR²⁶R²⁷, C₃₋₈ cycloalkyl, C₂₋₄ alkenylene having one double bond, C₂₋₄ alkynylene having one triple bond,
      4-6 membered heterocycloalkyl comprising one to three heteroatoms independently selected from among N, O, and S,
      C₆₋₁₀ aryl optionally substituted with one or more independently selected R¹¹ groups, and
      5-6 membered heteroaryl comprising one to four heteroatoms independently selected from N, O, and S;
   - C₃₋₈ cycloalkyl, optionally substituted with one or more independently selected R¹¹ groups;
   - C₄₋₈ cycloalkenyl comprising one double bond, optionally substituted with one or more independently selected R¹¹ groups;
   - 4-8 membered heterocycloalkyl comprising one to three heteroatoms independently selected from N, O, and S, optionally substituted with one or more independently selected R¹¹ groups;
   - C₆₋₁₀ aryl optionally substituted with one or more independently selected R¹¹ groups; and
   - 5-10 membered heteroaryl comprising one to three heteroatoms independently selected from N, O, and S, optionally substituted with one or more independently selected R¹¹ groups;
**R¹³** and **R¹⁴** are each independently selected from H and R¹²;
**R¹⁵, R¹⁶, R¹⁷, R¹⁸, R¹⁹, R²⁰, R²¹, R²², R²³, R²⁴, R²⁵, R²⁶,** and **R²⁷** are each independently selected from H and C₁₋₈ alkyl optionally substituted with one or more halo and/or -OH groups.

Said CRISPR-Cas associated activity or CRISPR-dCas associated activity may be expressed *in vitro,* or in a biological cell selected from the group consisting of a bacterial, yeast, fungal, insect, plant, and somatic mammalian cell.

A second embodiment of the invention provides a compound selected from the group consisting of: *N*-(5-methylisoxazol-3-yl)-3'-(4-methylpiperidine-1-carbonyl)-[1,1'-biphenyl]-4-sulfonamide; *N*-(isoxazol-3-yl)-3'-(4-methylpiperidine-1-carbonyl)-[1,1'-biphenyl]-4-sulfonamide; 3'-(4-methylpiperidine-1-carbonyl)-[1,1'-biphenyl]-4-sulfonamide; *N*-(5-methylisoxazol-3-yl)-3'-(piperidine-1-carbonyl)-[1,1'-biphenyl]-4-sulfonamide; 4'-(*N*-(5-methylisoxazol-3-yl)sulfamoyl)-[1,1'-biphenyl]-3-carboxamide; *N*-(isoxazol-3-yl)-[1,1'-biphenyl]-4-sulfonamide; *N*-(5-methylisoxazol-3-yl)-[1,1'-biphenyl]-3-sulfonamide; 1-([1,1'-biphenyl]-3-ylsulfonyl)-4-methylpiperidine; and *N-*(5-methylisoxazol-3-yl)-[1,1'-biphenyl]-3-carboxamide and *N*-([1,1'-biphenyl]-3-ylmethyl)-5-methylisoxazol-3-amine.

A third embodiment of the invention provides a compound for use as a medicament, wherein said compound is for use in regulating CRISPR-Cas associated activity and/or CRISPR-dCas associated activity in a somatic mammalian cell, said compound having formula I: 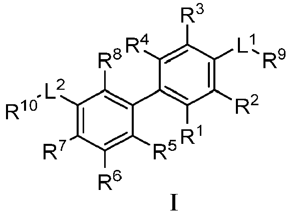 wherein
**R¹, R², R³, R⁴, R⁵, R⁶, R⁷,** and **R⁸** are each independently selected from R¹¹;
**R⁹** is R¹¹ or R¹²;
**R¹⁰** is selected from among: R¹¹, R¹², and -NR¹³R¹⁴;
**L¹** is selected from among: absent, -S(=O)₂N(R¹⁵)-, -C(=O)-, -C(=O)N(R¹⁶)-, - CH₂N(R¹⁷)-, -N(R¹⁷)CH₂-, -CH₂O-, -OCH₂-, C₁₋₃ alkylene, C₂₋₃ alkenylene having one double bond, and C₂₋₃ alkynylene having one triple bond;
**L²** is selected from among: absent, -C(=O)-, -S(=O)₂-, and C₁₋₃ alkylene;
**R¹¹** is selected from among: H, halo, -NO₂, -CN, -OR¹⁸, -NR¹⁹R²¹, -C(=O)OR²¹, - C(=O)NR²²R²³, Ph, -OPh,
C₁₋₈ alkyl optionally substituted with one to three halo and/or -OH groups,
C₃₋₈ cycloalkyl optionally substituted with one to three halo and/or -OH groups,
4-8 membered heterocycloalkyl comprising one to three heteroatoms independently selected from among N, O, and S, and
5-6 membered heteroaryl comprising one to three heteroatoms independently selected from among N, O, and S;
**R¹²** is selected from among:
   - C₁₋₈ alkyl optionally substituted with one to three groups independently selected from among:
      halo, -OR²⁴, -C(=O)OR²⁵, -C(=O)NR²⁶R²⁷, C₃₋₈ cycloalkyl, C₂₋₄ alkenylene having one double bond, C₂₋₄ alkynylene having one triple bond,
      4-6 membered heterocycloalkyl comprising one to three heteroatoms independently selected from among N, O, and S,
      C₆₋₁₀ aryl optionally substituted with one or more independently selected R¹¹ groups, and
      5-6 membered heteroaryl comprising one to four heteroatoms independently selected from N, O, and S;
   - C₃₋₈ cycloalkyl, optionally substituted with one or more independently selected R¹¹ groups;
   - C₄₋₈ cycloalkenyl comprising one double bond, optionally substituted with one or more independently selected R¹¹ groups;
   - 4-8 membered heterocycloalkyl comprising one to three heteroatoms independently selected from N, O, and S, optionally substituted with one or more independently selected R¹¹ groups;
   - C₆₋₁₀ aryl optionally substituted with one or more independently selected R¹¹ groups; and
   - 5-10 membered heteroaryl comprising one to three heteroatoms independently selected from N, O, and S, optionally substituted with one or more independently selected R¹¹ groups;
**R¹³** and **R¹⁴** are each independently selected from H and R¹²;
**R¹⁵, R¹⁶**, **R¹⁷, R¹⁸, R¹⁹, R²⁰, R²¹, R²², R²³, R²⁴, R²⁵, R²⁶,** and **R²⁷** are each independently selected from H and C₁₋₈ alkyl optionally substituted with one or more halo and/or -OH groups.

### Description of the invention

### Figures:

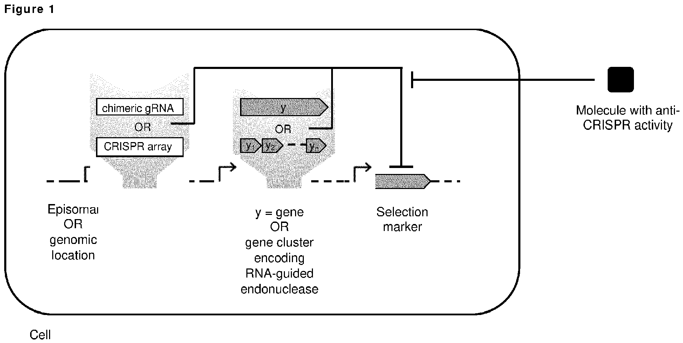

**Figure 1** is a cartoon illustrating a host cell comprising a genetic circuit used in the detection of inhibitors of CRISPR-Cas systems. The host cell comprises a gene or gene cluster encoding a protein or protein complex having RNA-guided endonuclease activity, a nucleic acid molecule encoding chimeric gRNA (sgRNA) or a CRISPR array, as well as a reporter gene encoding a selection marker. Anti-CRISPR activity of candidate small molecule contacted with said host cell, is detected by means of the selection marker, whose expression is dependent on inhibitors that prevent inactivation of the reporter gene or its transcript by the RNA-guided endonuclease.

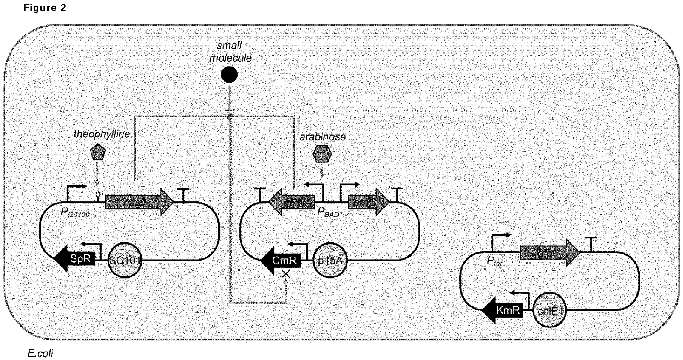

**Figure 2** shows a cartoon illustrating a cell comprising the pCasens3 and pDual3 plasmids and their interaction with an anti-CRISPR small molecule. Plasmid pCasens3 (middle) comprises: the *Streptococcus pyogenes* Cas9 gene (spCas9) [SEQ ID No.:150] cloned into the backbone of the plasmid pSEVA47. Additionally, a sigma70 constitutive promoter (P₂₃₁₀₀) [SEQ ID No.: 148], adjacent to a DNA molecule encoding a theophylline translational riboswitch [SEQ ID No.:149] were inserted upstream of the spCas9 gene in pSEVA47, in order to control its expression. pSEVA47 comprises a low copy number origin of replication, SC101 [SEQ ID No.: 155]; and the antibiotic resistance gene aadA [SEQ ID No.:153] under the control of its native (constitutive) promoter and ribosomal binding site, conferring resistance against Spectinomycin.
   Plasmid pDual3 (to the right) comprises a chloramphenicol resistance gene (CmR) [SEQ ID No.:164] operably linked to an upstream constitutive promoter and RBS; and a DNA sequence encoding a sgRNA [SEQ ID No.:161] whose transcription/expression is regulated by the inducible pBAD promoter [SEQ ID No.: 158], located upstream of the respective sequences. The plasmid further comprises an araC L-arabinose sensor gene [SEQ ID No.:159] that regulates the pBAD promoter in response to the metabolite, L-arabinose. pDual3 comprises a low copy number origin of replication, p15A [SEQ ID No.: 163]. A positive control plasmid comprising a gene [SEQ ID No.:146] encoding ACRIIA2 protein under the control of a constitutive pTET promoter [SEQ ID No.: 139]. The cell further comprises a plasmid comprising a gene [SEQ ID No.:140] encoding Green Fluorescent Protein (GFP) under the control of a constitutive pTET promoter [SEQ ID No.:139]; and a kanamycin resistance gene [SEQ ID No.:144] under the control of its native (constitutive) promoter and ribosomal binding site and a colE1 origin of replication [SEQ ID No.:143].

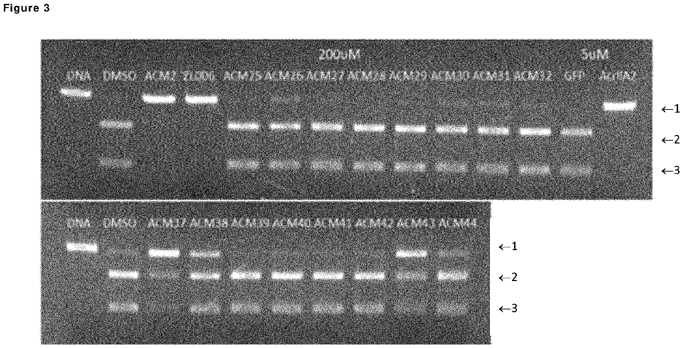

**Figure 3** shows two 1% agarose gels comprising the size-separated cleavage products (2, 3) resulting from an *in vitro* assay of cleavage of linear double-stranded DNA template (1) by the spCas9-sgRNA complex in the absence (lane 2 of gels) or presence of candidate Anti-CRISPR Small Molecules [ACMs]; or presence of AcrIIA2 anti-CRISPR peptide; or presence of GFP. The linear dsDNA template (lane 1 of gels) comprises a 20 base pair target sequence recognised spCas9-sgRNA complex.

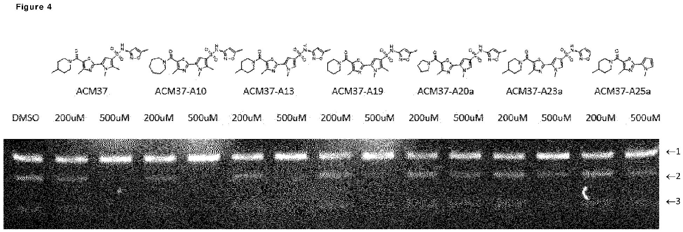

**Figure 4** shows a 1% agarose gel comprising the size-separated cleavage products (2, 3) resulting from an *in vitro* assay of cleavage of linear double-stranded DNA template (1) by the spCas9-sgRNA complex in the absence (gel lane 1) or presence of 200µM or 500µM of each ACM37 pyrrolylthiazole analogue. The structural formula of ACM37 and each tested ACM37 pyrrolylthiazole analogue is shown above the gel lane comprising the corresponding *in vitro* assay product. The linear dsDNA template comprises a 20 base pair target sequence recognised spCas9-sgRNA complex.

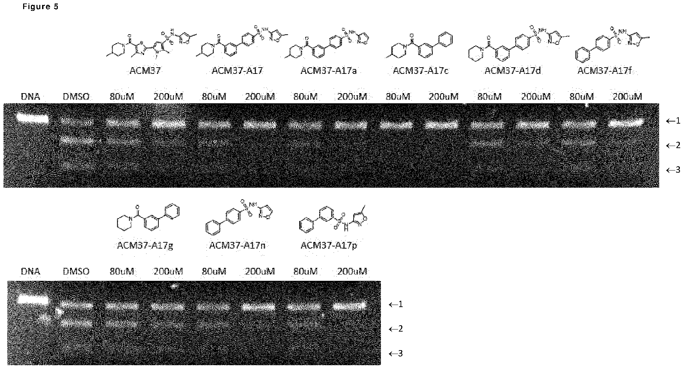

**Figure 5** shows two 1% agarose gels comprising the size-separated cleavage products (2, 3) resulting from an *in vitro* assay of cleavage of linear double-stranded DNA template (1) by the spCas9-sgRNA complex in the absence (lane 2 of gels) or presence of 80µM or 200µM of ACM37 and each ACM37 biphenyl analogue. The structural formula of ACM37 and each tested ACM37 biphenyl analogue is shown above the gel lane comprising the corresponding *in vitro* assay products. The linear dsDNA template comprises a 20 base pair target sequence recognised spCas9-sgRNA complex.

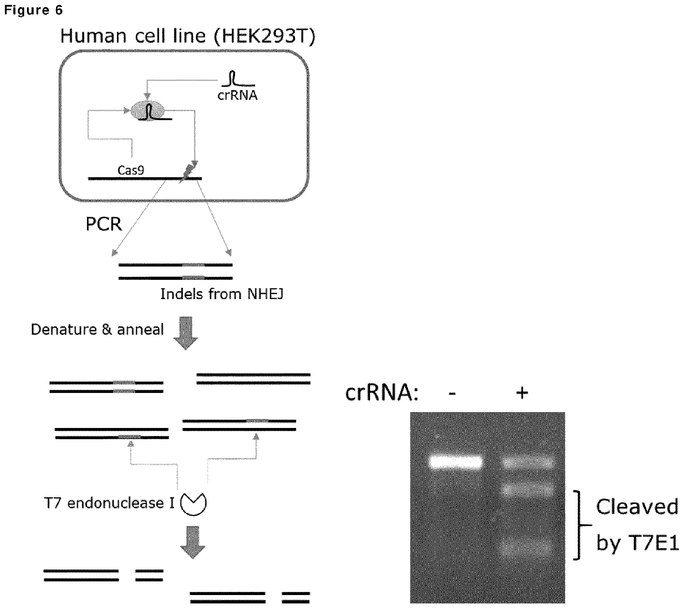

**Figure 6** Cartoon showing design of T7E1 assay for Anti-CRISPR activity in a human cell line (left hand) and image of agarose gel showing DNA fragments following T7 endonuclease I (T7E1) cleavage of Cas9 target DNA amplified from said cell line. The cartoon shows a human cell line (HEK293T) expressing Cas9, that together with guide RNA (crRNA) introduces a double-stranded break in a target locus on the genome, which is then repaired by a non-homologous end joining mechanism (NHEJ), leaving insertion or deletion mutations (indels). Genomic DNA is extracted from the cells; the target locus is then amplified; and the PCR products are then denatured and annealed to generate mismatched DNA hybrids around the target site. T7 endonuclease I cleaves the mismatched DNA hybrids resulting in cleaved products detectable on an agarose gel.

### Abbreviations and definition of terms:

**Amino acid sequence identity:** The term "sequence identity" as used herein, indicates a quantitative measure of the degree of homology between two amino acid sequences of substantially equal length. The two sequences to be compared must be aligned to give a best possible fit, by means of the insertion of gaps or alternatively, truncation at the ends of the protein sequences. The sequence identity can be calculated as ((Nref-Ndif)100)/(Nref), wherein Ndif is the total number of non-identical residues in the two sequences when aligned and wherein Nref is the number of residues in one of the sequences. Sequence identity between each AcrIIA and its homologues is calculated by the BLAST program e.g. the BLASTP program (Pearson W.R and D.J. Lipman (1988)) (www.ncbi.nlm.nih.gov/cgi-bin/BLAST). The identity matrix between AcrIIA1-9 is determined by the sequence alignment method ClustalW with default parameters as described by Thompson J., et al 1994, available at http://www2.ebi.ac.uk/clustalw/.

Preferably, the numbers of substitutions, insertions, additions or deletions of one or more amino acid residues in the polypeptide as compared to its comparator polypeptide is limited, i.e. no more than 1, 2, 3, 4, 5, 6, 7, 8, 9, or 10 substitutions, no more than 1, 2, 3, 4, 5, 6, 7, 8, 9, or 10 insertions, no more than 1, 2, 3, 4, 5, 6, 7, 8, 9, or 10 additions, and no more than 1, 2, 3, 4, 5, 6, 7, 8, 9, or 10 deletions. Preferably the substitutions are conservative amino acid substitutions: limited to exchanges within members of group 1: Glycine, Alanine, Valine, Leucine, Isoleucine; group 2: Serine, Cysteine, Selenocysteine, Threonine, Methionine; group 3: proline; group 4: Phenylalanine, Tyrosine, Tryptophan; Group 5: Aspartate, Glutamate, Asparagine, Glutamine.

**Cis element:** is a sequence of DNA that is capable of conferring inducible expression of a gene that is functionally linked to a promoter; where the cis element may induce expression by regulating transcription of the gene or by regulating translation of transcript product of the gene.

**Cognate:** is used to refer to interacting pairs of functional entities; more specifically that each protein or protein cluster having RNA-guided endonuclease activity has a cognate mat-crRNA, and in the case of Type II CRISPR-Cas systems both a cognate mat-crRNA and cognate tracrRNA, that are required for their activity.

**CRISPR:** is C lustered R egularly Interspaced S hort P alindromic R epeats.

**CRISPR-Cas system:** is a bacterial immune system that employs CRISPR-associated protein(s) to bind to and cleave DNA or RNA in a sequence specific manner, and whose endonuclease activity is mediated by a RNA guided nuclease.

**CRISPR-dCas:** is a mutant form of CRISPR-Cas system where the CRISPR-associated protein(s) bind to a DNA or RNA in a sequence specific manner, but is devoid of endonuclease activity.

***E. coli* Top10:** is *E*. *coli* having chromosomal Genotype mcrA, Δ(mrr-hsdRMS-mcrBC), Phi80lacZ(del)M15, ΔlacX74, deoR, recA1, araD139, Δ(ara-leu)7697, galU, galK, rpsL(SmR), endA1, nupG

**gi number:** (genInfo identifier) is a unique integer which identifies a particular sequence, independent of the database source, which is assigned by NCBI to all sequences processed into Entrez, including nucleotide sequences from DDBJ/EMBL/GenBank, protein sequences from SWISS-PROT, PIR and many others.

**Nucleic acid molecule:** according to the invention comprises a gene whose nucleic acid sequence encodes a AcrIIA protein of the invention.

**Promoter:** is a region of DNA that initiates transcription of a particular gene. Promoters are located near the transcription start sites of genes, on the same strand and upstream on the DNA (towards the 5' region of the sense strand). A promoter that is "functionally linked" to a gene is capable of driving expression of said gene. A promoter may be drive expression constitutively or only in response to an inducer.

**RBS: R**ibosomal **B**inding **S**ite is a sequence of nucleotides upstream of the start codon of an mRNA transcript that is responsible for the recruitment of a ribosome during the initiation of protein translation.

**Heterologous gene and heterologous DNA molecule:** have a different genetic origin from the recombinant cell in which they are expressed; and this also applies to the protein or transcript that they encode. The nucleotide sequence of the heterologous gene or heterologous DNA molecule may be optimized (e.g. codon optimization) with respect to the recombinant cell in which they are expressed. Heterologous gene and heterologous DNA molecule may be located on (and therefor be a part of) the chromosome or a episome of the recombinant cell, and may be inserted into this location by recombinant DNA cloning.

**RNA guided endonuclease-guide RNA complex:** complex formed between a protein or protein complex having endonuclease activity and its cognate palindromic repeat sequence (mat-crRNA), and in the case of Type II CRISPR-Cas systems both a cognate mat-crRNA and cognate tracrRNA

**RNA guided DNA binding-guide RNA complex:** complex formed between a protein having DNA binding activity and its cognate palindromic repeat sequence (mat-crRNA), and cognate tracrRNA.

### Detailed description of the invention

The Anti-CRISPR Small Molecules (ACMs) disclosed herein find use in methods for regulating CRISPR-Cas and/or CRISPR-dCas associated activity. The regulation conferred by these ACMs may provide an enhancement, or more preferably an inhibition of CRISPR-Cas and/or CRISPR-dCas associated activity. Furthermore, the disclosed ACMs have broad application, since they can be used to regulate more than one CRISPR-Cas system and/or CRISPR-dCas system. Examples of systems having CRISPR-Cas and CRISPR-dCas associated activity, their component molecules, and their expression are detailed in section VII.

The ACMs of the invention were initially identified by screening libraries of small molecules using a high through-put screening assay (as described in Example 1 and 2, Figures 1-3). The structural identify of the identified ACMs and their analogues for use in compositions, pharmaceutical compositons and in methods for regulating for regulating CRISPR-Cas associated activity and/or CRISPR-dCas associated activity, is detailed below.

### I Anti-CRISPR Small Molecules having a biphenyl core

One embodiment of the invention employs ACMs, characterized by an biphenyl core, for regulating (e.g. inhibiting) CRISPR-Cas associated activity and/or CRISPR-dCas associated activity.
**Ii** The biphenyl molecule is a compound having formula I: 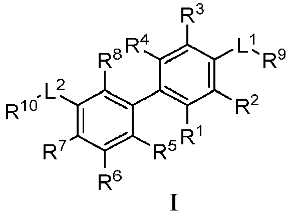 wherein
   **R¹, R², R³, R⁴, R⁵, R⁶, R⁷,** and **R⁸** are each independently selected from R¹¹;
   **R⁹** is R¹¹ or R¹²;
   **R¹⁰** is selected from among: R¹¹, R¹², and -NR¹³R¹⁴;
   **L¹** is selected from among: absent, -S(=O)₂N(R¹⁵)-, -C(=O)-, -C(=O)N(R¹⁶)-, - CH₂N(R¹⁷)-, -N(R¹⁷)CH₂-, -CH₂O-, -OCH₂-, C₁₋₃ alkylene, C₂₋₃ alkenylene having one double bond, and C₂₋₃ alkynylene having one triple bond;
   **L²** is selected from among: absent, -C(=O)-, -S(=O)₂-, and C₁₋₃ alkylene;
   **R¹¹** is selected from among: H, halo, -NO₂, -CN, -OR¹⁸, -NR¹⁹R²¹, -C(=O)OR²¹, - C(=O)NR²²R²³, Ph, -OPh,
   C₁₋₈ alkyl optionally substituted with one to three halo and/or -OH groups,
   C₃₋₈ cycloalkyl optionally substituted with one to three halo and/or -OH groups,
   4-8 membered heterocycloalkyl comprising one to three heteroatoms independently selected from among N, O, and S, and
   5-6 membered heteroaryl comprising one to three heteroatoms independently selected from among N, O, and S;
   **R¹²** is selected from among:
      - C₁₋₈ alkyl optionally substituted with one to three groups independently selected from among:
         halo, -OR²⁴, -C(=O)OR²⁵, -C(=O)NR²⁶R²⁷, C₃₋₈ cycloalkyl, C₂₋₄ alkenylene having one double bond, C₂₋₄ alkynylene having one triple bond,
         4-6 membered heterocycloalkyl comprising one to three heteroatoms independently selected from among N, O, and S,
         C₆₋₁₀ aryl optionally substituted with one or more independently selected R¹¹ groups, and
         5-6 membered heteroaryl comprising one to four heteroatoms independently selected from N, O, and S;
      - C₃₋₈ cycloalkyl, optionally substituted with one or more independently selected R¹¹ groups;
      - C₄₋₈ cycloalkenyl comprising one double bond, optionally substituted with one or more independently selected R¹¹ groups;
      - 4-8 membered heterocycloalkyl comprising one to three heteroatoms independently selected from N, O, and S, optionally substituted with one or more independently selected R¹¹ groups;
      - C₆₋₁₀ aryl optionally substituted with one or more independently selected R¹¹ groups; and
      - 5-10 membered heteroaryl comprising one to three heteroatoms independently selected from N, O, and S, optionally substituted with one or more independently selected R¹¹ groups;
   **R¹³** and **R¹⁴** are each independently selected from H and R¹²;
   **R¹⁵, R¹⁶**, **R¹⁷, R¹⁸, R¹⁹, R²⁰, R²¹, R²², R²³, R²⁴, R²⁵, R²⁶,** and **R²⁷** are each independently selected from H and C₁₋₈ alkyl optionally substituted with one or more halo and/or -OH groups.
**Iii** In a further aspect, said biphenyl molecule is a compound having formula II: 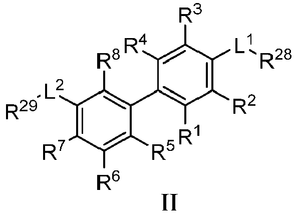 wherein
   **R¹, R², R³, R⁴, R⁵, R⁶, R⁷,** and **R⁸** are as defined for biphenyl of formula I,
   **R²⁸** is R¹¹, or 5-10 membered heteroaryl comprising one to three heteroatoms independently selected from N, O, and S, optionally substituted with one or more independently selected R¹¹ groups;
   **R¹¹** as defined in claim 1;
   **R²⁹** is R¹¹ or -NR³⁰R³¹;
   **L¹** and **L²** as defined in claim 1;
   **R³⁰** and **R³¹** are each independently selected from H and R¹²;
   **R¹²** as defined for biphenyl of formula I.
**Iiii** In a further aspect, said biphenyl molecule is a compound having formula III: 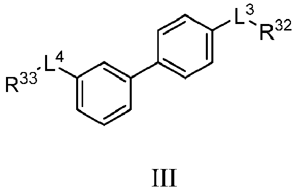 wherein
   **R³²** is selected from among: H, 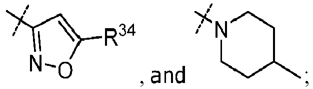
   **R³³** is selected from among: H, -NR³⁵R³⁶, and 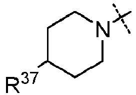
   **L³** is selected from among: absent, -S(=O)₂N(H)-, and -C(=O)-;
   **L⁴** is selected from among: absent, -C(=O)-, -S(=O)₂-, and -CH₂-;
   **R³⁴** is H or Me;
   **R³⁵** and **R³⁶** are independently selected from among: H,
   C₁₋₄ alkyl, and 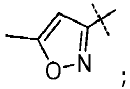
   **R³⁷** is H or Me;
   while excluding compounds having formula III wherein
   **R³²** is H, **R³³** is H, **L³** is absent or -S(=O)₂N(H)-, and **L⁴** is absent.
**Iiv** In a further aspect the biphenyl-based molecule is a compound selected from among: 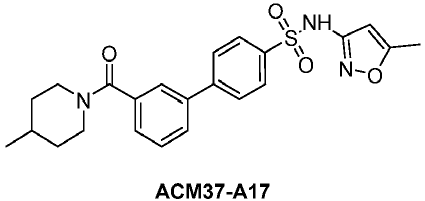 *N*-(5-methylisoxazol-3-yl)-3'-(4-methylpiperidine-1-carbonyl)-[1,1'-biphenyl]-4-sulfonamide; 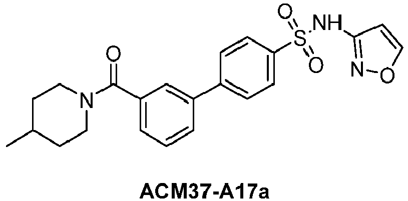 *N*-(isoxazol-3-yl)-3'-(4-methylpiperidine-1-carbonyl)-[1,1'-biphenyl]-4-sulfonamide; 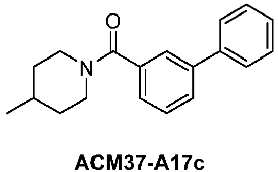 [1,1'-biphenyl]-3-yl(4-methylpiperidin-1-yl)metha none; 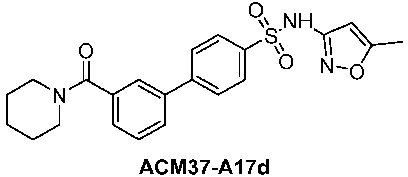 *N*-(5-methylisoxazol-3-yl)-3'-(piperidine-1-carbonyl)-[1,1'-biphenyl]-4-sulfonamide; 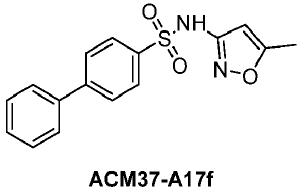 *N*-(5-methylisoxazol-3-yl)-[1,1'-biphenyl]-4-sulfonamide; 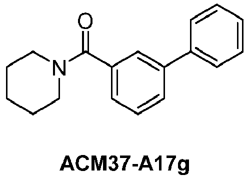 [1,1'-biphenyl]-3-yl(piperidin-1-yl)metha none; 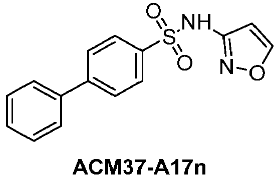 *N*-(isoxazol-3-yl)-[1,1'-biphenyl]-4-sulfonamide; and 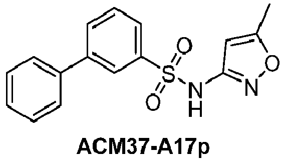 *N*-(5-methylisoxazol-3-yl)-[1,1'-biphenyl]-3-sulfonamide.

In a particular embodiment the biphenyl-based molecule is a compound selected from among: **ACM37-A17, ACM37-A17a, ACM37-A17b, ACM37-A17c, ACM37-A17d, ACM37-A17e, ACM37-A17n, ACM37-A17p, ACM37-A17v, ACM37-A17w,** and **ACM37-A17y.**

### II Anti-CRISPR Small Molecules having a pyrrolylthiazole core

A further embodiment of the invention employs ACMs, characterized by a pyrrolylthiazole core, for regulating CRISPR-Cas associated activity and/or CRISPR-dCas associated activity.
**IIi.** The pyrrolylthiazole-based molecule is a compound having formula I: 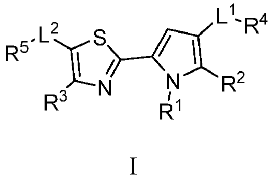 wherein
   **R¹** is selected from among: R⁶, -C(=O)R⁷, and -S(=O)₂R⁸;
   **R², R³,** and **R⁴** is independently selected from H and R⁶;
   **R⁵** is selected from among: R⁶, -OR⁹, and -NR¹⁰R¹¹;
   **L¹** is selected from among: absent, -S(=O)₂NR¹²-, -C(=O)NR¹³-, -CH₂NR¹⁴-, -NR¹⁴ CH₂-, -CH₂O-, -OCH₂-, C₁₋₃ alkylene, C₂₋₃ alkenylene having one double bond, and C₂₋₃ alkynylene having one triple bond;
   **L²** is selected from among: -C(=O)-, -S(=O)₂-, C₁₋₃ alkylene, and C₂₋₃ alkenylene having one double bond;
   **R⁶** is selected from among:
      - C₁₋₈ alkyl optionally substituted with one to three groups independently selected from among:
         halo, -OR¹⁵, -C(=O)OR¹⁶, -C(=O)NR¹⁷R¹⁸, C₃₋₈ cycloalkyl, C₂₋₄ alkenylene having one double bond, C₂₋₄ alkynylene having one triple bond,
         4-6 membered heterocycloalkyl comprising one to three heteroatoms independently selected from N, O, and S,
         C₆₋₁₀ aryl optionally substituted with one or more independently selected R¹⁹ group, and
         5-6 membered heteroaryl comprising one to four heteroatoms independently selected from N, O, and S;
      - C₃₋₈ cycloalkyl optionally substituted with one or more independently selected R¹⁹ group,
      - C₄₋₈ cycloalkenyl comprising one double bond optionally substituted with one or more independently selected R¹⁹ groups,
      - 4-8 membered heterocycloalkyl comprising one to three heteroatoms independently selected from N, O, and S, optionally substituted with one or more independently selected R¹⁹ groups,
      - C₆₋₁₀ aryl optionally substituted with one or more independently selected R¹⁹ groups, or
      - 5-10 membered heteroaryl comprising one to three heteroatoms independently selected from N, O, and S, optionally substituted with one or more independently selected R¹⁹ groups;
   Each of **R⁷** and **R⁸** is independently selected from the R⁶ groups;
   Each of **R⁹, R¹⁰, R¹¹, R¹², R¹³**, and **R¹⁴** is independently selected from H and the R⁶ groups;
   Each of **R¹⁵, R¹⁶, R¹⁷,** and **R¹⁸,** is independently selected from H and C₁₋₈ alkyl optionally substituted with one or more groups selected from halo and -OH;
   **R¹⁹** is selected from among:
      halo, -NO₂, -CN, -OR²⁰, -NR²¹R²², -C(=O)OR²³, -C(=O)NR²⁴R²⁵, Ph, -OPh, C₁₋₈ alkyl optionally substituted with one to three groups selected from halo and -OH, C₃₋₈ cycloalkyl optionally substituted with one to three groups selected from halo and -OH,
      4-8 membered heterocycloalkyl comprising one to three heteroatoms independently selected from N, O, and S,
      5-6 membered heteroaryl comprising one to three heteroatoms independently selected from N, O, and S;
   Each of **R²⁰, R²¹, R²², R²³, R²⁴,** and **R²⁵** is independently selected from H and C₁₋₈ alkyl optionally substituted with one or more groups selected from halo and -OH.
**IIii** In a further aspect, said pyrrolylthiazole-based molecule is a compound having formula II: 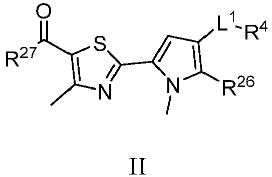 wherein
   **R⁴** is as defined for formula I;
   **R²⁶** is H or Me;
   **R²⁷** is 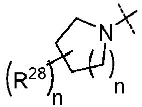 or -NR¹⁰R¹¹ wherein
      **R²⁸** is H or R¹⁹; **n** is an integer from 1 to 3; and
      **R¹⁰, R¹¹, R¹⁹,** and **L¹** are as defined for formula I.
**IIiii** In a further aspect, said pyrrolylthiazole-based molecule is a compound having formula III: 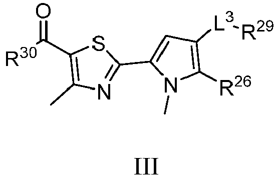 wherein
   **R²⁶** is as defined for formula II;
   **R²⁹** is H, or 3-isoxazole, optionally substituted with Me;
   **R³⁰** is encompassed in R⁵ but here limited to 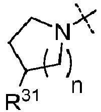
   wherein **R³¹** is H, or Me; n is an integer from 1 to 3;
   **L³** is absent, or -S(=O)₂NH-;
   while excluding compounds having formula III wherein **R²⁶** is H, **R²⁸** is H, **R³⁰** is Me, **n** is 2, and **L³** is -S(=O)₂NH-.
**IIiv** In a further aspect the pyrrolylthiazole-based molecule is a compound selected from among: 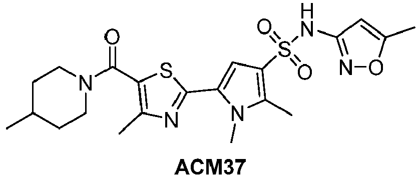 1,2-Dimethyl-5-(4-methyl-5-(4-methylpiperidine-1-carbonyl)thiazol-2-yl)-*N*-(5-methylisoxazol-3-yl)-1*H*-pyrrole-3-sulfonamide; 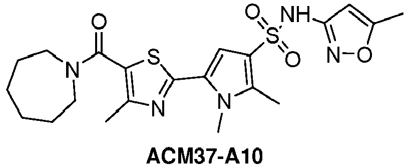 5-(5-(Azepane-1-carbonyl)-4-methylthiazol-2-yl)-1,2-dimethyl-*N*-(5-methylisoxazol-3-yl)-1*H*-pyrrole-3-sulfonamide; 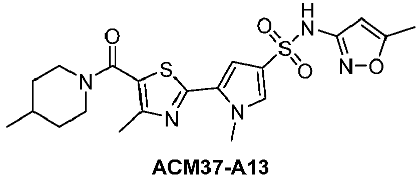 1-Methyl-5-(4-methyl-5-(4-methylpiperidine-1-carbonyl)thiazol-2-yl)-*N*-(5-methylisoxazol-3-yl)-1*H*-pyrrole-3-sulfonamide; 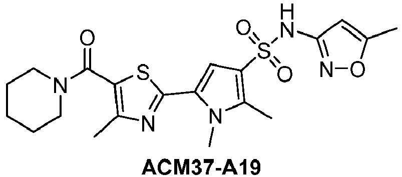 1,2-Dimethyl-5-(4-methyl-5-(piperidine-1-carbonyl)thiazol-2-yl)-*N*-(5-methylisoxazol-3-yl)-1*H*-pyrrole-3-sulfonamide; 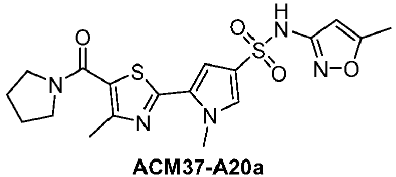 1-Methyl-5-(4-methyl-5-(pyrrolidine-1-carbonyl)thiazol-2-yl)-*N*-(5-methylisoxazol-3-yl)-1*H*-pyrrole-3-sulfonamide; 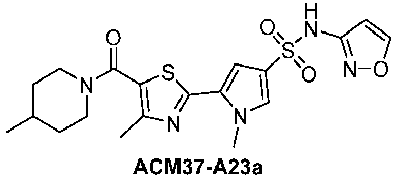 *N*-(Isoxazol-3-yl)-1-methyl-5-(4-methyl-5-(4-methylpiperidine-1-carbonyl)thiazol-2-yl)-1*H*-pyrrole-3-sulfonamide; and 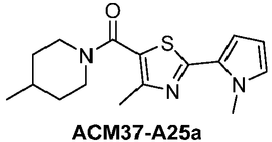 (4-Methyl-2-(1-methyl-1*H*-pyrrol-2-yl)thiazol-5-yl)(4-methylpiperidin-1-yl)methanone.

In a particular embodiment the pyrrolylthiazole-based molecule is a compound selected from among: **ACM37-A13, ACM37-A19, ACM37-A20a, ACM37-A23a, and ACM37-A25a.**

### III Pharmaceutically acceptable acid-addition salt of the Anti-CRISPR Small Molecule

In a further aspect, is provided a pharmaceutically acceptable acid addition salt of said biphenyl-based or pyrrolylthiazole-based compounds (described in I and II), wherein the salt is selected from among: sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, iso-butyrate, caproate, heptanoate, propiolate, oxalate, malonate, succinate, hemisuccinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, sulfonate, xylenesulfonate, phenylacetate, phenylpropionate, phenylbutyrate, citrate, lactate, β-hydroxybutyrate, glycolate, tartrate, methanesulfonate, propanesulfonate, naphthalene-1-sulfonate, naphthalene-2-sulfonate, and mandalate.

### IV Synthesis of the Anti-CRISPR Small Molecules

Methods for synthesis and identification of biphenyl-based and pyrrolylthiazole-based compounds (described in I and II) capable of regulating the activity of a CRISPR-Cas and/or CRISPR-dCas systems are detailed in Example 4.1 and 4.2.

### V Structure-Activity Relationships of the Anti-CRISPR Small Molecules

The ACMs disclosed herein are capable of regulating the activity of a CRISPR-Cas and/or CRISPR-dCas system. The ability of said ACMs to regulate a CRISPR-Cas system is illustrated in example 2. Using an *in vivo* assay, the ACMs were shown to inhibit CRISPR-Cas9 editing of a target DNA sequence in a microbial cell, whereby those cells exposed to the active ACM retained a functional target DNA sequence (an antibiotic resistance gene) and were able to grow under selective conditions (antibiotic supplemented growth medium). A regulatory effect of said ACMs on CRISPR-Cas editing was further demonstrated in an *in vitro* assay where inhibition of CRISPR-Cas9 nuclease cleavage of a target DNA sequence was detected (example 3, figures 3 - 5). ACMs characterised by a biphenyl core were found to be the most potent inhibitors of CRISPR-Cas-mediated gene editing (example 3ii, figure 5).

### VI A Kit comprising an Anti-CRISPR Small Molecule

According to a further embodiment the invention provides a kit comprising an ACM (as disclosed herein) and a CRISPR-Cas or CRISPR-dCas expression system, where the kit may be a pharmaceutical kit. A suitable expression system includes a self-replicating genetic element comprising one or more nucleic acid molecules encoding a CRISPR-Cas or CRISPR-dCas expression system that is capable of directing the expression of the components of the CRISPR-Cas or CRISPR-dCas system, as detailed in section VII. Alternatively, the kit may comprise an ACM (as defined in section I or II) and an isolated CRISPR-Cas endonuclease complex or CRISPR-dCas complex and their cognate mat-crRNA and/or cognate tracrRNA (as defined in section VIIi).

### VII Use of the Anti-CRISPR small molecule to regulate CRISPR-Cas or CRISPR-dCas associated activity

ACMs, as disclosed herein, find use in a method of regulating (e.g. inhibiting) CRISPR-Cas- and/or CRISPR-dCas- associated activity. For example they may be used in a method to regulate (e.g. inhibit) the activity of a CRISPR-Cas system, wherein said system is selected from the group consisting of Type 1, Type II, Type III; Type IV, Type V, and Type VI or a CRISPR-dCas system.

**VIIi** CRISPR-Cas systems: The CRISPR-Cas associated activity that can be regulated by said ACMs share the common functional property of modifying or cleaving a target DNA molecule or its transcript; and thereby allowing genetic material to be added, removed, or altered at particular locations in the genome of a cell or in a DNA molecule. The genome of a cell or the DNA molecule comprising the target DNA to be modified or cleaved by CRISPR-Cas associated activity, can be present in a biological cell as defined in section VIIii. In general terms, such a system is located in a CRISPR locus consisting of a CRISPR array, comprising short variable DNA sequences (called spacer sequence(s)) interspersed by short palindromic repeat sequences (called repeat sequence(s)), this being flanked by diverse cognate cas genes. The CRISPR-Cas systems may for example be those that have been classified into 2 classes; whereby members of Class 1 comprise a multi-subunit protein complex, each subunit encoded by a gene in the CRISPR locus; while members of Class 2 comprise a single multidomain protein encoded by a single gene. Class 1, includes type I, III and IV CRISPR-Cas systems; while Class 2, includes type II, V and VI CRISPR-Cas systems.

Thus, in total there are six types, which have been further classified into over 28 subtypes; based on their protein structure, signature Cas genes and operon arrangements (Koonin, E. V. et al., 2017).

The genetic loci encoding Class 1, type I CRISPR-Cas systems share in common a signature gene cas3 (or its variant cas3') encoding a nuclease that both unwinds and cleaves target double-stranded DNA (dsDNA) and RNA-DNA duplexes. Type I systems comprise seven subtypes, I-A to I-F, and I-U. The genetic loci encoding Class 1, type III CRISPR-Cas systems all contain the signature gene cas10, as well as genes encoding Cas5 gene and several paralogues of Cas7 that co-transcriptionally target RNA and DNA. The Type III systems comprise four subtypes, III-A to III-D. The loci comprising Class 1, type IV CRISPR-Cas system comprises two subtypes, IV-A and IV-B; where the Csf1 can serve as as signature gene.

The genetic loci encoding the Class 2, Type II CRISPR-Cas systems, comprise the signature Cas9 gene encoding a multidomain protein that combines the functions of a crRNA-effector complex and cleaves target DNA. All Type II loci are characterised by a nucleic acid sequence encoding a tracrRNA, which is partially complementary to the repeats within the respective CRISPR array. Type II systems comprise three subtypes, II-A to II-C. Class 2, Type V CRISPR-Cas systems comprise the signature gene Cpf1, also encoding a single multidomain protein. Unlike Cas9, the Cpf1 gene is commonly located outside a CRISPR-Cas locus. Type V systems are currently divided into five subtypes, V-A to V-E.

For the purpose of expression of members of the Class 2 type II CRISPR-Cas systems in a cell, it is sufficient to express the signature Cas gene encoding the multidomain protein having RNA guided-endonuclease activity. Expression of all other Class 1 and 2 CRISPR-Cas systems can be achieved by co-expressing the several genes encoding the Cas proteins that constitute the multi-subunit protein complex having RNA guided-endonuclease activity.

When expression of the CRISPR locus is activated, transcription of the CRISPR array yields a pre-crRNA, that is processed into individual mature crRNAs (mat-crRNA). Each mat-crRNA sequence, aided by its cognate single or multi-subunit Cas protein(s) (effector complex), functions as a guide to specifically target the effector complex to modify or cleave a target nucleic acid molecule (DNA or RNA). Processing of the pre-crRNA is mediated by either an endonuclease subunit of the effector complex; or in the case of type II CRISPR-cas system by a combination of a RNase III protein together with a trans-activating CRISPR RNA (tracrRNA); where tracrRNA serves to recruit the components of the type II effector complex.

For the purpose of expression of any member of the Class 1 or 2 CRISPR-Cas systems in a cell, an individual mat-crRNA comprising at least one spacer sequence, flanked at least at one end (preferably at both ends) by a palindromic repeat sequence, is sufficient to specifically guide the cognate effector complex to cleave a target nucleic acid molecule (DNA or RNA). Alternatively, a complete CRISPR array (pre-crRNA) can be expressed. The target nucleic acid molecule comprises a sequence that is complementary to the spacer sequence. A tracrRNA is additionally necessary for the expression of a Type II CRISPR-cas system. Synthetic DNA molecules can conveniently be engineered to express a RNA transcript comprising a spacer sequence flanked, at least at one end, by a palindromic repeat sequence (i.e. a mat-RNA), for use in the expression of the Class 1 or 2 CRISPR-Cas systems. However, in the case a Type II CRISPR-cas system, the RNA transcript of the synthetic DNA molecule can comprise a a mat-RNA fused with a tracrRNA sequence, also called single-guide RNA (sgRNA).

### VIIii Methods of regulating CRISPR-Cas or CRISPR-dCas associated activity

A further embodiment of the invention provides a method for regulating (e.g. inhibiting) CRISPR-Cas associated activity or CRISPR-dCas associated activity comprising the step of contacting an RNA guided endonuclease-guide RNA complex (or the Cas protein complex and mat-crRNA and/or tracRNA components thereof, as defined in section VIIi) a RNA guided DNA binding-guide RNA complex (or the Cas protein and tracRNA component thereof, as defined in section VIIi) respectively with an ACM, wherein said molecule is a compound as defined in section I or II. When the CRISPR-Cas associated activity or CRISPR-dCas associated activity is to be regulated in an *in vitro* assay method, then the assay may be performed using an isolated RNA guided endonuclease-guide RNA complex or isolated RNA guided DNA binding-guide RNA complex, or their respective isolated protein/protein complex and cognate RNA components.

The RNA guided endonuclease-guide RNA complex or RNA guided DNA binding-guide RNA complex may be expressed in vitro or in vivo in a biological cell. The cell may be a prokaryotic or eukaryotic cell, and may be present in, or derived from, a bacterium, yeast, fungus, insect, plant or mammal. When the biological cell is a eukaryotic cell, the cell may be an isolated cell or it may be present in a biological tissue; where the tissue may be healthy, diseased, and/or have genetic mutations. Such cell may be a somatic mammalian cell. The biological tissue may include any single tissue (e.g., a collection of cells that may be interconnected) or a group of tissues making up an organ or part or region of the body of an organism and can include lung tissue, skeletal tissue, and/or muscle tissue. Exemplary tissues include, but are not limited to those derived from liver, lung, thyroid, skin, pancreas, blood vessels, bladder, kidneys, brain, biliary tree, duodenum, abdominal aorta, iliac vein, heart and intestines, including any combination thereof.

The method for regulating CRISPR-Cas associated activity or CRISPR-dCas associated activity may further comprise:
a. providing a biological cell comprising a target DNA sequence;
b. contacting said cell with said ACM molecule,
   wherein said cell either comprises or is additionally contacted with either:
c. an RNA guided endonuclease-guide RNA complex expression system comprising:
   i. a first gene encoding a polypeptide, or a first gene cluster encoding a polypeptide complex, said polypeptide or polypeptide complex having RNA-guided endonuclease activity, wherein said gene or gene cluster is operably linked to a promoter; and
   ii. a nucleic acid molecule operably linked to a promoter, wherein said nucleic acid molecule encodes one or more RNA molecules capable of guiding said polypeptide or polypeptide complex having RNA-guided endonuclease activity to a target gene or its transcript,
      or
d. an RNA guided DNA binding-guide RNA complex expression system comprising:
   iii. a first gene encoding a polypeptide or a first gene cluster encoding a polypeptide complex, said polypeptide or polypeptide complex having RNA-guided DNA binding activity, wherein said gene or gene cluster is operably linked to a promoter; and
   iv. nucleic acid molecule operably linked to a promoter, said nucleic acid molecule encoding one or more RNA molecules capable of guiding said polypeptide or polypeptide complex having RNA-guided DNA binding activity to said target DNA sequence or its transcript;
wherein said small molecule is capable of regulating said CRISPR-Cas associated activity or CRISPR-dCas associated activity in said cell. The polypeptide or polypeptide complex having RNA-guided endonuclease activity may be selected from the group of CRISPR-Cas systems consisting of Type 1, Type II, Type III; Type IV, Type V, and Type VI and that is capable of modifying or inactivating said target gene or its transcript.

More specifically, said nucleic acid molecule may encode any one of:
- a pre-crRNA molecule (encoded by a DNA molecule comprising a CRISPR array) wherein said pre-crRNA comprises a spacer sequence complementary to a nucleic acid sequence of at least 15 nucleotides of at least one strand of said target gene or its transcript;
- a mat-crRNA molecule comprising at least one spacer sequence flanked by at least one palindromic repeat sequence, wherein the spacer sequence is complementary to a nucleic acid sequence of at least 15 nucleotides of at least one strand of said target gene or its transcript;
- said pre-crRNA molecule and a trans-activating crRNA; or
- said mat-crRNA molecule and a trans-activating crRNA (or a fusion thereof),
wherein the palindromic repeat sequence and the trans-activating crRNA are cognate with respect to the polypeptide or polypeptide complex having RNA-guided endonuclease activity (or RNA-guided DNA binding activity).

Examples of the components of polypeptide or polypeptide complex that make up the CRISPR-Cas systems that may be expressed in a cell, and be regulated by the ACMs are listed in Table 1 below, together with their DNA coding sequences. In a preferred embodiment, the amino acid sequence of the polypeptide or polypeptides of a protein complex having RNA-guided endonuclease activity has at least 70, 75, 80, 81, 82, 83, 84, 85, 86, 87, 89, 90, 91, 92, 93, 94, 95, 96, 97, 98, 99, or 100% amino acid sequence identity to an amino acid sequence selected from Table 1. These polypeptide or polypeptide complexes each selectively associate with a cognate palindromic repeat sequence (mat-crRNA) and, where applicable also a cognate tracrRNA. Thus, when the polypeptide or polypeptide complex is selected from Table 1, then the cognate palindromic repeat sequence and cognate tracrRNA are encoded by the nucleotide sequences listed in the adjacent boxes in Table 1.

| **Type** - | **Strain designation & Genome ID** | **Locus id** | **Cas protein or protein complex sequences** | | **Cognate palindromic repeat (mat-crRNA) SEQ ID No.:** | **tracRNA SEQ ID No.:** |
|---|---|---|---|---|---|---|
| **Sub-type** | | | **Protein id** | **SEQ ID** No.: | | |
| I A | *Archaeoglobus fulgidus* | AF1870 | WP_010879363.1 | SEQ ID No: 1 | SEQ ID: 109 | N/A |
| | DSM 4304 | AF1871 | WP_010879364.1 | SEQ ID No: 2 | | |
| | | AF1872 | WP_010879365.1 | SEQ ID No: 3 | | |
| | NC_000917.1 | AF1873 | WP_010879366.1 | SEQ ID No: 4 | | |
| | | AF1874 | WP_010879367.1 | SEQ ID No: 5 | | |
| | | AF1875 | WP_010879368.1 | SEQ ID No: 6 | | |
| | | AF1876 | WP_010879369.1 | SEQ ID No: 7 | | |
| | | AF1877 | WP_010879370.1 | SEQ ID No: 8 | | |
| | | AF1878 | WP_010879371.1 | SEQ ID No: 9 | | |
| | | AF1879 | WP_010879372.1 | SEQ ID No: 10 | | |
| I B | *Clostridium difficile* 630 | CD630_29770* | YP_001089495.1 | SEQ ID No: 11 | SEQ ID: 110 | N/A |
| | | CD630_29820 | YP_001089500.1 | SEQ ID No: 12 | | |
| | NC_009089.1 | CD630_29810 | YP_001089499.1 | SEQ ID No: 13 | | |
| | | CD630_29800 | YP_001089498.1 | SEQ ID No: 14 | | |
| | | CD630_29780 | YP_001089496.1 | SEQ ID No: 15 | | |
| | | CD630_29790 | YP_001089497.1 | SEQ ID No: 16 | | |
| I C | C-125 DNA | BH0336 | WP_010896517 | SEQ ID No: 17 | SEQ ID: 111 | N/A |
| | *Bacillus halodurans* | BH0337 | WP_010896518.1 | SEQ ID No: 18 | | |
| | | BH0338 | WP_010896519.1 | SEQ ID No: 19 | | |
| | NC_002570.2 | BH0339 | WP_010896520.1 | SEQ ID No: 20 | | |
| | | BH0340 | BAB04059.1 | SEQ ID No: 21 | | |
| | | BH0341 | WP_010896522.1 | SEQ ID No: 22 | | |
| | | BH0342 | WP_010896523.1 | SEQ ID No: 23 | | |
| I D | *Cyanothece sp.* 8802 | Cyan8802_0527 | WP_015783167.1 | SEQ ID No: 24 | SEQ ID: 112 | N/A |
| | | Cyan8802_0526 | WP_015783166.1 | SEQ ID No: 31 | | |
| | NC_013161.1 | Cyan8802_0525 | WP_015783165.1 | SEQ ID No: 30 | | |
| | | Cyan8802_0524 | WP_015783164.1 | SEQ ID No: 29 | | |
| | | Cyan8802_0523 | WP_015783163.1 | SEQ ID No: 28 | | |
| | | Cyan8802_0522 | WP_015783162.1 | SEQ ID No: 27 | | |
| | | Cyan8802_0521 | WP_015783161.1 | SEQ ID No: 26 | | |
| | | Cyan8802_0520 | WP_015783160.1 | SEQ ID No: 25 | | |
| I E | *Escherichia coli* | ygcB | NP_417241.1 | SEQ ID No: 32 | SEQ ID: 113 | N/A |
| | *K-12* NC_000913.3 | ygbF | NP_417234.2 | SEQ ID No: 33 | | |
| IF | *Yersinia pseudotuberculosis* | YPK_1644 | WP_072080408.1 | SEQ ID No: 34 | SEQ ID: 114 | N/A |
| | | YPK_1645 | WP_012303962.1 | SEQ ID No: 35 | | |
| | *YPIII* | YPK_1646 | WP_012303963.1 | SEQ ID No: 36 | | |
| | NC_010465.1 | YPK_1647 | WP_012303964.1 | SEQ ID No: 37 | | |
| | | YPK_1648 | WP_011192624.1 | SEQ ID No: 38 | | |
| | | YPK_1649 | WP_002211866.1 | SEQ ID No: 39 | | |
| IF* (variant) | *Shewanella putrefaciens CN-32* | Sputcn32_1819 | WP_011789486.1 | SEQ ID No: 40 | SEQ ID: 115 | N/A |
| | | Sputcn32_1820 | WP_000989810.1 | SEQ ID No: 41 | | |
| | | Sputcn32_1821 | WP_011919225.1 | SEQ ID No: 42 | | |
| | NC_009438.1 | Sputcn32_1822 | WP_011919226.1 | SEQ ID No: 43 | | |
| | | Sputcn32_1823 | WP_011919227.1 | SEQ ID No: 44 | | |
| II A | *Streptococcus pyogenes MGAS10270* | RS04390 | WP_020905136.1 | SEQ ID No: 45 | SEQ ID: 116 | SEQ ID: 134 |
| | NC_008022.1 | | | | | |
| II B | *Legionella pneumophila str. Paris* | Lpp0160 | WP_011212792.1 | SEQ ID No: 46 | SEQ ID: 117 | SEQ ID: 135 |
| | NC_006368.1 | | | | | |
| II C | Neisseria meningitidis 8013 NC_017501.1 | NMV_RS09835 | WP_014574210.1 | SEQ ID No: 47 | SEQ ID: 118 | SEQ ID: 136 |
| III A | *Staphylococcus epidermidis RP62A* | SERP2455 | WP_002486027.1 | SEQ ID No: 48 | SEQ ID: 119 | N/A |
| | | SERP2456 | WP_002486034.1 | SEQ ID No: 49 | | |
| | | SERP2457 | WP_002486031.1 | SEQ ID No: 50 | | |
| | NC_002976.3 | SERP2458 | WP_002486041.1 | SEQ ID No: 51 | | |
| | | SERP2459 | WP_002486018.1 | SEQ ID No: 52 | | |
| | | SERP2460 | WP_002486044.1 | SEQ ID No: 53 | | |
| | | SERP2461 | WP_002486045.1 | SEQ ID No: 54 | | |
| | | SERP2462 | WP_002469399.1 | SEQ ID No: 55 | | |
| | | SERP2463 | WP_002486040.1 | SEQ ID No: 56 | | |
| III B | *Pyrococcus furiosus DSM 3638* | PF1124 | WP_011012264.1 | SEQ ID No: 57 | SEQ ID: 120 | N/A |
| | | PF1125 | WP_011012265.1 | SEQ ID No: 58 | | |
| | NC_003413.1 | PF1126 | WP_011012266.1 | SEQ ID No: 59 | | |
| | | PF1127 | WP_011012267.1 | SEQ ID No: 60 | | |
| | | PF1128 | WP_011012268.1 | SEQ ID No: 61 | | |
| | | PF1129 | WP_011012269.1 | SEQ ID No: 62 | | |
| | | PF1130 | WP_011012270.1 | SEQ ID No: 63 | | |
| | | PF1131 | WP_011012271.1 | SEQ ID No: 64 | | |
| III B* (variant) | *Marinomonas mediterranea MMB_1* | Marme_0668 | WP_013659857.1 | SEQ ID No: 65 | SEQ ID: 121 | N/A |
| | | Marme_0669 | WP_013659858.1 | SEQ ID No: 66 | | |
| | | Marme_0670 | WP_013659859.1 | SEQ ID No: 67 | | |
| | NC_015276.1 | Marme_0671 | WP_013659861.1 | SEQ ID No: 68 | | |
| | | Marme_0673 | WP_083799838.1 | SEQ ID No: 69 | | |
| | | Marme_0674 | WP_013659863.1 | SEQ ID No: 70 | | |
| | | Marme_0675 | WP_013659864.1 | SEQ ID No: 71 | | |
| | | Marme_0676 | WP_013659865.1 | SEQ ID No: 72 | | |
| | | Marme_0677 | WP_013659866.1 | SEQ ID No: 73 | | |
| III C | *Methanothermobacter thermautotrophicus str.* | MTH323 | WP_010875962.1 | SEQ ID No: 74 | SEQ ID: 122 | N/A |
| | | MTH324 | WP_010875963.1 | SEQ ID No: 75 | | |
| | *Delta H* | MTH325 | WP_010875964.1 | SEQ ID No: 76 | | |
| | NC_000916.1 | MTH326 | WP_010875965.1 | SEQ ID No: 77 | | |
| | | MTH327 | WP_010875966.1 | SEQ ID No: 78 | | |
| | | MTH328 | WP_010875967.1 | SEQ ID No: 79 | | |
| III D | *Synechocystis sp. 6803* | sII7063 | WP_011153732.1 | SEQ ID No: 80 | SEQ ID: 123 | N/A |
| | NC_005230.1 | sII7064 | WP_011153733.1 | SEQ ID No: 81 | | |
| | | sII7065 | WP_011153734.1 | SEQ ID No: 82 | | |
| | | sII7066 | WP_011153735.1 | SEQ ID No: 83 | | |
| | | sII7067 | WP_011153736.1 | SEQ ID No: 84 | | |
| | | slr7068 | WP_011153737.1 | SEQ ID No: 85 | | |
| IV | *Thioalkalivibrio sp. K90mix* | TK90_2699 | WP_013006550.1 | SEQ ID No: 86 | SEQ ID: 124 | N/A |
| | | TK90_2700 | WP_013006551.1 | SEQ ID No: 87 | | |
| | | TK90_2701 | WP_018940624.1 | SEQ ID No: 88 | | |
| | NC_013930.1 | TK90_2702 | WP_013006553.1 | SEQ ID No: 89 | | |
| | | TK90_2703 | WP_013006554.1 | SEQ ID No: 90 | | |
| V A | *Francisella cf. novicida Fx1* | FNFX1_1431 | WP_014550095.1 | SEQ ID No: 91 | SEQ ID: 125 | N/A |
| | NC_017450.1 | | | | | |
| V B | *Alicyclobacillus acidoterrestris ATCC* | N007_06525 | WP_021296342.1 | SEQ ID No: 92 | SEQ ID: 126 | SEQ ID: 137 |
| | | N007_06530 | WP_021296343.1 | SEQ ID No: 93 | | |
| | *49025* | N007_06535 | WP_021296344.1 | SEQ ID No: 94 | | |
| | NZ_AURB01000127.1 | | | | | |
| V C | *Oleiphilus sp. HI0009* | A3715_16885 | KZX85786.1 | SEQ ID No: 95 | SEQ ID: 127 | N/A |
| | *HI0009_c113* | A3715_16890 | KZX85787.1 | SEQ ID No: 96 | | |
| | LWEK01000147.1 | | | | | |
| V D | *Bacterium CG09_39_24* | BK003_02070 | OJI08768.1 | SEQ ID No: 97 | SEQ ID: 128 | N/A |
| | *isolate CG09_39_24* | BK003_02075 | OJI08769.1 | SEQ ID No: 98 | | |
| | MOEH01000029.1 | | | | | |
| V E | *Deltaproteobacteria bacterium* | A2Z89_08250 | OGP07438.1 | SEQ ID No: 99 | SEQ ID: 129 | SEQ ID: 138 |
| | | A2Z89_08255 | OGP07439.1 | SEQ ID No: 100 | | |
| | *GWA2_43_19* | A2Z89_08260 | OGP07440.1 | SEQ ID No: 101 | | |
| | *gwa2_scaffold_7984* | A2Z89_08265 | OGP07441.1 | SEQ ID No: 102 | | |
| | MGPG01000094.1 | | | | | |
| VI A | *Leptotrichia shahii DSM 19757* | B031_RS011044 5 | WP_018451595.1 | SEQ ID No: 103 | SEQ ID: 130 | N/A |
| | *B031DRAFT_scaffold_9 .10* | | | | | |
| | NZ_KB890278.1 | | | | | |
| VI B1 | *Prevotella buccae* ATCC 33574 SCAFFOLD1 NZ_GL586311.1 | HMPREF6485_RS00335 | WP_004343973.1 | SEQ ID No: 104 | SEQ ID: 131 | N/A |
| | | HMPREF6485_R S00340 | WP_004343974.1 | SEQ ID No: 105 | | |
| VI B2 | *Bergeyella zoohelcum* ATCC 43767 JH932293.1 | HMPREF9699_02005 | EKB54193.1 | SEQ ID No: 106 | SEQ ID: 132 | N/A |
| | | HMPREF9699_0 2006 | EKB54194.1 | SEQ ID No: 107 | | |
| VI C | *Fusobacterium perfoetens* ATCC 29250 NZ_JHXW01000011.1 | T364_RS010511 0 | WP_027128616.1 | SEQ ID No: 108 | SEQ ID: 133 | N/A |

| | | | | | | |
|---|---|---|---|---|---|---|
| N/A = not applicable or required; * non-essential Cas component of Type 1B | | | | | | |

The target genomic sequence (or gene) may comprise a protospacer adjacent motif (PAM) located adjacent to the sequence complementary to the mat-crRNA spacer sequence, in particular where the RNA-guided endonuclease polypeptide is a Type II A Cas (e.g. Cas9). A canonical PAM, recognized by SpCas9, is the sequence 5'-NGG-3' where "N" is any nucleobase. The skilled man, when implementing the invention with any one of the CRISPR-Cas systems described herein, may employ a compatible PAM sequence present in the target sequence, using those known in the art.

In one embodiment, the CRISPR-Cas system regulated by the ACM is a Class 2 type II A Cas9 system. In another embodiment, the CRISPR-Cas system regulated by the ACM is a Class 2 type V A system comprising the signature gene Cpf1 encoding a Cpf1 protein (e.g. WP_014550095.1). In one embodiment, the CRISPR-Cas system regulated by the ACM is a Class 1 type I system comprising the signature gene Cas3 encoding a Cas3 protein.

In one embodiment, the CRISPR-Cas system regulated by the ACM is a CRISPR-dCas expression system comprising the signature gene dCas9, in substitution for the Cas9 system shown in Table 1. The dCas9 gene encodes the mutant amino acid sequence of dCas9 protein (SEQ ID No.: 168), corresponding to the wild type Cas9 SEQ ID No.: 45 (WP_020905136.1), but containing 2 point mutations in D10A and H840A, which deactivates the endonuclease functionality (Jinek et al., 2012).

Each of said first gene, first gene cluster, and nucleic acid molecule may independently be a component of a self-replicating genetic element (episome) that is stably replicated in a biological cell or is an integrated component of its chromosome.

### VIII Applications of the Anti-CRISPR Small Molecule

The ACMs, disclosed herein, capable of regulating CRISPR-dCas and CRISPR-Cas gene editing activity, provide an advantageous adjunct (e.g. by co-administration) in permitting safe and practical biological therapeutics through spatial or temporal control of CRISPR-Cas/dCas activity; temporal control of CRISPR-Cas/dCas-based gene drives that propogate lethal traits used in animal husbandry and population expansion for field studies; and contributing to general research into various biotechnological, agricultural, and medical applications of gene editing technologies to correct genetic defects that cause illness in a subject and who are then in need of therapeutic treatment.
**VIIIi** Dose and temporal control to minimize Off-Target Effects: The ACMs find use in a method of controlling the duration of CRISPR-Cas/dCas activity. For example, a CRISPR-Cas/dCas expression system delivered into a cell, can be expressed, and allowed to function for a predetermined duration shown empirically to yield efficient on-target editing, and then inactivated through the co- or post-delivery of the ACM. In some cases (for example in germline editing of non-human primate embryos) it could be important to stop Cas9 activity after the initial rounds of mitosis, otherwise it could give rise to mosaic genotypes.
**VIIIii** Control Of Spatial Activity: The ACMs finds use in a method of controlling the spacial activity of a CRISPR-Cas having two or more tissue or cell type specific activities where the Anti-CRISPR Molecule selectively regulates at least one of said plurality of tissue- or cell type-specific activities. The co- or post-delivery of the ACM to a cell with a CRISPR-Cas expression system will result in CRISPR-Cas activity being confined to a selected subset of possible cell types or tissues.
**VIIIiii** Gene Drives: The ACMs may be used to temporarily switch off CRISPR-Cas mediated gene drives when used in: i) preventing the spread of genes in insect vectors of infectious disease (malaria, dengue, Zika, chikungunya etc.); ii) preventing the ability of a general population to act as a host for a pathogen; iii) immunizing animal reservoirs of disease; and iv) rendering a specific animal/plant population sub-fertile or even sterile.

### VIIIiv Pharmaceutical compositions

A composition comprising the ACM is provided for use as a medicament, where the composition may optionally comprise ingredients required for administration of said composition to a subject in need thereof. Said composition finds application when provided as an adjunct (e.g. co-administration) to the administration of a CRISPR-Cas expression system to the subject, whereby said ACM is one that is capable of regulating the CRISPR-Cas gene editing activity of said CRISPR-Cas expression system such that target DNA in the subject is edited, while limiting or preventing off-target DNA editing. The DNA in the subject that is the target for DNA editing by the CRISPR-Cas expression system is DNA in a somatic cell (or a germ-line cell of a non-human primate).

The ACMs may also be incorporated into a heterobifunctional molecule, comprising the ACM linked to a ubiquitin-ligase binding domain; finding use in promoting Cas protein (Cas9) proteosomal degradation; such as to reduce the half-life of administered Cas protein and reduce Cas immune responses in a subject.

### EXAMPLES

### Example 1 Construction and cloning of a genetic circuit [GC] for screening for polypeptide inhibitors of CRISPR-Cas associated activity

The genetic circuit was cloned on two plasmids, where pCasens3 comprises a cas9 gene having theophylline-inducible expression; and pDual3 comprises an arabinose-inducible sgRNA gene and a constitutively expressed chloramphenicol-resistance gene (CmR), which is the Cas9-sgRNA target.

### 1.1 Construction of pCasens3 and pDual3 plasmids

The pCasens3 and pDual3 plasmids, illustrated in Figure 2 (left-hand central and plasmids respectively), were constructed as follows:
Plasmid pCasens3 containing the *Streptococcus pyogenes* Cas9 gene (spCas9) [SEQ ID No.: 151] was constructed in a single step by using USER cloning (Genee et al. 2014). A DNA fragment containing spCas9 gene [SEQ ID No.:150] and its endogenous terminator [SEQ ID No.:152] was PCR amplified from a plasmid DS-SPcas (supplied by addgene: Plasmid #48645) (Esvelt et al. 2013); and the amplified DNA fragment was cloned into the backbone of the plasmid pSEVA47 (as described by Martinez-Garcia et al. 2014). The plasmid pSEVA47 contains: the low copy number origin of replication pSC101 [SEQ ID No.: 155], and the antibiotic resistance gene aadA [SEQ ID No.: 153] that confers resistance against Spectinomycin and a restriction site for insertion of a gene. A DNA molecule [SEQ ID No.:149] encoding a theophylline translational riboswitch was placed in front of Cas9 using a long forward primer: 5'AAGTCTAGCGAACCGCACTTAATACGACTCACTATAGGTACCGGTGATACCAGCATCGTCTT GATGCCCTTGGCAGCACCCTGCTAAGGTAACAACAAGATGATGGATAAGAAATACTCAATAGG CTTAGATATCGGCAC-3' [SEQ ID No.:156]). Additionally, a sigma70 constitutive promoter (J23100; as defined by http://parts.igem.org/Promoters/Catalog/Anderson) was also introduced using a reverse primer in substitution for the endogenous of spCas9 promoter:
5'-ctctagTagctagcactgtacctaggactgagctagccgtcaaGTTAGCTGTGCTCTAGAAGCTAGCAG-3' [SEQ ID No.:157].

Plasmid pDual3 for arabinose-inducible sgRNA expression was constructed by using USER cloning (Genee et al. 2014) to insert the respective genes into the backbone of the plasmid was from pSEVA3610, described by Martínez-Garćía et al., 2015. The chimeric gRNA encoding sequence [SEQ ID No.:161] with a TrrnB terminator [SEQ ID No.:162] and a chloramphenicol resistance gene (CmR) [SEQ ID No.:164] were cloned downstream of the inducible pBAD promoter [SEQ ID No.:158] and araC gene [SEQ ID No.:159] encoding an L-arabinose-inducible transcription factor. In the absence of L-arabinose, the AraC protein binds to operator sites within pBAD effectively repressing transcription. On addition of L-arabinose, AraC protein binds to L-arabinose to form a complex having a DNA-binding conformation that activates pBAD leading to induction of transcription of its cognate genes, namely sgRNA and CmR. The plasmid comprises a low copy number origin of replication, p15A [SEQ ID No.:163]. The first twenty nucleotide sequence of the encoded gRNA transcript is complementary to nucleotides 96:115 of the + strand in the CmR gene having [SEQ ID No.:164].
Positive strand nucleotides 96:115: CTATAACCAGACCGTTCAGC [SEQ ID No.:166]

### 1.2 Construction of GFP plasmid for detecting host cell growth

A GFP plasmid (Figure 2) was constructed by inserting a gfp gene [SEQ ID No.:140] with rrnB T1 terminator [SEQ ID No.:142] capable of expression of GFP; under the control of a constitutive pTET promoter (lacking the TET repressor) and RBS [SEQ ID No.:139] into a plasmid have a ColE1 ori [SEQ ID No.:143] and kanamycin resistance gene [SEQ ID No.:144].

### 1.3 Construction of AcrIIA2 plasmid for monitoring in vitro anti-CRISPR assay

A control plasmid for testing the genetic circuit was constructed (Figure 2A) by inserting an acrIIa2 gene [SEQ ID No.:159] capable of expression of the anti-CRISPR protein, AcrIIA2, under the control of a constitutive pTET promoter (lacking the TET repressor) and RBS [SEQ ID No.:139] into a plasmid have a ColE1 ori [SEQ ID No.:143] and kanamycin resistance gene [SEQ ID No.:144].

The expression of the anti-CRISPR protein, AcrIIA2 is capable of inhibiting Cas9 mediated inactivation of the CmR gene.

### 1.4 Host cells transformed with the GC (pCasens3 and pDual3 plasmids)

E.coli TOP10 were made electro-competent and transformed with the genetic circuit comprising the plasmids: pCasens3 and pDual3 (obtained in example 1.1) as follows:
i. A 40 µl sample of electrocompetant E.coli TOP10 cells were transferred to microcentrifuge tubes; to which a 1 µl sample of pCasens3 and pDual3 DNA was added; and this DNA-cell mixture was then transferred to a cold cuvette and electroporated with a pulse having 1.8 kv, 200 ohms and 25 µF.
ii. Immediately thereafter, a 975 µl volume of 37°C SOC [Ausubel F.M. et al. (1987)] was added to the electroporated cells; and then incubated on rollers at 37°C for 1 h;
iii. The recovered transformed cells were diluted 10^0 - 10^8; plated on selective solid media comprising Luria Broth agar supplemented with: 50 µg/mL spectinomycin; 30 µg/mL chloramphenicol and 50 µg/mL kanamycin; and incubate overnight at 37°C.
iv. Transformed cell colonies comprising the pCasens3 and pDual3 plasmids (*E. coli* genetic circuit [E *coli-GC*]) were selected; amplified and stored.

### 1.5 E. coli-GC cells transformed with GFP plasmid

E.coli TOP10 containing the genetic circuit comprising the plasmids: pCasens3 and pDual3 (E. coli-GC) were made electro-competent and transformed with a plasmid expressing GFP, employing the transformation protocol described in Example 1.2, but where the host cells were E. coli-GC.

### Example 2 In vivo and in vitro screening identifies small molecule inhibitors of CRISPR-Cas associated activity

50,000 compounds from small molecule libraries were screened for anti-CRISPR activity *in vivo.*

### 2.1 In vivo screening assay with E. coli-GC cells detects small molecules having anti-CRISPR activity

*E coli*-GC cells comprising the genetic circuit (GC) and GFP plasmid (Figure 2); were use to screen libraries of small molecules for anti-CRISPR activity as follows:
i. Overnight cultures of said *E.coli*-GC cells were prepared by culture in 3ml filtered M9 minimal medium (http://cshprotocois.cship.org/content/2010/8/pdb.rec12295.short), comprising gluclose as sole carbon source, and supplemented with the following antibiotics (30 µg/mL chloramphenicol; 50 µg/mL kanamycin; and 30 µg/mL spectinomycin) at 37°C and 250RPM;
ii. The wells of 384-well plates (Corning 3710) were prefilled with 30 µL of M9 minimal media supplemented with 2mM theophylline (for inducing Cas9 expression), and 1% arabinose (for inducing gRNA transcription) and antibiotics (50 µg/mL spectinomycin; 30 µg/mL chloramphenicol and 50 µg/mL kanamycin); and then each well was inoculated with 1:1000 of the cultured *E.coli*-GC cells of step (i).
iii. 300 nL of each compound of the small molecule libraries were pin-transferred to an assigned well on duplicate plates. The absorbance (OD 600nm) and sfGFP fluorescence signal (Ex485/Em515nm) of each well of the plates was measured at t=0 using a PerkinElmer EnVision in order to identify compounds that have an inherent absorption at 600nm or in the same wavelength as sfGFP signal. Plates, covered with lids (Corning 3009), were incubated at 37 °C overnight for 16 h.
iv. The absorbance and sfGFP signal of each well of the duplicate plates were measured again at 16h. Growth of the *E.coli*-GC cells in each well was determined by subtracting the subtracting the absorbance and sfGFP fluorescence signal values at t=0h from t=16h in order to remove inherent colour or fluorescence of some of the screened compounds.

Results: Chloramphenical-resistant cell growth (over baseline) was detected in 52 wells, thereby identifying 52 compounds capable of inhibiting Cas9-sgRNA mediated cleavage of the CmR gene (data not shown).

### 2.2 In vitro screening assay validates anti-CRISPR activity of candidate small molecules

The 52 selected candidate compounds from small molecule libraries were re-screened for anti-CRISPR activity *in vitro.*

The in vitro DNA cleavage assay was carried out as described by (Pawluk, N et al., 2016) with the following modifications. SpyCas9 (New England Biolabs) (5nM) was incubated with candidate compound (50∼500µM) for 15 min at 37°C in Cas9 nuclease buffer (New England Biolabs). Alt-R crRNA:tracrRNA (Integrated DNA Technologies) (6nM) was added later and incubated again for 15min at 37°C. Finally, target dsDNA (1nM) was added and incubated for 10 min at 37°C for the cleavage. The reaction was stopped by adding Proteinase K. The cleaved and un-cleaved fraction of target dsDNA were visualized after separation on a 1% agarose gel.

Control assays were performed in parallel, where the candidate compound was substituted with 5µM AcrIIA2, as positive control; either 5µM GFP protein or no protein (DMSO) as negative control; and phenolic Mannich base inhibitor, ZL006 (Sigma-Aldrich). The AcrIIA2 and GFP proteins were obtained from *E*. *coli* cells transformed with the AcrIIA2- and GFP-expression plasmids (see example 1.2 and 1.3).

Results: Of the tested candidate compounds (designated **A**nti-**C**RISPR **M**olecules [ACM]) the compound ACM 37 was identified as the most potent and specific inhibitor of cleavage of a target DNA molecule by the spCas9:gRNA complex in the *in vitro* assay, are shown in Figure 3.

### Example 3 The structure-activity relationship of ACM37 and its analogues

The small molecule, ACM37, identified as having anti-CRISPR activity, is characterized by a pyrrolylthiazole core structure (specifically a 2-(1*H*-pyrrol-2-yl)thiazole)), where the thiazole ring is amide-linked to a piperidine ring; and the pyrrole is sulfonamide linked to an oxazole ring (Figure 4).

### 3.i Structural analogues of ACM37 having a pyrrolylthiazole-based core

ACM37 analogues (Figure 4) were screened for anti-CRISPR activity employing the *in vitro* assay described in Example 2.2. The tested pyrrolylthiazole-based ACM37 analogues were: ACM37-A10 (where the piperidine ring was replaced with azepane); ACM37-A13 (where the pyrrole 2-methyl group is deleted); ACM37-A19 (where the piperidine 4-methyl group is deleted); ACM37-A20a (where piperidine ring is replaced with a pyrrolidine); ACM37-A23a (where oxazole methyl is deleted); and ACM37-A25a (where the oxazole ring and sulphonamide linker are deleted).

Results: ACM37 and its pyrrolylthiazole-based analogues ACM37-A10; ACM37-A13; ACM37-A19 and ACM37-A23a, were identified as the most potent inhibitors of spCas9:gRNA mediated target DNA cleavage.

### 3ii Structural analogues of ACM37 having a biphenyl core

ACM37 analogues (Figure 5) were screened for anti-CRISPR activity employing the *in vitro* assay described in Example 2.2. The following biphenyl-based ACM37 analogues tested were: ACM37-A17 (pyrrolylthiazole core of ACM37 replaced with a biphenyl core); ACM37-A17a (where the oxazole methyl is deleted); ACM37-A17c (where the oxazole ring and sulphonamide linker are deleted); ACM37-A17d (where the piperidine 4-methyl group is deleted); ACM37-A17f (where the piperidine ring and amide linker are deleted); ACM37-A17g (where the oxazole ring, sulphonamide linker and piperidine 4-methyl group are deleted); ACM37-A17n (where the piperidine ring, amide linker and oxazole methyl are deleted); and ACM37-A17p (where the piperidine ring and amide linker are deleted; and with a *meta*-regioisomeric oxazole ring).

Results: ACM37-A17 and each of the tested biphenyl-based analogues were identified as inhibitors of spCas9:gRNA mediated target DNA cleavage, ACM37-A17c being the most potent, followed by ACM37-A17, ACM37-A17p, ACM37-A17a, ACM37-A17n, and ACM37-A17f.

### Example 4 Synthesis of the Anti-CRISPR Molecules of the invention

### 4.1 General method for the preparation of pyrrolylthiazole-based compounds

The synthesis of pyrrolylthiazole compounds may be accomplished as outlined below in **schemes 1** and **2**. The groups used below are as defined for the compounds of formula I-III, but additional definitions are also employed to indicate variable functional groups included in the structure of intermediate compounds towards the final target molecules.

As depicted in **scheme 1,** the central pyrrolylthiazole core of the compounds of formula III can be constructed by a *de novo* thiazole ring synthesis, cyclizing an appropriately substituted α-halo-1,3-dicarbonyl compound (e.g. an α-chloro-1,3-dicarbonyl) and a pyrrole-2-carbothioamide. The other compounds encompassed in formula I and II may be prepared using the same overall cyclization strategy. The skilled artisan will appreciate that the synthesis of the dicarbonyl and thioamide building blocks as well as the wise placement of the cyclization step in the synthetic sequence will vary dependent on the nature of the specific linkers and substituent groups on the target molecule. The skilled artisan will also appreciate that if a linker or substituent is not compatible with the reaction conditions of the cyclization step, suitable protecting groups may be employed.

By way of example, the detailed synthetic routes employed to prepare the compounds encompassed in formula III **(ACM37-A)** are shown in **scheme 2.** The pyrrole-2-carbothioamides are prepared from the corresponding carboxamides by treating these with Lawesson's reagent in tetrahydrofuran (THF). Introduction of an α-methyl group on the pyrrole building block is carried out using a mild two-step C-H functionalization strategy reported by Gui et al., (2014) consisting of an initial radical-based (phenylsulfonyl)methylation and a subsequent reductive desulfonylation to de-mask the methyl group. A distal ring moiety connected to the pyrrolylthiazole core via a sulfonamide linker is introduced by treating the pyrrolylthiazole intermediate with chlorosulfonic acid in acetonitrile (MeCN) (which regioselectively introduces the chlorosulfonyl group in the least sterically hindered pyrrole β-position) followed by coupling with an amine in dichloromethane (DCM) and using pyridine as a catalyst/cosolvent. A distal ring moiety connected to the pyrrolylthiazole core via an amide linker is introduced by having a masked carboxylic acid substituent in the α-chloro-1,3-dicarbonyl building, which is subsequently deprotected and then coupled with an amine using a carboxylic acid activation reagent (N-(3-dimethylaminopropyl)-N'-ethylcarbodiimide hydrochloride (EDC•HCl) or 1-[bis(dimethylamino)methylene]-1*H-*1,2,3-triazolo[**4,5**-*b*]pyridinium 3-oxide hexafluorophosphate (HATU)); the reaction is performed in Dimethylformamide (DMF) in the presence of a base (N,N-diisopropylethyl amine (DIPEA)) and optionally a nucleophilic catalyst (1-hydroxybenzotriazole (HOBt)).

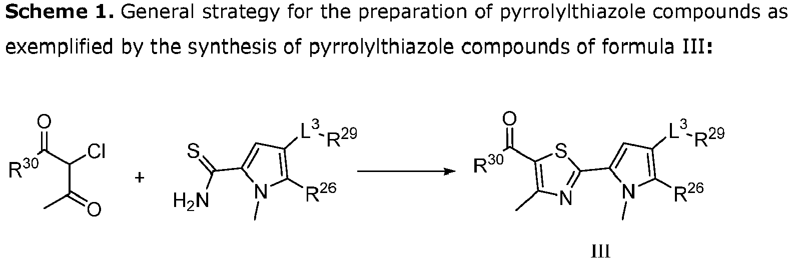

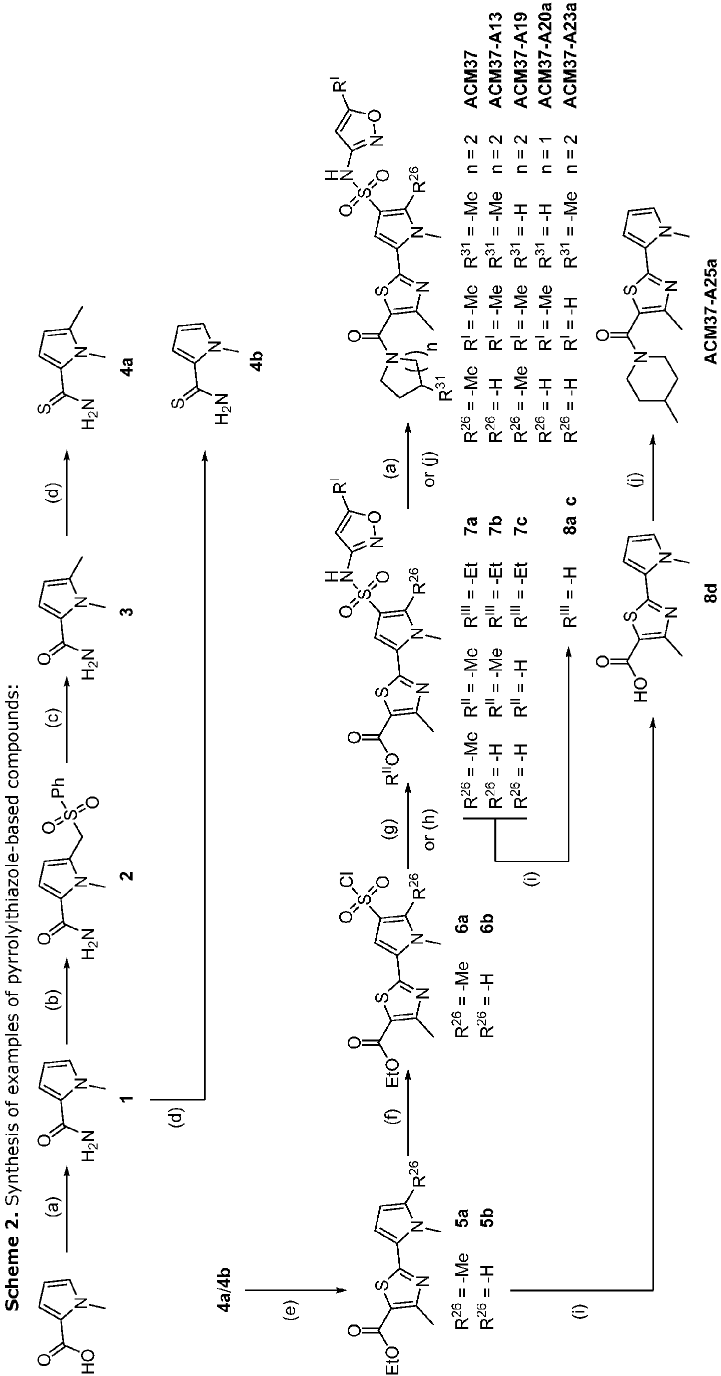

**Reagents and conditions in Scheme 2:** (a) EDC•HCl, HOBt, DIPEA, N,N-dimethylformamide (DMF), 0 °C, 30 min; then amine or amine•HCl, RT, 14-47 h, 55-70 %; (b) zinc bis[(phenylsulfonyl)methanesulfinate], 70 % tert-butyl hydroperoxide (TBHP) (aq), PhCF₃/H₂O, 0 °C-RT, 25 h, quant.; (c) SmI₂, THF/H₂O, RT, 30 min, 88 %; (d) Lawesson's reagent, THF, RT, 15 h, 54-64 %; (e) ethyl 2-chloro-3-oxobutanoate, pyridine, abs. EtOH, reflux, 4-5 h, 74-85 %; (f) CISO₃H, MeCN, 0 °C-RT, 16-18 h, 94 %-quant.; (g) amine, pyridine, DCM, MW irradiation, 50 °C, 2 h, 49 %; (h) amine, pyridine, DCM, reflux, 12-14 h, 63-72 %; (i) 1M NaOH (aq), abs. EtOH, RT, 14-21 h, 60 %-quant.; (j) HATU, DIPEA, DMF, 0 °C, 30 min; then amine, RT, 2.5-18 h, 36-73 %.

### Preparation and analytical conditions for obtaining exemplified pyrrolylthiazole-based compounds:

All temperatures are given in degrees Celsius (°C). Room temperature (RT) refers to temperatures of 20-25 °C. All reactions were conducted under anhydrous conditions in a nitrogen (N₂) atmosphere unless otherwise stated. All chemicals and solvents were purchased from chemical vendors and used without prior purification.

Microwave (MW) assisted synthesis was carried out using a Biotage® Initiator+ apparatus, employing the high absorbance setting.

Thin-layer chromatography (TLC) analyses was performed using TLC silica gel 60 F₂₅₄ aluminum plates (Merck).

¹H NMR and ¹³C NMR-spectra were recorded using a 600 MHz Bruker Avance III HD instrument equipped with a cryogenically cooled 5 mm dual probe or a 400 MHz Bruker Avance III instrument equipped with a 5 mm broad band probe. Samples were dissolved in DMSO-*d₆* (VWR Chemicals, 99.80% D) or chloroform-*d* (Cambridge Isotope Laboratories, Inc., 99.8% D) and analyzed at 300 K. Chemical shifts (δ) are reported in ppm, referenced to DMSO-d₆ (¹H: 2.500 ppm, ¹³C: 39.520 ppm) or chloroform-*d* (¹H: 7.260 ppm, ¹³C: 77.160 ppm). Coupling constants (J) are reported in Hz. Abbreviations for multiplicities: s, singlet; d, doublet; t, triplet; q, quartet; m, multiplet; dd, double doublet; ddd, doublet of doublet of doublets; dt, doublet of triplets; td, triplet of doublets; ttt, triplet of triplet of triplets.

LC-MS-A analyses were carried out using an Agilent 6410 Triple Quadrupole Mass Spectrometer instrument with electron spray ionization (ESI) coupled to an Agilent 1200 HPLC system, a C18 reverse phase column (Zorbax Eclipse XBD-C18, 4.6 mm × 50 mm), autosampler and diode array detector, and a linear gradient of buffer A (milliQ H₂O:MeCN:formic acid, 95:5:0.1 v/v%) to buffer B (milliQ H₂O:MeCN:formic acid, 5:95:0.043 v/v%) with a flow rate of 1 mL/min. LC-MS was performed with positive (pos.) ion mode detection.

LC-MS-B analyses were carried out using an Agilent 6130 Mass Spectrometer instrument using ESI coupled to an Agilent 1200 HPLC system with a C18 reverse phase column (Zorbax Eclipse XBD-C18, 4.6 mm × 50 mm), autosampler and diode array detector, using a linear gradient of the binary solvent system of buffer A (milliQ:H₂O:MeCN:formic acid, 95:5:0.1 v/v%) to buffer B (MeCN:formic acid, 100:0.1 v/v%) with a flow rate of 1 mL/min.

UPLC-MS analyses were carried out using a Waters Acquity H-class UPLC with a Sample Manager FTN and a TUV dual wavelength detector coupled to a QDa single quadrupole analyser using ESI. UPLC separation was achieved with a C18 reversed-phase column (Acquity UPLC BEH C18, 2.1 mm × 50 mm, 1.7 µm) operated at 40 °C, using a linear gradient of the binary solvent system of buffer A (milliQ H₂O:MeCN:formic acid, 95:5:0.1 v/v%) to buffer B (MeCN:formic acid, 100:0.1 v/v%) from 0 to 100 % B in 3.5 min, then 1 min at 100 % buffer B, maintaining a flow rate of 0.8 mL/min.

Silica gel flash chromatography was carried out using prepacked RediSep Rf silica flash cartridges on a CombiFlash® Rf+ apparatus equipped with a detector with UV wavelengths at 254 and 280 nm. Heptane/EtOAc or DCM/MeOH were used as eluent systems.

Reverse phase preparative HPLC was performed using an Agilent 1200 series HPLC preparative system with an Agilent Zorbax 300-SB-C18 column (21.2 x 250 mm). A binary solvent system consisting of buffer A (milliQ H₂O:MeCN:TFA 95:5:0.1 v/v%) and buffer B (milliQ H₂O:MeCN:TFA 5:95:0.1 v/v%) was employed in various gradients.

### 1-Methyl-1H-pyrrole-2-carboxamide (1) in scheme 2:

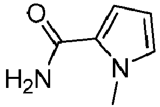

To a round-bottomed flask charged with a magnetic stirring bar was added a solution of 1-methyl-1*H*-pyrrole-2-carboxylic acid (0.626 g, 5.00 mmol, 1.00 equiv) in DMF (20.0 mL). Under cooling at 0 °C were then added EDC·HCl (1.438 g, 7.50 mmol, 1.50 equiv), HOBt (1.013 g, 7.50 mmol, 1.50 equiv) and DIPEA (2.61 mL, 15.0 mmol, 3.00 equiv). The solution was stirred at 0 °C for 30 min. After pre-activation, NH₄Cl (0.535 g, 10.0 mmol, 2.00 equiv) was then added and the mixture stirred at RT for 14 h. Upon reaction completion as determined by LC-MS-A, the reaction was quenched by the addition of H₂O (100 mL). The mixture was extracted with EtOAc (4 x 100 mL), and the combined organic layers were dried over anhydrous Na₂SO₄, filtered and evaporated to dryness *in vacuo* (co-evaporating with toluene two times) to furnish a brown oil. The crude was purified by silica gel flash chromatography (DCM/MeOH, 0-10 %) to afford the desired product **1** as a white solid (0.34 g, 2.74 mmol, 55 %). ¹H NMR (400 MHz, DMSO-*d*₆) δ 7.40 (s, 1H), 6.86 (t, *J* = 2.2 Hz, 1H), 6.78 (dd, *J* = 3.9, 1.8 Hz, 1H), 5.98 (dd, *J* = 3.9, 2.5 Hz, 1H), 3.82 (s, 3H). NMR data matches those previously reported (Smaliy, R. V. et al., 2003).

### 1-Methyl-5-((phenylsulfonyl)methyl)-1H-pyrrole-2-carboxamide (2) in scheme 2:

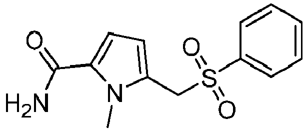

A protocol by Gui *et al.* (2014) was followed. To a vial charged with a magnetic stirring bar were added 1-methyl-1*H*-pyrrole-2-carboxamide (**1**; 0.124 g, 1.00 mmol, 1.00 equiv) and zinc bis[(phenylsulfonyl)methanesulfinate] (Baran PSMS reagent, synthesized according to previously reported protocol;¹ 0.756 g, 1.45 mmol, 1.45 equiv) in PhCF₃ (5.0 mL) and H₂O (2.0 mL). Under cooling at 0 °C was then added 70 % aq. TBHP (0.684 mL, 5.00 mmol, 5.00 equiv). The mixture was stirred at 0 °C for 5 min and then stirred at RT for 25 h. Upon reaction completion as determined by UPLC-MS, the reaction was quenched by the addition of sat. aq. NaHCO₃ (10 mL) and 5 % aq. EDTA-2Na solution (10 mL). The mixture was extracted with DCM (25 mL) and EtOAc (3 x 20 mL). The combined organic layers were dried over anhydrous Na₂SO₄, filtered and evaporated to dryness *in vacuo* to afford the desired crude product **2** (0.31 g, ∼1.0 mmol, ∼quant.) used without further purification. ¹H NMR (400 MHz, DMSO-*d*₆) δ 7.81-7.71 (m, 3H), 7.65-7.58 (m, 2H), 7.47 (s, 1H), 6.91 (s, 1H), 6.68 (d, *J* = 4.0 Hz, 1H), 5.77 (d, *J* = 3.9 Hz, 1H), 4.79 (s, 2H), 3.71 (s, 3H).

### 1,5-Dimethyl-1H-pyrrole-2-carboxamide (3) in scheme 2:

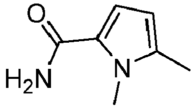

A protocol by Gui *et al.* (2014) was followed. To a vial charged with a magnetic stirring bar was added a solution of 1-methyl-5-((phenylsulfonyl)methyl)-1*H*-pyrrole-2-carboxamide (**2**; 0.214 g, 0.77 mmol, 1.00 equiv) in THF (15.0 mL) and H₂O (1.5 mL). The solution was degassed with N₂ for 20 min. Then 0.1 M SmI₂ in THF (46.1 mL, 4.61 mmol, 6.00 equiv) was added in one portion. The mixture was stirred at RT for 30 min. Upon reaction completion as determined by UPLC-MS, the reaction was quenched by opening the mixture to air, and the mixture was subsequently concentrated *in vacuo* to remove THF. The residue was added sat. aq. NaHCO₃ (20 mL) and extracted with EtOAc (4 x 25 mL). The combined organic layers were dried over anhydrous Na₂SO₄, filtered and evaporated to dryness *in vacuo* to afford a brown oily solid. The crude was purified by silica gel flash chromatography (DCM/MeOH, 0-5 %) to afford the desired product **3** as a light yellow solid (0.094 g, 0.68 mmol, 88 %). ¹H NMR (400 MHz, DMSO-*d*₆) δ 7.26 (s, 1H), 6.69 (d, *J* = 3.8 Hz, 1H), 5.79 (dd, *J* = 3.8, 0.9 Hz, 1H), 3.74 (s, 3H), 2.17 (s, 3H).

### 1,5-Dimethyl-1-pyrrole-2-carbothioamide (4a) in scheme 2:

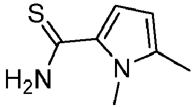

To a round-bottomed flask charged with a magnetic stirring bar was added a solution of 1,5-dimethyl-1*H*-pyrrole-2-carboxamide (**3**; 0.12 g, 0.85 mmol, 1.00 equiv) in dry THF (10.0 mL). The solution was degassed with N₂ for 5 min. Then at RT was added Lawesson's reagent (0.41 g, 1.02 mmol, 1.20 equiv). The mixture was stirred at RT for 15 h. Upon reaction completion as determined by UPLC-MS, the mixture was concentrated *in vacuo* to remove THF. The crude was purified by silica gel flash chromatography (DCM) to afford the desired product **4a** as a light yellow solid (0.070 g, 0.45 mmol, 54 %). ¹H NMR (600 MHz, DMSO-*d*₆) δ 8.90 (s, 1H), 8.73 (s, 1H), 6.59 (d, *J* = 3.8 Hz, 1H), 5.84 (dd, *J* = 3.9, 0.9 Hz, 1H), 3.84 (s, 3H), 2.19 (d, *J* = 0.7 Hz, 3H).

### 1-Methyl-1H-pyrrole-2-carbothioamide (4b) in scheme 2:

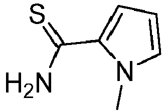

To a round-bottomed flask charged with a magnetic stirring bar was added a solution of 1-methyl-1*H*-pyrrole-2-carboxamide (0.248 g, 2.00 mmol, 1.00 equiv) in dry THF (20.0 mL). The solution was degassed with N₂ for 10 min. Then at RT was added Lawesson's reagent (0.971 g, 2.40 mmol, 1.20 equiv). The mixture was stirred at RT for 15 h. Upon reaction completion as determined by UPLC-MS, the mixture was concentrated *in vacuo* to remove THF. The crude was purified by silica gel flash chromatography (DCM/MeOH, 0-10 %) to afford the desired product **4b** as a yellow oil (0.18 g, 1.27 mmol, 64 %). ¹H NMR (400 MHz, Chloroform-d) δ 7.07-6.88 (m, 2H), 6.85 (t, *J* = 2.2 Hz, 1H), 6.62 (dd, *J* = 4.1, 1.7 Hz, 1H), 6.11 (dd, *J* = 4.0, 2.6 Hz, 1H), 4.08 (s, 3H).

### Ethyl 2-(1,5-dimethyl-1H-pyrrol-2-yl)-4-methylthiazole-5-carboxylate (5a) in scheme 2:

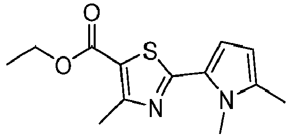

To a round-bottomed flask charged with a magnetic stirring bar was added a solution of 1,5-dimethyl-1-pyrrole-2-carbothioamide (**4a**; 0.070 g, 0.45 mmol, 1.00 equiv) in dry EtOH (10.0 mL). Then ethyl 2-chloro-3-oxobutanoate (0.062 mL, 0.45 mmol, 1.00 equiv) and pyridine (0.036 mL, 0.45 mmol, 1.00 equiv) were added. The mixture was stirred at reflux for 4 h. Upon reaction completion as determined by UPLC-MS, the mixture was concentrated *in vacuo* to remove EtOH. The crude was purified by silica gel flash chromatography (heptane/EtOAc, 0-10 %) to afford the desired product **5a** as a white crystalline solid (0.088 g, 0.33 mmol, 74 %). ¹H NMR (400 MHz, DMSO-*d*₆) δ 6.75 (d, *J* = 3.9 Hz, 1H), 5.97 (dd, *J* = 3.9, 0.9 Hz, 1H), 4.26 (q, *J* = 7.1 Hz, 2H), 3.89 (s, 3H), 2.63 (s, 3H), 2.25 (s, 3H), 1.28 (t, *J* = 7.1 Hz, 3H).

### Ethyl 4-methyl-2-(1-methyl-1H-pyrrol-2-yl)thiazole-5-carboxylate (5b) in scheme 2:

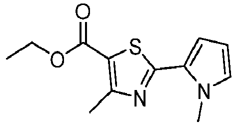

To a round-bottomed flask charged with a magnetic stirring bar was added a solution of 1-methyl-1*H*-pyrrole-2-carbothioamide (**4b**; 0.52 g, 3.71 mmol, 1.00 equiv) in dry EtOH (50.0 mL). Then ethyl 2-chloro-3-oxobutanoate (0.513 mL, 3.71 mmol, 1.00 equiv) and pyridine (0.300 mL, 3.71 mmol, 1.00 equiv) were added. The mixture was stirred at reflux for 5 h. Upon reaction completion as determined by TLC, the mixture was concentrated *in vacuo* to remove EtOH. The crude was purified by silica gel flash chromatography (heptane/EtOAc, 0-20 %) to afford the desired product **5b** as a yellowish solid (0.79 g, 3.16 mmol, 85 %). ¹H NMR (400 MHz, DMSO-*d*₆) δ 7.07 (t, *J* = 2.2 Hz, 1H), 6.83 (dd, *J* = 4.0, 1.7 Hz, 1H), 6.14 (dd, *J* = 4.0, 2.6 Hz, 1H), 4.27 (q, *J* = 7.1 Hz, 2H), 3.96 (s, 3H), 2.64 (s, 3H), 1.29 (t, *J* = 7.1 Hz, 3H).

### Ethyl 2-(4-(chlorosulfonyl)-1,5-dimethyl-1H-pyrrol-2-yl)-4-methylthiazole-5-carboxylate (6a) in scheme 2:

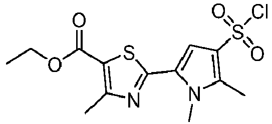

To a vial charged with a magnetic stirring bar was added **e**thyl 2-(1,5-dimethyl-1*H-*pyrrol-2-yl)-4-methylthiazole-5-carboxylate (**5a**; 0.088 g, 0.33 mmol, 1.00 equiv) in dry MeCN (2.0 mL). Under cooling at 0 °C was then added ClSO₃H (0.110 mL, 1.65 mmol, 5.00 equiv). The mixture was stirred at 0 °C and allowed to slowly reach RT over 16 h. Upon reaction completion as determined by UPLC-MS, the reaction was quenched by the addition of ice-cold water (5 mL). The mixture was extracted with EtOAc (3 x 10 mL), and the combined organic layers were dried over anhydrous Na₂SO₄, filtered and evaporated to dryness *in vacuo* to afford the desired crude product **6a** as a light yellow solid (0.11 g, 0.31 mmol, 94 %) used without further purification. ¹H NMR (400 MHz, DMSO-*d*₆) δ 6.74 (s, 1H), 4.27 (q, *J* = 7.1 Hz, 2H), 3.86 (s, 3H), 2.63 (s, 3H), 2.37 (s, 3H), 1.29 (t, *J* = 7.1 Hz, 3H). ¹³C NMR (101 MHz, DMSO-*d*₆) δ 162.15, 161.52, 159.70, 133.92, 129.53, 121.92, 117.49, 112.93, 60.92, 32.60, 17.31, 14.18, 10.60.

### Ethyl 2-(4-(chlorosulfonyl)-1-methyl-1H-pyrrol-2-yl)-4-methylthiazole-5-carboxylate (6b) in scheme 2:

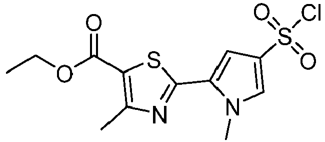

To a vial charged with a magnetic stirring bar was added ethyl 4-methyl-2-(1-methyl-1*H*-pyrrol-2-yl)thiazole-5-carboxylate (**5b**; 0.50 g, 2.00 mmol, 1.00 equiv) in dry MeCN (10.0 mL). Under cooling at 0 °C was then added ClSO₃H (0.66 mL, 10.0 mmol, 5.00 equiv). The mixture was stirred at 0 °C for 5 min and then stirred at RT for 18 h. Upon reaction completion as determined by UPLC-MS, the reaction was quenched by the addition of ice-cold water (20 mL). The mixture was extracted with EtOAc (3 x 30 mL), and the combined organic layers were dried over anhydrous Na₂SO₄, filtered and evaporated to dryness *in vacuo* to afford the desired crude product **6b** as a light yellow solid (0.82 g, ∼2.0 mmol, ∼quant.) used without further purification. ¹H NMR (400 MHz, DMSO-*d*₆) δ 7.16 (d, *J* = 1.8 Hz, 1H), 6.75 (d, *J* = 1.9 Hz, 1H), 4.28 (q, *J* = 7.1 Hz, 2H), 3.92 (s, 3H), 2.64 (s, 3H), 1.29 (t, *J* = 7.1 Hz, 3H).

### Ethyl 2-(1,5-dimethyl-4-(N-(5-methylisoxazol-3-yl)sulfamoyl)-1H-pyrrol-2-yl)-4-methylthiazole-5-carboxylate (7a) in scheme 2:

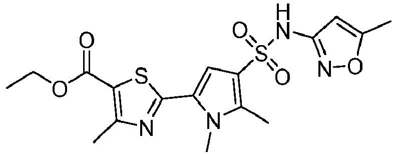

To a pressure vial charged with a magnetic stirring bar were added ethyl 2-(4-(chlorosulfonyl)-1,5-dimethyl-1*H*-pyrrol-2-yl)-4-methylthiazole-5-carboxylate (**6a;** 0.11 g, 0.31 mmol, 1.00 equiv), 5-methylisoxazol-3-amine (0.185 g, 1.89 mmol, 6.08 equiv), pyridine (0.20 mL, 2.48 mmol, 8.00 equiv) and DCM (2.0 mL). The vial was capped and the mixture subjected to MW irradiation at 50 °C for 2 h. Upon reaction completion as determined by TLC, the reaction was quenched by the addition of 1 M aq. HCl (5 mL). The mixture was extracted with DCM (3 x 10 mL), and the combined organic layers were washed with 1 M aq. HCl (10 mL), dried over anhydrous Na₂SO₄, filtered and evaporated to dryness in vacuo to afford a yellow oil. The crude was purified by silica gel flash chromatography (heptane/EtOAc, 0-50 %) to afford the desired product **7a** as a white powder (0.065 g, 0.15 mmol, 49 %). ¹H NMR (600 MHz, DMSO-*d*₆) δ 11.19 (s, 1H), 7.05 (s, 1H), 6.14 (q, *J* = 0.8 Hz, 1H), 4.29 (q, *J* = 7.1 Hz, 2H), 3.89 (s, 3H), 2.65 (s, 3H), 2.46 (s, 3H), 2.29 (d, *J* = 0.9 Hz, 3H), 1.29 (t, *J* = 7.1 Hz, 3H). ¹³C NMR (151 MHz, DMSO-*d*₆) δ 170.00, 161.29, 160.53, 159.71, 157.67, 137.55, 124.42, 119.54, 119.33, 113.32, 95.25, 61.16, 33.12, 17.22, 14.12, 12.03, 10.63.

### Ethyl 4-methyl-2-(1-methyl-4-(N-(5-methylisoxazol-3-yl)sulfamoyl)-1H-pyrrol-2-yl)thiazole-5-carboxylate (7b) in scheme 2:

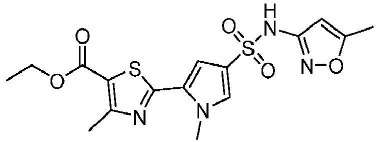

To a pressure vial charged with a magnetic stirring bar were added ethyl 2-(4-(chlorosulfonyl)-1-methyl-1*H*-pyrrol-2-yl)-4-methylthiazole-5-carboxylate (**6b**; 0.52 g, 1.50 mmol, 1.00 equiv), 5-methylisoxazol-3-amine (0.88 g, 9.00 mmol, 6.00 equiv), pyridine (0.97 mL, 12.00 mmol, 8.00 equiv) and DCM (15.0 mL). The vial was capped and the mixture stirred at 50 °C for 14 h. Upon reaction completion as determined by UPLC-MS, the reaction was quenched by the addition of 1 M aq. HCl (10 mL). The mixture was extracted with DCM (2 x 10 mL), and the combined organic layers were washed with 1 M aq. HCl (10 mL), dried over anhydrous Na₂SO₄, filtered and evaporated to dryness *in vacuo* to afford a brown oil. The crude was purified by silica gel flash chromatography (heptane/EtOAc, 0-60 %) to afford the desired product **7b** as a white solid (0.45 g, 1.09 mmol, 72 %). ¹H NMR (600 MHz, DMSO-*d*₆) δ 11.15 (s, 1H), 7.75 (d, *J* = 1.9 Hz, 1H), 7.05 (d, *J* = 1.9 Hz, 1H), 6.17 (d, *J* = 1.0 Hz, 1H), 4.29 (q, *J* = 7.1 Hz, 2H), 3.98 (s, 3H), 2.65 (s, 3H), 2.32 (d, *J* = 0.9 Hz, 3H), 1.30 (t, *J* = 7.1 Hz, 3H).

### Ethyl 2-(4-(N-(isoxazol-3-yl)sulfamoyl)-1-methyl-1H-pyrrol-2-yl)-4-methylthiazole-5-carboxylate (7c) in scheme 2:

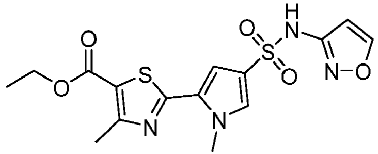

To a pressure vial charged with a magnetic stirring bar were added ethyl 2-(4-(chlorosulfonyl)-1-methyl-1*H*-pyrrol-2-yl)-4-methylthiazole-5-carboxylate (**6b**; 0.105 g, 0.30 mmol, 1.00 equiv), isoxazol-3-amine (0.133 mL, 1.80 mmol, 6.00 equiv), pyridine (0.194 mL, 2.40 mmol, 8.00 equiv) and DCM (3.0 mL). The vial was capped and the mixture stirred at 50 °C for 12 h. Upon reaction completion as determined by UPLC-MS, the reaction was quenched by the addition of 1 M aq. HCl (5 mL). The mixture was extracted with DCM (4 x 5 mL), and the combined organic layers were washed with 1 M aq. HCl (10 mL), dried over anhydrous Na₂SO₄, filtered and evaporated to dryness *in vacuo* to afford a slightly yellow viscous oil. The crude was purified by silica gel flash chromatography (heptane/EtOAc, 0-60 %) to afford the desired product **7c** as a white powder (0.076 g, 0.19 mmol, 63 %). ¹H NMR (400 MHz, DMSO-*d*₆) δ 11.31 (s, 1H), 8.74 (d, *J* = 1.8 Hz, 1H), 7.78 (d, *J* = 2.0 Hz, 1H), 7.06 (d, *J* = 2.0 Hz, 1H), 6.48 (d, *J* = 1.8 Hz, 1H), 4.29 (q, *J* = 7.1 Hz, 2H), 3.97 (s, 3H), 2.65 (s, 3H), 1.29 (t, *J* = 7.1 Hz, 3H). ¹³C NMR (101 MHz, DMSO-*d*₆) δ 161.25, 160.67, 159.82, 159.76, 157.19, 130.54, 126.82, 122.03, 119.92, 111.68, 98.18, 61.22, 37.18, 17.19, 14.11.

### 2-(1,5-Dimethyl-4-(N-(5-methylisoxazol-3-yl)sulfamoyl)-1H-pyrrol-2-yl)-4-methylthiazole-5-carboxylic acid (8a) in scheme 2:

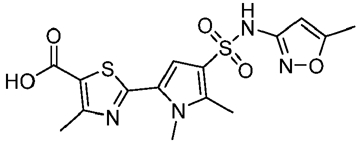

To a vial charged with a magnetic stirring bar was added a solution of ethyl 2-(1,5-dimethyl-4-(*N*-(5-methylisoxazol-3-yl)sulfamoyl)-1*H*-pyrrol-2-yl)-4-methylthiazole-5-carboxylate (**7a**; 0.065 g, 0.15 mmol, 1.00 equiv) in abs. EtOH (1.5 mL). This solution was then treated with 1 M aq. NaOH (0.60 mL, 0.60 mmol, 4.00 equiv). The mixture was stirred at RT for 14 h. Upon reaction completion as determined by UPLC-MS, the mixture was pH-adjusted to 1-2 with 1 M aq. HCl. The mixture was extracted with EtOAc (3 x 10 mL), and the combined organic layers were dried over anhydrous Na₂SO₄, filtered and evaporated to dryness *in vacuo* to afford the desired crude product **8a** as a white solid (0.060 g, ∼0.15 mmol, ∼quant.) used without further purification. UPLC-MS (ESI, pos. mode): m/z 397.0 [M+1]⁺, *t*_{R} = 2.01 min.

### 4-Methyl-2-(1-methyl-4-(N-(5-methylisoxazol-3-yl)sulfamoyl)-1H-pyrrol-2-yl)thiazole-5-carboxylic acid (8b) in scheme 2:

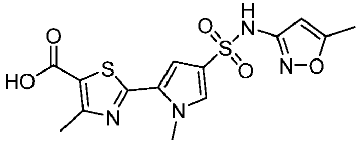

To a vial charged with a magnetic stirring bar was added a solution of ethyl 4-methyl-2-(1-methyl-4-(*N*-(5-methylisoxazol-3-yl)sulfamoyl)-1*H*-pyrrol-2-yl)thiazole-5-carboxylate (**7b**; 0.45 g, 1.09 mmol, 1.00 equiv) in abs. EtOH (10.0 mL). This solution was then treated with 1 M aq. NaOH (4.37 mL, 4.37 mmol, 4.00 equiv). The mixture was stirred at RT for 21 h. Upon reaction completion as determined by UPLC-MS, the mixture was pH-adjusted to 1-2 with 2 M aq. HCl. The mixture was extracted with EtOAc (3 x 15 mL) and 15 % *i*-PrOH in EtOAc (2 x 15 mL), and the combined organic layers were dried over anhydrous Na₂SO₄, filtered and evaporated to dryness *in vacuo* to afford the desired crude product **8b** as a white solid (0.41 g, 1.08 mmol, 98 %) used without further purification. ¹H NMR (400 MHz, DMSO-*d*₆) δ 11.14 (s, 1H), 7.74 (d, *J* = 1.9 Hz, 1H), 7.01 (d, *J* = 1.9 Hz, 1H), 6.17 (d, *J* = 1.0 Hz, 1H), 3.97 (s, 3H), 2.63 (s, 3H), 2.32 (d, *J* = 0.9 Hz, 3H).

### 2-(4-(N-(Isoxazol-3-yl)sulfamoyl)-1-methyl-1H-pyrrol-2-yl)-4-methylthiazole-5-carboxylic acid (8c) in scheme 2:

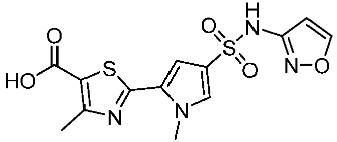

To a vial charged with a magnetic stirring bar was added a solution of ethyl 2-(4-(*N-*(isoxazol-3-yl)sulfamoyl)-1-methyl-1*H*-pyrrol-2-yl)-4-methylthiazole-5-carboxylate (**7c**; 0.075 g, 0.19 mmol, 1.00 equiv) in abs. EtOH (3.0 mL). This solution was then treated with 1 M aq. NaOH (0.76 mL, 0.76 mmol, 4.00 equiv). The mixture was stirred at RT for 16 h. Upon reaction completion as determined by UPLC-MS, the mixture was pH-adjusted to 1-2 with 1 M aq. HCl. The mixture was extracted with EtOAc (3 x 10 mL), and the combined organic layers were dried over anhydrous Na₂SO₄, filtered and evaporated to dryness *in vacuo* to afford the desired crude product **8c** as a white solid (0.070 g, 0.19 mmol, quant.) used without further purification. ¹H NMR (400 MHz, DMSO-*d*₆) δ 13.31 (s, 1H), 11.29 (s, 1H), 8.74 (d, *J* = 1.8 Hz, 1H), 7.76 (d, *J* = 1.9 Hz, 1H), 7.01 (d, *J* = 1.9 Hz, 1H), 6.47 (d, *J* = 1.8 Hz, 1H), 3.97 (s, 3H), 2.63 (s, 3H).

### 4-Methyl-2-(1-methyl-1H-pyrrol-2-yl)thiazole-5-carboxylic acid (8d).

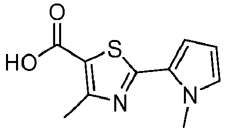

To a vial charged with a magnetic stirring bar was added a solution of ethyl 4-methyl-2-(1-methyl-1*H*-pyrrol-2-yl)thiazole-5-carboxylate (**5b**; 0.15 g, 0.60 mmol, 1.00 equiv) in abs. EtOH (6.0 mL). This solution was then treated with 1 M aq. NaOH (2.40 mL, 2.40 mmol, 4.00 equiv). The mixture was stirred at RT for 17 h. Upon reaction completion as determined by UPLC-MS, the mixture was pH-adjusted to 1-2 with 1 M HCl. The mixture was extracted with EtOAc (2 x 10 mL), and the combined organic layers were dried over anhydrous Na₂SO₄, filtered and evaporated to dryness *in vacuo* to afford the desired crude product **8d** as a brown solid (0.078 g, 0.35 mmol, 60 %) used without further purification. ¹H NMR (400 MHz, DMSO-*d*₆) δ 13.13 (s, 1H), 7.06 (t, *J* = 2.1 Hz, 1H), 6.80 (dd, *J* = 3.9, 1.7 Hz, 1H), 6.14 (dd, *J* = 3.9, 2.6 Hz, 1H), 3.97 (s, 3H), 2.63 (s, 3H).

### 1,2-Dimethyl-5-(4-methyl-5-(4-methylpiperidine-1-carbonyl)thiazol-2-yl)-N-(5-methylisoxazol-3-yl)-1H-pyrrole-3-sulfonamide (ACM37) in scheme 2:

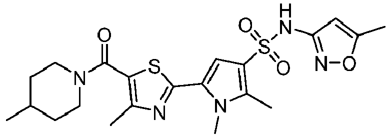

To a vial charged with a magnetic stirring bar was added a solution of 2-(1,5-dimethyl-4-(*N*-(5-methylisoxazol-3-yl)sulfamoyl)-1*H*-pyrrol-2-yl)-4-methylthiazole-5-carboxylic acid (**8a**; 0.030 g, 0.076 mmol, 1.00 equiv) in DMF (2.0 mL). Under cooling at 0 °C were then added HATU (0.058 g, 0.152 mmol, 2.00 equiv) and DIPEA (0.040 mL, 0.228 mmol, 3.00 equiv). The solution was stirred at 0 °C for 30 min. After pre-activation, 4-methylpiperidine (0.018 mL, 0.152 mmol, 2.00 equiv) was added and the mixture stirred at RT for 18 h. Upon reaction completion as determined by UPLC-MS, the mixture was directly purified by reverse phase preparative HPLC (buffer A/buffer B, 0-70 %, 40 min) to afford the desired product **ACM37** as a yellowish solid (0.022 g, 0.047 mmol, 62 %). ¹H NMR (600 MHz, DMSO-*d*₆) δ 11.14 (s, 1H), 6.89 (s, 1H), 6.12 (d, *J* = 1.1 Hz, 1H), 3.87 (s, 3H), 2.96 (s, 2H), 2.45 (s, 3H), 2.35 (s, 3H), 2.29 (d, *J* = 0.9 Hz, 3H), 1.72-1.58 (m, 3H), 1.12-1.00 (m, 2H), 0.92 (d, *J* = 6.3 Hz, 3H). ¹³C NMR (151 MHz, DMSO-*d*₆) δ 169.97, 160.76, 157.71, 157.62, 150.87, 136.75, 124.47, 123.57, 119.02, 112.03, 95.25, 33.83, 32.87, 30.27, 21.45, 16.16, 12.03, 10.58. UPLC-MS (ESI, pos. mode): m/z 478.2 [M+1]⁺, *t*_{R} = 2.42 min. Purity > 95 % (UV, subtracting blank DMSO).

### 1-Diethyl-5-(4-methyl-5-(4-methylpiperidine-1-carbonyl)thiazol-2-yl)-N-(5-methylisoxazol-3-yl)-1H-pyrrole-3-sulfonamide (ACM37-A13) in scheme 2:

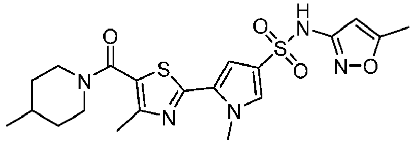

To a vial charged with a magnetic stirring bar was added a solution of 4-methyl-2-(1-methyl-4-(*N*-(5-methylisoxazol-3-yl)sulfamoyl)-1*H*-pyrrol-2-yl)thiazole-5-carboxylic acid (**8b**; 0.024 g, 0.063 mmol, 1.00 equiv) in DMF (2.0 mL). Under cooling at 0 °C were then added EDC•HCl (0.024 g, 0.127 mmol, 2.00 equiv), HOBt (0.017 g, 0.127 mmol, 2.00 equiv) and DIPEA (0.044 mL, 0.252 mmol, 4.00 equiv). The solution was stirred at 0 °C for 30 min. After pre-activation, 4-methylpiperidine (0.030 mL, 0.252 mmol, 4.00 equiv) was added and the mixture stirred at RT for 47 h. Upon reaction completion as determined by LC-MS-A, the mixture was directly purified by reverse phase preparative HPLC (buffer A/buffer B, 0-80 %, 40 min) to afford the desired product **ACM37-A13** as a white solid (0.020 g, 0.044 mmol, 70 %). ¹H NMR (600 MHz, DMSO-*d*₆) δ 11.11 (s, 1H), 7.70 (d, *J* = 2.0 Hz, 1H), 6.90 (d, *J* = 2.0 Hz, 1H), 6.16 (d, *J* = 1.1 Hz, 1H), 3.95 (s, 3H), 2.95 (s, 2H), 2.35 (s, 3H), 2.32 (d, *J* = 0.9 Hz, 3H), 1.71-1.57 (m, 3H), 1.05 (p, *J* = 9.5, 7.6 Hz, 2H), 0.92 (d, *J* = 6.2 Hz, 3H). ¹³C NMR (151 MHz, DMSO-*d*₆) δ 170.01, 160.70, 157.76, 156.98, 150.96, 129.73, 126.80, 124.03, 121.86, 110.53, 95.32, 40.06, 39.92, 39.17, 36.93, 33.85, 30.27, 21.45, 16.12, 12.08. LC-MS-B (ESI, pos. mode): m/z 464.2 [M+1]⁺, 927.3 [2M+1]⁺, *t*_{R} = 4.26 min. Purity > 95 % (UV).

### 1,2-Dimethyl-5-(4-methyl-5-(piperidine-1-carbonyl)thiazol-2-yl)-N-(5-methylisoxazol-3-yl)-1H-pyrrole-3-sulfonamide (ACM37-A19).

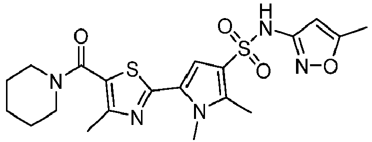

To a vial charged with a magnetic stirring bar was added a solution of 2-(1,5-dimethyl-4-(*N*-(5-methylisoxazol-3-yl)sulfamoyl)-1*H*-pyrrol-2-yl)-4-methylthiazole-5-carboxylic acid (**8a**; 0.030 g, 0.076 mmol, 1.00 equiv) in DMF (2.0 mL). Under cooling at 0 °C were then added HATU (0.058 g, 0.152 mmol, 2.00 equiv) and DIPEA (0.040 mL, 0.228 mmol, 3.00 equiv). The solution was stirred at 0 °C for 30 min. After pre-activation, piperidine (0.015 mL, 0.152 mmol, 2.00 equiv) was added and the mixture stirred at RT for 18 h. Upon reaction completion as determined by UPLC-MS, the mixture was directly purified by reverse phase preparative HPLC (buffer A/buffer B, 0-70 %, 40 min) to afford the desired product **ACM37-A19** as a yellowish solid (0.026 g, 0.055 mmol, 73 %). ¹H NMR (600 MHz, DMSO-*d*₆) δ 11.14 (s, 1H), 6.89 (s, 1H), 6.12 (d, *J* = 1.0 Hz, 1H), 3.87 (s, 3H), 3.48 (s, 4H), 2.45 (s, 3H), 2.35 (s, 3H), 2.29 (d, *J* = 0.9 Hz, 3H), 1.62 (d, *J* = 5.7 Hz, 2H), 1.52 (d, *J* = 6.1 Hz, 4H). ¹³C NMR (151 MHz, DMSO-*d*₆) δ 169.97, 160.75, 157.70, 157.60, 150.84, 136.74, 124.47, 123.52, 119.01, 112.02, 95.26, 32.87, 25.70, 23.85, 16.14, 12.03, 10.58. LC-MS-A (ESI, pos. mode): m/z 464.2 [M+1]⁺, 927.4 [2M+1]⁺, *t*_{R} = 3.24 min. Purity > 95 % (UV).

### 1-Methyl-5-(4-methyl-5-(pyrrolidine-1-carbonyl)thiazol-2-yl)-N-(5-methylisoxazol-3-yl)-1H-pyrrole-3-sulfonamide (ACM37-A20a) in scheme 2:

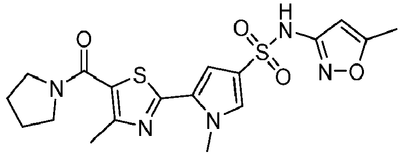

To a vial charged with a magnetic stirring bar was added a solution of 4-methyl-2-(1-methyl-4-(*N*-(5-methylisoxazol-3-yl)sulfamoyl)-1*H*-pyrrol-2-yl)thiazole-5-carboxylic acid (**8b**; 0.076 g, 0.20 mmol, 1.00 equiv) in DMF (5.0 mL). Under cooling at 0 °C were then added HATU (0.152 g, 0.40 mmol, 2.00 equiv) and DIPEA (0.105 mL, 0.60 mmol, 3.00 equiv). The solution was stirred at 0 °C for 30 min. After pre-activation, pyrrolidine (0.033 mL, 0.40 mmol, 2.00 equiv) was added and the mixture stirred at RT for 15 h. Upon reaction completion as determined by UPLC-MS, the reaction was quenched by the addition of 1 M aq. HCl (15 mL). The mixture was extracted with EtOAc (3 x 20 mL), and the combined organic layers were dried over anhydrous Na₂SO₄, filtered and evaporated to dryness *in vacuo.* The crude was purified by reverse phase preparative HPLC (buffer A/buffer B, 0-70 %, 40 min) to afford the desired product **ACM37-A20a** as a white to slight yellow solid (0.031 g, 0.071 mmol, 36 %). ¹H NMR (600 MHz, DMSO-*d*₆) δ 11.12 (s, 1H), 7.71 (d, *J* = 2.0 Hz, 1H), 6.91 (d, *J* = 2.0 Hz, 1H), 6.16 (d, *J* = 1.0 Hz, 1H), 3.96 (s, 3H), 3.45 (s, 4H), 2.43 (s, 3H), 2.32 (d, *J* = 0.9 Hz, 3H), 1.91-1.83 (m, 4H). ¹³C NMR (151 MHz, DMSO-*d*₆) δ 170.02, 160.39, 157.75, 156.76, 153.01, 129.78, 126.79, 124.20, 121.88, 110.51, 95.32, 48.38, 46.18, 36.94, 25.75, 23.87, 16.62, 12.08. LC-MS-A (ESI, pos. mode): m/z 436.2 [M+1]⁺, 871.3 [2M+1]⁺, *t*_{R} = 2.90 min. Purity > 95 % (UV).

### N-(isoxazol-3-yl)-1-methyl-5-(4-methyl-5-(4-methylpiperidine-1-carbonyl)thiazol-2-yl)-1H-pyrrole-3-sulfonamide (ACM37-A23a) in scheme 2:

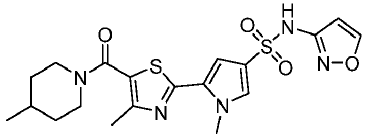

To a vial charged with a magnetic stirring bar was added a solution of 2-(4-(*N-*(isoxazol-3-yl)sulfamoyl)-1-methyl-1*H*-pyrrol-2-yl)-4-methylthiazole-5-carboxylic acid (**8c**; 0.070 g, 0.19 mmol, 1.00 equiv) in DMF (5.0 mL). Under cooling at 0 °C were then added HATU (0.144 g, 0.38 mmol, 2.00 equiv) and DIPEA (0.099 mL, 0.57 mmol, 3.00 equiv). The solution was stirred at 0 °C for 30 min. After pre-activation, 4-methylpiperidine (0.045 mL, 0.38 mmol, 2.00 equiv) was added and the mixture stirred at RT for 18 h. Upon reaction completion as determined by UPLC-MS, the reaction was quenched by the addition of 1 M aq. HCl (20 mL). The mixture was extracted with EtOAc (4 x 20 mL), and the combined organic layers were washed with sat. brine (20 mL), dried over anhydrous Na₂SO₄, filtered and evaporated to dryness *in vacuo* to afford a golden-brown oil. The crude was purified by reverse phase preparative HPLC (buffer A/buffer B, 0-70 %, 40 min) to afford the desired product **ACM37-A23a** as a white to slight yellow solid (0.041 g, 0.091 mmol, 48 %). ¹H NMR (600 MHz, DMSO-*d*₆) δ 11.30 (s, 1H), 8.74 (d, *J* = 1.8 Hz, 1H), 7.75 (d, *J* = 1.9 Hz, 1H), 6.92 (d, *J* = 2.0 Hz, 1H), 6.47 (d, *J* = 1.8 Hz, 1H), 3.95 (s, 3H), 3.14-2.82 (m, 4H), 2.34 (s, 3H), 1.72-1.55 (m, 3H), 1.05 (q, *J* = 11.5 Hz, 2H), 0.91 (d, *J* = 6.4 Hz, 3H). ¹³C NMR (151 MHz, DMSO-*d*₆) δ 160.69, 160.67, 157.25, 156.95, 150.96, 129.86, 126.87, 124.04, 121.66, 110.53, 110.51, 98.21, 98.17, 36.98, 36.90, 33.85, 30.27, 21.45, 16.12. UPLC-MS (ESI, pos. mode): m/z 450.2 [M+1]⁺, *t*_{R} = 2.27 min. Purity > 95 % (UV).

### (4-Methyl-2-(1-methyl-1H-pyrrol-2-yl)thiazol-5-yl)(4-methylpiperidin-1-yl)methanone (ACM37-A25a) in scheme 2:

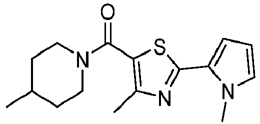

To a vial charged with a magnetic stirring bar was added a solution of 4-Methyl-2-(1-methyl-1*H*-pyrrol-2-yl)thiazole-5-carboxylic acid (**8d**; 0.078 g, 0.35 mmol, 1.00 equiv) in DMF (5.0 mL). Under cooling at 0 °C were then added HATU (0.267 g, 0.70 mmol, 2.00 equiv) and DIPEA (0.183 mL, 1.05 mmol, 3.00 equiv). The solution was stirred at 0 °C for 30 min. After pre-activation, 4-methylpiperidine (0.083 mL, 0.70 mmol, 2.00 equiv) was added and the mixture stirred at RT for 2.5 h. Upon reaction completion as determined by UPLC-MS, the reaction was quenched by the addition of 1 M aq. HCl (20 mL). The mixture was extracted with EtOAc (3 x 20 mL), and the combined organic layers were washed with sat. brine (20 mL), dried over anhydrous Na₂SO₄, filtered and evaporated to dryness *in vacuo* to afford a brown oil. The crude was purified by reverse phase preparative HPLC (buffer A/buffer B, 0-70 %, 40 min) to afford the desired product **ACM37-A25a** as a white to light-brown solid (0.077 g, 0.25 mmol, 70 %). ¹H NMR (400 MHz, DMSO-*d*₆) δ 7.01 (t, *J* = 2.2 Hz, 1H), 6.67 (dd, *J* = 3.9, 1.7 Hz, 1H), 6.11 (dd, *J* = 3.9, 2.6 Hz, 1H), 3.94 (s, 3H), 3.51 (s, 2H), 2.95 (s, 2H), 2.33 (s, 3H), 1.73-1.55 (m, 3H), 1.13-0.97 (m, 2H), 0.92 (d, *J* = 6.1 Hz, 3H). ¹³C NMR (101 MHz, DMSO-*d*₆) δ 161.19, 159.14, 150.71, 127.95, 125.11, 122.05, 112.96, 108.37, 39.19, 36.26, 33.88, 30.30, 21.46, 16.24. UPLC-MS (ESI, pos. mode): m/z 450.2 [M+1]⁺, *t*_{R} = 2.27 min. Purity > 95 % (UV).

### 4.2 General method for the preparation of biphenyl-based compounds

The synthesis of the compounds of the biphenyl compounds may be accomplished as outlined below in **schemes 3** and **4.** The groups used below are as defined above for the biphenyl compounds of formula I-III, but additional definitions are also employed to indicate variable functional groups included in the structure of intermediate compounds towards the final target molecules. Abbreviations are as defined in the Preparation of examples of biphenyl-based compounds section.

As depicted in **scheme 3,** the biphenyl core of the compounds of formula III can be constructed through a central transition metal-catalyzed cross-coupling reaction, e.g. a Suzuki-Miyaura (SM) reaction, between an appropriately substituted aryl halide (e.g. an aryl bromide) and an organometallic species (e.g. a boronic acid in case of the SM reaction). The other biphenyl compounds encompassed in formula I and II may be prepared using the same overall cross-coupling strategy. The skilled artisan will appreciate that dependent on the presence and nature of groups substituted on the biphenyl core, this cross-coupling step can be strategically placed on the synthetic sequence so as to avoid chemoselectivity or stability issues known to the skilled artisan. Alternatively, if a linker or substituent is not compatible with the reaction conditions of the cross-coupling step, suitable protecting groups may be employed. The skilled artisan will also appreciate that the synthesis of the building blocks for the cross-coupling will depend on the specific linkers and substituent groups on the target molecule.

As an example, the detailed synthetic routes employed to prepare the compounds encompassed in formula III (**ACM37-A**) are shown in **scheme 4.** A distal ring moiety connected to the biphenyl core via a sulfonamide linker is introduced by reacting appropriately substituted sulfonyl chlorides with amines in DCM and using pyridine as a catalyst/co-solvent. A distal ring moiety connected to the core via an amide linker is introduced by coupling appropriately substituted carboxylic acids with amines using a carboxylic acid activation reagent (EDC•HCl or HATU); the reaction is performed in DMF in the presence of a base (DIPEA) and optionally a nucleophilic catalyst (HOBt).

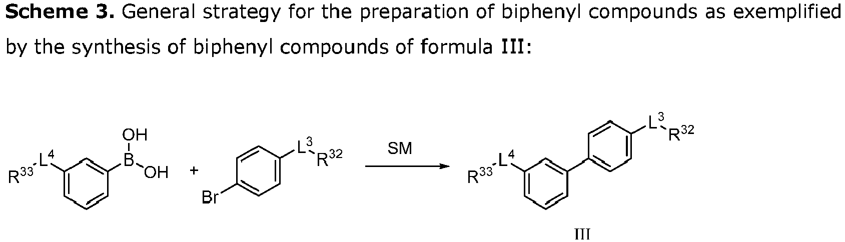

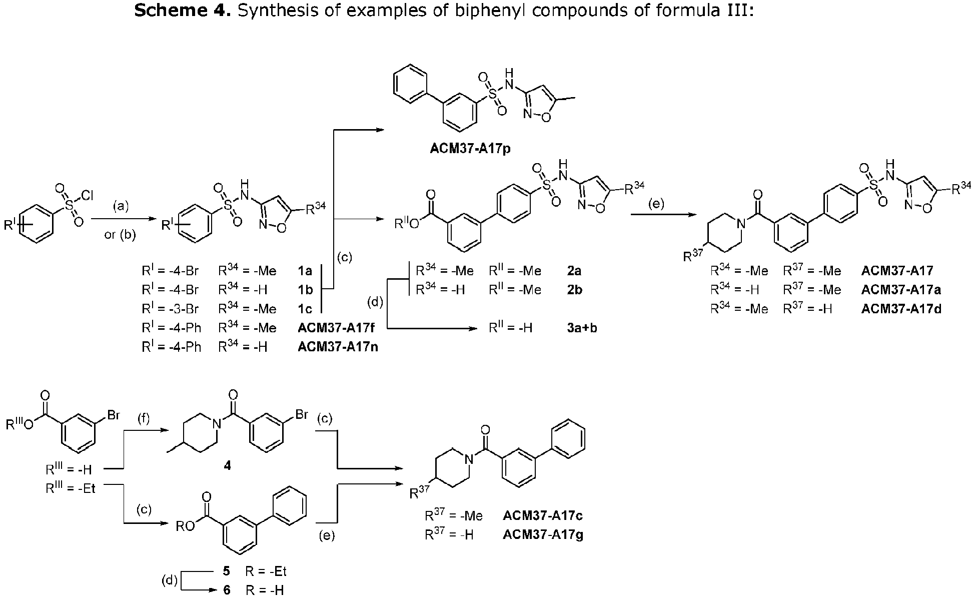

**Reagents and conditions in Scheme 4:** (a) Amine, pyridine, DCM, MW irradiation, 50 °C, 20 min, 89 %; (b) amine, pyridine, DCM, reflux, 5-7 h, 54-82 %; (c) boronic acid, Pd(PPh₃)₄, K₂CO₃ or Cs₂CO₃, 1,4-dioxane/H₂O, 90 °C, 1-14 h, 18-84 %; (d) 1 M NaOH (aq), abs. EtOH, RT, 14-24 h, 83 %-quant.; (e) EDC•HCl, HOBt, DIPEA, DMF, 0 °C, 30-40 min; then amine, RT, 17-23 h, 21-72 %; (f) HATU, DIPEA, DMF, 0 °C, 30 min; then amine, RT, 1.5 h, 82 %.

### Preparation and analytical conditions for obtaining exemplified biphenyl-based compounds:

All temperatures are given in degrees Celsius (°C). Room temperature (RT) refers to temperatures of 20-25 °C. All reactions were conducted under anhydrous conditions in a nitrogen (N₂) atmosphere unless otherwise stated. All chemicals and solvents were purchased from chemical vendors and used without prior purification.

Microwave (MW) assisted synthesis was carried out using a Biotage® Initiator+ apparatus, employing the high absorbance setting.

Thin-layer chromatography (TLC) analyses were performed using TLC silica gel 60 F₂₅₄ aluminum plates (Merck).

¹H NMR and ¹³C NMR-spectra were recorded using a 600 MHz Bruker Avance III HD instrument equipped with a cryogenically cooled 5 mm dual probe or a 400 MHz Bruker Avance III instrument equipped with a 5 mm broad band probe. Samples were dissolved in DMSO-*d₆* (VWR Chemicals, 99.80% D) or chloroform-*d* (Cambridge Isotope Laboratories, Inc., 99.8% D) and analyzed at 300 K. Chemical shifts (δ) are reported in ppm, referenced to DMSO-d₆ (¹H: 2.500 ppm, ¹³C: 39.520 ppm) or chloroform-*d* (¹H: 7.260 ppm, ¹³C: 77.160 ppm). Coupling constants (*J*) are reported in Hz. Abbreviations for multiplicities: s, singlet; d, doublet; t, triplet; q, quartet; m, multiplet; dd, double doublet; ddd, doublet of doublet of doublets; dt, doublet of triplets; td, triplet of doublets; ttt, triplet of triplet of triplets.

LC-MS analyses were carried out using an Agilent 6410 Triple Quadrupole Mass Spectrometer instrument with electron spray ionization (ESI) coupled to an Agilent 1200 HPLC system, a C18 reverse phase column (Zorbax Eclipse XBD-C18, 4.6 mm × 50 mm), autosampler and diode array detector, and a linear gradient of buffer A (milliQ H₂O:MeCN:formic acid, 95:5:0.1 v/v%) to buffer B (milliQ H₂O:MeCN:formic acid, 5:95:0.043 v/v%) with a flow rate of 1 mL/min. LC-MS was performed with positive (pos.) ion mode detection.

UPLC-MS analyses were carried out using a Waters Acquity H-class UPLC with a Sample Manager FTN and a TUV dual wavelength detector coupled to a QDa single quadrupole analyser using ESI. UPLC separation was achieved with a C18 reversed-phase column (Acquity UPLC BEH C18, 2.1 mm × 50 mm, 1.7 µm) operated at 40 °C, using a linear gradient of the binary solvent system of buffer A (milliQ H₂O:MeCN:formic acid, 95:5:0.1 v/v%) to buffer B (MeCN:formic acid, 100:0.1 v/v%) from 0 to 100 % B in 3.5 min, then 1 min at 100 % buffer B, maintaining a flow rate of 0.8 mL/min.

Silica gel flash chromatography was carried out using prepacked RediSep Rf silica flash cartridges on a CombiFlash® Rf+ apparatus equipped with a detector with UV wavelengths at 254 and 280 nm. Heptane/EtOAc or DCM/MeOH were used as eluent systems.

Reverse phase preparative HPLC was performed using an Agilent 1200 series HPLC preparative system with an Agilent Zorbax 300-SB-C18 column (21.2 x 250 mm). A binary solvent system consisting of buffer A (milliQ H₂O:MeCN:TFA 95:5:0.1 v/v%) and buffer B (milliQ H₂O:MeCN:TFA 5:95:0.1 v/v%) was employed in various gradients.

### 4-Bromo-N-(5-methylisoxazol-3-yl)benzenesulfonamide (1a) of scheme 4:

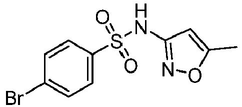

To a pressure vial charged with a magnetic stirring bar were added 5-methylisoxazol-3-amine (0.491 g, 5.00 mmol, 1.00 equiv), pyridine (0.809 mL, 10.0 mmol, 2.00 equiv) and DCM (10.0 mL). The solution was then treated with 4-bromobenzenesulfonyl chloride (1.278 g, 5.00 mmol, 1.00 equiv). The vial was capped and the mixture subjected to MW irradiation at 50 °C for 20 min. Upon reaction completion as determined by TLC, the reaction was quenched by the addition of 1 M aq. HCl (20 mL). The mixture was extracted with DCM (2 x 20 mL), and the combined organic layers were dried over anhydrous Na₂SO₄, filtered and evaporated to dryness *in vacuo* to afford the desired crude product **1a** as a light-brown solid (1.42 g, 4.48 mmol, 89 %) used without further purification. ¹H NMR (400 MHz, Chloroform-*d*) δ 7.75-7.66 (m, 2H), 7.65-7.58 (m, 2H), 6.22 (d, *J* = 1.0 Hz, 1H), 2.38 (d, *J* = 0.9 Hz, 3H).

### 4-Bromo-N-(isoxazol-3-yl)benzenesulfonamide (1b) of scheme 4:

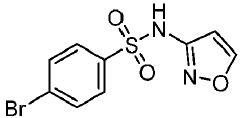

To a pressure vial charged with a magnetic stirring bar were added isoxazol-3-amine (0.44 mL, 6.00 mmol, 6.00 equiv), pyridine (0.65 mL, 8.00 mmol, 8.00 equiv) and DCM (5.0 mL). The solution was then treated with 4-bromobenzenesulfonyl chloride (0.26 g, 1.00 mmol, 1.00 equiv). The vial was capped and the mixture stirred at reflux (50 °C) for 5 h. Upon reaction completion as determined by UPLC-MS, the reaction was quenched by the addition of 1 M aq. HCl (5 mL). The mixture was extracted with DCM (2 x 5 mL), and the combined organic layers were dried over anhydrous Na₂SO₄, filtered and evaporated to dryness *in vacuo.* The crude was purified silica gel flash chromatography (heptane/EtOAc, 0-70 %) to afford the desired product **1b** as a white solid (0.25 g, 0.82 mmol, 82 %). ¹H NMR (400 MHz, Chloroform-*d*) δ 8.31 (s, 1H), 8.27 (d, *J* = 1.7 Hz, 1H), 7.74-7.67 (m, 2H), 7.66-7.60 (m, 2H), 6.60 (d, *J* = 1.7 Hz, 1H).

### 3-Bromo-N-(5-methylisoxazol-3-yl)benzenesulfonamide (1c) of scheme 4:

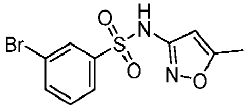

To a round-bottomed flask charged with a magnetic stirring bar were added 5-methylisoxazol-3-amine (1.96 g, 20.0 mmol, 2.55 equiv), pyridine (4.04 mL, 50.0 mmol, 6.39 equiv) and DCM (50.0 mL). The solution was then treated with 3-bromobenzenesulfonyl chloride (2.00 g, 7.83 mmol, 1.00 equiv). The flask was equipped with a reflux condenser and the mixture stirred at reflux for 6 h. Upon reaction completion as determined by TLC, the reaction mixture was washed with 2 M aq. HCl (2 x 30 mL), sat. brine (30 mL), and the organic layer dried over anhydrous Na₂SO₄, filtered and evaporated to dryness *in vacuo* to afford the desired crude product **1c** as a light-yellow solid (3.17 g, 4.60 mmol, 59 %) used without further purification. ¹H NMR (600 MHz, Chloroform-d) δ 9.61 (s, 1H), 7.97 (t, *J* = 1.9 Hz, 1H), 7.75 (ddd, *J* = 8.0, 1.8, 1.0 Hz, 1H), 7.67 (ddd, *J* = 8.0, 1.9, 1.0 Hz, 1H), 7.33 (t, *J* = 8.0 Hz, 1H), 6.25 (d, *J* = 1.0 Hz, 1H), 2.40 (d, *J* = 0.9 Hz, 3H).

### Methyl 4'-(N-(5-methylisoxazol-3-yl)sulfamoyl)-[1,1'-biphenyl]-3-carboxylate (2a) of scheme 4:

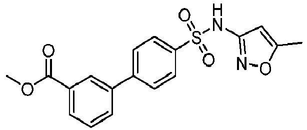

A pressure vial charged with a magnetic stirring bar were added 4-bromo-*N*-(5-methylisoxazol-3-yl)benzenesulfonamide (**1a**; 0.317 g, 1.00 mmol, 1.00 equiv), (3-(methoxycarbonyl)phenyl)boronic acid (0.270 g, 1.50 mmol, 1.50 equiv) and Cs₂CO₃ (0.977 g, 3.00 mmol; 3.00 equiv), 1,4-dioxane (8.0 mL) and H₂O (2.0 mL). The solution was degassed with N₂ for 10 min. To this solution was then added Pd(PPh₃)₄ (0.116 g, 0.10 mmol) against a positive flow of N₂. The vial was capped and the mixture stirred at 90 °C for 1 h. Upon reaction completion as determined by TLC, the reaction was quenched by the addition of H₂O (20 mL). The mixture was extracted with EtOAc (3 x 20 mL), and the combined organic layers were dried over anhydrous Na₂SO₄, filtered and evaporated to dryness *in vacuo* to afford a golden-brown oil. The crude was purified silica gel flash chromatography (heptane/EtOAc, 0-50 %) to afford the desired product **2a** as a yellowish powder (0.14 g, 0.38 mmol, 38 %). ¹H NMR (400 MHz, DMSO-*d*₆) δ 11.52 (s, 1H), 8.24 (d, *J* = 2.5 Hz, 2H), 8.08-7.92 (m, 6H), 7.67 (t, *J* = 7.8 Hz, 1H), 6.18 (d, *J* = 1.0 Hz, 1H), 3.89 (s, 3H), 2.30 (s, 3H).

### Methyl 4'-(N-(isoxazol-3-yl)sulfamoyl)-[1,1'-biphenyl]-3-carboxylate (2b) of scheme 4:

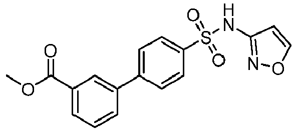

A pressure vial charged with a magnetic stirring bar were added 4-bromo-*N*-(isoxazol-3-yl)benzenesulfonamide (**1b**; 0.30 g, 1.00 mmol, 1.00 equiv), (3-(methoxycarbonyl)phenyl)boronic acid (0.270 g, 1.50 mmol, 1.50 equiv) and K₂CO₃ (0.415 g, 3.00 mmol; 3.00 equiv), 1,4-dioxane (8.0 mL) and H₂O (2.0 mL). The solution was degassed with N₂ for 10 min. To this solution was then added Pd(PPh₃)₄ (0.116 g, 0.10 mmol) against a positive flow of N₂. The vial was capped and the mixture stirred at 90 °C for 1 h. Upon reaction completion as determined by TLC, the reaction was quenched by the addition of H₂O (15 mL) and the mixture extracted with EtOAc (3 x 15 mL). The aq. phase was acidified to pH 1-2 with 1 M aq. HCl and further extracted with EtOAc (2 x 10 mL), and the combined organic layers were dried over anhydrous Na₂SO₄, filtered and evaporated to dryness *in vacuo* to afford a golden-brown oil. The crude was purified silica gel flash chromatography (heptane/EtOAc, 0-75 %) to afford the desired product **2b** as a white sticky solid (0.30 g, 0.84 mmol, 84 %). ¹H NMR (600 MHz, Chloroform-d) δ 8.27 (d, *J* = 1.8 Hz, 1H), 8.24 (t, *J* = 1.8 Hz, 1H), 8.08 (dt, *J* = 7.7, 1.4 Hz, 1H), 7.96-7.90 (m, 2H), 7.75 (ddd, *J* = 7.8, 2.0, 1.1 Hz, 1H), 7.73-7.70 (m, 2H), 7.55 (t, *J* = 7.8 Hz, 1H), 6.65 (d, *J* = 1.8 Hz, 1H), 3.95 (s, 3H).

### 4'-(N-(5-Methylisoxazol-3-yl)sulfamoyl)-[1,1'-biphenyl]-3-carboxylic acid (3a) of scheme 4:

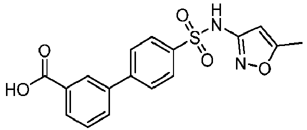

To a vial charged with a magnetic stirring bar was added a solution of methyl 4'-(*N*-(5-methylisoxazol-3-yl)sulfamoyl)-[1,1'-biphenyl]-3-carboxylate (**3a**; 0.17 g, 0.46 mmol, 1.00 equiv) in MeOH (5.0 mL). This solution was then treated with 1 M aq. NaOH (1.83 mL, 1.83 mmol, 4.00 equiv). The mixture was stirred at RT for 14 h. Upon reaction completion as determined by UPLC-MS, the mixture was pH-adjusted to 1-2 with 1 M aq. HCl. The mixture was extracted with EtOAc (3 x 10 mL), and the combined organic layers were dried over anhydrous Na₂SO₄, filtered and evaporated to dryness *in vacuo* to afford the desired crude product **3a** as a light-brown solid (0.13 g, 0.37 mmol, 83 %) used without further purification. ¹H NMR (600 MHz, DMSO-*d*₆) δ 13.09 (s, 1H), 11.52 (s, 1H), 8.23 (t, *J* = 1.8 Hz, 1H), 8.02-7.98 (m, 2H), 7.96-7.94 (m, 4H), 7.65 (t, *J* = 7.7 Hz, 1H), 6.18 (d, *J* = 1.0 Hz, 1H), 2.31 (d, *J* = 0.9 Hz, 3H).

### 4'-(N-(Isoxazol-3-yl)sulfamoyl)-[1,1'-biphenyl]-3-carboxylic acid (3b) of scheme 4:

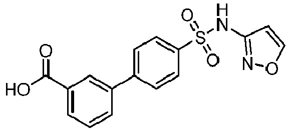

To a vial charged with a magnetic stirring bar was added a solution of methyl 4'-(*N-*(isoxazol-3-yl)sulfamoyl)-[1,1'-biphenyl]-3-carboxylate (**2b**; 0.30 g, 0.84 mmol, 1.00 equiv) in MeOH (8.0 mL). This solution was then treated with 1 M aq. NaOH (3.36 mL, 3.36 mmol, 4.00 equiv). The mixture was stirred at RT for 16 h. Upon reaction completion as determined by UPLC-MS, the mixture was pH-adjusted to 1-2 with 1 M aq. HCl. The mixture was extracted with EtOAc (3 x 15 mL), and the combined organic layers were dried over anhydrous Na₂SO₄, filtered and evaporated to dryness *in vacuo* to afford the desired crude product **3b** as a white solid (0.35 g, ∼1.00 mmol, ∼quant.) used without further purification. ¹H NMR (400 MHz, DMSO-*d*₆) δ 11.67 (s, 1H), 8.74 (d, *J* = 1.8 Hz, 1H), 8.23 (t, *J* = 1.8 Hz, 1H), 8.07-7.88 (m, 6H), 7.64 (t, *J* = 7.8 Hz, 1H), 6.47 (d, *J* = 1.8 Hz, 1H).

### (3-Bromophenyl)(4-methylpiperidin-1-yl)methanone (4) of scheme 4:

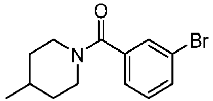

To a vial charged with a magnetic stirring bar was added a solution of 3-bromobenzoic acid (0.20 g, 1.00 mmol, 1.00 equiv) in DMF (5.0 mL). Under cooling at 0 °C were then added HATU (0.76 g, 2.00 mmol, 2.00 equiv) and DIPEA (0.523 mL, 3.00 mmol, 3.00 equiv). The solution was stirred at 0 °C for 30 min. After pre-activation, 4-methylpiperidine (0.237 mL, 2.00 mmol, 2.00 equiv) was added and the mixture stirred at RT for 1.5 h. Upon reaction completion as determined by UPLC-MS, the reaction was quenched by the addition of H₂O. The mixture was extracted with EtOAc (3 x 20 mL), and the combined organic layers were washed with sat. brine (20 mL), dried over anhydrous Na₂SO₄, filtered and evaporated to dryness *in vacuo.* The crude was purified silica gel flash chromatography (heptane/EtOAc, 0-40 %) to afford the desired product **4** as a colorless oil (0.23 g, 0.82 mmol, 82 %). ¹H NMR (400 MHz, Chloroform-*d*) δ 7.59-7.45 (m, 2H), 7.35-7.22 (m, 2H), 4.64 (s, 1H), 3.66 (s, 1H), 2.99 (s, 1H), 2.77 (s, 1H), 1.86-1.50 (m, 3H), 1.36-1.02 (m, 2H), 0.98 (d, *J* = 6.4 Hz, 3H).

### Ethyl [1,1'-biphenyl]-3-carboxylate (5) of scheme 4:

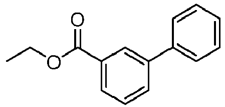

To a round-bottomed flask charged with a magnetic stirring bar were added ethyl 3-bromobenzoate (1.60 mL, 10.0 mmol, 1.00 equiv), phenylboronic acid (1.83 g, 15.0 mmol, 1.50 equiv) and K₂CO₃ (4.15 g, 30.0 mmol; 3.00 equiv), 1,4-dioxane (40.0 mL) and H₂O (10.0 mL). The solution was degassed with N₂ for 10 min. To this solution was then added Pd(PPh₃)₄ (0.58 g, 0.50 mmol, 0.05 equiv) against a positive flow of N₂. The flask was equipped with a reflux condenser, capped and the entire system purged with N₂. The mixture was stirred at 90 °C for 4 h. Upon reaction completion as determined by TLC, the reaction mixture was concentrated *in vacuo* to remove 1,4-dioxane. The residue was added H₂O (20 mL), extracted with EtOAc (3 x 20 mL), and the combined organic layers were dried over anhydrous Na₂SO₄, filtered and evaporated to dryness *in vacuo* to afford a dark-red oily crystalline solid. The crude was purified silica gel flash chromatography (heptane/EtOAc, 0-30 %) to afford the desired product **5** as a colorless to slightly yellow-green oil (1.58 g, 6.98 mmol, 70 %). ¹H NMR (600 MHz, Chloroform-d) δ 8.29 (t, *J* = 1.8 Hz, 1H), 8.04 (dt, *J* = 7.7, 1.4 Hz, 1H), 7.78 (dt, *J* = 7.8, 1.2 Hz, 1H), 7.63 (dd, *J* = 8.1, 1.4 Hz, 2H), 7.52 (t, *J* = 7.7 Hz, 1H), 7.47 (t, *J* = 7.7 Hz, 2H), 7.41-7.36 (m, 1H), 4.42 (q, *J* = 7.1 Hz, 2H), 1.42 (t, *J* = 7.1 Hz, 3H).

### [1,1'-Biphenyl]-3-carboxylic acid (6) of scheme 4:

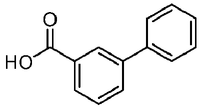

To a round-bottomed flask charged with a magnetic stirring bar was added a solution of ethyl [1,1'-biphenyl]-3-carboxylate (**5**; 1.58 g, 6.98 mmol, 1.00 equiv) in abs. EtOH (70.0 mL). This solution was then treated with 1 M aq. NaOH (28 mL, 27.92 mmol, 4.00 equiv). The mixture was stirred at RT for 22 h. Upon reaction completion as determined by TLC, the mixture was pH-adjusted to 1-2 with conc. aq. HCl. The mixture was extracted with EtOAc (3 x 20 mL), and the combined organic layers were dried over anhydrous Na₂SO₄, filtered and evaporated to dryness *in vacuo* to afford the desired crude product **6** as a white solid (1.38 g, 6.60 mmol, 95 %) used without further purification. ¹H NMR (400 MHz, DMSO-*d*₆) δ 13.07 (s, 1H), 8.18 (t, *J* = 1.8 Hz, 1H), 7.97-7.89 (m, 2H), 7.73-7.67 (m, 2H), 7.60 (t, *J* = 7.7 Hz, 1H), 7.50 (dd, *J* = 8.3, 6.9 Hz, 2H), 7.44-7.37 (m, 1H). NMR data matches those previously reported (Herschhorn, A. et al, 2007).

### N-(5-Methylisoxazol-3-yl)-3'-(4-methylpiperidine-1-carbonyl)-[1,1'-biphenyl]-4-sulfonamide (ACM37-A17) of scheme 4:

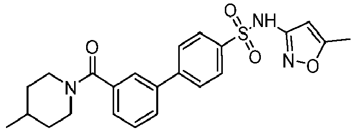

To a vial charged with a magnetic stirring bar was added a solution of 4'-(*N*-(5-methylisoxazol-3-yl)sulfamoyl)-[1,1'-biphenyl]-3-carboxylic acid (**3a**; 0.13 g, 0.36 mmol, 1.00 equiv) in DMF (2.0 mL). Under cooling at 0 °C were then added EDC•HCl (0.10 g, 0.54 mmol, 1.50 equiv), HOBt (0.073 g, 0.54 mmol, 1.50 equiv) and DIPEA (0.188 mL, 1.08 mmol, 3.00 equiv). The solution was stirred at 0 °C for 30 min. After pre-activation, 4-methylpiperidine (0.085 mL, 0.72 mmol, 2.00 equiv) was added and the mixture stirred at RT for 17 h. Upon reaction completion as determined by LC-MS, the mixture was concentrated *in vacuo* to remove DMF. The residue was added H₂O (5 mL), extracted with EtOAc (3 x 5 mL), and the combined organic layers dried over anhydrous Na₂SO₄, filtered and evaporated to dryness *in vacuo* (co-evaporating with toluene two times) to afford a golden-brown oil. The crude was purified by reverse phase preparative HPLC (buffer A/buffer B, 0-90 %, 40 min) to afford the desired product **ACM37-A17** as a white powder (0.059 g, 0.13 mmol, 37 %). ¹H NMR (600 MHz, DMSO-*d*₆) δ 11.51 (s, 1H), 7.96-7.91 (m, 4H), 7.80 (dt, *J* = 8.0, 1.4 Hz, 1H), 7.69 (t, *J* = 1.8 Hz, 1H), 7.57 (t, *J* = 7.7 Hz, 1H), 7.42 (dt, *J* = 7.5, 1.3 Hz, 1H), 6.18 (d, *J* = 1.0 Hz, 1H), 4.46 (s, 1H), 3.55 (s, 1H), 3.04 (s, 1H), 2.77 (s, 1H), 2.30 (d, *J* = 0.8 Hz, 3H), 1.78-1.46 (m, 3H), 1.09 (s, 2H), 0.92 (d, *J* = 6.5 Hz, 3H). ¹³C NMR (151 MHz, DMSO-*d*₆) δ 170.41, 168.35, 157.44, 143.97, 138.49, 138.35, 137.50, 129.28, 127.88, 127.74, 127.39, 126.74, 125.14, 95.42, 47.35, 41.66, 34.03, 33.37, 30.43, 21.54, 12.05. UPLC-MS (ESI, pos. mode): m/z 440.2 [M+1]⁺, *t*_{R} = 2.49 min. Purity > 95 % (UV).

### N-(Isoxazol-3-yl)-3'-(4-methylpiperidine-1-carbonyl)-[1,1'-biphenyl]-4-sulfonamide (ACM37-A17a) of scheme 4:

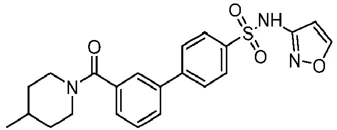

To a vial charged with a magnetic stirring bar was added a solution of 4'-(*N*-(isoxazol-3-yl)sulfamoyl)-[1,1'-biphenyl]-3-carboxylic acid (**3b**; 0.35 g, 0.84 mmol, 1.00 equiv) in DMF (5.0 mL). Under cooling at 0 °C were then added EDC•HCl (0.242 g, 1.26 mmol, 1.50 equiv), HOBt (0.170 g, 1.26 mmol, 1.50 equiv) and DIPEA (0.439 mL, 2.52 mmol, 3.00 equiv). The solution was stirred at 0 °C for 40 min. After pre-activation, 4-methylpiperidine (0.199 mL, 1.68 mmol, 2.00 equiv) was added and the mixture stirred at RT for 22 h. Upon reaction completion as determined by UPLC-MS, the reaction was quenched by the addition of 1 M aq. HCl (20 mL). The mixture was extracted with EtOAc (3 x 20 mL), and the combined organic layers were dried over anhydrous Na₂SO₄, filtered and evaporated to dryness *in vacuo.* The crude was purified by silica gel flash chromatography (DCM/MeOH, 0-10 %) to afford the desired product **ACM37-A17a** as a white solid (0.20 g, 0.46 mmol, 54 %). ¹H NMR (600 MHz, DMSO-*d*₆) δ 11.66 (s, 1H), 8.75 (d, *J* = 1.8 Hz, 1H), 7.96-7.89 (m, 4H), 7.80 (ddd, *J* = 7.9, 2.0, 1.1 Hz, 1H), 7.69 (t, *J* = 1.8 Hz, 1H), 7.56 (t, *J* = 7.7 Hz, 1H), 7.42 (dt, *J* = 7.6, 1.3 Hz, 1H), 6.47 (d, *J* = 1.8 Hz, 1H), 4.46 (s, 1H), 3.55 (s, 1H), 3.03 (s, 1H), 2.76 (s, 1H), 1.70 (s, 1H), 1.61 (ttt, *J* = 10.6, 6.8, 3.9 Hz, 1H), 1.54 (s, 1H), 1.09 (s, 2H), 0.92 (d, *J* = 6.5 Hz, 3H). ¹³C NMR (151 MHz, DMSO-*d*₆) δ 168.35, 160.99, 156.93, 144.05, 138.33, 137.50, 129.27, 127.89, 127.75, 127.44, 126.76, 125.13, 98.35, 47.35, 41.66, 34.03, 33.37, 30.43, 21.54. UPLC-MS (ESI, pos. mode): m/z 426.2 [M+1]⁺, *t*_{R} = 2.39 min. Purity > 95 % (UV).

### [1,1'-Biphenyl]-3-yl(4-methylpiperidin-1-yl)methanone (ACM37-A17c) of scheme 4:

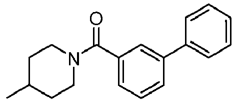

To a round-bottomed flask charged with a magnetic stirring bar were added (3-bromophenyl)(4-methylpiperidin-1-yl)methanone (**4**; 0.23 g, 0.82 mmol, 1.00 equiv), phenylboronic acid (0.15 g, 1.23 mmol, 1.50 equiv) and K₂CO₃ (0.34 g, 2.46 mmol; 3.00 equiv), 1,4-dioxane (8.0 mL) and H₂O (2.0 mL). The solution was degassed with N₂ for 10 min. To this solution was then added Pd(PPh₃)₄ (0.095 g, 0.082 mmol, 0.10 equiv) against a positive flow of N₂. The flask was equipped with a reflux condenser, capped and the entire system purged with N₂. The mixture was stirred at 90 °C for 14 h. Upon reaction completion as determined by UPLC-MS, the reaction was quenched by the addition of H₂O (15 mL). The mixture was extracted with EtOAc (3 x 15 mL), and the combined organic layers were dried over anhydrous Na₂SO₄, filtered and evaporated to dryness *in vacuo* to afford a dark-brown oil. The crude was purified by silica gel flash chromatography (heptane/EtOAc, 0-60 %) to afford the desired product **ACM37-A17c** as a dark-green oil (0.23 g, 0.64 mmol, 77 %). ¹H NMR (400 MHz, DMSO-*d*₆) δ 7.73 (dt, *J* = 7.9, 1.4 Hz, 1H), 7.71-7.66 (m, 2H), 7.61 (t, *J* = 1.8 Hz, 1H), 7.57-7.44 (m, 3H), 7.43-7.32 (m, 2H), 4.47 (s, 1H), 3.59 (s, 1H), 3.04 (s, 1H), 2.77 (s, 1H), 1.81-1.47 (m, 3H), 1.09 (s, 2H), 0.92 (d, *J* = 6.4 Hz, 3H). ¹³C NMR (101 MHz, DMSO-*d*₆) δ 168.63, 140.23, 139.38, 137.28, 129.06, 128.99, 127.75, 127.46, 126.76, 125.57, 124.66, 47.32, 41.66, 39.21, 34.08, 33.41, 30.45, 21.55. UPLC-MS (ESI, pos. mode): m/z 280.1 [M+1]⁺, *t*_{R} = 2.72 min. Purity > 95 % (UV, subtracting blank DMSO).

### N-(5-Methylisoxazol-3-yl)-3'-(piperidine-1-carbonyl)-[1,1'-biphenyl]-4-sulfonamide (ACM37-A17d) of scheme 4:

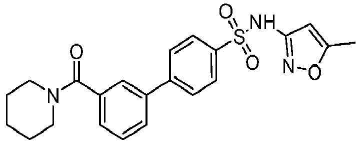

To a vial charged with a magnetic stirring bar was added a solution of 4'-(*N*-(5-methylisoxazol-3-yl)sulfamoyl)-[1,1'-biphenyl]-3-carboxylic acid (**3a**; 0.072 g, 0.20 mmol, 1.00 equiv) in DMF (1.0 mL). Under cooling at 0 °C were then added EDC•HCl (0.058 g, 0.30 mmol, 1.50 equiv), HOBt (0.041 g, 0.30 mmol, 1.50 equiv) and DIPEA (0.105 mL, 0.60 mmol, 3.00 equiv). The solution was stirred at 0 °C for 30 min. After pre-activation, piperidine (0.040 mL, 0.40 mmol, 2.00 equiv) was added and the mixture stirred at RT for 23 h. Upon reaction completion as determined by UPLC-MS, the mixture was concentrated *in vacuo* to remove DMF. The residue was added H₂O (5 mL), extracted with EtOAc (3 x 5 mL), and the combined organic layers dried over anhydrous Na₂SO₄, filtered and evaporated to dryness *in vacuo* (co-evaporating with toluene two times). The crude was purified by reverse phase preparative HPLC (buffer A/buffer B, 0-70 %, 40 min) to afford the desired product **ACM37-A17d** as a white solid (0.018 g, 0.042 mmol, 21 %). ¹H NMR (600 MHz, DMSO-*d*₆) δ 11.51 (s, 1H), 7.97-7.89 (m, 4H), 7.80 (ddd, *J* = 7.8, 2.0, 1.1 Hz, 1H), 7.69 (t, *J* = 1.8 Hz, 1H), 7.57 (t, *J* = 7.7 Hz, 1H), 7.42 (dt, *J* = 7.6, 1.3 Hz, 1H), 6.18 (d, *J* = 1.0 Hz, 1H), 3.60 (s, 2H), 3.29 (s, 2H), 2.30 (d, *J* = 0.9 Hz, 3H), 1.61 (q, *J* = 6.4 Hz, 2H), 1.57 (s, 2H), 1.46 (s, 2H). ¹³C NMR (151 MHz, DMSO-*d*₆) δ 170.42, 168.35, 157.44, 143.96, 138.49, 138.34, 137.49, 129.28, 127.86, 127.73, 127.39, 126.73, 125.12, 95.42, 48.07, 42.32, 25.91, 25.19, 24.02, 12.05. UPLC-MS (ESI, pos. mode): m/z 426.2 [M+1]⁺, *t*_{R} = 2.31 min. Purity > 95 % (UV, subtracting blank DMSO).

### N-(5-Methylisoxazol-3-yl)-[1,1'-biphenyl]-4-sulfonamide (ACM37-A17f) of scheme 4:

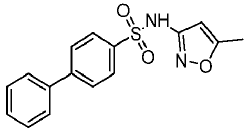

To a pressure vial charged with a magnetic stirring bar were added 5-methylisoxazol-3-amine (0.59 g, 6.00 mmol, 6.00 equiv), pyridine (0.65 mL, 8.00 mmol, 8.00 equiv) and DCM (7.0 mL). The solution was then treated with [1,1'-biphenyl]-4-sulfonyl chloride (0.25 g, 1.00 mmol, 1.00 equiv). The vial was capped and the mixture stirred at reflux (50 °C) for 7 h. Upon reaction completion as determined by UPLC-MS, the mixture was diluted with DCM (5 mL) and washed with 1 M aq. HCl. The aq. phase was re-extracted with DCM (10 mL), and the combined organic layers were dried over anhydrous Na₂SO₄, filtered and evaporated to dryness *in vacuo* to afford a yellowish oily solid. The crude was purified by silica gel flash chromatography (heptane/EtOAc, 0-70 %) to afford the desired product **ACM37-A17f** as a white crystalline solid (0.22 g, 0.70 mmol, 70 %). ¹H NMR (400 MHz, Chloroform-d) δ 8.90 (s, 1H), 7.96-7.87 (m, 2H), 7.70-7.62 (m, 2H), 7.58-7.51 (m, 2H), 7.49-7.37 (m, 3H), 6.29 (d, *J* = 1.0 Hz, 1H), 2.38 (d, *J* = 0.8 Hz, 3H). ¹³C NMR (101 MHz, Chloroform-*d*) δ 171.23, 157.73, 146.61, 139.16, 137.66, 129.20, 128.78, 128.03, 127.72, 127.46, 95.72, 12.90. UPLC-MS (ESI, pos. mode): m/z 315.0 [M+1]⁺, *t*_{R} = 2.42 min. Purity > 95 % (UV).

### [1,1'-Biphenyl]-3-yl(piperidin-1-yl)methanone (ACM37-A17g) of scheme 4:

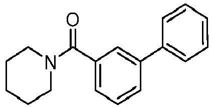

To a vial charged with a magnetic stirring bar was added a solution of [1,1'-biphenyl]-3-carboxylic acid (**6**; 0.694 g, 3.50 mmol, 1.00 equiv) in DMF (7.0 mL). Under cooling at 0 °C were then added EDC•HCl (1.006 g, 5.25 mmol, 1.50 equiv), HOBt (0.709 g, 5.25 mmol, 1.50 equiv) and DIPEA (1.828 mL, 10.50 mmol, 3.00 equiv). The solution was stirred at 0 °C for 30 min. After pre-activation, ∼1/7 of the reaction mixture was transferred to a vial charged with a magnetic stirring bar, treated with piperidine (0.099 mL, 1.00 mmol, 2.00 equiv), and the mixture stirred at RT for 18 h. Upon reaction completion as determined by UPLC-MS, the reaction was quenched by the addition of 1 M aq. HCl (5 mL). The mixture was extracted with EtOAc (4 x 5 mL), and the combined organic layers dried over anhydrous Na₂SO₄, filtered and evaporated to dryness *in vacuo.* The crude was purified by silica gel flash chromatography (heptane/EtOAc, 0-70 %) to afford the desired product **ACM37-A17g** as a colorless oil (0.095 g, 0.36 mmol, 72 %). ¹H NMR (600 MHz, DMSO-*d*₆) δ 7.73 (ddd, *J* = 7.9, 2.0, 1.2 Hz, 1H), 7.71-7.67 (m, 2H), 7.61 (t, *J* = 1.8 Hz, 1H), 7.53 (t, *J* = 7.7 Hz, 1H), 7.50-7.45 (m, 1H), 7.42-7.37 (m, 1H), 7.35 (dt, *J* = 7.6, 1.4 Hz, 1H), 3.60 (s, 2H), 3.31 (s, 2H), 1.62 (td, *J* = 6.9, 4.3 Hz, 2H), 1.57 (s, 2H), 1.47 (s, 2H). ¹³C NMR (151 MHz, DMSO-*d*₆) δ 168.63, 140.22, 139.38, 137.27, 129.07, 129.00, 127.76, 127.44, 126.76, 125.56, 124.66, 48.05, 42.29, 25.94, 25.22, 24.04. UPLC-MS (ESI, pos. mode): m/z 266.0 [M+1]⁺, *t*_{R} = 2.57 min. Purity > 95 % (UV).

### N-(Isoxazol-3-yl)-[1,1'-biphenyl]-4-sulfonamide (ACM37-A17n) of scheme 4:

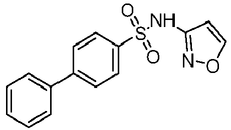

To a pressure vial charged with a magnetic stirring bar were added isoxazol-3-amine (0.44 mL, 6.00 mmol, 6.00 equiv), pyridine (0.65 mL, 8.00 mmol, 8.00 equiv) and DCM (5.0 mL). The solution was then treated with [1,1'-biphenyl]-4-sulfonyl chloride (0.25 g, 1.00 mmol, 1.00 equiv). The vial was capped and the mixture stirred at reflux (50 °C) for 5.5 h. Upon reaction completion as determined by TLC, the mixture was evaporated to dryness *in vacuo.* The crude was purified by silica gel flash chromatography (heptane/EtOAc, 0-80 %) to afford the desired product **ACM37-A17n** as a colorless oil (0.16 g, 0.54 mmol, 54 %). ¹H NMR (400 MHz, Chloroform-*d*) δ 8.26 (d, *J* = 1.8 Hz, 1H), 7.96-7.88 (m, 2H), 7.73-7.65 (m, 2H), 7.59-7.53 (m, 2H), 7.51-7.38 (m, 3H), 6.65 (d, *J* = 1.8 Hz, 1H). ¹³C NMR (101 MHz, Chloroform-*d*) δ 160.04, 157.04, 146.88, 139.08, 137.34, 129.23, 128.86, 128.12, 127.77, 127.48, 98.73. UPLC-MS (ESI, pos. mode): m/z 300.9 [M+1]⁺, *t*_{R} = 2.36 min. Purity > 95 % (UV).

### N-(5-Methylisoxazol-3-yl)-[1,1'-biphenyl]-3-sulfonamide (ACM37-A17p) of scheme 4:

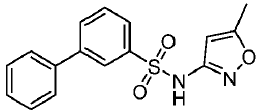

To a pressure vial charged with a magnetic stirring bar were added 3-Bromo-*N*-(5-methylisoxazol-3-yl)benzenesulfonamide (**1c**; 0.19 g, 0.60 mmol, 1.00 equiv), phenylboronic acid (0.11 g, 0.90 mmol, 1.50 equiv) and K₂CO₃ (0.25 g, 1.80 mmol; 3.00 equiv), 1,4-dioxane (4.8 mL) and H₂O (1.2 mL). The solution was degassed with N₂ for 10 min. To this solution was then added Pd(PPh₃)₄ (0.069 g, 0.060 mmol, 0.10 equiv) against a positive flow of N₂. The vial was capped and the mixture stirred at 90 °C for 2 h. Upon reaction completion as determined by UPLC-MS, the reaction was quenched by the addition of H₂O (10 mL). The mixture was extracted with EtOAc (3 x 10 mL), and the combined organic layers were dried over anhydrous Na₂SO₄, filtered and evaporated to dryness *in vacuo* to afford a yellow-green oil. The crude was purified by reverse phase preparative HPLC (buffer A/buffer B, 0-100 %, 40 min) to afford the desired product **ACM37-A17p** as a white solid (0.035 g, 0.11 mmol, 18 %). ¹H NMR (400 MHz, DMSO-*d*₆) δ 11.47 (s, 1H), 8.09 (t, *J* = 1.9 Hz, 1H), 7.98 (dt, *J* = 7.8, 1.5 Hz, 1H), 7.84 (dt, *J* = 7.9, 1.4 Hz, 1H), 7.78-7.64 (m, 3H), 7.58-7.49 (m, 2H), 7.49-7.41 (m, 1H), 6.20 (s, 1H), 2.30 (s, 3H). ¹³C NMR (101 MHz, DMSO-*d*₆) δ 170.48, 157.46, 141.17, 140.14, 138.27, 131.53, 130.15, 129.23, 128.42, 126.81, 125.44, 124.55, 95.47, 12.02. UPLC-MS (ESI, pos. mode): m/z 315.0 [M+1]⁺, *t*_{R} = 2.42 min. Purity > 95 % (UV).

### Example 5 Physicochemical properties of biphenyl-based Anti-CRISPR Molecules of the invention

The physicochemical properties of selected biphenyl-based compounds of the invention having anti-CRISPR activity are set out below:

| Name | AC37-A17C | ACM37-A17p | ACM37-A17 |
|---|---|---|---|
| Structure | 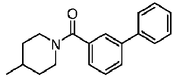 | 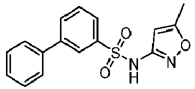 | 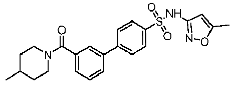 |
| Molecular weight (Da)*^{a}* | 279.38 | 314.36 | 439.53 |
| ClogP*^{b}* | 4.06 | 3.27 | 3.70 |
| ClogD at pH 7.40*^{b}* | 4.06 | 2.40 | 2.84 |
| HBD count*^{c}* | 0 | 1 | 1 |
| HBA count*^{c}* | 2 | 3 | 5 |
| Matching Lipinski's Ro5 | 4/4 | 4/4 | 4/4 |
| Aq. solubility (mg/mL)*^{d}* | ≤0.01 | ≤0.01 | ≤0.01 |
| pKa of most basic site*^{c}* | -0.99 | 0.38 | 0.40 |
| tPSA (Å²)*^{a}* | 20.31 | 67.76 | 88.07 |

| | | | |
|---|---|---|---|
| *^{a}*Calculated using ChemDraw Professional 15.1 *^{b}*Calculated using MarvinSketch 18.24, a consensus method for determining logP value of a compound (which is the logarithm of its partition coefficient between n-octanol and water log(Coctanol/Cwater)); and logD value of a compound (which is the logarithm of its distribution coefficient between n-octanol and water log(Coctanol/Cwater)), which is a well established method for measurement of a compound's hydrophilicity. *^{c}*H-bond donor [HBD] and H-bond acceptor [HBA] counted using Reaxys ^{d}Predicted using MarvinSketch 18.24 | | | |

Each of the selected biphenyl based-compounds is relatively lipophilic by nature, which is indirectly apparent from their partition/distribution coefficient values (ClogP/ClogD); which is generally found to confer a higher cell permeability.

### Example 6. Detection of ACR activity in human cells

Cells of the human cell line, HEK293T, stably expressing a Cas9 gene from an AAS1 site (supplied by GeneCopoeia, MD, USA), with or without transfection with Alt-R crRNA/tracrRNA (supplied by Integrated DNA technologies, IA, USA) using PolyFect Transfection Reagent (Qiagen, Venlo, Netherlands), were used to observe the effect of small molecule inhibitors on guide Cas9-mediated gene editing of a target locus in the host cell genome. The candidate small molecules were incubated with the cells for 1 day prior to crRNA transfection. The cells were then transfected with crRNA, and cultured for 24 hours, allowing for Cas9-induced double-strand breaks introduced in the targeted locus on the chromosome of the human cell to be repaired by non-homologous end joining mechanism (NHEJ) leaving insertion or deletion mutation (indels). A T7E1 assay (see figure 6) was then used, according to the manufacturer's protocol (New England Biolabs, MA, USA), to detect indels in the targeted locus in the genomic DNA extracted from the human cells. The assay was performed by amplifying the target locus by PCR reaction; denaturing and re-annealing the PCR products to create mismatched DNA region around the target site. T7 endonuclease I was then used to detect and cleave the mismatched DNA region resulting in cleaved product appeared on the agarose gel.

A reduction in CRISPR-Cas9 mediated double-stranded breaks in the host cells is detected using the T7E1 assay in cells contacted with small molecule inhibitors of the invention.

### References

Ausubel F.M. et al. (1987) Current Protocols in Molecular Biology
Bondy-Denomy, J., Pawluk, A., Maxwell, K.L., and Davidson, A.R. (2013). Bacteriophage genes that inactivate the CRISPR/Cas bacterial immune system. Nature 493, 429-432.
Esvelt, K. M., Mali, P., Braff, J. L., Moosburner, M., Yaung, S. J., & Church, G. M. (2013). Orthogonal Cas9 proteins for RNA-guided gene regulation and editing. Nature Methods, 10(11), 1116-21. https://doi.org/10.1038/nmeth.2681
Genee, H. J., Bonde, M. T., Bagger, F. O., Jespersen, J. B., Sommer, M. O. a, Wernersson, R., & Olsen, L. R. (2014). Software-supported USER cloning strategies for site-directed mutagenesis and DNA assembly. ACS Synthetic Biology, 4(3), 342-349. https://doi.org/10.1021/sb500194z
Genee H J, Bali A P, Petersen SD, Siedler, S, Bonde MT, Gronenberg LS, Kristensen, M and Harrison SJ and MOA Sommer (2016) Functional mining of transporters using synthetic selections. Nature Chemical Biology 12, 1015-1022*;*
Gui, J.; Zhou, Q.; Pan, C.-M.; Yabe, Y.; Burns, A. C.; Collins, M. R.; Ornelas, M. A.; Ishihara, Y.; Baran, P. S., (2014) C-H Methylation of Heteroarenes Inspired by Radical SAM Methyl Transferase. Journal of the American Chemical Society, 136 (13), 4853-4856.
Fremaux, C., and Moineau, S. (2017). An anti-CRISPR from a virulent streptococcal phage inhibits Streptococcus pyogenes Cas9. Nat. Microbiol. 2, 1374-1380.
Herschhorn, A.; Lerman, L.; Weitman, M.; Gleenberg, I. O.; Nudelman, A.; Hizi, A., (2007) De Novo Parallel Design, Synthesis and Evaluation of Inhibitors against the Reverse Transcriptase of Human Immunodeficiency Virus Type-1 and Drug-Resistant Variants. Journal of Medicinal Chemistry, 50 (10), 2370-2384.
Hynes, A.P., Rousseau, G.M., Agudelo, D., Goulet, A., Amigues, B., Loehr, J., Romero, D.A., Fremaux, C., Horvath, P., Doyon, Y., et al. (2018). Widespread anti-CRISPR proteins in virulent bacteriophages inhibit a range of Cas9 proteins. Nat. Commun. 9, 2919.
Jinek, M., Chylinski, K., Fonfara, I., Hauer, M., Doudna, J.A., and Charpentier, E. (2012) A Programmable Dual-RNA-Guided DNA Endonuclease in Adaptive Bacterial Immunity. Science (80-.) 337, 816-821.
Koonin, E. V., Makarova, K. S., & Zhang, F. (2017) Diversity, classification and evolution of CRISPR-Cas systems. Current Opinion in Microbiology, 37, 67-78. https://doi.org/10.1016/j.mib.2017.05.008
Lutz, R., Bujard, H., (1997) Independent and tight regulation of transcriptional units in Escherichia coli via the LacR / O, the TetR / O and AraC / I 1 -I 2 regulatory elements. Pharmacia 25: 1203-1210*.*
Martínez-Garćía, E., Aparicio, T., Goñi-Moreno, A., Fraile, S., & De Lorenzo, V. (2015). SEVA 2.0: An update of the Standard European Vector Architecture for de-/re-construction of bacterial functionalities. Nucleic Acids Research, 43(D1), D1183-D1189. https://doi.org/10.1093/nar/gku1114
Pawluk, A., N. Amrani, Y. Zhang, B. Garcia, Y. Hidalgo-Reyes, J. Lee, A. Edaki, M. Shah, E.J. Sontheimer, K.L. Maxwell, A.R. Davidson (2016) Naturally Occurring Off-Switches for CRISPR-Cas9 Article Naturally Occurring Off-Switches for CRISPR-Cas9. Cell. 167, 1829-1834.e9.
Rauch, B.J., Silvis, M.R., Hultquist, J.F., Waters, C.S., McGregor, M.J., Krogan, N.J., and Bondy-Denomy, J. (2017). Inhibition of CRISPR-Cas9 with Bacteriophage Proteins. Cell 168, 150-158.e10.
Smaliy, R. V.; Chaikovskaya, A. A.; Pinchuk, A. M.; Tolmachev, A. A., (2003) Isocyanatophosphoryl Dichloride as Reagent for Introduction of Carbamoyl Group into Molecules of n-Excessive Heterocycles and Enamines. Synthesis 16: 2525-2529.

## Claims

1. A method for regulating CRISPR-Cas associated activity and/or CRISPR-dCas associated activity, the method comprising contacting an RNA guided endonuclease-guide RNA complex or a RNA guided DNA binding-guide RNA complex, respectively with a small molecule, wherein said molecule is a compound having formula I: 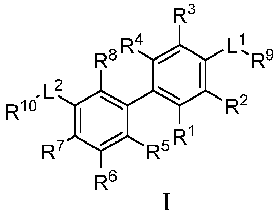 wherein
**R¹, R², R³, R⁴, R⁵, R⁶, R⁷,** and **R⁸** are each independently selected from R¹¹;
**R⁹** is R¹¹ or R¹²;
**R¹⁰** is selected from among: R¹¹, R¹², and -NR¹³R¹⁴;
**L¹** is selected from among: absent, -S(=O)₂N(R¹⁵)-, -C(=O)-, -C(=O)N(R¹⁶)-, -CH₂N(R¹⁷)-, -N(R¹⁷)CH₂-, -CH₂O-, -OCH₂-, C₁₋₃ alkylene, C₂₋₃ alkenylene having one double bond, and C₂₋₃ alkynylene having one triple bond;
**L²** is selected from among: absent, -C(=O)-, -S(=O)₂-, and C₁₋₃ alkylene;
**R¹¹** is selected from among: H, halo, -NO₂, -CN, -OR¹⁸, -NR¹⁹R²⁰, -C(=O)OR²¹, -C(=O)NR²²R²³, Ph, -OPh,
C₁₋₈ alkyl optionally substituted with one to three halo and/or -OH groups,
C₃₋₈ cycloalkyl optionally substituted with one to three halo and/or -OH groups,
4-8 membered heterocycloalkyl comprising one to three heteroatoms independently selected from among N, O, and S,
5-6 membered heteroaryl comprising one to three heteroatoms independently selected from among N, O and S;
**R¹²** is selected from among:
- C₁₋₈ alkyl optionally substituted with one to three groups independently selected from among:
halo, -OR²⁴, -C(=O)OR²⁵, -C(=O)NR²⁶R²⁷, C₃₋₈ cycloalkyl, C₂₋₄ alkenylene having one double bond, C₂₋₄ alkynylene having one triple bond,
4-6 membered heterocycloalkyl comprising one to three heteroatoms independently selected from among N, O, and S, C₆₋₁₀ aryl optionally substituted with one or more independently selected R¹¹ groups, and
5-6 membered heteroaryl comprising one to four heteroatoms independently selected from N, O, and S,
- C₃₋₈ cycloalkyl, optionally substituted with one or more independently selected R¹¹ groups,
- C₄₋₈ cycloalkenyl comprising one double bond, optionally substituted with one or more independently selected R¹¹ groups,
- 4-8 membered heterocycloalkyl comprising one to three heteroatoms independently selected from N, O, and S, optionally substituted with one or more independently selected R¹¹ groups,
- C₆₋₁₀ aryl optionally substituted with one or more independently selected R¹¹ groups, and
- 5-10 membered heteroaryl comprising one to three heteroatoms independently selected from N, O, and S, optionally substituted with one or more independently selected R¹¹ groups;
**R**^{**1**3} and **R¹⁴** are each independently selected from H and R¹²;
**R¹⁵, R¹⁶, R¹⁷, R¹⁸, R¹⁹, R²⁰, R²¹, R²², R²³, R²⁴, R²⁵, R²⁶,** and **R²⁷** are each independently selected from H and C₁₋₈ alkyl optionally substituted with one or more halo and/or -OH groups.

2. The method for regulating CRISPR-Cas associated activity and/or CRISPR-dCas associated activity according to claim 1, wherein said small molecule is a compound having formula II: 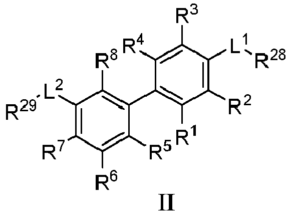 wherein
**R¹, R², R³, R⁴, R⁵, R⁶, R⁷,** and **R⁸** are as defined in claim 1,
**R²⁸** is R¹¹ or 5-10 membered heteroaryl comprising one to three heteroatoms independently selected from N, O, and S, optionally substituted with one or more independently selected R¹¹ groups;
**R¹¹** as defined in claim 1;
**R²⁹** is R¹¹ or -NR³⁰R³¹;
**L¹** and **L²** as defined in claim 1;
**R³⁰** and **R³¹** are each independently selected from H and R¹²;
**R¹²** as defined in claim 1.

3. The method for regulating CRISPR-Cas associated activity and/or CRISPR-dCas associated activity according to claim 1 or 2, wherein said small molecule is a compound having formula III: 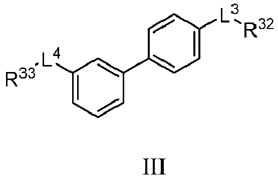 wherein
**R³²** is selected from among: H, 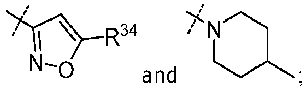
**R³³** is selected from among: H, -NR³⁵R³⁶, and 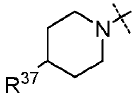
**L³** is selected from among: absent, -S(=O)₂N(H)-, and
-C(=O)-;
**L⁴** is selected from among: absent, -C(=O)-, -S(=O)₂-, and
-CH₂-;
**R³⁴** is H or Me;
**R³⁵** and **R³⁶** are independently selected from among: H,
C₁₋₄ alkyl, and 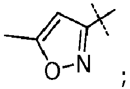
**R³⁷** is H or Me;
while excluding compounds having formula III wherein
**R³²** is H, **R³³** is H, **L³** is absent or -S(=O)₂N(H)-, and **L⁴** is absent.

4. The method for regulating CRISPR-Cas associated activity and/or CRISPR-dCas associated activity according to claim 1 or 2, wherein said small molecule is a compound selected from the group consisting of:
• *N*-(5-Methylisoxazol-3-yl)-3'-(4-methylpiperidine-1-carbonyl)-[1,1'-biphenyl]-4-sulfonamide,
• *N*-(Isoxazol-3-yl)-3'-(4-methylpiperidine-1-carbonyl)-[1,1'-biphenyl]-4-sulfonamide,
• 3'-(4-Methylpiperidine-1-carbonyl)-[1,1'-biphenyl]-4-sulfonamide,
• *N*-(5-Methylisoxazol-3-yl)-3'-(piperidine-1-carbonyl)-[1,1'-biphenyl]-4-sulfonamide,
• 4'-(*N*-(5-Methylisoxazol-3-yl)sulfamoyl)-[1,1'-biphenyl]-3-carboxamide,
• *N*-(Isoxazol-3-yl)-[1,1'-biphenyl]-4-sulfonamide,
• *N*-(5-Methylisoxazol-3-yl)-[1,1'-biphenyl]-3-sulfonamide,
• 1-([1,1'-Biphenyl]-3-ylsulfonyl)-4-methylpiperidine,
• *N*-(5-Methylisoxazol-3-yl)-[1,1'-biphenyl]-3-carboxamide and
• *N*-([1,1'-Biphenyl]-3-ylmethyl)-5-methylisoxazol-3-amine.

5. The method for regulating CRISPR-Cas associated activity and/or CRISPR-dCas associated activity according to any one of claims 1 to 4, wherein said activity is expressed *in vitro,* or in a biological cell selected from the group consisting of a bacterial, yeast, fungal, insect, plant, and somatic mammalian cell.

6. The method for regulating CRISPR-Cas associated activity and/or CRISPR-dCas associated activity according to any one of claims 1 to 5, further comprising
a. providing a biological cell comprising a target DNA sequence;
b. contacting said cell with said small molecule,
wherein said cell either comprises or is additionally contacted with either:
c. an RNA guided endonuclease-guide RNA complex expression system comprising:
i. a first gene encoding a polypeptide or a first gene cluster encoding a polypeptide complex, said polypeptide or polypeptide complex having RNA-guided endonuclease activity, wherein said gene or gene cluster is operably linked to a promoter; and
ii. a nucleic acid molecule operably linked to a promoter, said nucleic acid molecule encoding one or more RNA molecules capable of guiding said polypeptide or polypeptide complex having RNA-guided endonuclease activity to said target DNA sequence;
or
d. an RNA guided DNA binding-guide RNA complex expression system comprising:
iii. a first gene encoding a polypeptide or a first gene cluster encoding a polypeptide complex, said polypeptide or polypeptide complex having RNA-guided DNA binding activity, wherein said gene or gene cluster is operably linked to a promoter; and
iv. a nucleic acid molecule operably linked to a promoter, said nucleic acid molecule encoding one or more RNA molecules capable of guiding said polypeptide or polypeptide complex having RNA-guided DNA binding activity to said target DNA sequence;
wherein said small molecule is capable of regulating said CRISPR-Cas associated activity or CRISPR-dCas associated activity in said cell.

7. The method for regulating CRISPR-Cas associated activity or CRISPR-dCas associated activity according to claim 6, wherein said polypeptide or polypeptide complex having RNA-guided endonuclease activity is selected from the group of CRISPR-Cas systems consisting of Type 1, Type II, Type III; Type IV, Type V, and Type VI.

8. The method for regulating CRISPR-Cas associated activity according to claim 6 or 7, wherein
i. said first gene encodes a Cas9 polypeptide having RNA-guided endonuclease activity, and wherein
ii. one of said RNA molecules is a mat-crRNA and one of said RNA molecules is a cognate trans-activating crRNA, or said one RNA molecule is a fusion of a mat-crRNA and a cognate trans-activating crRNA.

9. The method for regulating CRISPR-Cas associated activity or CRISPR-dCas associated activity according to any one of claims 6 to 8, wherein the amino acid sequence(s) of said polypeptide or each member of said polypeptide complex having RNA-guided endonuclease activity has at least 80% amino acid sequence identity to an amino acid sequence or a corresponding member of a set of amino acid sequences selected from among Group A; and
wherein one of said RNA molecules comprises at least one cognate palindromic repeat sequence and one of said RNA molecules comprises a cognate trans-activating crRNA or one of said RNA molecules comprises a fusion at least one cognate palindromic repeat sequence and one cognate trans-activating crRNA, wherein said repeat sequence is encoded by a nucleotide sequence at a corresponding position in the list of Group B; and wherein said cognate trans-activating crRNA is encoded by a nucleotide sequence at a corresponding position in the list of Group C
| | Group A | Group B | Group C |
|---|---|---|---|
| Type II A | SEQ ID No.: 45 | SEQ ID No.: 116 | SEQ ID No.:134 |
| Type II B | SEQ ID No.: 46 | SEQ ID No.: 117 | SEQ ID No.:135 |
| Type II C | SEQ ID No.: 47 | SEQ ID No.: 118 | SEQ ID No.:136 |
| Type V B | SEQ ID No.: 92-94 | SEQ ID No.: 126 | SEQ ID No.:137 |
| Type V E | SEQ ID No.: 99-102 | SEQ ID No.: 129 | SEQ ID No.:138. |

10. The method for regulating CRISPR-Cas associated activity or CRISPR-dCas associated activity according to any one of claims 6 to 8, wherein the amino acid sequence of each member of said polypeptide complex having RNA-guided endonuclease activity has at least 80% amino acid sequence identity to a corresponding member of a set of amino acid sequences selected from among Group A;
wherein said one or more RNA molecules comprises at least one cognate palindromic repeat sequence, wherein said repeat sequence is encoded by a nucleotide sequence at a corresponding position in the list of Group B
| | Group A | Group B |
|---|---|---|
| Type I A | SEQ ID No.: 1-10 | SEQ ID No.: 109 |
| Type I B | SEQ ID No.: 12-16 | SEQ ID No.: 110 |
| Type I C | SEQ ID No.: 17-23 | SEQ ID No.: 111 |
| Type I D | SEQ ID No.: 24-31 | SEQ ID No.: 112 |
| Type I E | SEQ ID No.: 32-33 | SEQ ID No.: 113 |
| Type I F | SEQ ID No.: 34-39 | SEQ ID No.: 114 |
| Type I F* | SEQ ID No.: 40-44 | SEQ ID No.: 115 |
| Type III A | SEQ ID No.: 48-56 | SEQ ID No.: 119 |
| Type III B | SEQ ID No.: 57-64 | SEQ ID No.: 120 |
| Type III B* | SEQ ID No.: 65-73 | SEQ ID No.: 121 |
| Type III C | SEQ ID No.: 74-79 | SEQ ID No.: 122 |
| Type III D | SEQ ID No.: 80-85 | SEQ ID No.: 123 |
| Type IV | SEQ ID No.: 86-90 | SEQ ID No.: 124 |
| Type V A | SEQ ID No.: 91 | SEQ ID No.: 125 |
| Type V C | SEQ ID No.: 95-96 | SEQ ID No.: 127 |
| Type V D | SEQ ID No.: 97-98 | SEQ ID No.: 128 |
| Type VI A | SEQ ID No.: 103 | SEQ ID No.: 130 |
| Type VI B1 | SEQ ID No.: 104-105 | SEQ ID No.: 131 |
| Type VI B2 | SEQ ID No.: 106-107 | SEQ ID No.: 132 |
| Type VI C | SEQ ID No.: 108 | SEQ ID No.: 133. |

11. A compound selected from the group consisting of:
• *N*-(5-Methylisoxazol-3-yl)-3'-(4-methylpiperidine-1-carbonyl)-[1,1'-biphenyl]-4-sulfonamide,
• *N*-(Isoxazol-3-yl)-3'-(4-methylpiperidine-1-carbonyl)-[1,1'-biphenyl]-4-sulfonamide,
• 3'-(4-Methylpiperidine-1-carbonyl)-[1,1'-biphenyl]-4-sulfonamide,
• *N*-(5-Methylisoxazol-3-yl)-3'-(piperidine-1-carbonyl)-[1,1'-biphenyl]-4-sulfonamide,
• 4'-(*N*-(5-Methylisoxazol-3-yl)sulfamoyl)-[1,1'-biphenyl]-3-carboxamide,
• *N*-(Isoxazol-3-yl)-[1,1'-biphenyl]-4-sulfonamide,
• *N*-(5-Methylisoxazol-3-yl)-[1,1'-biphenyl]-3-sulfonamide,
• 1-([1,1'-biphenyl]-3-ylsulfonyl)-4-methylpiperidine,
• *N*-(5-Methylisoxazol-3-yl)-[1,1'-biphenyl]-3-carboxamide and
• *N*-([1,1'-Biphenyl]-3-ylmethyl)-5-methylisoxazol-3-amine.

12. A compound for use as a medicament, wherein said compound is for use in regulating CRISPR-Cas associated activity and/or CRISPR-dCas associated activity in a somatic mammalian cell, said compound having the structure of formula I: 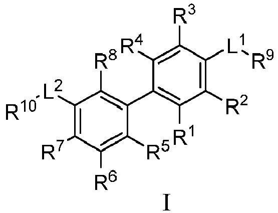 wherein
**R¹, R², R³, R⁴, R⁵, R⁶, R⁷,** and **R⁸** are each independently selected from R¹¹;
**R⁹** is R¹¹ or R¹²;
**R¹⁰** is selected from among: R¹¹, R¹², and -NR¹³R¹⁴;
**L¹** is selected from among: absent, -S(=O)₂N(R¹⁵)-, -C(=O)-, -C(=O)N(R¹⁶)-,-CH₂N(R¹⁷)-, -N(R¹⁷)CH₂-, -CH₂O-, -OCH₂-, C₁₋₃ alkylene,
C₂₋₃ alkenylene having one double bond, and
C₂₋₃ alkynylene having one triple bond;
**L²** is selected from among: absent, -C(=O)-, -S(=O)₂-, and e₁₋₃ alkylene;
**R¹¹** is selected from among: H, halo, -NO₂, -CN, -OR¹⁸, -NR¹⁹R²⁰, -C(=O)OR²¹, -C(=O)N R²²R²³, Ph, -OPh,
C₁₋₈ alkyl optionally substituted with one to three halo and/or -OH groups,
C₃₋₈ cycloalkyl optionally substituted with one to three halo and/or -OH groups,
4-8 membered heterocycloalkyl comprising one to three heteroatoms independently selected from among N, O, and S,
5-6 membered heteroaryl comprising one to three heteroatoms independently selected from among N, O, and S;
**R¹²** is selected from among:
- C₁₋₈ alkyl optionally substituted with one to three groups independently selected from among:
halo, -OR²⁴, -C(=O)OR²⁵, -C(=O)NR²⁶R²⁷, C₃₋₈ cycloalkyl, C₂₋₄ alkenylene having one double bond, C₂₋₄ alkynylene having one triple bond,
4-6 membered heterocycloalkyl comprising one to three heteroatoms independently selected from among N, O, and S, C₆₋₁₀ aryl optionally substituted with one or more independently selected R¹¹ groups, and
5-6 membered heteroaryl comprising one to four heteroatoms independently selected from N, O, and S,
- C₃₋₈ cycloalkyl, optionally substituted with one or more independently selected R¹¹ groups,
- C₄₋₈ cycloalkenyl comprising one double bond, optionally substituted with one or more independently selected R¹¹ groups,
- 4-8 membered heterocycloalkyl comprising one to three heteroatoms independently selected from N, O, and S, optionally substituted with one or more independently selected R¹¹ groups,
- C₆₋₁₀ aryl optionally substituted with one or more independently selected R¹¹ groups, and
- 5-10 membered heteroaryl comprising one to three heteroatoms independently selected from N, O, and S, optionally substituted with one or more independently selected R¹¹ groups;
**R¹³** and **R¹⁴** are each independently selected from H and R¹²;
**R¹⁵, R¹⁶**, **R¹⁷, R¹⁸, R¹⁹, R²⁰, R²¹, R²², R²³, R²⁴, R²⁵, R²⁶,** and **R²⁷** are each independently selected from H and C₁₋₈ alkyl optionally substituted with one or more halo and/or -OH groups.

13. The compound for use as a medicament according to claim 12, said compound having the structure of formula II: 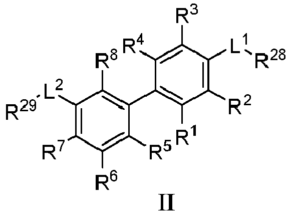 wherein
**R¹, R², R³, R⁴, R⁵, R⁶, R⁷,** and **R⁸** are as defined in claim 1,
**R²⁸** is R¹¹ or 5-10 membered heteroaryl comprising one to three heteroatoms independently selected from N, O, and S, optionally substituted with one or more independently selected R¹¹ groups;
**R¹¹** as defined in claim 1;
**R²⁹** is **R¹¹** or -NR³⁰R³¹;
**L¹** and **L²** as defined in claim 1;
**R³⁰** and **R³¹** are each independently selected from H and R¹²;
**R¹²** as defined in claim 1.

14. The compound for use as a medicament according to claim 12 or 13, said compound having the structure of formula III: 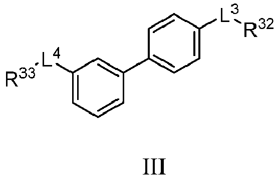 wherein
**R³²** is selected from among: H, 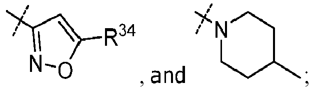
**R³³** is selected from among: H, -NR³⁵R³⁶, and 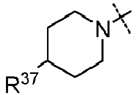
**L³** is selected from among: absent, -S(=O)₂N(H)-, and
-C(=O)-;
**L⁴** is selected from among: absent, -C(=O)-, -S(=O)₂-, and
-CH₂-;
**R³⁴** is H or Me;
**R³⁵** and **R³⁶** are independently selected from among: H,
C₁₋₄ alkyl, and 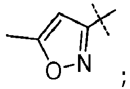
**R³⁷** is H or Me;
while excluding compounds having formula III wherein
**R³²** is H, **R³³** is H, **L³** is absent or -S(=O)₂N(H)-, and **L⁴** is absent.

15. A pharmaceutical kit comprising a compound according to any one of claims 11 to 14 in combination with a RNA guided endonuclease-guide RNA complex expression system or a RNA guided DNA binding-guide RNA complex expression system for use as a medicament,
wherein said expression system comprises:
- a first gene encoding a polypeptide or a first gene cluster encoding a polypeptide complex, said polypeptide or polypeptide complex having RNA-guided endonuclease activity, wherein said gene or gene cluster is operably linked to a promoter; and
- a nucleic acid molecule operably linked to a promoter, said nucleic acid molecule encoding one or more RNA molecules capable of guiding said polypeptide or polypeptide complex having RNA-guided endonuclease activity to a target genomic sequence; and
wherein said compound is for use in regulating CRISPR-Cas associated activity and/or CRISPR-dCas associated activity in a somatic mammalian cell.


SEQUENCE LISTING

<110>  Danmarks Tekniske Universitet

<120>  Small molecule Inhibitors of CRISPR-Cas associated  activity

<130>  P2449EP00

<160>  168

<170>  PatentIn version 3.5

<210>  1
<211>  116
<212>  PRT
<213>  Archaeoglobus fulgidus


<220>
<221>  PEPTIDE
<222>  (1)..(116)
<223>  type I-A CRISPR-associated protein Csa5: WP_010879363.1

<400>  1

Met Ser Ser Thr Met Ser Phe Asp Asn Leu Ala Lys Val Leu Ala Val
1               5                   10                  15


Leu Val Ala Glu Gln Gly Ser Tyr Thr Tyr Val Asp Lys Leu Gly Tyr
            20                  25                  30


Val Pro Ser Lys Asp Leu Ala Val Phe Tyr Leu Lys Glu Ala Leu Arg
        35                  40                  45


Asp Leu His Ser Ile Gln Gln Lys Glu Lys Phe Glu Asn Glu Lys Ala
    50                  55                  60


Arg Glu Leu Val Gly Lys Ile Asp Tyr Glu Arg Val Glu Lys Glu Leu
65                  70                  75                  80


Glu Asp Ile Ala Lys Thr Asp Glu Arg Lys Glu Leu Arg Glu Lys Thr
                85                  90                  95


Ser Leu Ile Ala Ala Lys Ala Leu Ala Leu Ser Ala Lys Leu Gly Gly
            100                 105                 110


Gly Ser Gly Glu
        115


<210>  2
<211>  319
<212>  PRT
<213>  Archaeoglobus fulgidus

<220>
<221>  PEPTIDE
<222>  (1)..(319)
<223>  type I-A CRISPR-associated protein Cas7/Csa2: WP_010879364.1

<400>  2

Met Val Val Val Ser Asp Val Phe Val Ser Val Arg Gly Arg Val Met
1               5                   10                  15

Leu Asn Val Glu Ala Met Asn Met Thr Glu Ser Val Gly Asn Tyr Val
            20                  25                  30

Lys His Arg Arg Val Pro Val Val Leu Pro Asp Ala Lys Tyr Thr Thr
        35                  40                  45

Tyr Phe Val Pro Ala Ile Ser Gly Glu Ser Ile Ala His Gly Phe Gln
    50                  55                  60

Glu Val Leu Ala Glu Val Gly Lys Lys Asn Gly Leu Lys Val Cys Lys
65                  70                  75                  80

Leu Cys Glu Lys Gly Ile Phe Leu Lys Ser Thr Asn Glu Asn Val Phe
                85                  90                  95

Lys Glu Ser Phe Ser Ser Asp Pro Pro Lys Asp Asp Phe Glu Phe Glu
            100                 105                 110

Lys Thr Val Ile Glu Asn Cys Ile Val Glu Asp Val Gly Gly Phe Leu
        115                 120                 125

Tyr Ala Pro Arg Ala Gly Gly Asn Val Lys Arg Thr Ser Asn Phe Tyr
    130                 135                 140

Thr Gly Tyr Met Ile Pro Val Arg Glu Ser Ile Glu Gly Ala Val Ile
145                 150                 155                 160

Glu Pro Gln Leu His Ser Arg Tyr Ala Leu Gly Thr Pro Phe Val Glu
                165                 170                 175

Gly Gly Gln Gly Gln Met Ile Tyr Tyr Val Glu Leu Ser Ser Ala Val
            180                 185                 190

Tyr Thr Phe Ser Phe Asp Leu Asp Thr Arg Tyr Ile Gly Arg Thr Thr
        195                 200                 205

Phe Ser Tyr Glu Lys Ala Gly Thr Glu Val Gly Lys Arg Ser Glu Arg
    210                 215                 220

Ile Asn Ala Ala Leu Glu Ala Leu Lys Lys Phe Leu Ile Glu Phe Ala
225                 230                 235                 240

Phe Gly Ala Lys Lys Thr Arg Phe Leu Pro Val Met Glu Trp Asp Ser
                245                 250                 255

Leu Val Val Ala Val Ser Asp Asp Val Trp Thr Val Pro Ser Pro Tyr
            260                 265                 270

Thr Ala Gly Tyr Ile Asp Asn Ala Arg Lys Lys Lys Glu Lys Val Asn
        275                 280                 285

Phe Asn Thr Lys Leu Phe Val Tyr Pro Glu Gly Gly Ser Phe Glu Glu
    290                 295                 300

Val Val Val Glu Ala Ile Glu Glu Ala Lys Glu Arg Ala Gly Lys
305                 310                 315

<210>  3
<211>  266
<212>  PRT
<213>  Archaeoglobus fulgidus

<220>
<221>  PEPTIDE
<222>  (1)..(266)
<223>  type I-A CRISPR-associated protein Cas5: WP_010879365.1

<400>  3

Met Ile Gly Tyr Leu Val Asp Val Glu Phe Val Trp Gly Phe Gln Ala
1               5                   10                  15

Arg Ile Ala Gly Leu Ser Lys Thr Ser Pro Ser Phe Tyr Tyr Pro Pro
            20                  25                  30

Pro Thr Thr Phe Leu Gly Ala Val Ala Glu Ala Ile Ala Lys Asp Lys
        35                  40                  45

Gly Ile Gly Glu Gln Arg Gly Lys Glu Ile Ile Ser Glu Leu Gly Glu
    50                  55                  60

Asn Leu Leu Ala Ile Gly Trp Lys Ala Leu Asn Cys Thr Pro Leu Arg
65                  70                  75                  80

Tyr Ser Asp Ile Asn Arg Ile Leu Ala Val Lys Ile Thr Ser Gly Val
                85                  90                  95

Leu Tyr Pro Asn Pro Gln Asp Leu Ala Lys Ser Phe Asp Ser Pro Ala
            100                 105                 110

Arg Gly Lys Thr Ile Leu Ser Ser Leu Asn Asp Glu Ala Pro Lys Ile
        115                 120                 125

Arg Trp Phe Leu Val Phe Lys Glu Glu Ala Val Glu Glu Lys Ile Leu
    130                 135                 140

Trp Lys Ile His Arg Ile Gly Ser Lys Glu Ser Arg Val Ala Val Val
145                 150                 155                 160

Asp Val Lys Lys Val Lys Val Thr Gln Lys Asp Gly Leu Ile Ser Thr
                165                 170                 175

Asp Tyr Ser Phe Pro Ala Glu Asp Gly Val Glu Leu Arg Gly Ile Leu
            180                 185                 190

Ser Gln Arg Trp Glu Phe Glu Val Tyr Leu Asn Pro Phe Glu Val Lys
        195                 200                 205

Tyr Ser Glu Lys Glu Asn Pro Leu Met Ser Tyr Ile Ser Gly Lys Lys
    210                 215                 220

Ala Val Leu Tyr Arg Ile Pro Ile Met Thr Ser Ile Phe Ser Thr Pro
225                 230                 235                 240

Glu Cys Leu Val Glu Val Gly Gly Asp Phe Lys Ala Tyr Glu Ala Gly
                245                 250                 255

Gly Glu Val Val Ile Gly Arg Cys Ser Lys
            260                 265

<210> 4
<211> 390
<212> PRT
<213> Archaeoglobus fulgidus

<220>
<221> PROPEP
<222> (1)..(390)
<223> hypothetical protein; WP_010879366.1

<400> 4

Met Leu Lys Val Lys Leu Ser Leu Pro Pro Asp Ser Thr Phe Leu Ser
1               5                   10                  15

Gln Leu Ile Tyr Glu Ser Leu Leu Tyr Leu Ser Gly Lys Lys Leu Ala
            20                  25                  30

Lys Phe Glu Asn Gly Glu Leu Leu Ile Glu Ser Ile Ser Lys Ala Leu
        35                  40                  45

Glu Thr Phe Asp Asp Glu Arg Val Gly Glu Ile Arg Ile Val Met Ser
    50                  55                  60

Gly Asn Asp Asn Ile Asn Ala Lys Ile Phe Glu Thr Phe Gly Ile Gly
65                  70                  75                  80

Gly Val Lys Ser Arg Lys Thr Tyr Tyr Asp Leu Ile Ala Leu Leu Lys
                85                  90                  95

Glu His Arg Asp Lys Ile Ser Thr Lys Asp Glu Ser Glu Ile His Leu
            100                 105                 110

Asn Ile Lys Gly Lys Asp Val Phe Met Asp Ile Asn Ser Lys Ser Asp
        115                 120                 125

Gly Ile Ala Ala Pro Gln Leu Leu Lys Val Asp Arg Tyr Thr Gly Phe
    130                 135                 140

Ser Ser Leu Asp Thr Pro Tyr Thr Ser Gln Gln Leu Thr Phe Tyr Leu
145                 150                 155                 160

Ser Lys Glu Val Ala Leu Leu Ala Leu Leu Gly Ile Tyr Ser Ser Phe
                165                 170                 175

Val Thr Asn Val Arg Gln Gln Gln Gln Ser Tyr Tyr Tyr Phe Leu Phe
            180                 185                 190

Phe Ser Pro Glu Glu Val Ala Gly Leu Leu Asn Ser Pro Ser Leu Ala
        195                 200                 205

Glu Lys Phe Phe Leu Val Lys Glu Ser Val Arg Glu Ser Leu Arg Glu
    210                 215                 220

Leu Leu Arg Arg Thr Thr Phe Asn Glu Leu Leu Leu Ile Glu Thr Thr
225                 230                 235                 240

Leu Asn Leu Glu Leu Gln Arg Leu Met Glu Thr Glu Asn Leu Asp Lys
                245                 250                 255

Ile Ser Leu Thr Leu Phe Lys Ile Ala His Glu Gly Gln Thr Tyr Lys
            260                 265                 270

Ile Tyr Glu Gln Ile Pro Ile Thr Val Tyr Arg Asn Pro Ala Phe Tyr
        275                 280                 285

Glu Thr Ala Lys Lys Tyr Phe Arg Asp Pro Leu Lys Phe Ser Glu Lys
    290                 295                 300

Leu Arg Lys Ala Ile Thr Gln Gly Val Ile Pro Ser Ala Leu Ala Ile
305                 310                 315                 320

Leu Ser Thr Lys Asn Lys Tyr Ser Glu Ala Asp Asn Ile Leu Lys Ala
                325                 330                 335

Ile Gln Met Leu Tyr Arg Phe Val Val Leu Gly Asp Leu Gln Gly Trp
            340                 345                 350

Phe Gly Phe Leu Arg Glu Leu Asn Asn Ala His Ser Lys Leu Lys Asn
        355                 360                 365

Ser Ser Asp Gly Arg Glu Lys Lys Arg Ala Gly Gln Tyr Leu Asn Ile
    370                 375                 380

Val Lys Glu Val Ala Trp
385                 390

<210>  5
<211>  546
<212>  PRT
<213>  Archaeoglobus fulgidus

<220>
<221>  PEPTIDE
<222>  (1)..(546)
<223>  CRISPR-associated helicase Cas3': WP_010879367.1

<400>  5

Met Val Thr Leu Ser Glu Ile Tyr Gly Glu Ile Val Glu Lys Trp Phe
1               5                   10                  15

Gly Gly Arg Asp Glu Arg Pro Phe Ile Ala Asn Ala Ile Arg Glu Ile
            20                  25                  30

Glu Lys Asn Trp Asp Arg Asn Ile Phe Val Ile Glu Ala Pro Thr Gly
        35                  40                  45

Tyr Gly Lys Ser Thr Ile Ser Ala Thr Ile Ala Ala Tyr Ser Leu Gln
    50                  55                  60

Glu Glu Leu Lys Ser Ile Ile Ala Phe Pro Leu Arg Thr Leu Leu Glu
65                  70                  75                  80

Asp Gln Tyr Asn Lys Phe Lys Gly Leu Phe Gly Asn Lys Leu Gly Lys

85                  90                  95

Arg Tyr Met His Asn Pro Asp Ser Pro Tyr Leu Ile Lys Pro Val Thr
            100                 105                 110

Leu Thr Thr Ile Asp Thr Leu Ala Leu Thr Leu Phe Gly Ile Pro Pro
        115                 120                 125

Glu Asp Leu Asp Lys Ala Val Lys Tyr Trp Asp Gly Thr Ser Ser Gly
    130                 135                 140

Ser Leu Gly His Tyr Leu Phe Ser Gln Ala Ser Val Ala Leu Ser Asn
145                 150                 155                 160

Ile Val Leu Asp Glu Val His Leu Leu Ala Asp Ser Thr Lys Ser Leu
                165                 170                 175

Asn Phe Leu Phe Ala Leu Met His Ala Ala Ile Asp Asn Asp Gln Lys
            180                 185                 190

Leu Val Leu Met Ser Ala Thr Ile Pro Glu Ala Leu Lys Lys Arg Ile
        195                 200                 205

Lys Glu Phe Asn Pro Asp Val Cys Met Val Glu Phe Asn Glu Ser Phe
    210                 215                 220

Asp Arg Lys Phe Val Glu Glu Arg Arg Lys Lys Lys Tyr Arg Ile Asn
225                 230                 235                 240

Leu Ile Ala Leu Ala Ser Val Glu Lys Phe Gly Ser Leu Glu Arg Ile
                245                 250                 255

Leu Leu Glu Gly Ser Lys Lys Phe Ser Lys Ile Thr Val Val Phe Asn
            260                 265                 270

Thr Ile Gly Asp Ala Val Tyr Phe Tyr Asn Asn Ile Lys Glu Lys Ile
        275                 280                 285

Pro Glu Phe Lys Lys Leu Leu Leu His Ser Arg Phe Ser Glu Met Asp
    290                 295                 300

Arg Glu Lys Lys Ala Glu Leu Leu Ala Glu Met Lys Lys Ser Glu Lys
305                 310                 315                 320

Phe Val Ile Val Thr Thr Gln Val Ile Glu Ala Gly Val Asp Ile Ser
                325                 330                 335

Ser Asn Leu Phe Ile Thr Glu Ile Ala Pro Ala Ser Ser Leu Ile Gln
                340                 345                 350

Arg Leu Gly Arg Phe Leu Arg Tyr Gly Glu Arg Glu Gly Glu Val Tyr
            355                 360                 365

Ile Trp Tyr Glu Val Asp Asp Arg Gly Glu Leu Ala Arg Trp Lys Asp
    370                 375                 380

Asp Arg Tyr Lys Val Tyr Asp Tyr Ser Leu Thr Ala Leu Thr Leu Glu
385                 390                 395                 400

Lys Leu Ala Glu Met Lys Asn Phe Ser Val His Val Asp Tyr Asp Arg
                405                 410                 415

Leu Leu Asp Val Tyr Gly Glu Phe Glu Val Asp Glu Arg Gly Val Asn
                420                 425                 430

Asp Leu Arg Arg Ile Leu Met Asn Phe Glu Asn Ile Asn Ser Ala Val
            435                 440                 445

Glu Lys Phe Phe Glu Leu Glu Gly Ser Phe Val Arg Asp Ser Phe Ile
    450                 455                 460

Ile Pro Val Val Pro Tyr Ser Val Ile Glu Glu Lys Arg Arg Ser Ser
465                 470                 475                 480

Lys Val Pro Val Ser Phe Glu Thr Phe Lys Asn Phe Lys Val Tyr Gly
                485                 490                 495

Cys Ile Val Ser Asp Glu Phe Val Ser Ala Glu Lys Leu Lys Cys Asn
                500                 505                 510

Leu Ser Ser Pro Lys Ala Leu Leu Arg Asp Met Ile Arg Lys Ser Ile
            515                 520                 525

Ala Ala Phe Val Val Asp Gly Asp Tyr Asp Ser Glu Met Gly Leu Val
    530                 535                 540

Lys Arg
545

<210>  6
<211>  241
<212>  PRT
<213>  Archaeoglobus fulgidus

<220>

<221>  PROPEP
<222>  (1)..(241)
<223>  CRISPR-associated endonuclease Cas3':WP_010879368.1

<400>  6

Met Arg Ile Tyr Ser Phe Tyr Asn Glu Asn Ala Arg Arg Glu Thr Leu
1               5                   10                  15

Glu Glu His Val Ala Thr Ala Leu Lys Leu Leu Asp Ser Ala Glu Asn
            20                  25                  30

Ser Lys Leu Val Lys Tyr Gly Lys Lys Leu Ser Pro Glu Phe Glu Arg
        35                  40                  45

Ala Val Lys Leu Ala Ile Ile Phe His Asp Thr Gly Lys Val Phe Phe
    50                  55                  60

Gln Ile Lys Lys Gly Tyr Gly Tyr Met Ser Phe Arg Gly His Glu Tyr
65                  70                  75                  80

Phe Ser Thr Tyr Ile Phe Asp Val Phe Ala Arg Arg Leu Ile Lys Ser
                85                  90                  95

Glu Ile Glu Asn Tyr Glu Ala Leu Ser Glu Leu Arg Lys Ile Cys Asn
            100                 105                 110

Phe Ser Ile Phe Phe His His His Ala Met Asn Ile Arg Met Arg Glu
        115                 120                 125

Pro Glu Ala Gly Ser Asn Ile Lys Ala Gly Leu Lys Leu Leu Asp Val
    130                 135                 140

Leu Tyr Ser Glu Val Lys Arg Phe Leu Glu Asp Lys Glu Lys Lys Ala
145                 150                 155                 160

Leu Lys Ala Ala Leu Glu Glu Val Lys Glu Lys Gly Thr Ala Asn Leu
                165                 170                 175

Thr Gly Tyr Val Lys Gly Glu Ile Val Arg Glu Val Trp Arg Asn Ala
            180                 185                 190

Met Asp Ser Ser Lys Leu Lys Arg Leu Cys Tyr Leu Asn Leu Leu Val
        195                 200                 205

Leu Ile Ala Ala Asp Tyr Ser Ser Ala Ala Leu Asn Arg Gly Gly Asn
    210                 215                 220

Lys Thr Pro Phe Gly Arg Val Val Glu Glu Phe Ser Glu Phe Tyr Leu

225                 230                 235                 240

Lys

<210>  7
<211>  94
<212>  PRT
<213>  Archaeoglobus fulgidus

<220>
<221>  PEPTIDE
<222>  (1)..(94)
<223>  CRISPR-associated endonuclease Cas2: WP_010879369.1

<400>  7

Met Leu His Leu Val Val Tyr Asp Ile Ser Asp Asp Gly Ser Arg Ala
1               5                   10                  15

Arg Leu Ala Lys Leu Leu Glu Lys Phe Gly Leu Gln Arg Val Gln Tyr
            20                  25                  30

Ser Ala Phe Arg Gly Glu Leu Asn Pro Asn Asp Arg Glu Val Leu Ala
        35                  40                  45

Arg Gln Val Gly Lys Phe Val Arg Asp Asp Arg Asp Cys Ile Phe Ile
    50                  55                  60

Ile Pro Leu Cys Gln Arg Cys Ser Ser Thr Ala Ile Val Ile Ser Asn
65                  70                  75                  80

Thr Gly Val Glu Leu Val Lys Glu Lys Gly Val Glu Phe Val
                85                  90

<210>  8
<211>  189
<212>  PRT
<213>  Archaeoglobus fulgidus

<220>
<221>  PEPTIDE
<222>  (1)..(189)
<223>  CRISPR-associated protein Cas4: WP_010879370.1

<400>  8

Met Arg Gly Leu Phe Arg Ser Gly Lys Met Asp Val Ser Asp Ile Val
1               5                   10                  15

Gln Tyr Leu Tyr Cys Pro Arg Lys Leu Tyr Phe Leu Lys Ile Ala Gly
            20                  25                  30

Ile Arg Ile Ser Lys Pro Lys Met Glu Glu Gly Lys Ala Ala Gln Glu
        35                  40                  45

Glu Ala Arg Gly Lys Leu Arg Ser Phe Ala Arg Lys Leu Lys Gly Glu
    50                  55                  60

Leu Leu Thr Ser Val Tyr Leu Glu Ser Lys Arg Tyr Gly Leu Lys Gly
65                  70                  75                  80

Val Leu Asp Ala Leu Val Ile Val Asp Gly Gly Ala Phe Pro Val Asp
                85                  90                  95

Val Lys Leu Thr Arg Phe Gln Ser Ile Thr Tyr Ala Trp Lys Met Gln
            100                 105                 110

Leu Thr Ala Tyr Ala Leu Leu Val Glu Glu Asn Tyr Ala Val Glu Val
        115                 120                 125

Arg Lys Gly Tyr Leu Phe Leu Leu Asn Gln Gly Lys Phe Val Glu Val
    130                 135                 140

Ser Ile Ser Pro Asp Asp Arg Lys Ala Leu Leu Arg Ile Ile Lys Glu
145                 150                 155                 160

Ile Glu Arg Leu Ile Glu Glu Glu Lys Tyr Pro Arg Thr Ser Lys Ser
                165                 170                 175

Lys Lys Cys Gly Tyr Cys Glu Val Arg Glu Phe Cys Val
            180                 185

<210>  9
<211>  345
<212>  PRT
<213>  Archaeoglobus fulgidus

<220>
<221>  PEPTIDE
<222>  (1)..(345)
<223>  CRISPR-associated protein Cas1: WP_010879371.1

<400>  9

Met Arg Leu Val Val Asp Gly Phe Gly Lys Tyr Leu Gly Ile Glu Asn
1               5                   10                  15

Gly Leu Ile Val Val Lys Glu Lys Gly Lys Ala Leu Arg Lys Val Arg
            20                  25                  30

Pro Glu Asp Leu Lys Gln Val Leu Ile Ile Gly Lys Ala Ala Ile Ser
        35                  40                  45

Ser Asp Ala Ile Lys Leu Leu Leu Lys Asn Arg Val Asp Val Val Phe
    50                  55                  60

Leu Asp Phe Asn Gly Glu Ile Leu Gly Arg Leu Ser His Pro Leu Ile
65                  70                  75                  80

Gly Thr Ala Lys Thr Arg Arg Glu Gln Tyr Leu Ala Tyr Gly Asp Lys
                85                  90                  95

Arg Gly Val His Leu Ala Lys Glu Phe Ile Lys Ala Lys Met Ala Asn
            100                 105                 110

Gln Met Ala Ile Leu Thr Asn Leu Ala Lys Ala Arg Lys Asp Ser Asn
        115                 120                 125

Pro Glu Val Ala Glu Ser Leu Leu Lys Ala Lys Lys Glu Ile Asp Ala
    130                 135                 140

Cys Leu Asn Glu Leu Asp Gly Val Glu Ala Glu Met Ile Asp Lys Val
145                 150                 155                 160

Arg Glu Arg Leu Leu Gly Ile Glu Gly Lys Ala Ser Lys His Tyr Trp
                165                 170                 175

Asp Ala Ile Ser Leu Val Ile Pro Glu Glu Tyr Arg Phe Asn Gly Arg
            180                 185                 190

Arg Gly Ile Glu Ile Gly Ser Pro Arg Tyr Ala Lys Asp Ile Val Asn
        195                 200                 205

Ala Met Leu Asn Tyr Gly Tyr Ser Ile Leu Leu Ala Glu Cys Val Lys
    210                 215                 220

Ala Val Glu Leu Ala Gly Leu Asp Pro Tyr Ala Gly Phe Leu His Val
225                 230                 235                 240

Asp Val Ser Gly Arg Ser Ser Leu Ala Ile Asp Leu Met Glu Asn Phe
                245                 250                 255

Arg Gln Gln Val Val Asp Arg Val Val Leu Arg Leu Ile Ser Tyr Arg
            260                 265                 270

Gln Ile Lys Pro Glu Asp Cys Glu Lys Arg Asn Met Val Cys Gln Leu
        275                 280                 285

Ser Asp Asn Ala Arg Arg Leu Leu Leu Ala Ser Leu Leu Glu Arg Leu
    290                 295                 300

Asp Ser Lys Thr Gln Tyr Arg Gly Arg Asn Leu Ala Tyr Ser Ser Ile
305                 310                 315                 320

Ile Leu Leu His Ala Arg Asp Val Val Ala Phe Leu Arg Gly Glu Arg
                325                 330                 335

Arg Tyr Glu Gly Phe Val Gln Lys Trp
            340                 345

<210>  10
<211>  281
<212>  PRT
<213>  Archaeoglobus fulgidus

<220>
<221>  PEPTIDE
<222>  (1)..(281)
<223>  type I-A CRISPR-associated protein Cas4/Csa1: WP_010879372.1

<400>  10

Met Val Tyr Phe Leu Asn Glu Leu Glu His Lys Phe Leu Thr Leu Glu
1               5                   10                  15

Leu Arg Pro Arg Ile Arg Gln Asn Pro Val Pro Glu Glu Leu Arg Gly
            20                  25                  30

Trp Asn Trp His Ser Pro Pro Leu Lys Pro Tyr Tyr Asn Val Lys Leu
        35                  40                  45

Pro Met Tyr Leu Ile Cys Ser Arg Tyr Cys Pro Thr Ser Arg Asp Val
    50                  55                  60

Tyr Leu Thr Met Val Glu Lys Val Arg Gly Glu Glu Ser Phe Pro Val
65                  70                  75                  80

Ser Leu Gly Lys Ala Ile His Glu Ala Val Ala Lys Ala Ile Arg Glu
                85                  90                  95

Ala Lys Ser Lys Asn Phe Ser Ala Glu Pro Pro Asn Tyr Asp Asn Glu
            100                 105                 110

Met Ile Asn Val Ala Val Lys Ala Val Trp Glu Tyr Thr Met Ser Leu
        115                 120                 125

Cys Gln Ser Ser Tyr Leu Lys Val Arg Ser Glu Gln Pro Tyr Ala Thr

130                 135                 140

Glu Glu Gly Val Leu Leu Thr Ser Met Pro Phe Leu Val Glu His Arg
145                 150                 155                 160

Met Asp Gly Ser Leu Leu Gly Cys Ser Gly Leu Ile Ser Val Asp Cys
                165                 170                 175

Tyr Asp Tyr Leu Arg Asn Val Val Phe Asp Leu Lys Val Gly Glu Phe
            180                 185                 190

Asn Glu Glu Met Arg Leu Tyr Thr Thr Gly Tyr Ala Leu Val Leu Glu
        195                 200                 205

Ser Ile His Glu Ile Pro Val Asp Val Gly Cys Ile Ile Ser Ile Thr
    210                 215                 220

Phe Asn Gly Glu Arg Met Arg Val Ser Lys Asp Leu His Tyr Ile Asp
225                 230                 235                 240

Ala Asn Leu Arg Ser Trp Trp Ile Glu Glu Arg Asp Arg Lys Ala Glu
                245                 250                 255

Ile Val Phe Asn Glu Ala Asp Pro Gly Val Ala Lys Ser Cys Ser Ser
            260                 265                 270

Asn Cys Leu Phe Asn Ala Phe Cys Leu
        275                 280

<210>  11
<211>  172
<212>  PRT
<213>  Clostridium difficile

<220>
<221>  PEPTIDE
<222>  (1)..(172)
<223>  CRISPR-associated Cas4 family protein: YP_001089495.1

<400>  11

Met Pro Ile Asp Leu Glu Val Leu Lys Val Gln Gly Val Lys Ile Asn
1               5                   10                  15

Tyr Tyr Tyr Val Cys Lys Arg Lys Leu Trp Leu Phe Ser Lys Gly Ile
            20                  25                  30

Thr Met Glu Gln Asn Ser Ser Arg Val Leu Ser Gly Lys Ile Val His
        35                  40                  45

Glu Asp Ser Tyr Lys Arg Met Lys Asn Lys Glu Ile Leu Ile Asp Asp
    50                  55                  60

Ile Leu Lys Leu Asp Ile Ile Lys Asp Glu Tyr Val Lys Glu Val Lys
65                  70                  75                  80

Ile Ser Ser Lys Met Lys Glu Ala Asp Glu Met Gln Leu Tyr Tyr Tyr
                85                  90                  95

Leu Tyr Tyr Leu Lys Asn Ser Met Gly Ile Glu Lys Lys Gly Thr Ile
            100                 105                 110

Asn Tyr Val Lys Glu Lys Lys Gln Glu Glu Leu Ile Leu Thr Glu Glu
        115                 120                 125

Lys Glu Arg Ile Ile Glu Asn Thr Leu Ile Asn Ile Asn Glu Ile Asn
    130                 135                 140

Met Gln Leu Ser Pro Pro Lys Ile Glu Lys Lys His Tyr Cys Thr Lys
145                 150                 155                 160

Cys Ala Tyr Tyr Glu Phe Cys Phe Val Lys Glu Glu
                165                 170

<210>  12
<211>  245
<212>  PRT
<213>  Clostridium difficile

<220>
<221>  PEPTIDE
<222>  (1)..(245)
<223>  CRISPR/Cas system-associated RAMP superfamily protein Cas6:
       YP_001089500.1

<400>  12

Met Lys Met Lys Ile Glu Phe Ser Thr Gly Cys Ile Pro Ile Ser Tyr
1               5                   10                  15

Asn Ser Leu Phe Met Ser Ile Ile Lys Glu Ala Ile Arg Lys Ser Asn
            20                  25                  30

Glu Asp Tyr Tyr Lys Asn Met Tyr Tyr Tyr Lys Glu Lys Asn Asn Lys
        35                  40                  45

Lys Thr Lys Asn Phe Thr Phe Ser Val Tyr Ile Lys Lys Tyr Ser Ile
    50                  55                  60

Glu Gly Asp Asn Phe Ile Ile Glu Asp Lys Val Ile Leu Asn Ile Ser
65                  70                  75                  80

Thr Pro Asp Leu Glu Leu Gly Phe His Ile Tyr Asn Gly Leu Met Thr
                85                  90                  95

Ser Lys Lys Cys Leu Tyr Lys Asp Tyr Glu Leu Thr Arg Ile Arg Ile
            100                 105                 110

Asp Leu Ser Arg Glu Lys Lys Val Thr Glu Glu Arg Val Leu Phe Asn
        115                 120                 125

Ala Leu Ser Pro Ile Cys Val Lys Ser Lys Glu Gly Lys Phe Leu Glu
    130                 135                 140

Ile Thr Asp Asp Arg Tyr Ile Glu Glu Leu Asn Tyr Ile Thr Asn Glu
145                 150                 155                 160

Val Val Lys Asn Tyr Arg Gly Asn Gly Leu Lys Arg Lys Leu Glu Phe
                165                 170                 175

Glu Asn Val Gly Phe Lys Lys Val Val Val Lys Glu Ser Leu Arg Glu
            180                 185                 190

Phe Lys Lys Ile Thr Gly Lys Glu Tyr Gln Tyr Ile Asn Gly Tyr Lys
        195                 200                 205

Gly Lys Phe Ile Leu Lys Gly Asp Ile Asp Asp Leu Asn Leu Ile Tyr
    210                 215                 220

Asn Leu Gly Ile Gly Phe Arg Arg Ala Gln Gly Phe Gly Asp Val Asp
225                 230                 235                 240

Ile Leu Glu Trp Arg
                245

<210>  13
<211>  625
<212>  PRT
<213>  Clostridium difficile

<220>
<221>  PEPTIDE
<222>  (1)..(625)
<223>  CRISPR-associated protein YP_001089499.1

<400>  13

Met Lys Leu Arg Val Tyr Arg Gly Asp Trp Phe Phe Asn Met Gly Ile
1               5                   10                  15

Val Gly Phe Leu Asn Ile Ile Lys Lys Ala Asp Lys Gln Ala Glu Ile
        20                  25                  30

Phe Ile Met Glu Asp Tyr Ile Glu Phe Asp Ser Leu Phe Leu Glu Asn
    35                  40                  45

Phe His Glu Tyr Tyr Phe Asn Tyr Phe Met Asp Glu Tyr Asp Val Ser
    50                  55                  60

Lys Arg Ile Lys Lys Asn Ile Asp Tyr Ser Ile Asn Phe Ile Lys Ser
65                  70                  75                  80

Lys Pro Asp Arg Ile Lys Asp Gly Ile Lys Lys Ile Lys Asp Ser Val
                85                  90                  95

Lys Gln Gln Asn Asp Lys Ile Lys Lys Phe Asp Glu Glu Lys Phe Asn
            100                 105                 110

Leu Ile Lys Glu Lys Leu Asp Ser Met Ser Lys Ile Lys Ser Tyr Asp
        115                 120                 125

Glu Phe Asp Ser Leu Glu Ala Leu Val Asp Glu Thr Ile Asp Ile Phe
    130                 135                 140

Lys Ile Lys Ser Ile Asn Asp Arg Leu Thr Ala Asn Leu Tyr Lys Tyr
145                 150                 155                 160

Val Val Gln Asp Asn Tyr Phe Gly Gln Val Ser Phe Phe Asn Val Ala
                165                 170                 175

Lys Ala Lys Leu Asp Leu Asp Gly Leu Lys Gln Val Met Phe Asn Asp
            180                 185                 190

Tyr Leu Arg Gln Ile Ile Tyr Phe Gly Glu Leu Glu Asp Leu Leu Glu
        195                 200                 205

Glu Asn Asp Tyr Asp Lys Leu Lys Asn Tyr Leu Asn Asp Arg Leu Asn
    210                 215                 220

Ser Ile Asp Lys Asp Ile Asn Glu Lys Lys Val Ser Lys Ser Ser Ile
225                 230                 235                 240

Asn Thr Ile Glu Lys Ile Met Lys Glu Ile Asn Lys Asn Phe Ile Lys
                245                 250                 255

Lys Asn Lys Ser Ile Glu Asp Ile Lys Glu Tyr Met Asp Ser Leu Glu

            260                 265                 270

Val Cys Glu Met Cys Gly Leu Tyr Lys Gly Ile Leu Asp Glu Tyr Ser
        275                 280                 285

Glu Ser Asn Phe Ala Pro Leu Gly Val Ser Thr Asn Asn Ala Arg Asn
    290                 295                 300

Met Phe Trp Asn Gln Ala Tyr Val Pro Ser Ile Cys Asp Ile Cys Lys
305                 310                 315                 320

Leu Ile Leu Phe Cys Thr Pro Ala Gly Ala Thr Tyr Thr Arg Lys Asn
                325                 330                 335

Tyr Leu Ile Asn Glu Glu Asn Glu Phe Tyr Leu Phe Val Asn Met Asp
            340                 345                 350

Thr Cys Ile Asn Glu Leu Phe Glu Arg Asn Asn Ser Leu Lys Val Gln
        355                 360                 365

Lys Ser Glu Ser Ser Asp Ser Lys Asp Glu Asn Pro Phe Asn Gln Leu
    370                 375                 380

Ile Lys Ser Ile Val Glu Glu Asn Thr Leu Lys Ser Glu Trp Gln Leu
385                 390                 395                 400

Arg Asn Ile Leu Phe Val Glu Phe Lys Ala Ser Ile Asp Ser Lys Lys
                405                 410                 415

Cys Lys Ile Asn Tyr Phe Asn Ile Pro Thr Tyr Leu Ala Lys Phe Phe
            420                 425                 430

Thr Asp Lys Tyr Ala Asn Lys Lys Ile Gln Ser Ile Tyr Asp Tyr Lys
        435                 440                 445

Leu Lys Ser Asn Val Leu Asp Leu Leu Leu Lys Asn Arg Asp Leu Lys
    450                 455                 460

His Ile Thr Asn Asn Ile Leu Arg Asn Lys Val Lys Asn Asp Met Glu
465                 470                 475                 480

Ser Asn Asn Lys Ser Asn Ile Ser Gly Ile Asp Cys Phe Arg Val Val
                485                 490                 495

Gln Leu Arg Val Leu Ile Asn Ser Tyr Lys Lys Gly Val Tyr Lys Met
            500                 505                 510

Asp Ser Lys Asn Leu Glu Lys Asn Asp Glu Lys Leu Lys Ile Ile Tyr
        515                 520                 525

Tyr Leu Gly Cys Asp Ile His Asp Tyr Phe Val Asn Lys Asn Ser Lys
    530                 535                 540

Asn Lys Ile Asp Gly Val Ser Tyr Lys Leu Leu Asn Ser Val Lys Val
545                 550                 555                 560

Gly Asn Lys Ser Asp Phe Met Asp Thr Ile Ile Arg Val Phe Met Ser
                565                 570                 575

Ala Glu Lys Gln Ile Pro Ala Phe Ile Leu Asp Ile Glu Ile Glu Lys
            580                 585                 590

Asp Leu Asp Phe Glu Ser Ile Gly His Ala Phe Ile Ser Gly Leu Ile
        595                 600                 605

Ser Gly Lys Tyr Glu Gly Lys Asp Lys Leu Pro Asn Glu Lys Glu Glu
    610                 615                 620

Lys
625

<210>  14
<211>  304
<212>  PRT
<213>  Clostridium difficile

<220>
<221>  PEPTIDE
<222>  (1)..(304)
<223>  CRISPR-associated autoregulator DevR family:YP_001089498.1

<400>  14

Met Lys Arg Gly Leu Thr Phe Thr Val Ile Ala Gln Ala Gln Ser Leu
1               5                   10                  15

Asn Tyr Gly Glu Gly Ile Gly Asn Ile Ser Glu Leu Lys Lys Leu Ser
            20                  25                  30

Arg Asp Asp Gly Asn Met Tyr Thr Phe Ala Ser Arg Gln Cys Leu Arg
        35                  40                  45

Tyr Asp Ile Val Arg Leu Ala Ala Glu Leu Phe Asn Trp Asn Leu Gln
    50                  55                  60

Val Val Asp Lys Glu Lys Gly Thr Ile Gln Phe Lys Asp Asn Ile Ser
65                  70                  75                  80

Ile Arg Glu Ser Glu Glu Met Asp Leu Phe Gly Tyr Met Lys Thr Asn
                85                  90                  95

Lys Lys Asp Glu Lys Asp Lys Lys Gly Gly Ser Leu Thr Arg Glu Ala
            100                 105                 110

Thr Val Arg Leu Ser Asn Ala Ile Ser Leu Glu Pro Tyr Arg Ser Asp
        115                 120                 125

Met Asp Phe Leu Asn Asn Lys Gly Phe Ala Asp Arg Ile Gly Glu His
    130                 135                 140

Pro Asn Leu Ala Asn Ile Glu Gln His Leu Ser Tyr Tyr Thr Tyr Thr
145                 150                 155                 160

Val Thr Ile Asp Leu Ser Lys Ile Gly Lys Asp Gly Glu Ile Glu Leu
                165                 170                 175

Asp Asn Lys Glu Lys Cys Arg Arg Val Val Glu Phe Leu Glu Ile Ile
            180                 185                 190

Lys Val Leu Asn Arg Asn Ile Arg Gly Arg Gln Glu Asn Leu Ser Pro
        195                 200                 205

Leu Phe Val Val Gly Gly Val Tyr Glu Ile Ala Asn Pro Phe Phe Leu
    210                 215                 220

Gly Arg Ile Lys Leu Lys Gly Asp Lys Asn Gly Phe Lys Ile Asn Lys
225                 230                 235                 240

Gln Ala Ile Glu Glu Val Ile Gln Gly Thr Phe Leu Gly Lys Asp Leu
                245                 250                 255

Lys Glu Phe Thr Tyr Val Gly Met Val Asp Gly Val Phe Ile Asn Lys
            260                 265                 270

Glu Glu Phe Lys Gly Leu Phe Glu Asp Asn Phe Leu Ser Val Asp Lys
        275                 280                 285

Phe Phe Gly Gln Leu Val Lys Glu Val Lys Glu Tyr Tyr Gly Val Asn
    290                 295                 300

<210>  15
<211>  773
<212>  PRT
<213>  Clostridium difficile

<220>
<221>  PEPTIDE
<222>  (1)..(773)
<223>  CRISPR-associated Cas3 family helicase: YP_001089496.1

<400>  15

Met Leu Tyr Ala Lys Ser Asn Pro Val Glu Thr Leu Arg Glu His Thr
1               5                   10                  15

Asp Glu Leu Leu Lys Gln Met Asn Val Leu Arg Glu Ser Tyr Gly Lys
            20                  25                  30

Asn Ile Asn Ser Leu Asp Phe Leu Glu Glu Glu Ile Phe Trp Gln Leu
        35                  40                  45

Leu Asp Phe Val Ile Glu Phe His Asp Ile Gly Lys Ala Phe Ser Pro
    50                  55                  60

Phe Gln Glu Leu Ile Lys Ser Arg Met Asp Thr Lys Ile Asn Glu Pro
65                  70                  75                  80

Lys Ile Val Thr His Leu Glu Asn Asn Val Gly His Asn Tyr Leu Ser
                85                  90                  95

Pro Ala Phe Ile Asp Tyr Ser Tyr Ile Asp Arg Lys Lys Asn Lys Glu
            100                 105                 110

Leu Arg Ala Val Leu Asn Gln Val Ile Val Tyr His His Glu Arg Asp
        115                 120                 125

Ile Phe Ile Asp Lys Asp Phe Lys Asn Leu Ile Gln Lys Ile Leu Asp
    130                 135                 140

Glu Asp Leu Ile Asn Lys Val Tyr Glu Leu Gln His Glu Phe Lys Val
145                 150                 155                 160

Arg Tyr Pro Ile Lys Thr Glu Lys Leu Ser Lys Val Tyr Leu Gln Ser
                165                 170                 175

Val Glu Lys Arg Ile Asp Lys Asn His Lys Tyr Tyr Asn Leu Tyr Ile
            180                 185                 190

Met Leu Lys Gly Ile Leu His Arg Leu Asp His Ser Ala Ser Ala His
        195                 200                 205

Glu Val Val Glu Cys Asn Asn Val Ile Asn Ile Gly Glu Gln Thr Glu
    210                 215                 220

Asn Tyr Leu Met Lys Glu Phe Gly Ser Leu Arg Glu Ala Gln Ser Phe
225                 230                 235                 240

Ala Lys Ser Asn Arg Asn Lys Asn Ile Ile Leu Ile Ala Pro Thr Gly
                245                 250                 255

Met Gly Lys Thr Glu Thr Ala Leu Ile Trp Ile Asp Lys Asp Lys Ala
            260                 265                 270

Phe Phe Thr Leu Pro Leu Arg Val Ser Ile Asn Ala Leu Phe Asp Arg
        275                 280                 285

Ala Lys Asn Ile Val Gly Val Gly Glu Ser Asn Asp Thr Phe Leu Gly
    290                 295                 300

Leu Leu His Ser Thr Ala Ile Asp Tyr Leu Glu Glu Ser Asn Gln Glu
305                 310                 315                 320

Asn Ser Ser Glu Ile Val Asp Leu Ala Lys Leu Leu Ser Cys Lys Leu
                325                 330                 335

Thr Phe Ser Thr Ile Asp Gln Ile Phe Lys Phe Pro Phe Leu Tyr Arg
            340                 345                 350

Gly Tyr Glu Lys Val Tyr Ser Thr Leu Ala Tyr Ser Lys Val Val Ile
        355                 360                 365

Asp Glu Ile Gln Ala Tyr Ser Pro Glu Ile Ala Ala Val Leu Val Lys
    370                 375                 380

Gly Ile Glu Met Ile His Lys Ile Gly Gly Arg Phe Met Ile Met Thr
385                 390                 395                 400

Ala Thr Met Pro Thr Ile Tyr Ile Asp Glu Leu Lys Lys Arg Gly Val
                405                 410                 415

Met Asn Ser Asn Leu Ala Glu Leu Thr Cys Asn Thr Glu Lys Ile Arg
            420                 425                 430

His Cys Val Ser Ile Val Glu Asn Ser Ile Asp Glu Asn Leu Gly Lys
        435                 440                 445

Ile Ile Gln Ser Gly Met Asn Lys Lys Val Leu Val Ile Val Asn Thr
    450                 455                 460

Val Lys Ser Ala Val Glu Lys Tyr Glu Leu Ile Glu Glu Ile Val Lys
465                 470                 475                 480

Pro Lys Gly Ile Asp Val Asn Leu Asn Leu Leu His Ser Met Tyr Ile
                485                 490                 495

Gln Glu Asp Arg Ala Lys Leu Glu Lys Tyr Ile Lys Glu Phe Ala Asp
            500                 505                 510

Ser Asp Ser Asn Gly Ile Trp Ile Thr Thr Gln Leu Val Glu Ala Ser
        515                 520                 525

Leu Asp Ile Asp Phe Asp Glu Leu His Thr Glu Asn Ser Thr Leu Asp
    530                 535                 540

Ser Leu Phe Gln Arg Phe Gly Arg Cys Tyr Arg Ser Arg Glu Tyr Glu
545                 550                 555                 560

Glu Asn Ser Pro Asn Ile Phe Ile Tyr Thr Glu Lys Ala Thr Gly Ile
                565                 570                 575

Gly Ser Ile Tyr Asp Lys Asp Ile Val Glu Lys Gly Leu Glu Leu Leu
            580                 585                 590

Lys Thr Phe Ile Asn Gly Lys Glu Cys Val Lys Met Lys Glu Lys Tyr
        595                 600                 605

Lys Val Glu Met Val Lys Ile Leu Tyr Ser Lys Glu Ser Leu Glu Gly
    610                 615                 620

Thr Ala Phe Glu Lys Arg Phe Thr Ser Ala Leu Asn Ile Leu Asp Thr
625                 630                 635                 640

Ile Thr Pro Tyr Ser Leu Gly Ser Lys Asp Ala Gln Asn Ile Leu Arg
                645                 650                 655

Asn Ile Glu Gly Tyr Thr Val Ile Pro Glu Asp Ile Tyr Asn Lys Ile
            660                 665                 670

Glu Glu Thr Leu Ile Lys Asp Tyr Glu Glu Leu Gly Gln Glu Leu Ile
        675                 680                 685

Asn Ala Tyr Ser Asn Asp Asp Arg Gln Leu Ile Asn Lys Ile Lys Ser
    690                 695                 700

Lys Arg Lys Lys Ala Arg Arg Glu Ile Ile Lys Lys Thr Val Asn Leu
705                 710                 715                 720

Pro Ile Tyr Lys Ala Lys Gln Asn Val Ile Asp Ile Gln Val Lys Gly

                725                 730                 735

Leu Glu Asp Leu Lys Ile Leu Leu Tyr Lys Tyr Asp Ile Tyr Glu Asn
            740                 745                 750

Lys His Thr Lys Gln Ile Phe Gly Lys Gly Val Leu Ile Ser Asn Glu
        755                 760                 765

Ile Asp Gln Phe Ile
    770

<210> 16
<211> 233
<212> PRT
<213> Clostridium difficile

<220>
<221> PEPTIDE
<222> (1)..(233)
<223> CRISPR-associated Cas5 family protein:YP_001089497.1

<400> 16

Met Lys Val Leu Lys Leu Asp Leu Phe Gln Glu Thr Ala Cys Tyr Lys
1               5                   10                  15

Lys Pro Phe Ala Met Lys Ile Thr Glu Thr Tyr Pro Leu Pro Pro Tyr
            20                  25                  30

Ser Thr Ile Asn Gly Leu Ile His Lys Ile Leu Asn Ala Thr Glu Tyr
        35                  40                  45

Ile Pro Phe Asn Ile Ser Val Gln Gly Thr Tyr Glu Ser Ile Phe Asn
    50                  55                  60

Asn Tyr Gln Thr Thr Tyr Phe Tyr Lys Lys Asp Ser Ile Thr Ser Met
65                  70                  75                  80

Pro Met Asn Ser His Met Leu Leu Asn Val Asn Leu Ile Ile His Ile
                85                  90                  95

Gly Gly Asp Phe Asp Leu Leu Glu Glu Ile Tyr Asp Ser Leu Leu Asn
            100                 105                 110

Tyr Asp Glu His Leu Ser Leu Gly Arg Lys Glu Asp Leu Val Arg Ile
        115                 120                 125

Asn Asp Ile Arg Phe Val Glu Val Asn Lys Phe Glu Val Asp Glu Asp
    130                 135                 140

Ile Gln Leu Asp Asp Tyr Glu Asn Tyr Lys Leu Ser Glu Cys Asn Ile
145                 150                 155                 160

Arg Met Pro Ile Tyr Ile Pro Lys Thr Ser Leu Gln Asp Thr Asp Ile
                165                 170                 175

Asp Gly Ile Ser Tyr Arg Leu Asn Asn Tyr Tyr Asp Asn Asn Ala Val
            180                 185                 190

Glu Asp Lys Lys Arg Val Trp Asn Lys Val Asp Ser Tyr Tyr Val Glu
        195                 200                 205

Asp Gly Asn Thr Ile Ser Ile Gly Asp Ile Leu Leu Asp Asn Glu Gly
    210                 215                 220

Asp Leu Leu Tyr Phe Asn Asn Ile Asn
225                 230

<210>  17
<211>  800
<212>  PRT
<213>  Bacillus halodurans

<220>
<221>  PEPTIDE
<222>  (1)..(800)
<223>  CRISPR/Cas system-associated endonuclease/helicase [Cas3]:
       WP_010896517

<400>  17

Met Tyr Ile Ala His Ile Arg Glu Val Asp Lys Val Ile Gln Thr Leu
1               5                   10                  15

Lys Glu His Leu Cys Gly Val Gln Cys Leu Ala Glu Thr Phe Gly Ala
            20                  25                  30

Lys Leu Arg Leu Gln His Val Ala Gly Leu Ala Gly Leu Leu His Asp
        35                  40                  45

Leu Gly Lys Tyr Thr Asn Glu Phe Lys Asp Tyr Ile Tyr Lys Ala Val
    50                  55                  60

Phe Glu Pro Glu Leu Ala Glu Lys Lys Arg Gly Gln Val Asp His Ser
65                  70                  75                  80

Thr Ala Gly Gly Arg Leu Leu Tyr Gln Met Leu His Asp Arg Glu Asn
                85                  90                  95

Ser Phe His Glu Lys Leu Leu Ala Glu Val Val Gly Asn Ala Ile Ile
        100                 105                 110

Ser His His Ser Asn Leu Gln Asp Tyr Ile Ser Pro Thr Ile Glu Ser
    115                 120                 125

Asn Phe Leu Thr Arg Val Leu Glu Lys Glu Leu Pro Glu Tyr Glu Ser
    130                 135                 140

Ala Val Glu Arg Phe Phe Gln Glu Val Met Thr Glu Ala Glu Leu Ala
145                 150                 155                 160

Arg Tyr Val Ala Lys Ala Val Asp Glu Ile Lys Gln Phe Thr Asp Asn
                165                 170                 175

Ser Pro Thr Gln Ser Phe Phe Leu Thr Lys Tyr Ile Phe Ser Cys Leu
        180                 185                 190

Ile Asp Ala Asp Arg Thr Asn Thr Arg Met Phe Asp Glu Gln Ala Arg
        195                 200                 205

Glu Glu Glu Pro Thr Gln Pro Gln Gln Leu Phe Glu His Tyr His Gln
    210                 215                 220

Gln Leu Leu Asn His Leu Ala Ser Leu Lys Glu Ser Asp Ser Ala Gln
225                 230                 235                 240

Lys Pro Ile Asn Val Leu Arg Ser Ala Met Ser Glu Gln Cys Glu Ser
                245                 250                 255

Phe Ala Met Arg Pro Ser Gly Ile Tyr Thr Leu Ser Ile Pro Thr Gly
        260                 265                 270

Gly Gly Lys Thr Leu Ala Ser Leu Arg Tyr Ala Leu Lys His Ala Gln
        275                 280                 285

Glu Tyr Asn Lys Gln Arg Ile Ile Tyr Ile Val Pro Phe Thr Thr Ile
    290                 295                 300

Ile Glu Gln Asn Ala Gln Glu Val Arg Asn Ile Leu Gly Asp Asp Glu
305                 310                 315                 320

Asn Ile Leu Glu His His Ser Asn Val Val Glu Asp Ser Glu Asn Gly
                325                 330                 335

Asp Glu Gln Glu Asp Gly Val Ile Thr Lys Lys Glu Arg Leu Arg Leu
        340                 345                 350

Ala Arg Asp Asn Trp Asp Arg Pro Ile Ile Phe Thr Thr Leu Val Gln
        355                 360                 365

Phe Leu Asn Val Phe Tyr Ala Lys Gly Asn Arg Asn Thr Arg Arg Leu
    370                 375                 380

His Asn Leu Ser His Ser Val Leu Ile Phe Asp Glu Val Gln Lys Val
385                 390                 395                 400

Pro Thr Lys Cys Val Ser Leu Phe Asn Glu Ala Leu Asn Phe Leu Lys
                405                 410                 415

Glu Phe Ala His Cys Ser Ile Leu Leu Cys Thr Ala Thr Gln Pro Thr
            420                 425                 430

Leu Glu Asn Val Lys His Ser Leu Leu Lys Asp Arg Asp Gly Glu Ile
        435                 440                 445

Val Gln Asn Leu Thr Glu Val Ser Glu Ala Phe Lys Arg Val Glu Ile
    450                 455                 460

Leu Asp Lys Thr Asp Gln Pro Met Thr Asn Glu Arg Leu Ala Glu Trp
465                 470                 475                 480

Val Arg Asp Glu Ala Pro Ser Trp Gly Ser Thr Leu Ile Ile Leu Asn
                485                 490                 495

Thr Lys Lys Val Val Lys Asp Leu Tyr Glu Lys Leu Glu Gly Gly Pro
            500                 505                 510

Leu Pro Val Phe His Leu Ser Thr Ser Met Cys Ala Ala His Arg Lys
        515                 520                 525

Asp Gln Leu Asp Glu Ile Arg Ala Leu Leu Lys Glu Gly Thr Pro Phe
    530                 535                 540

Ile Cys Val Thr Thr Gln Leu Ile Glu Ala Gly Val Asp Val Ser Phe
545                 550                 555                 560

Lys Cys Val Ile Arg Ser Leu Ala Gly Leu Asp Ser Ile Ala Gln Ala
                565                 570                 575

Ala Gly Arg Cys Asn Arg His Gly Glu Glu Gln Leu Gln Tyr Val Tyr
            580                 585                 590

Val Ile Asp His Ala Glu Glu Thr Leu Ser Lys Leu Lys Glu Ile Glu
        595                 600                 605

Val Gly Gln Glu Ile Ala Gly Asn Val Leu Ala Arg Phe Lys Lys Lys
    610                 615                 620

Ala Glu Lys Tyr Glu Gly Asn Leu Leu Ser Gln Ala Ala Met Arg Glu
625                 630                 635                 640

Tyr Phe Arg Tyr Tyr Tyr Ser Lys Met Asp Ala Asn Leu Asn Tyr Phe
                645                 650                 655

Val Lys Glu Val Asp Lys Asp Met Thr Lys Leu Leu Met Ser His Ala
            660                 665                 670

Val Glu Asn Ser Tyr Val Thr Tyr Tyr Gln Lys Asn Thr Gly Thr His
        675                 680                 685

Phe Pro Leu Leu Leu Asn Gly Ser Tyr Lys Thr Ala Ala Asp His Phe
    690                 695                 700

Arg Val Ile Asp Gln Asn Thr Thr Ser Ala Ile Val Pro Tyr Gly Glu
705                 710                 715                 720

Gly Gln Asp Ile Ile Ala Gln Leu Asn Ser Gly Glu Trp Val Asp Asp
                725                 730                 735

Leu Ser Lys Val Leu Lys Lys Ala Gln Gln Tyr Thr Val Asn Leu Tyr
            740                 745                 750

Ser Gln Glu Ile Asp Gln Leu Lys Lys Glu Gly Ala Ile Val Met His
        755                 760                 765

Leu Asp Gly Met Val Tyr Glu Leu Lys Glu Ser Trp Tyr Ser His Gln
    770                 775                 780

Tyr Gly Val Asp Phe Lys Gly Glu Gly Gly Met Asp Phe Met Ser Phe
785                 790                 795                 800

<210>  18
<211>  236
<212>  PRT
<213>  Bacillus halodurans

<220>
<221>  PEPTIDE
<222>  (1)..(236)
<223>  CRISPR/Cas system-associated protein Cas5: WP_010896518.1

<400>  18

Met Arg Asn Glu Val Gln Phe Glu Leu Phe Gly Asp Tyr Ala Leu Phe
1               5                   10                  15

Thr Asp Pro Leu Thr Lys Ile Gly Gly Glu Lys Leu Ser Tyr Ser Val
            20                  25                  30

Pro Thr Tyr Gln Ala Leu Lys Gly Ile Ala Glu Ser Ile Tyr Trp Lys
        35                  40                  45

Pro Thr Ile Val Phe Val Ile Asp Glu Leu Arg Val Met Lys Pro Ile
    50                  55                  60

Gln Met Glu Ser Lys Gly Val Arg Pro Ile Glu Tyr Gly Gly Gly Asn
65                  70                  75                  80

Thr Leu Ala His Tyr Thr Tyr Leu Lys Asp Val His Tyr Gln Val Lys
                85                  90                  95

Ala His Phe Glu Phe Asn Leu His Arg Pro Asp Leu Ala Phe Asp Arg
            100                 105                 110

Asn Glu Gly Lys His Tyr Ser Ile Leu Gln Arg Ser Leu Lys Ala Gly
        115                 120                 125

Gly Arg Arg Asp Ile Phe Leu Gly Ala Arg Glu Cys Gln Gly Tyr Val
    130                 135                 140

Ala Pro Cys Glu Phe Gly Ser Gly Asp Gly Phe Tyr Asp Gly Gln Gly
145                 150                 155                 160

Lys Tyr His Leu Gly Thr Met Val His Gly Phe Asn Tyr Pro Asp Glu
                165                 170                 175

Thr Gly Gln His Gln Leu Asp Val Arg Leu Trp Ser Ala Val Met Glu
            180                 185                 190

Asn Gly Tyr Ile Gln Phe Pro Arg Pro Glu Asp Cys Pro Ile Val Arg
        195                 200                 205

Pro Val Lys Glu Met Glu Pro Lys Ile Phe Asn Pro Asp Asn Val Gln
    210                 215                 220

Ser Ala Glu Gln Leu Leu His Asp Leu Gly Gly Glu
225                 230                 235

<210>  19
<211>  627
<212>  PRT

<213>  Bacillus halodurans

<220>
<221>  PEPTIDE
<222>  (1)..(627)
<223>  CRISPR/Cas system-associated protein Cas8C/Csd1 : WP_010896519.1

<400>  19

Met Ser Trp Leu Leu His Leu Tyr Glu Thr Tyr Glu Ala Asn Leu Asp
1               5                   10                  15

Gln Val Gly Lys Thr Val Lys Lys Gly Glu Asp Arg Glu Tyr Thr Leu
            20                  25                  30

Leu Pro Ile Ser His Thr Thr Gln Asn Ala His Ile Glu Val Thr Leu
        35                  40                  45

Asp Glu Asp Gly Asp Phe Leu Arg Ala Lys Ala Leu Thr Lys Glu Ser
    50                  55                  60

Thr Leu Ile Pro Cys Thr Glu Glu Ala Ala Ser Arg Ser Gly Ser Lys
65                  70                  75                  80

Val Ala Pro Tyr Pro Leu His Asp Lys Leu Ser Tyr Val Ala Gly Asp
                85                  90                  95

Phe Val Lys Tyr Gly Gly Lys Ile Lys Asn Gln Asp Asp Ala Pro Phe
            100                 105                 110

Asp Thr Tyr Ile Lys Asn Leu Gly Glu Trp Ala Asn Ser Pro Tyr Ala
        115                 120                 125

Thr Glu Lys Val Lys Cys Ile Tyr Thr Tyr Leu Lys Lys Gly Arg Leu
    130                 135                 140

Ile Glu Asp Leu Val Asp Ala Gly Val Leu Lys Leu Asp Glu Asn Gln
145                 150                 155                 160

Gln Leu Ile Glu Lys Trp Glu Lys Arg Tyr Glu Glu Leu Leu Gly Glu
                165                 170                 175

Lys Pro Ala Ile Phe Ser Ser Gly Ala Thr Asp Gln Ala Ser Ala Phe
            180                 185                 190

Val Arg Phe Asn Val Phe His Pro Glu Ser Ile Asp Asp Val Trp Lys
        195                 200                 205

Asp Lys Glu Met Phe Asp Ser Phe Ile Ser Phe Tyr Asn Asp Lys Leu

210                 215                 220

Gly Glu Glu Asp Ile Cys Phe Val Thr Gly Asn Arg Leu Pro Ser Thr
225                 230                 235                 240

Glu Arg His Ala Asn Lys Ile Arg His Ala Ala Asp Lys Ala Lys Leu
                245                 250                 255

Ile Ser Ala Asn Asp Asn Ser Gly Phe Thr Phe Arg Gly Arg Phe Lys
            260                 265                 270

Thr Ser Arg Glu Ala Val Gly Ile Ser Tyr Glu Val Ser Gln Lys Ala
        275                 280                 285

His Asn Ala Leu Lys Trp Leu Ile His Arg Gln Ser Lys Ser Ile Asp
    290                 295                 300

Asp Arg Val Phe Leu Val Trp Ser Asn Asp Asn Ser Leu Val Pro Asn
305                 310                 315                 320

Pro Asp Glu Asp Ala Val Asp Ile Met Lys His Ala Asn Arg Glu Leu
                325                 330                 335

Glu Arg Asp Pro Asp Thr Gly Gln Ile Phe Ala Gly Glu Val Lys Lys
            340                 345                 350

Ala Ile Gly Gly Tyr Arg Ser Asp Leu Asn Tyr Gln Pro Glu Val His
        355                 360                 365

Ile Leu Val Leu Asp Ser Ala Thr Thr Gly Arg Met Ala Val Leu Tyr
    370                 375                 380

Tyr Arg Ser Leu Asn Lys Glu Leu Tyr Leu Asn Arg Leu Glu Ala Trp
385                 390                 395                 400

His Asp Ser Cys Ala Trp Glu His Arg Tyr Arg Arg Asp Glu Lys Glu
                405                 410                 415

Phe Ile Ser Phe Tyr Gly Ala Pro Ala Thr Lys Asp Ile Ala Phe Ala
            420                 425                 430

Ala Tyr Gly Pro Arg Ala Ser Glu Lys Val Ile Lys Asp Leu Met Glu
        435                 440                 445

Arg Met Leu Pro Cys Ile Val Asp Gly Arg Arg Val Pro Lys Asp Ile
    450                 455                 460

Val Arg Ser Ala Phe Gln Arg Ala Ser Asn Pro Val Ser Met Glu Arg
465                 470                 475                 480

Trp Glu Trp Glu Lys Thr Leu Ser Ile Thr Cys Ala Leu Ile Arg Lys
                485                 490                 495

Met His Ile Glu Gln Lys Glu Glu Trp Gly Val Pro Leu Asp Lys Ser
            500                 505                 510

Ser Thr Asp Arg Ser Tyr Leu Phe Gly Arg Leu Leu Ala Val Ala Asp
        515                 520                 525

Val Leu Glu Arg Gly Ala Leu Gly Lys Asp Glu Thr Arg Ala Thr Asn
    530                 535                 540

Ala Ile Arg Tyr Met Asn Ser Tyr Ser Lys Asn Pro Gly Arg Thr Trp
545                 550                 555                 560

Lys Thr Ile Gln Glu Ser Leu Gln Pro Tyr Gln Ala Lys Leu Gly Thr
                565                 570                 575

Lys Ala Thr Tyr Leu Ser Lys Leu Val Asp Glu Ile Gly Asp Gln Phe
            580                 585                 590

Glu Pro Gly Asp Phe Asn Asn Asn Pro Leu Thr Glu Gln Tyr Leu Leu
        595                 600                 605

Gly Phe Tyr Ser Gln Arg Arg Glu Leu Tyr Lys Lys Lys Glu Glu Glu
    610                 615                 620

Thr Asn Gln
625

<210>  20
<211>  283
<212>  PRT
<213>  Bacillus halodurans

<220>
<221>  PEPTIDE
<222>  (1)..(283)
<223>  CRISPR/Cas system-associated type I-C Cas7/Csd2B : WP_010896520.1

<400>  20

Met Thr Ile Leu Asp His Lys Ile Asp Phe Ala Val Ile Leu Ser Val
1               5                   10                  15

Thr Lys Ala Asn Pro Asn Gly Asp Pro Leu Asn Gly Asn Arg Pro Arg
            20                  25                  30

Gln Asn Tyr Asp Gly His Gly Glu Ile Ser Asp Val Ala Ile Lys Arg
        35                  40                  45

Lys Ile Arg Asn Arg Leu Leu Asp Met Glu Glu Pro Ile Phe Val Gln
    50                  55                  60

Ser Asp Asp Arg Lys Ala Asp Ser Phe Lys Ser Leu Arg Asp Arg Ala
65                  70                  75                  80

Asp Ser Asn Pro Glu Leu Ala Lys Met Leu Lys Ala Lys Asn Ala Ser
                85                  90                  95

Val Asp Glu Phe Ala Lys Ile Ala Cys Gln Glu Trp Met Asp Val Arg
            100                 105                 110

Ser Phe Gly Gln Val Phe Ala Phe Lys Gly Ser Asn Leu Ser Val Gly
        115                 120                 125

Val Arg Gly Pro Val Ser Ile His Thr Ala Thr Ser Ile Asp Pro Ile
    130                 135                 140

Asp Ile Val Ser Thr Gln Ile Thr Lys Ser Val Asn Ser Val Thr Gly
145                 150                 155                 160

Asp Lys Arg Ser Ser Asp Thr Met Gly Met Lys His Arg Val Asp Phe
                165                 170                 175

Gly Val Tyr Val Phe Lys Gly Ser Ile Asn Thr Gln Leu Ala Glu Lys
            180                 185                 190

Thr Gly Phe Thr Asn Glu Asp Ala Glu Lys Ile Lys Arg Ala Leu Ile
        195                 200                 205

Thr Leu Phe Glu Asn Asp Ser Ser Ser Ala Arg Pro Asp Gly Ser Met
    210                 215                 220

Glu Val His Lys Val Tyr Trp Trp Glu His Ser Ser Lys Leu Gly Gln
225                 230                 235                 240

Tyr Ser Ser Ala Lys Val His Arg Ser Leu Lys Ile Glu Ser Lys Thr
                245                 250                 255

Asp Thr Pro Lys Ser Phe Asp Asp Tyr Ala Val Glu Leu Tyr Glu Leu
            260                 265                 270

Asp Gly Leu Gly Val Glu Val Ile Asp Gly Gln

275                 280

<210>  21
<211>  174
<212>  PRT
<213>  Bacillus halodurans

<220>
<221>  PROPEP
<222>  (1)..(174)
<223>  CRISPR/Cas system-associated exonuclease Cas4, RecB: BAB04059.1

<400>  21

Met His Lys Lys Ala Asp Gln Pro Phe Met Lys Glu Lys Arg Gly Ser
1               5                   10                  15

Lys Leu Thr Val Arg Ala Met Pro Ile Gln Ser Lys Asn Leu Gln Ile
            20                  25                  30

Ser Gly Ile Cys Asp Val Val Glu Phe Val Gln Asp Ser Glu Gly Ile
        35                  40                  45

Glu Leu Ser Gly Val Ser Gly Ser Tyr Lys Ala Phe Pro Val Glu Tyr
    50                  55                  60

Lys Arg Gly Lys Pro Lys Lys Gly Asp Glu Asp Ile Val Gln Leu Val
65                  70                  75                  80

Ala Gln Ala Met Cys Leu Glu Glu Met Leu Val Cys Arg Ile Asp Lys
                85                  90                  95

Gly Tyr Leu Phe Tyr Asn Glu Ile Lys His Arg Val Glu Val Pro Ile
            100                 105                 110

Thr Asp Ala Leu Arg Asp Lys Val Val Gln Met Ala Lys Glu Met His
        115                 120                 125

His Tyr Tyr Glu Asn Arg His Thr Pro Lys Val Lys Thr Gly Pro Phe
    130                 135                 140

Cys Asn Asn Cys Ser Leu Gln Ser Ile Cys Leu Pro Lys Leu Met Asn
145                 150                 155                 160

Lys Arg Ser Val Lys Arg Tyr Ile Glu Gly Arg Leu Ser Glu
                165                 170

<210>  22
<211>  343
<212>  PRT

<213>  Bacillus halodurans

<220>
<221>  PEPTIDE
<222>  (1)..(343)
<223>  CRISPR-associated endonuclease Cas1, subtype I-C: WP_010896522.1

<400>  22

Met Lys Lys Leu Leu Asn Thr Leu Tyr Val Thr Gln Pro Asp Thr Tyr
1               5                   10                  15

Leu Ser Leu Asp Gly Asp Asn Val Val Leu Leu Lys Glu Gln Glu Lys
            20                  25                  30

Leu Gly Arg Leu Pro Leu His Asn Leu Glu Ala Ile Val Gly Phe Gly
        35                  40                  45

Tyr Thr Gly Ala Ser Pro Ala Leu Met Gly Tyr Cys Ala Glu Arg Asn
    50                  55                  60

Ile Ser Ile Thr Phe Leu Thr Lys Asn Gly Arg Phe Leu Ala Arg Val
65                  70                  75                  80

Val Gly Glu Ser Arg Gly Asn Val Val Leu Arg Lys Thr Gln Tyr Arg
                85                  90                  95

Ile Ser Glu Asn Asp Gln Glu Ser Thr Lys Ile Ala Arg Asn Phe Ile
            100                 105                 110

Thr Gly Lys Val Tyr Asn Ser Lys Trp Met Leu Glu Arg Met Thr Arg
        115                 120                 125

Glu His Pro Leu Arg Val Asn Val Glu Gln Phe Lys Ala Thr Ser Gln
    130                 135                 140

Leu Leu Ser Val Met Met Gln Glu Ile Arg Asn Cys Asp Ser Leu Glu
145                 150                 155                 160

Ser Leu Arg Gly Trp Glu Gly Gln Ala Ala Ile Asn Tyr Asn Lys Val
                165                 170                 175

Phe Asp Gln Met Ile Leu Gln Gln Lys Glu Glu Phe Ala Phe His Gly
            180                 185                 190

Arg Ser Arg Arg Pro Pro Lys Asp Asn Val Asn Ala Met Leu Ser Phe
        195                 200                 205

Ala Tyr Thr Leu Leu Ala Asn Asp Val Ala Ala Ala Leu Glu Thr Val

210                 215                 220

Gly Leu Asp Ala Tyr Val Gly Phe Met His Gln Asp Arg Pro Gly Arg
225                 230                 235                 240

Ala Ser Leu Ala Leu Asp Leu Met Glu Glu Leu Arg Gly Leu Tyr Ala
                245                 250                 255

Asp Arg Phe Val Leu Ser Leu Ile Asn Arg Lys Glu Met Thr Ala Asp
            260                 265                 270

Gly Phe Tyr Lys Lys Glu Asn Gly Ala Val Leu Met Thr Asp Glu Ala
        275                 280                 285

Arg Lys Thr Phe Leu Lys Ala Trp Gln Thr Lys Lys Gln Glu Lys Ile
    290                 295                 300

Thr His Pro Tyr Leu Gly Glu Lys Met Ser Trp Gly Leu Val Pro Tyr
305                 310                 315                 320

Val Gln Ala Leu Leu Leu Ala Arg Phe Leu Arg Gly Asp Leu Asp Glu
                325                 330                 335

Tyr Pro Pro Phe Leu Trp Lys
            340

<210>  23
<211>  96
<212>  PRT
<213>  Bacillus halodurans

<220>
<221>  PEPTIDE
<222>  (1)..(96)
<223>  CRISPR/Cas system-associated protein Cas2: WP_010896523.1

<400>  23

Met Leu Val Leu Ile Thr Tyr Asp Val Gln Thr Ser Ser Met Gly Gly
1               5                   10                  15

Thr Lys Arg Leu Arg Lys Val Ala Lys Ala Cys Gln Asn Tyr Gly Gln
            20                  25                  30

Arg Val Gln Asn Ser Val Phe Glu Cys Ile Val Asp Ser Thr Gln Leu
        35                  40                  45

Thr Ser Leu Lys Leu Glu Leu Thr Ser Leu Ile Asp Glu Glu Lys Asp
    50                  55                  60

Ser Leu Arg Ile Tyr Arg Leu Gly Asn Asn Tyr Lys Thr Lys Val Glu
65                  70                  75                  80

His Ile Gly Ala Lys Pro Ser Ile Asp Leu Glu Asp Pro Leu Ile Phe
                85                  90                  95

<210>  24
<211>  701
<212>  PRT
<213>  Cyanothece spp

<220>
<221>  PEPTIDE
<222>  (1)..(701)
<223>  type I-D CRISPR-associated helicase Cas3: WP_015783167.1

<400>  24

Met Lys Ile Asn Leu Lys Pro Leu Tyr Ser Lys Leu Asn Ala Gly Val
1               5                   10                  15

Gly Asn Cys Pro Leu Gly Cys Gln Glu Met Cys Arg Val Gln Gln Gln
            20                  25                  30

Ala Pro Gln Phe Lys Ala Pro Ser Gly Cys Asn Cys Pro Leu Tyr Gln
        35                  40                  45

His Gln Ala Glu Ser Tyr Pro Tyr Leu Thr Lys Gly Asp Thr Asp Ile
    50                  55                  60

Ile Phe Ile Thr Ala Pro Thr Ala Gly Gly Lys Ser Leu Leu Ala Ser
65                  70                  75                  80

Leu Pro Ser Leu Leu Asp Pro Asn Phe Arg Met Met Gly Leu Tyr Pro
                85                  90                  95

Thr Ile Glu Leu Val Glu Asp Gln Thr Glu Gln Gln Asn Asn Tyr His
            100                 105                 110

Asn Leu Phe Gly Leu Asn Ser Glu Glu Arg Ile Asp Lys Leu Phe Gly
        115                 120                 125

Val Glu Leu Thr Gln Arg Ile Lys Glu Phe Asn Ser Asn Arg Phe Gln
    130                 135                 140

Gln Leu Trp Leu Ala Ile Glu Thr Lys Glu Val Ile Leu Thr Asn Pro
145                 150                 155                 160

Asp Ile Phe His Leu Met Thr His Phe Arg Tyr Arg Asp Asn Ala Tyr

                165                 170                 175

Gly Thr Asp Glu Leu Pro Leu Ala Leu Ala Lys Phe Pro Asp Leu Trp
            180                 185                 190

Val Phe Asp Glu Phe His Ile Phe Gly Ala His Gln Glu Thr Ala Val
        195                 200                 205

Leu Asn Ser Met Met Leu Ile Arg Arg Thr Gln Gln Gln Lys Lys Arg
    210                 215                 220

Phe Leu Phe Thr Ser Ala Thr Val Lys Thr Asp Phe Val Glu Gln Leu
225                 230                 235                 240

Lys Gln Thr Gly Leu Lys Ile Lys Glu Ile Ala Gly Glu Tyr Lys Ser
                245                 250                 255

Glu Ala Gln Gln Gly Tyr Arg Gln Ile Leu Gln Ala Val Glu Leu Ser
            260                 265                 270

Ile Ile Asn Leu Lys Glu Glu Asp Gly Phe Ser Trp Leu Ile Asn Asn
        275                 280                 285

Ala Ala Lys Ile Arg Lys Ile Leu Lys Ala Glu Asp Lys Gly Arg Gly
    290                 295                 300

Leu Ile Ile Leu Asn Ser Val Val Met Val Arg Arg Ile Ser Gln Glu
305                 310                 315                 320

Leu Gln Ser Leu Leu Pro Glu Ile Val Val Arg Glu Ile Ser Gly Arg
                325                 330                 335

Ile Asp Arg Lys Glu Arg Ser Gln Thr Gln Gln Leu Leu Gln Glu Glu
            340                 345                 350

Glu Lys Pro Val Leu Val Val Ala Thr Ser Ala Val Asp Val Gly Val
        355                 360                 365

Asp Phe Arg Ile His Leu Leu Ile Thr Glu Ser Ser Asp Ser Ala Thr
    370                 375                 380

Val Ile Gln Arg Leu Gly Arg Leu Gly Arg His Ser Gly Phe Ser Asn
385                 390                 395                 400

Tyr Gln Ala Phe Leu Leu Leu Ser Gly Arg Thr Pro Trp Val Ile Asn
                405                 410                 415

Arg Leu Gln Glu Lys Leu Glu Ser Lys Gln Asp Val Thr Arg Glu Glu
        420                 425                 430

Leu Ile Glu Ala Ile Gln Tyr Ala Phe Asp Pro Pro Lys Glu Tyr Gln
    435                 440                 445

Glu Tyr Arg Asn Arg Trp Gly Ala Ile Gln Val Gln Gly Met Phe Ser
    450                 455                 460

Gln Met Met Gly Ser Asn Ala Lys Val Met Gln Ser Ile Lys Glu Arg
465                 470                 475                 480

Ile Ser Glu Asp Leu Lys Arg Ile Tyr Gly Asn Thr Leu Asp Asn Lys
                485                 490                 495

Ala Trp Tyr Ala Met Gly His Asn Cys Leu Gly Lys Ala Ile Gln Ser
        500                 505                 510

Glu Leu Leu Arg Phe Arg Gly Gly Ser Thr Leu Gln Ala Ala Val Trp
    515                 520                 525

Asp Glu Gln Arg Phe Tyr Thr Tyr Asp Leu Leu Arg Leu Leu Pro Tyr
    530                 535                 540

Ala Thr Val Asp Ile Leu Asp Arg Glu Thr Phe Leu Lys Ala Ala Thr
545                 550                 555                 560

Lys Ala Gly His Ile Glu Glu Ala Phe Pro Ser Gln Tyr Leu Gln Val
                565                 570                 575

Tyr Leu Arg Ile Glu Gln Trp Leu Asp Lys Arg Leu Asn Leu Asn Leu
        580                 585                 590

Phe Cys Asn Arg Glu Ser Asp Glu Leu Leu Val Gly Lys Leu Phe Leu
    595                 600                 605

Ile Thr Arg Leu Lys Leu Asp Gly His Pro Gln Ser Asp Val Ile Ser
    610                 615                 620

Cys Leu Ser Arg Cys Asn Leu Leu Thr Phe Leu Val Pro Val Asp Arg
625                 630                 635                 640

Ser Arg Thr Gln Ser His Trp Glu Val Ser Tyr Cys Leu His Leu Asn
                645                 650                 655

Pro Leu Phe Gly Leu Tyr Arg Leu Lys Asp Ala Ser Glu Gln Ala Tyr
        660                 665                 670

Ala Cys Ala Phe Asn Gln Asp Ala Leu Leu Leu Glu Ala Leu Asn Trp
        675                 680                 685

Lys Leu Thr Lys Phe Tyr Arg Glu Arg Ser Leu Ile Phe
    690                 695                 700

<210>  25
<211>  97
<212>  PRT
<213>  Cyanothece spp

<220>
<221>  PROPEP
<222>  (1)..(97)
<223>  CRISPR/Cas system-associated endonuclease Cas2: WP_015783160.1

<400>  25

Met Asn Thr Leu Phe Tyr Leu Ile Ile Tyr Asp Leu Pro Ala Thr Lys
1               5                   10                  15

Ala Gly Asn Lys Arg Arg Lys Arg Leu Tyr Glu Met Leu Cys Gly Tyr
            20                  25                  30

Gly Asn Trp Thr Gln Phe Ser Val Phe Glu Cys Phe Leu Thr Ala Val
        35                  40                  45

Gln Phe Ala Asn Leu Gln Ser Lys Leu Glu Asn Leu Ile Gln Pro Asn
    50                  55                  60

Glu Asp Ser Val Arg Ile Tyr Ile Leu Asp Ala Gly Ser Val Arg Lys
65                  70                  75                  80

Thr Leu Thr Tyr Gly Ser Glu Lys Pro Arg Gln Val Asp Thr Leu Ile
                85                  90                  95

Leu

<210>  26
<211>  325
<212>  PRT
<213>  Cyanothece spp

<220>
<221>  PEPTIDE
<222>  (1)..(325)
<223>  type I-D CRISPR-associated endonuclease Cas1: WP_015783161.1

<400>  26

Met Ser Ile Leu Tyr Leu Thr Gln Pro Asp Ala Val Leu Ser Lys Lys
1               5                   10                  15

Gln Glu Ala Phe His Val Ala Leu Lys Gln Glu Asp Gly Ser Trp Lys
            20                  25                  30

Lys Gln Leu Ile Pro Ala Gln Thr Val Glu Gln Ile Val Leu Ile Gly
        35                  40                  45

Tyr Pro Ser Ile Thr Gly Glu Ala Leu Cys Tyr Ala Leu Glu Leu Gly
    50                  55                  60

Ile Pro Val His Tyr Leu Ser Cys Phe Gly Lys Tyr Leu Gly Ser Ala
65                  70                  75                  80

Leu Pro Gly Tyr Ser Arg Asn Gly Gln Leu Arg Leu Ala Gln Tyr His
                85                  90                  95

Val His Asp Asn Glu Glu Gln Arg Leu Ala Leu Val Lys Thr Val Val
            100                 105                 110

Thr Gly Lys Ile His Asn Gln Tyr His Val Leu Tyr Arg Tyr Gln Gln
        115                 120                 125

Lys Asp Asn Pro Leu Lys Glu His Lys Gln Leu Val Lys Ser Lys Thr
    130                 135                 140

Thr Leu Glu Gln Val Arg Gly Val Glu Gly Leu Ala Ala Lys Asp Tyr
145                 150                 155                 160

Phe Asn Gly Phe Lys Leu Ile Leu Asp Ser Gln Trp Asn Phe Asn Gly
                165                 170                 175

Arg Asn Arg Arg Pro Pro Thr Asp Pro Val Asn Ala Leu Leu Ser Phe
            180                 185                 190

Ala Tyr Gly Leu Leu Arg Val Gln Val Thr Ala Ala Val His Ile Ala
        195                 200                 205

Gly Leu Asp Pro Tyr Ile Gly Tyr Leu His Glu Thr Thr Arg Gly Gln
    210                 215                 220

Pro Ala Met Val Leu Asp Leu Met Glu Glu Phe Arg Pro Leu Ile Ala
225                 230                 235                 240

Asp Ser Leu Val Leu Ser Val Ile Ser His Lys Glu Ile Lys Pro Thr
                245                 250                 255

Asp Phe Asn Glu Ser Leu Gly Ala Tyr Leu Leu Ser Asp Ser Gly Arg
        260                 265                 270

Lys Thr Phe Leu Gln Ala Phe Glu Arg Lys Leu Asn Thr Glu Phe Lys
    275                 280                 285

His Pro Val Phe Gly Tyr Gln Cys Ser Tyr Arg Arg Ser Ile Glu Leu
    290                 295                 300

Gln Ala Arg Leu Phe Ser Arg Tyr Leu Gln Glu Asn Ile Pro Tyr Lys
305                 310                 315                 320

Ser Leu Ser Leu Arg
                325

<210>  27
<211>  196
<212>  PRT
<213>  Cyanothece spp

<220>
<221>  PEPTIDE
<222>  (1)..(196)
<223>  CRISPR-associated protein Cas4: WP_015783162.1

<400>  27

Met Ile Asp Asn Tyr Leu Pro Leu Ala Tyr Leu Asn Ala Phe Glu Tyr
1               5                   10                  15

Cys Thr Arg Arg Phe Tyr Trp Glu Tyr Val Leu Gly Glu Met Ala Asn
            20                  25                  30

Asn Glu His Ile Ile Ile Gly Arg His Leu His Arg Asn Ile Asn Gln
        35                  40                  45

Glu Gly Ile Ile Lys Glu Glu Asp Thr Ile Ile His Arg Gln Gln Trp
    50                  55                  60

Val Trp Ser Asp Arg Leu Gln Ile Lys Gly Ile Ile Asp Ala Val Glu
65                  70                  75                  80

Glu Lys Glu Ser Ser Leu Val Pro Val Glu Tyr Lys Lys Gly Arg Met
                85                  90                  95

Ser Gln His Leu Asn Asp His Phe Gln Leu Cys Ala Ala Ala Leu Cys
            100                 105                 110

Leu Glu Glu Lys Thr Gly Lys Ile Ile Thr Tyr Gly Glu Ile Phe Tyr

115                 120                 125

His Ala Asn Arg Arg Arg Gln Arg Val Asp Phe Ser Asp Arg Leu Arg
    130                 135                 140

Cys Ser Thr Glu Gln Ala Ile His His Ala His Glu Leu Val Asn Gln
145                 150                 155                 160

Lys Met Pro Ser Pro Ile Asn Asn Ser Lys Lys Cys Arg Asp Cys Ser
                165                 170                 175

Leu Lys Thr Met Cys Leu Pro Lys Glu Val Lys Gln Leu Arg Asn Ser
            180                 185                 190

Leu Ile Ser Asp
        195

<210>  28
<211>  269
<212>  PRT
<213>  Cyanothece spp

<220>
<221>  PEPTIDE
<222>  (1)..(269)
<223>  CRISPR system precrRNA processing endoribonuclease RAMP protein
       Cas6: WP_015783163.1

<400>  28

Met Val Gln Asp Ile Leu Pro Gln Leu His Lys Tyr Gln Leu Gln Ser
1               5                   10                  15

Leu Val Ile Glu Leu Gly Val Ala Lys Gln Gly Lys Leu Pro Ala Thr
            20                  25                  30

Leu Ser Arg Ala Ile His Ala Cys Val Leu Asn Trp Leu Ser Leu Ala
        35                  40                  45

Asp Ser Gln Leu Ala Asn Gln Ile His Asp Ser Gln Ile Ser Pro Leu
    50                  55                  60

Cys Leu Ser Gly Leu Ile Gly Asn Arg Arg Gln Pro Tyr Ser Leu Leu
65                  70                  75                  80

Gly Asp Tyr Phe Leu Leu Arg Ile Gly Val Leu Gln Pro Ser Leu Ile
                85                  90                  95

Lys Pro Leu Leu Lys Gly Ile Glu Ala Gln Glu Thr Gln Thr Leu Glu
            100                 105                 110

Leu Gly Lys Phe Pro Phe Ile Ile Arg Gln Val Tyr Ser Met Pro Gln
        115                 120                 125

Ser His Lys Leu Ser Gln Leu Thr Asp Tyr Tyr Ser Leu Ala Leu Tyr
    130                 135                 140

Ser Pro Thr Met Thr Glu Ile Gln Leu Lys Phe Leu Ser Pro Thr Ser
145                 150                 155                 160

Phe Lys Gln Ile Gln Gly Val Gln Pro Phe Pro Leu Pro Glu Leu Val
                165                 170                 175

Phe Asn Ser Leu Leu Arg Lys Trp Asn His Phe Ala Pro Gln Glu Leu
            180                 185                 190

Lys Phe Pro Glu Ile Gln Trp Gln Ser Phe Val Ser Ala Phe Glu Leu
        195                 200                 205

Lys Thr His Ala Leu Lys Met Glu Gly Gly Ala Gln Ile Gly Ser Gln
    210                 215                 220

Gly Trp Ala Lys Tyr Cys Phe Lys Asp Thr Glu Gln Ala Arg Ile Ala
225                 230                 235                 240

Ser Ile Leu Ser His Phe Ala Phe Tyr Ala Gly Val Gly Arg Lys Thr
                245                 250                 255

Thr Met Gly Met Gly Gln Thr Gln Leu Leu Val Asn Thr
            260                 265

<210>  29
<211>  258
<212>  PRT
<213>  Cyanothece spp

<220>
<221>  PEPTIDE
<222>  (1)..(258)
<223>  type I-D CRISPR-associated protein Cas5/Csc1: WP_015783164.1

<400>  29

Met Asn Ile Tyr Tyr Cys Gln Leu Thr Leu His Asp Asn Ile Phe Phe
1               5                   10                  15

Ala Thr Arg Glu Met Gly Leu Leu Tyr Glu Thr Glu Lys Tyr Leu His
            20                  25                  30

Asn Trp Ala Leu Ser Tyr Ala Phe Phe Lys Gly Thr Tyr Ile Pro His
        35                  40                  45

Pro Tyr Arg Leu Gln Gly Lys Ser Ala Gln Lys Pro Asp Tyr Leu Asp
    50                  55                  60

Ser Thr Gly Glu Gln Ser Leu Ala His Leu Asn Arg Leu Lys Ile Tyr
65                  70                  75                  80

Val Phe Pro Ala Lys Pro Leu Arg Trp Ser Tyr Gln Ile Asn Thr Phe
                85                  90                  95

Lys Ala Ala Gln Thr Thr Tyr Tyr Gly Lys Ser Gln Gln Phe Gly Asp
            100                 105                 110

Lys Gly Ala Asn Arg Asn Tyr Pro Ile Asn Tyr Gly Arg Ala Lys Glu
        115                 120                 125

Leu Ala Val Gly Ser Glu Tyr His Thr Phe Leu Ile Ser Ser Gln Glu
    130                 135                 140

Leu Asn Ile Pro His Trp Ile Arg Val Gly Lys Trp Ser Ala Lys Val
145                 150                 155                 160

Glu Val Thr Ser Tyr Leu Ile Pro Gln Lys Ala Ile Ser Gln His Ser
                165                 170                 175

Gly Ile Tyr Leu Cys Asp His Pro Leu Asn Pro Ile Asp Leu Pro Phe
            180                 185                 190

Asp Gln Glu Leu Leu Leu Tyr Asn Arg Ile Val Met Pro Pro Val Ser
        195                 200                 205

Leu Val Ser Gln Ala Gln Leu Gln Gly Asn Tyr Cys Lys Ile Asn Lys
    210                 215                 220

Asn Asn Trp Asn Asp Cys Pro Ser Asn Leu Thr Asp Leu Pro Gln Gln
225                 230                 235                 240

Ile Cys Leu Pro Leu Gly Val Asn Tyr Gly Ala Gly Tyr Ile Ala Ser
                245                 250                 255

Ala Ser

<210>  30
<211>  356
<212>  PRT

<213>  Cyanothece spp

<220>
<221>  PEPTIDE
<222>  (1)..(356)
<223>  type I-D CRISPR-associated protein Cas7/Csc2:WP_015783165.1

<400>  30

Met Ser Ile Leu Glu Thr Leu Lys Pro Gln Phe Gln Ser Ala Phe Pro
1               5                   10                  15

Arg Leu Ala Ser Ala Asn Tyr Val His Phe Ile Met Leu Arg His Ser
            20                  25                  30

Gln Ser Phe Pro Val Phe Gln Thr Asp Gly Val Leu Asn Thr Val Arg
        35                  40                  45

Thr Gln Ala Gly Leu Met Ala Lys Asp Ser Leu Ser Arg Leu Val Met
    50                  55                  60

Phe Lys Arg Lys Gln Thr Thr Pro Glu Arg Leu Thr Gly Arg Glu Leu
65                  70                  75                  80

Leu Arg Ser Leu Asn Ile Thr Thr Ala Asp Lys Asn Asp Lys Glu Lys
                85                  90                  95

Gly Cys Glu Tyr Asn Gly Glu Gly Ser Cys Lys Lys Cys Pro Asp Cys
            100                 105                 110

Ile Ile Tyr Gly Phe Ala Ile Gly Asp Ser Gly Ser Glu Arg Ser Lys
        115                 120                 125

Val Tyr Ser Asp Ser Thr Phe Ser Leu Ser Ala Tyr Glu Gln Ser His
    130                 135                 140

Arg Thr Phe Thr Phe Asn Ala Pro Phe Glu Gly Gly Thr Met Ser Glu
145                 150                 155                 160

Gln Gly Val Met Arg Ser Ala Ile Asn Glu Leu Asp His Ile Leu Pro
                165                 170                 175

Glu Ile Thr Phe Pro Asn Ile Glu Thr Leu Arg Asp Ser Thr Tyr Glu
            180                 185                 190

Gly Phe Ile Tyr Val Leu Gly Asn Ile Leu Arg Thr Lys Arg Tyr Gly
        195                 200                 205

Ala Gln Glu Ser Arg Thr Gly Thr Met Lys Asn His Leu Val Gly Ile

210                 215                 220

Ala Phe Cys Asp Gly Glu Ile Phe Ser Asn Leu Arg Phe Thr Gln Ala
225                 230                 235                 240

Leu Tyr Asp Gly Leu Glu Gly Asp Val Asn Lys Pro Ile Asp Glu Ile
                245                 250                 255

Cys Tyr Gln Ala Ser Gln Ile Val Gln Thr Leu Leu Ser Asp Glu Pro
            260                 265                 270

Val Arg Lys Ile Lys Thr Ile Phe Gly Glu Glu Leu Asn His Leu Ile
        275                 280                 285

Asn Glu Val Ser Gly Ile Tyr Gln Asn Asp Ala Leu Leu Thr Glu Thr
    290                 295                 300

Leu Asn Met Leu Tyr Gln Gln Thr Lys Thr Tyr Ser Glu Asn His Gly
305                 310                 315                 320

Ser Leu Ala Lys Ser Lys Pro Pro Lys Ala Glu Gly Asn Lys Ser Lys
                325                 330                 335

Gly Arg Thr Lys Lys Lys Gly Asp Asp Glu Gln Thr Ser Leu Asp Leu
            340                 345                 350

Asn Ile Glu Glu
        355

<210>  31
<211>  974
<212>  PRT
<213>  Cyanothece spp

<220>
<221>  PEPTIDE
<222>  (1)..(974)
<223>  type I-D CRISPR-associated protein Cas10d/Csc3: WP_015783166.1

<400>  31

Met Thr Leu Leu Gln Ile Leu Leu Leu Glu Thr Ile Ser Gln Asp Thr
1               5                   10                  15

Asp Pro Ile Leu Ile Ser Tyr Leu Glu Thr Val Leu Pro Ala Met Glu
            20                  25                  30

Pro Glu Phe Ala Leu Ile Pro Ala Leu Gly Gly Ser Gln Gln Ile His
        35                  40                  45

Tyr Gln Asn Leu Ile Ala Ile Gly Asn Arg Tyr Ala Gln Glu Asn Ala
    50                  55                  60

Lys Arg Phe Ser Asp Lys Ala Asp Gln Asn Leu Leu Val His Val Leu
65                  70                  75                  80

Asn Ala Leu Leu Thr Ala Trp Asn Leu Val Asp His Leu Thr Lys Pro
                85                  90                  95

Leu Ser Asp Ile Glu Lys Tyr Leu Leu Cys Leu Gly Leu Thr Leu His
            100                 105                 110

Asp Tyr Asn Lys Tyr Cys Leu Gly His Gly Glu Glu Ser Pro Lys Val
        115                 120                 125

Ser Asn Ile Asn Glu Ile Ile Asn Ile Cys Gln Glu Leu Gly Lys Lys
    130                 135                 140

Leu Asn Phe Gln Ala Phe Trp Ser Asp Trp Glu Gln Tyr Leu Pro Glu
145                 150                 155                 160

Ile Val Tyr Leu Ala Gln Asn Thr Gln Phe Lys Ala Gly Thr Asn Ala
                165                 170                 175

Ile Pro Ala Asn Tyr Pro Leu Phe Thr Leu Ala Asp Ser Arg Arg Leu
            180                 185                 190

Asp Leu Pro Leu Arg Arg Leu Leu Ala Phe Gly Asp Ile Ala Val His
        195                 200                 205

Leu Gln Asp Pro Ala Asp Ile Ile Ser Lys Thr Gly Gly Asp Arg Leu
    210                 215                 220

Arg Glu His Leu Arg Phe Leu Gly Ile Lys Lys Ala Leu Val Tyr His
225                 230                 235                 240

Arg Leu Arg Asp Thr Leu Gly Ile Leu Ser Asn Gly Ile His Asn Ala
                245                 250                 255

Thr Leu Arg Phe Ala Lys Asp Leu Asn Trp Gln Pro Leu Leu Phe Phe
            260                 265                 270

Ala Gln Gly Val Ile Tyr Leu Ala Pro Ile Asp Tyr Thr Ser Pro Glu
        275                 280                 285

Lys Met Glu Leu Gln Gly Phe Ile Trp Gln Glu Ile Ser Gln Leu Leu
    290                 295                 300

Ala Ser Ser Met Leu Lys Gly Glu Ile Gly Phe Lys Arg Asp Gly Lys
305                 310                 315                 320

Gly Leu Lys Val Ala Pro Gln Thr Leu Glu Leu Phe Thr Pro Val Gln
                325                 330                 335

Leu Ile Arg Asn Leu Ala Asp Val Ile Asn Val Lys Val Ala Asn Ala
            340                 345                 350

Lys Val Pro Ala Thr Pro Lys Arg Leu Glu Lys Leu Glu Leu Thr Asp
        355                 360                 365

Ile Glu Arg Gln Leu Leu Glu Lys Gly Ala Asp Leu Arg Ala Asp Arg
    370                 375                 380

Ile Ala Glu Leu Ile Ile Leu Ala Gln Arg Glu Phe Leu Ala Asp Ser
385                 390                 395                 400

Pro Glu Phe Ile Asp Trp Thr Leu Gln Phe Trp Gly Leu Glu Lys Gln
                405                 410                 415

Ile Thr Ala Glu Gln Thr Gln Glu Gln Ser Gly Gly Val Asn Tyr Gly
            420                 425                 430

Trp Tyr Arg Val Ala Ala Asn Tyr Ile Ala Asn His Ser Thr Leu Ser
        435                 440                 445

Leu Glu Asp Val Ser Gly Lys Leu Val Asp Phe Cys Gln Gln Leu Ala
    450                 455                 460

Asp Trp Ala Thr Ser Asn Gln Leu Leu Ser Ser His Ser Ser Ser Thr
465                 470                 475                 480

Phe Glu Val Phe Asn Ser Tyr Leu Glu Gln Tyr Leu Glu Ile Gln Gly
                485                 490                 495

Trp Gln Ser Ser Thr Pro Asn Phe Ser Gln Glu Leu Ser Thr Tyr Ile
            500                 505                 510

Met Ala Lys Thr Gln Ser Ser Lys Gln Pro Ile Cys Ser Leu Ser Ser
        515                 520                 525

Gly Glu Phe Ile Ser Glu Asp Gln Met Asp Ser Val Val Leu Phe Lys
    530                 535                 540

Pro Gln Gln Tyr Ser Asn Lys Asn Pro Leu Gly Gly Gly Lys Ile Lys

545                 550                 555                 560

Arg Gly Ile Ser Lys Ile Trp Ala Leu Glu Met Leu Leu Arg Gln Ala
                565                 570                 575

Leu Trp Thr Val Pro Ser Gly Lys Phe Glu Asp Gln Gln Pro Val Phe
            580                 585                 590

Leu Tyr Ile Phe Pro Ala Tyr Val Tyr Ser Pro Gln Ile Ala Ala Ala
        595                 600                 605

Ile Arg Ser Leu Val Asn Asp Met Lys Arg Ile Asn Leu Trp Asp Val
    610                 615                 620

Arg Lys His Trp Leu His Glu Asp Met Asn Leu Asp Ser Leu Arg Ser
625                 630                 635                 640

Leu Gln Trp Arg Lys Glu Glu Ala Glu Val Gly Arg Phe Lys Asp Lys
                645                 650                 655

Tyr Ser Arg Ala Asp Ile Pro Phe Met Gly Thr Val Tyr Thr Thr Thr
            660                 665                 670

Arg Gly Lys Thr Leu Thr Glu Ala Trp Ile Asp Pro Ala Phe Leu Thr
        675                 680                 685

Leu Ala Leu Pro Ile Leu Leu Gly Val Lys Val Ile Ala Thr Ser Ser
    690                 695                 700

Ser Val Pro Leu Tyr Asn Ser Asp Asn Asp Phe Leu Asp Ser Val Ile
705                 710                 715                 720

Leu Asp Ala Pro Ala Gly Phe Trp Gln Leu Leu Lys Leu Ser Thr Ser
                725                 730                 735

Leu Arg Ile Gln Glu Leu Ser Val Ala Leu Lys Arg Leu Leu Thr Ile
            740                 745                 750

Tyr Thr Ile His Leu Asp Asn Arg Ser Asn Pro Pro Asp Ala Arg Trp
        755                 760                 765

Gln Ala Leu Asn Ser Thr Val Arg Glu Val Ile Thr Asp Val Leu Asn
    770                 775                 780

Val Phe Ser Ile Ala Asp Glu Lys Leu Arg Glu Asp Gln Arg Glu Ala
785                 790                 795                 800

Ser Pro Gln Glu Val Gln Arg Tyr Trp Lys Phe Ala Glu Ile Phe Ala
                805                 810                 815

Gln Gly Asp Thr Ile Met Thr Glu Lys Leu Lys Leu Thr Lys Glu Leu
            820                 825                 830

Val Arg Gln Tyr Arg Thr Phe Tyr Gln Val Lys Trp Ser Glu Ser Ser
        835                 840                 845

His Thr Ile Leu Leu Pro Leu Thr Lys Ala Leu Glu Glu Ile Leu Ser
    850                 855                 860

Thr Pro Glu His Trp Asp Asp Glu Glu Leu Ile Leu Gln Gly Ala Gly
865                 870                 875                 880

Ile Leu Asn Asp Ala Leu Asp Arg Gln Glu Val Tyr Lys Arg Pro Leu
                885                 890                 895

Leu Gln Asp Lys Ser Ile Pro Tyr Glu Ile Arg Lys Gln Gln Glu Leu
            900                 905                 910

Gln Ala Ile His Gln Phe Met Thr Thr Cys Val Lys Glu Leu Phe Gly
        915                 920                 925

Gln Met Cys Lys Gly Asp Arg Ala Leu Leu Gln Glu Tyr Arg Asn Arg
    930                 935                 940

Ile Lys Ser Gly Ala Glu Ser Ala Tyr Lys Leu Leu Ala Phe Glu Glu
945                 950                 955                 960

Lys Ser Asn Ser Ser Gln Gln Gln Lys Ser Ser Glu Asp Gln
                965                 970

<210>  32
<211>  888
<212>  PRT
<213>  Escherichia coli

<220>
<221>  PEPTIDE
<222>  (1)..(888)
<223>  Cascade complex anti-viral R-loop helicase-annealase
       Cas3:NP_417241.1

<400>  32

Met Glu Pro Phe Lys Tyr Ile Cys His Tyr Trp Gly Lys Ser Ser Lys
1               5                   10                  15

Ser Leu Thr Lys Gly Asn Asp Ile His Leu Leu Ile Tyr His Cys Leu

20                  25                  30

Asp Val Ala Ala Val Ala Asp Cys Trp Trp Asp Gln Ser Val Val Leu
        35                  40                  45

Gln Asn Thr Phe Cys Arg Asn Glu Met Leu Ser Lys Gln Arg Val Lys
    50                  55                  60

Ala Trp Leu Leu Phe Phe Ile Ala Leu His Asp Ile Gly Lys Phe Asp
65                  70                  75                  80

Ile Arg Phe Gln Tyr Lys Ser Ala Glu Ser Trp Leu Lys Leu Asn Pro
                85                  90                  95

Ala Thr Pro Ser Leu Asn Gly Pro Ser Thr Gln Met Cys Arg Lys Phe
            100                 105                 110

Asn His Gly Ala Ala Gly Leu Tyr Trp Phe Asn Gln Asp Ser Leu Ser
        115                 120                 125

Glu Gln Ser Leu Gly Asp Phe Phe Ser Phe Phe Asp Ala Ala Pro His
    130                 135                 140

Pro Tyr Glu Ser Trp Phe Pro Trp Val Glu Ala Val Thr Gly His His
145                 150                 155                 160

Gly Phe Ile Leu His Ser Gln Asp Gln Asp Lys Ser Arg Trp Glu Met
                165                 170                 175

Pro Ala Ser Leu Ala Ser Tyr Ala Ala Gln Asp Lys Gln Ala Arg Glu
            180                 185                 190

Glu Trp Ile Ser Val Leu Glu Ala Leu Phe Leu Thr Pro Ala Gly Leu
        195                 200                 205

Ser Ile Asn Asp Ile Pro Pro Asp Cys Ser Ser Leu Leu Ala Gly Phe
    210                 215                 220

Cys Ser Leu Ala Asp Trp Leu Gly Ser Trp Thr Thr Thr Asn Thr Phe
225                 230                 235                 240

Leu Phe Asn Glu Asp Ala Pro Ser Asp Ile Asn Ala Leu Arg Thr Tyr
                245                 250                 255

Phe Gln Asp Arg Gln Gln Asp Ala Ser Arg Val Leu Glu Leu Ser Gly
            260                 265                 270

Leu Val Ser Asn Lys Arg Cys Tyr Glu Gly Val His Ala Leu Leu Asp
        275                 280                 285

Asn Gly Tyr Gln Pro Arg Gln Leu Gln Val Leu Val Asp Ala Leu Pro
    290                 295                 300

Val Ala Pro Gly Leu Thr Val Ile Glu Ala Pro Thr Gly Ser Gly Lys
305                 310                 315                 320

Thr Glu Thr Ala Leu Ala Tyr Ala Trp Lys Leu Ile Asp Gln Gln Ile
                325                 330                 335

Ala Asp Ser Val Ile Phe Ala Leu Pro Thr Gln Ala Thr Ala Asn Ala
            340                 345                 350

Met Leu Thr Arg Met Glu Ala Ser Ala Ser His Leu Phe Ser Ser Pro
        355                 360                 365

Asn Leu Ile Leu Ala His Gly Asn Ser Arg Phe Asn His Leu Phe Gln
    370                 375                 380

Ser Ile Lys Ser Arg Ala Ile Thr Glu Gln Gly Gln Glu Glu Ala Trp
385                 390                 395                 400

Val Gln Cys Cys Gln Trp Leu Ser Gln Ser Asn Lys Lys Val Phe Leu
                405                 410                 415

Gly Gln Ile Gly Val Cys Thr Ile Asp Gln Val Leu Ile Ser Val Leu
            420                 425                 430

Pro Val Lys His Arg Phe Ile Arg Gly Leu Gly Ile Gly Arg Ser Val
        435                 440                 445

Leu Ile Val Asp Glu Val His Ala Tyr Asp Thr Tyr Met Asn Gly Leu
    450                 455                 460

Leu Glu Ala Val Leu Lys Ala Gln Ala Asp Val Gly Gly Ser Val Ile
465                 470                 475                 480

Leu Leu Ser Ala Thr Leu Pro Met Lys Gln Lys Gln Lys Leu Leu Asp
                485                 490                 495

Thr Tyr Gly Leu His Thr Asp Pro Val Glu Asn Asn Ser Ala Tyr Pro
            500                 505                 510

Leu Ile Asn Trp Arg Gly Val Asn Gly Ala Gln Arg Phe Asp Leu Leu
        515                 520                 525

Ala His Pro Glu Gln Leu Pro Pro Arg Phe Ser Ile Gln Pro Glu Pro
    530                 535                 540

Ile Cys Leu Ala Asp Met Leu Pro Asp Leu Thr Met Leu Glu Arg Met
545                 550                 555                 560

Ile Ala Ala Ala Asn Ala Gly Ala Gln Val Cys Leu Ile Cys Asn Leu
                565                 570                 575

Val Asp Val Ala Gln Val Cys Tyr Gln Arg Leu Lys Glu Leu Asn Asn
            580                 585                 590

Thr Gln Val Asp Ile Asp Leu Phe His Ala Arg Phe Thr Leu Asn Asp
        595                 600                 605

Arg Arg Glu Lys Glu Asn Arg Val Ile Ser Asn Phe Gly Lys Asn Gly
    610                 615                 620

Lys Arg Asn Val Gly Arg Ile Leu Val Ala Thr Gln Val Val Glu Gln
625                 630                 635                 640

Ser Leu Asp Val Asp Phe Asp Trp Leu Ile Thr Gln His Cys Pro Ala
                645                 650                 655

Asp Leu Leu Phe Gln Arg Leu Gly Arg Leu His Arg His His Arg Lys
            660                 665                 670

Tyr Arg Pro Ala Gly Phe Glu Ile Pro Val Ala Thr Ile Leu Leu Pro
        675                 680                 685

Asp Gly Glu Gly Tyr Gly Arg His Glu His Ile Tyr Ser Asn Val Arg
    690                 695                 700

Val Met Trp Arg Thr Gln Gln His Ile Glu Glu Leu Asn Gly Ala Ser
705                 710                 715                 720

Leu Phe Phe Pro Asp Ala Tyr Arg Gln Trp Leu Asp Ser Ile Tyr Asp
                725                 730                 735

Asp Ala Glu Met Asp Glu Pro Glu Trp Val Gly Asn Gly Met Asp Lys
            740                 745                 750

Phe Glu Ser Ala Glu Cys Glu Lys Arg Phe Lys Ala Arg Lys Val Leu
        755                 760                 765

Gln Trp Ala Glu Glu Tyr Ser Leu Gln Asp Asn Asp Glu Thr Ile Leu
    770                 775                 780

Ala Val Thr Arg Asp Gly Glu Met Ser Leu Pro Leu Leu Pro Tyr Val
785                 790                 795                 800

Gln Thr Ser Ser Gly Lys Gln Leu Leu Asp Gly Gln Val Tyr Glu Asp
                805                 810                 815

Leu Ser His Glu Gln Gln Tyr Glu Ala Leu Ala Leu Asn Arg Val Asn
            820                 825                 830

Val Pro Phe Thr Trp Lys Arg Ser Phe Ser Glu Val Val Asp Glu Asp
        835                 840                 845

Gly Leu Leu Trp Leu Glu Gly Lys Gln Asn Leu Asp Gly Trp Val Trp
    850                 855                 860

Gln Gly Asn Ser Ile Val Ile Thr Tyr Thr Gly Asp Glu Gly Met Thr
865                 870                 875                 880

Arg Val Ile Pro Ala Asn Pro Lys
                885

<210>  33
<211>  94
<212>  PRT
<213>  Escherichia coli

<220>
<221>  PEPTIDE
<222>  (1)..(94)
<223>  CRISPR adaptation ssRNA endonuclease: NP_417234.2

<400>  33

<210> 34
<211> 328
<212> PRT
<213> Yersinia pseudotuberculosis

<220>
<221> PEPTIDE
<222> (1)..(328)
<223> type I-F CRISPR-associated endonuclease Cas1: WP_072080408.1

<400> 34

Met Ala Met Glu Asn Ala Ile His Ser Ser Asp Leu Lys Thr Ile Leu
1               5                   10                  15

His Ser Lys Arg Ser Asn Ile Tyr Tyr Leu Glu Tyr Cys Arg Val Leu
            20                  25                  30

Val Asn Gly Gly Arg Val Glu Tyr Val Thr Asp Glu Gly Lys Gln Ser
        35                  40                  45

Leu Tyr Trp Asn Ile Pro Ile Ala Asn Thr Thr Val Ile Met Leu Gly
    50                  55                  60

Thr Gly Thr Ser Val Thr Gln Ala Ala Met Arg Glu Phe Ala Arg Ala
65                  70                  75                  80

Gly Val Leu Val Gly Phe Cys Gly Gly Gly Gly Thr Pro Leu Phe Ala
                85                  90                  95

Ala Asn Asp Val Glu Val Asn Val Ser Trp Leu Thr Ala Gln Ser Glu
            100                 105                 110

Tyr Arg Pro Thr Glu Tyr Leu His Asp Trp Val Ser Phe Trp Phe Asp
        115                 120                 125

Asp Glu Lys Arg Leu Ala Ala Ala Val Ala Phe Gln Arg Ile Arg Ile
    130                 135                 140

Ala Gln Ile Gln Gln His Trp Leu Ser Ser His Ile Gln Arg Glu Ser
145                 150                 155                 160

Leu Phe Pro Val Asn His Asp Gln Leu Leu Phe Ile Leu Ser Arg Phe
                165                 170                 175

Glu Gln Asn Leu Ala Asn Cys Leu Thr Ser Asn Asp Leu Met Val Gln
            180                 185                 190

Glu Ala Val Leu Thr Lys Ala Leu Tyr Lys Leu Ala Ala Asn Thr Val
        195                 200                 205

Asn Tyr Gly Asp Phe Thr Arg Ala Lys Arg Gly Gly Gly Ile Asp Leu
    210                 215                 220

Ala Asn Arg Phe Leu Asp His Gly Asn Tyr Leu Ala Tyr Gly Leu Ala
225                 230                 235                 240

Ala Thr Ala Thr Trp Val Ile Gly Leu Pro His Gly Leu Ser Val Leu
                245                 250                 255

His Gly Lys Thr Arg Arg Gly Gly Leu Val Phe Asp Val Ala Asp Leu
            260                 265                 270

Ile Lys Asp Ala Leu Val Leu Pro Gln Ala Phe Ile Ala Ala Met Gln
        275                 280                 285

Gly Glu Glu Glu Gln Glu Phe Arg Gln Arg Cys Ile Ser Gly Phe Gln
    290                 295                 300

Arg Thr Glu Ala Leu Asp Val Met Ile Asp Gly Ile Lys Glu Thr Ala
305                 310                 315                 320

Ala Leu Cys Ser Gln Val Pro Arg
                325

<210>  35
<211>  1095
<212>  PRT
<213>  Yersinia pseudotuberculosis

<220>
<221>  PEPTIDE
<222>  (1)..(1095)
<223>  type I-F CRISPR-associated helicase Cas3: WP_012303962.1

<400>  35

Met Asn Ile Leu Leu Ile Ser Gln Cys His Lys Arg Ala Leu Asp Glu
1               5                   10                  15

Thr Arg Arg Ile Leu Asp Gln Phe Ala Glu Arg Lys Gly Asp Arg Thr
            20                  25                  30

Trp Gln Thr Ala Ile Thr Gln Glu Gly Leu Asn Thr Leu Arg Lys Leu
        35                  40                  45

Leu Arg Arg Thr Ala Arg Arg Asn Ser Ala Val Ala Cys His Trp Ile
    50                  55                  60

Lys Ser Asn Asn Gln Ser Glu Leu Leu Trp Ile Val Gly Asn Leu Arg
65                  70                  75                  80

Arg Phe Asn Ala Glu Gly Thr Val Pro Thr Asn Thr Thr Gln Arg Asp
                85                  90                  95

Ile Leu Arg Ser Gln Asp Glu Asn Asn Trp His Ser Val Glu Thr Ile
            100                 105                 110

Ala Leu Leu Ala Gly Ile Ala Gly Leu Phe His Asp Phe Gly Lys Ala
        115                 120                 125

Asn Val Leu Phe Gln Gln Gly Leu Gln Gly Gln Gly Lys Thr Tyr Gln
    130                 135                 140

Pro Tyr Arg His Glu Trp Val Ser Leu Arg Leu Phe Gln Ala Phe Val
145                 150                 155                 160

Glu Gln Lys Asn Asp Ile Gln Trp Leu Thr Ala Leu Ser Gln Ile Asn
                165                 170                 175

Pro Ala Asp Glu Thr Ala Leu Met Ser Asn Leu Val Lys Asp Gly Leu
            180                 185                 190

Asp Asp Tyr Thr Asn Pro Leu Ala Ala Leu Pro Pro Leu Ala Lys Thr
        195                 200                 205

Ile Ala Trp Leu Ile Leu Ser His His Arg Leu Pro Val Tyr Pro Ser
    210                 215                 220

Lys Asp Tyr His Arg Pro Pro Lys Val Glu Glu Ile Asp Asp Trp Leu
225                 230                 235                 240

His Gln Gln Leu Met Pro Val Trp Asn Ser Thr Asn Met Asp Ser Gly
                245                 250                 255

Asp Trp Arg Glu Lys Asp Lys Leu Asp Val Trp Arg Phe Pro His Gly
            260                 265                 270

Thr Pro Leu Gln Ser Ala Glu Trp Cys Lys Lys Ala Gln Lys Phe Ala
        275                 280                 285

Lys Arg Ala Leu Arg Gln Pro Ser Leu Val Asn Asp Gly His Leu Asn
    290                 295                 300

Gln Arg Phe Thr Ser His Met Ala Arg Leu Val Leu Met Leu Ala Asp
305                 310                 315                 320

His His Tyr Ser Ser Leu Pro Ala Thr Pro Gly Trp Gln Asp Lys Asn
                325                 330                 335

Tyr Gln Val Phe Ala Asn Thr His Asn Lys Met Arg Asp Ala Asn Ala
            340                 345                 350

His Ser Lys Thr Ile Glu Met Lys Gln Arg Leu Asp Glu His Asn Ile
        355                 360                 365

Gly Val Gly Gln Asn Ala Leu Leu Leu Gly Arg Ser Leu Pro His Ile
    370                 375                 380

Arg Lys Thr Leu Pro Ala Ile Thr Arg His Lys Gly Phe Lys Gln Arg
385                 390                 395                 400

Ser Val Gln Glu Lys Tyr Arg Trp Gln Asp Lys Ala Phe Asp Leu Ala
                405                 410                 415

Val Thr Leu Arg Glu Arg Ser Leu Gln Gln Gly Phe Phe Gly Val Asn
            420                 425                 430

Met Ala Ser Thr Gly Cys Gly Lys Thr Phe Ala Asn Ala Arg Ile Met
        435                 440                 445

Tyr Gly Leu Ser Asp Glu Lys Gln Gly Cys Arg Phe Ser Val Ala Leu
    450                 455                 460

Gly Leu Arg Thr Leu Thr Leu Gln Thr Gly Thr Ala Leu Gln Gln Lys
465                 470                 475                 480

Leu Thr Leu Gln Lys Asp Asp Leu Ala Val Leu Ile Gly Ser Gln Ser
                485                 490                 495

Val Leu Gln Leu Tyr Gln Leu Asn Asn Asp Lys Leu Glu Pro Arg Gly
            500                 505                 510

Thr Gly Ser Gln Ser Ala Asp Lys Phe Phe Ser Glu His Gln Tyr Val
        515                 520                 525

Ser Tyr Asp Gly Ser Leu Asp Asp Gly Arg Leu Ser Thr Trp Leu Ser
    530                 535                 540

Asn Asn Asp Arg Leu Lys Arg Leu Leu Cys Ala Pro Val Leu Val Cys
545                 550                 555                 560

Thr Ile Asp His Leu Ile Pro Ala Thr Glu Ser Leu Arg Gly Gly Lys
                565                 570                 575

Gln Ile Ala Pro Met Leu Arg Leu Leu Thr Ser Asp Leu Val Leu Asp
            580                 585                 590

Glu Pro Asp Asp Phe Asp Arg Asn Asp Leu Pro Ala Leu Cys Arg Leu
        595                 600                 605

Val Asn Trp Ala Gly Met Leu Gly Ser Arg Val Leu Leu Ser Ser Ala
    610                 615                 620

Thr Leu Pro Pro Ala Leu Val Gln Ala Leu Phe Asn Ala Tyr Arg Ala
625                 630                 635                 640

Gly Arg Ala Asp Tyr Gln Gln Ala Ser Gly Gln Pro Asp Thr Pro Val
                645                 650                 655

Asn Ile Cys Cys Ala Trp Phe Asp Glu His Gly Val Ala Gln Gln Asp
            660                 665                 670

Ile Gln Gln Ser Lys Asp Phe Ile Gln His His Gly Asp Phe Ile Thr
        675                 680                 685

Arg Arg Val Gly Lys Leu Ser Thr Ala Glu Val Leu Arg Arg Ala Glu
    690                 695                 700

Leu Ile Pro Val Ser Ser Ala Ser Val Lys Asn Gly Ser Val Leu Asp
705                 710                 715                 720

Ala Met Ala Ala Val Met Arg Asp Ser Met Leu Arg Leu His Gln Ala
                725                 730                 735

His His Gln Thr His Pro Ser Glu Lys Thr Val Ser Val Gly Val Val
            740                 745                 750

Arg Met Ala Asn Ile Asp Pro Leu Val Ala Ile Ala Gln Arg Leu Tyr
        755                 760                 765

Ala Met Ala Ala Pro Glu Asn Tyr Arg Ile His Tyr Cys Val Tyr His
    770                 775                 780

Ser Gln His Pro Leu Ala Val Arg Ser Ala Ile Glu Arg Arg Leu Asp
785                 790                 795                 800

Val Thr Leu Thr Arg Tyr Gln Pro Asn Ala Leu Trp Gln Val Ala Glu

                805                 810                 815

Ile Glu His Ala Leu Gln His His Pro Glu Gln His His Leu Phe Val
            820                 825                 830

Val Leu Ala Thr Ser Val Val Glu Val Gly Arg Asp His Asp Tyr Asp
        835                 840                 845

Trp Ala Leu Ala Glu Pro Ser Ser Met Arg Ser Phe Ile Gln Leu Ala
    850                 855                 860

Gly Arg Val Gln Arg His Arg Gln Val Ala Pro Gln Met Ala Asn Met
865                 870                 875                 880

His Ile Leu Gln Lys Asn Phe Lys Ala Leu Thr Gln Lys Asp Pro Ala
                885                 890                 895

Ile Pro Val Tyr Cys Lys Pro Gly Phe Glu Thr Thr Arg Cys Arg Leu
            900                 905                 910

Asn Thr His Asp Leu Asp Lys Leu Leu Leu Pro Ser Gln Tyr Lys Val
        915                 920                 925

Ile Asn Ala Ile Ser Arg Ile Gln Glu Arg Thr Lys Leu Glu Pro Arg
    930                 935                 940

Asp Asn Phe Val Asp Leu Glu His Leu Ser Leu Arg Leu Ile Leu Gln
945                 950                 955                 960

Gly Ser Ala Glu Pro Leu Lys Tyr Tyr Ala Ala Leu Trp Trp Arg Lys
                965                 970                 975

Gln Ala Thr Trp Ser Gly Glu Gln Gln Arg Cys Thr Pro Phe Arg Gln
            980                 985                 990

Ser Ser Pro Asp Glu Gln His Asn Leu Trp Ile Asp Asp Glu Ala Asp
        995                 1000                1005

Lys Pro Val Phe Lys Arg Leu Asp Ser Asp Gln Ile Glu Trp Lys
    1010                1015                1020

Val Ser Asp Gly Phe Gln Asp Val Glu Leu Thr Leu Ala Val Gly
    1025                1030                1035

Asn Ser Ala Trp Ile Asp Thr Asp Cys Leu Arg Ile Tyr Gln Trp
    1040                1045                1050

Leu Ala Glu Ser Leu Asn Lys Glu Leu Asn Glu Val Ser Arg Gln
    1055                1060                1065

Phe Gly Glu Val Arg Leu Pro Lys Lys Asp Gly Glu Arg Trp His
    1070                1075                1080

Tyr His Pro Leu Leu Gly Gly Phe Gly Ala Leu Asp
    1085                1090                1095

<210> 36
<211> 448
<212> PRT
<213> Yersinia pseudotuberculosis

<220>
<221> PEPTIDE
<222> (1)..(448)
<223> type I-F CRISPR-associated protein Csy1: WP_012303963.1

<400> 36

Met Glu Glu Lys Gly Leu Thr Ala Phe Ile Val Ser Tyr Ile Lys Ser
1               5                   10                  15

Arg Glu Gln Pro Lys Leu Glu Ala Phe Asp Lys Glu Ala Glu Lys Arg
            20                  25                  30

Leu Ala Gly Leu Thr Gln Ala Glu Asp Ile Ala Leu Ala Gln Gln Glu
        35                  40                  45

Ile Ala Gln Gln Arg Gln Glu Leu Val Ala Arg Tyr Glu Val Arg Asn
    50                  55                  60

Trp Leu Thr Asp Ala Ala Asn Arg Ala Gly Gln Ile Ser Leu Val Thr
65                  70                  75                  80

His Ala Leu Lys Phe Thr His Ser Asp Ala Lys Gly Ser Ser Val Phe
                85                  90                  95

Asn Leu Pro Ala Ala Glu Asn Asp Ala Ile Tyr Leu Ser Ser Ala Thr
            100                 105                 110

Leu Gly Gln Pro Ala Val Asp Ala Val Gly Asn Ala Ala Ala Leu Asp
        115                 120                 125

Val Ala Lys Leu Leu Gln Thr Glu His Asn Gly Asp Ser Leu Leu Ala
    130                 135                 140

Cys Leu Asn Arg Ala Asp Asn Ser Pro Leu Ala Ala Leu Ala Glu Asn
145                 150                 155                 160

Pro Gln Gln Leu Ala Leu Trp Val Glu Gly Phe Lys Gln Ala Leu Val
                165                 170                 175

Asp Lys Gln Leu Thr Ser His Lys Leu Ala Lys Gln Phe Tyr Phe Pro
            180                 185                 190

Val Gly Pro Asp Gln Tyr His Leu Leu Ser Pro Leu Phe Ser Ser Ser
        195                 200                 205

Leu Ala Gln Ala Met His Gln Arg Ile Ala Glu Ala Arg Phe Ser Asp
    210                 215                 220

Gln Ser Lys Glu Ala Lys Val Ala His Lys Ala Gly Lys Trp His Ser
225                 230                 235                 240

Glu Ala Arg Val Ile Tyr Leu Lys Thr Ala Val Gln Asn Ile Gly Gly
                245                 250                 255

Thr Lys Pro Gln Asn Ile Ser Tyr Leu Asn Ser Val Arg Gly Gly Lys
            260                 265                 270

Val Trp Leu Leu Pro Cys Gly Ala Pro Pro Trp Lys Asn Ile Gln Lys
        275                 280                 285

Pro Pro Ile Lys Tyr Arg Ser Ile Phe His Asp Arg Ser Glu Phe Thr
    290                 295                 300

Val Leu Ala Arg Asn Asn Leu Trp Gln Met Gln Gln Tyr Leu Leu Gly
305                 310                 315                 320

Val Lys Pro Leu Ser Asn Thr Met Asp Ile Arg Ala Val Arg Leu Ala
                325                 330                 335

Cys Ser Asp Glu Ile Ile Asp Ile Leu Phe Asn Tyr Val Ala Glu Ile
            340                 345                 350

Gln Asn Leu Asp Gly Thr Asn Gly Trp Ser Asp Ala Glu Asp Cys Lys
        355                 360                 365

Leu Lys Arg Ser Glu Gln Leu Trp Leu Asp Pro Asn Arg Ala Met Asp
    370                 375                 380

Asp Pro Val Phe Lys Leu Glu Arg Glu Lys Glu Asp Trp Lys Gln Glu
385                 390                 395                 400

Val Ser Gln Asp Phe Gly Tyr Trp Leu Asn Arg Trp Leu Gln His Asp

405                 410                 415

Glu Leu Val Phe Gly Tyr Val Glu Gln Arg Glu Trp Ser Ser Leu Phe
            420                 425                 430

Lys Gln Arg Leu Arg Glu Phe Glu Gln Gly Ser Leu Glu Thr Trp Ser
        435                 440                 445

<210>  37
<211>  316
<212>  PRT
<213>  Yersinia pseudotuberculosis

<220>
<221>  PEPTIDE
<222>  (1)..(316)
<223>  type I-F CRISPR-associated protein Csy2: WP_012303964.1

<400>  37

Met Ser Ser Leu Ile Val Leu His Gln Val Arg Val Glu Asn Ala Asn
1               5                   10                  15

Ala Ile Ala Gly Leu Thr Tyr Gly Phe Pro Ala Ile Thr His Phe Leu
            20                  25                  30

Gly Tyr Ser His Ala Leu Ser Arg Lys Leu Gln Ser Ser His Gly Leu
        35                  40                  45

Thr Leu Arg Gly Cys Ala Val Ile Cys His His Gln Gln Val His Ala
    50                  55                  60

Tyr Ser Ser Gly Arg Asp Tyr Val Phe Ser Leu Thr Arg Asn Pro Leu
65                  70                  75                  80

Thr Lys Glu Ala Lys Thr Ala Ala Phe Asn Glu Glu Gly Arg Met His
                85                  90                  95

Met Thr Val Ser Leu Val Ile Glu Cys Asp Gly Thr Ile Ala Asn Gly
            100                 105                 110

Asp Asp Gly Ala Asn Ala Leu Glu Lys His Leu Ala Ser Leu Cys Leu
        115                 120                 125

Thr Gln Arg Leu Ala Gly Gly Thr Ile Thr Asn Ile Arg Arg Val Lys
    130                 135                 140

Val Ile Asn Tyr Pro Glu Thr Glu Ala Ala Thr Arg Arg Leu Leu Cys
145                 150                 155                 160

His Leu Leu Pro Gly Phe Ala Leu Leu Asp Arg Ser Glu Leu Leu Ala
                165                 170                 175

Glu His Leu Asn Gln Leu Arg Gln Thr Asn Pro Gln Thr Glu Met Leu
            180                 185                 190

Asp Ala Trp Leu Asp Phe Ser Ala Leu Lys Ile Gln Ala Ile Pro Pro
        195                 200                 205

Gln Ser Asp Thr Pro Val Glu Ser Gly Ala Pro Ala Pro Trp Gly Tyr
    210                 215                 220

Val Gly Lys Pro Ala Ala Gly Tyr Leu Val Pro Ile Val Thr Gly Tyr
225                 230                 235                 240

Arg Ala Ile Ser Pro Leu Tyr Ala Pro Gly Glu Val Asp Lys Thr Arg
                245                 250                 255

Asp Pro His Thr Pro Phe Arg Phe Val Glu Ser Leu Tyr Gly Ile Gly
            260                 265                 270

Glu Trp Arg Gly Leu His Arg Ile Thr Asp Leu Arg Gln Leu Leu Trp
        275                 280                 285

Gly Tyr His Gln Glu Gly Asp Asn Tyr Leu Cys Arg Cys Ala Leu Pro
    290                 295                 300

Ala Ser Val Asp Thr Tyr Leu Leu Asn Asp Glu Glu
305                 310                 315

<210>  38
<211>  334
<212>  PRT
<213>  Yersinia pseudotuberculosis

<220>
<221>  PEPTIDE
<222>  (1)..(334)
<223>  type I-F CRISPR-associated protein Csy3: WP_011192624.1

<400>  38

Met Ala Lys Ala Thr Ile Lys Thr Ala Ser Val Leu Ala Phe Glu Arg
1               5                   10                  15

Lys Leu Ser Asn Ser Asp Ala Met Met Tyr Ala Gly Asn Trp Gly Gln
            20                  25                  30

Thr Asp Asn Trp Gln Pro Ile Ile Ile Lys Glu Lys Ala Val Arg Gly

        35                  40                  45

Thr Ile Ser Asn Arg Leu Lys Asn Ala Leu Ala Ser Asp Pro Val Lys
    50                  55                  60

Leu Asp Ala Glu Ile Gln Lys Ala Asn Leu Gln Arg Val Asp Val Ala
65                  70                  75                  80

Ala Leu Pro Phe Asp Thr Asp Thr Leu Lys Met Ser Phe Thr Leu Arg
                85                  90                  95

Val Leu Gly Asn Leu Ala Thr Pro Ser Val Cys Asn Asp Gln Glu Tyr
            100                 105                 110

Gln Ala Glu Leu Ala Ser Val Ile Asn Gly Tyr Ile Ser Glu Gln Ser
        115                 120                 125

Phe Ser Val Leu Ala Ala Arg Tyr Ala Glu Asn Leu Ala Asn Gly Arg
    130                 135                 140

Phe Leu Trp Arg Asn Arg Val Gly Ala Glu Asp Ile Lys Val Cys Ile
145                 150                 155                 160

Thr Ser Gln Thr Lys Ser Tyr Gln Phe Asp Ser Asn Glu Phe Ser Leu
                165                 170                 175

Arg Gln Phe Ser Gln Pro Ser Lys Glu Leu Thr Ala Leu Ala Gln Glu
            180                 185                 190

Ile Glu Gln Gly Leu Ala Gly Asn Gly Phe Ala Phe Phe Thr Val Asp
        195                 200                 205

Ala Phe Val Arg Leu Gly Asn Gly Gln Glu Val Phe Pro Ser Gln Glu
    210                 215                 220

Leu Val Leu Asp Ser Asn Ser Lys Lys Ser Lys Val Leu Tyr Gln Ile
225                 230                 235                 240

Asp Gly Ile Ala Ala Leu His Ser Gln Lys Val Gly Asn Ala Leu Arg
                245                 250                 255

Thr Ile Asp Asp Trp Tyr Pro Asp Ala Thr Glu Leu Ala Leu Gly Pro
            260                 265                 270

Ile Ala Val Glu Pro Tyr Gly Ser Val Thr Ser Arg Gly Lys Ala Tyr
        275                 280                 285

Arg Gln Pro Lys Gln Lys Met Asp Phe Tyr Thr Leu Leu Asp Asn Trp
    290                 295                 300

Val Thr Lys Gly Thr Val Pro Ala Val Glu Gln Gln His Tyr Val Met
305                 310                 315                 320

Ala Thr Leu Ile Arg Gly Gly Val Phe Gly Glu Lys Gly Glu
                325                 330

<210>  39
<211>  184
<212>  PRT
<213>  Yersinia pseudotuberculosis

<220>
<221>  PEPTIDE
<222>  (1)..(184)
<223>  type I-F CRISPR-associated endoribonuclease  Cas6/Csy4:
       WP_002211866.1

<400>  39

Met Asp His Tyr Leu Asp Ile Arg Val Leu Pro Asp Pro Glu Phe Ser
1               5                   10                  15

Ala Gln Thr Leu Leu Glu Ala Leu Phe Ala Lys Leu His Arg Ala Leu
            20                  25                  30

Val Ala Thr Ile Pro Gly Arg Val Gly Val Ser Phe Pro Thr Ala Gly
        35                  40                  45

Lys Thr Leu Gly Ser Gln Leu Arg Leu His Gly Ser Arg Gly Asp Leu
    50                  55                  60

Leu Glu Leu Gln Ser Ala Gly Trp Leu Lys Gly Leu Gln Asp Tyr Cys
65                  70                  75                  80

Glu Cys Ser Glu Ile Leu Pro Val Pro Ala Asp Val Lys His Arg Thr
                85                  90                  95

Ile Arg Arg Val Gln Val Lys Ser Ser Ala Gln Arg Leu Arg Arg Arg
            100                 105                 110

Ser Val Ser Lys Gly Trp Leu Thr Glu Glu Gln Ala Arg Leu Arg Ile
        115                 120                 125

Pro Asp Ser His Asp Lys Arg Cys Asp Leu Pro Phe Leu Arg Leu Lys
    130                 135                 140

Ser Arg Ser Ser Glu Gln Tyr Phe Leu Leu Phe Ile Glu Gln Gly Thr

145                 150                 155                 160

Leu Gln Ala Ser Ala Thr Thr Gly Glu Phe Ser Ala Tyr Gly Leu Ser
                165                 170                 175

Val Asn Ala Thr Ile Pro Trp Phe
            180

<210>  40
<211>  325
<212>  PRT
<213>  Shewanella putrefaciens

<220>
<221>  PEPTIDE
<222>  (1)..(325)
<223>  type I-F CRISPR-associated endonuclease Cas1:WP_011789486.1

<400>  40

Met Asp Asp Phe Ser Pro Ser Asp Leu Lys Thr Ile Leu His Ser Lys
1               5                   10                  15

Arg Ala Asn Met Tyr Tyr Leu Glu Tyr Cys Arg Val Met Gln Lys Asp
            20                  25                  30

Gly Arg Val Leu Tyr Leu Thr Glu Ala Lys Asn Glu Asn Gln Tyr Phe
        35                  40                  45

Asn Ile Pro Ile Ala Asn Thr Thr Val Leu Leu Leu Gly Asn Gly Thr
    50                  55                  60

Ser Ile Thr Gln Ala Ala Met Arg Met Leu Ala Gln Ala Gly Val Leu
65                  70                  75                  80

Val Gly Phe Cys Gly Gly Gly Gly Thr Pro Leu Tyr Met Thr Cys Glu
                85                  90                  95

Val Glu Trp Leu Thr Pro Gln Ser Glu Tyr Arg Pro Thr Glu Tyr Leu
            100                 105                 110

His Gly Trp Met Gln Phe Trp Phe Asp Asp Glu Lys Arg Leu Leu Ala
        115                 120                 125

Ala Lys Thr Phe Gln Gln Ala Arg Ile Gln Phe Ile Glu Gln Val Trp
    130                 135                 140

Gln Arg Asp Arg Glu Leu Lys Thr Glu Gly Phe Ile Phe Lys Asp Pro
145                 150                 155                 160

Ala Ile Gln Ala Ala Leu Glu Thr Phe His Ala Arg Thr Glu Ala Ala
                165                 170                 175

Thr Lys Gln Ser Asp Leu Leu Leu Thr Glu Ala Gln Leu Thr Lys Val
            180                 185                 190

Leu Tyr Lys His Ala Ala Asn Asn Thr Gln Leu Lys Asp Phe Thr Arg
        195                 200                 205

Gln His Gln Ser Ala Asp Ile Ala Asn Asp Phe Leu Asn His Gly Asn
    210                 215                 220

Tyr Leu Ala Tyr Gly Leu Ala Ala Ser Cys Leu Trp Val Leu Gly Ile
225                 230                 235                 240

Pro His Gly Phe Ala Val Met His Gly Lys Thr Arg Arg Gly Ala Leu
                245                 250                 255

Val Phe Asp Val Ala Asp Leu Ile Lys Asp Ala Ile Val Leu Pro Trp
            260                 265                 270

Ala Phe Val Cys Ala Lys Glu Lys Ala Ser Glu Gln Glu Phe Arg Gln
        275                 280                 285

Gln Val Leu Gln Ala Phe Thr Asp His Asn Ala Leu Asp Phe Met Phe
    290                 295                 300

Asn Thr Val Lys Gly Ile Ala Leu Gln Asp Tyr Ser Ala Glu Gln Ile
305                 310                 315                 320

Ala Ala Gln Gly Leu
                325

<210>  41
<211>  978
<212>  PRT
<213>  Shewanella putrefaciens

<220>
<221>  PEPTIDE
<222>  (1)..(978)
<223>  CRISPR-associated endonuclease Cas3'': WP_000989810.1

<400>  41

Met Met Val Thr Phe Ile Ser Gln Cys Glu Lys Asn Ala Leu Lys Lys
1               5                   10                  15

Thr Arg Arg Val Leu Asp Ala Phe Ala Asn Arg Ile Gly Asp Asn Thr

20                  25                  30

Trp Gln Thr Leu Ile Thr Glu Asp Gly Leu Leu Thr Val Lys Lys Met
        35                  40                  45

Leu Lys Gln Thr Ala Ser Lys Ser Thr Ala Val Ser Cys His Trp Ile
    50                  55                  60

Arg Ser Arg Ala Arg Ser Gln Leu Leu Trp Val Val Gly Asn Arg Leu
65                  70                  75                  80

Lys Phe Asn Glu Gln Gly Ile Val Pro Val Asn Ser Thr Val Lys Glu
                85                  90                  95

Val Phe Met Asp Val Val Thr Asn Lys Pro Lys Gln Gly Val Leu Tyr
            100                 105                 110

Ala Asn Thr Gln Leu Gln Pro Leu Ser Glu His Leu Phe Ala Val Gly
        115                 120                 125

Phe Val Ala Glu Leu Leu His Arg Gln Leu Phe Pro Glu Asn Thr Gln
    130                 135                 140

Phe Ser Ile Ala Asn Phe Ile Ala Gly Cys Leu His Asp Leu Gly Lys
145                 150                 155                 160

Ile Asp Pro Lys Phe Gln Glu Trp Val Thr Asn Pro Lys Lys Arg Asp
                165                 170                 175

Phe Ile Ala Glu Asp Gly Gln His Ile Asp Asp Ala Lys Phe Ser Phe
            180                 185                 190

Asp Lys His Pro Arg His Asn Glu Val Ser Leu Leu Leu Tyr Gln Leu
        195                 200                 205

Leu Asp Ser Thr Glu Leu Lys Leu Leu Asn Pro Ser Asn Lys Arg Ser
    210                 215                 220

Val Lys His Ala Leu Tyr Trp His His Ala Lys Pro Phe Arg Lys Glu
225                 230                 235                 240

Lys Asn Gly Phe Asp Thr Tyr Lys Asp Ile Ser Lys Lys Leu Val Ala
                245                 250                 255

Asn Gln Lys Glu Ile Gly Leu Ser Asp Ile Val Gly Gln Ala Gln Thr
            260                 265                 270

Leu Leu Arg Gln Val Ile Gly Ile Asp Lys Lys Tyr Arg Glu Leu Thr
        275                 280                 285

Asn Arg Leu Leu Glu Lys Ala Phe Leu Ala Glu Val Glu Gln Asp Leu
    290                 295                 300

Glu Ala Asn Leu Pro Asp Ala Pro Ala Leu Pro Ser Tyr Lys Glu Tyr
305                 310                 315                 320

Glu Phe Glu Glu Ser Val Ser Ala Asn Ser Thr Asn Val Lys Leu Asn
                325                 330                 335

Ala Leu Asn Asn Ile Val Arg Ala Cys Leu Ile Ser Ala Asp Arg Thr
            340                 345                 350

Val Ser Ala Leu Ser Ala Ala Glu Leu His Thr Ala Ile Phe Glu Arg
        355                 360                 365

Gln Leu Glu Glu Ile Val Thr Glu Leu Leu Glu Val Glu Thr Thr Leu
    370                 375                 380

Ser Ser Asp Ile Asp Thr Cys Leu Asn Ala Phe Phe Pro Thr Ser Glu
385                 390                 395                 400

Arg Ser Gln Lys Gln Arg Asp Ile Ala Lys Gln Leu Thr Thr Ala Glu
                405                 410                 415

Asp Val Ala Val Leu Ala Gly Ala Ala Gly Cys Gly Lys Thr Lys Ile
            420                 425                 430

Ala Leu Glu Trp Ala Lys Leu Gln Gly Val Lys Arg Ile Ile Trp Val
        435                 440                 445

Cys Pro Arg Val Gln Val Cys Gln Gly Leu Phe Tyr Glu Leu Thr Ser
    450                 455                 460

Glu Gln Tyr Leu Pro Asn Ser Thr Ile Glu Ile Asn Thr Gly Glu Phe
465                 470                 475                 480

Lys Phe His Asn Gln Trp Asp Asn Pro Ile Pro Asp Asp Glu Asn Phe
                485                 490                 495

Ser Gly Asp Ile Val Leu Thr Thr Ile Asp Gln Ile Leu Gly Ser Val
            500                 505                 510

Ile Ser His Thr Lys Ala Asn Thr Leu Ile Asp Phe Leu Asn Val His
        515                 520                 525

Val Val Phe Asp Glu Phe His Glu Tyr Val Asn Met Pro Ala Phe Asn
    530                 535                 540

Leu Leu Phe Ala Glu Leu Ile Ala Ala Lys Lys Met Gln Lys Asp Gln
545                 550                 555                 560

Ala Lys Thr Leu Leu Val Ser Ala Thr Pro His Tyr Val Phe Val Glu
                565                 570                 575

Asp Leu Leu Gly Ile His Pro Asp Asp Ile Lys Ile Met Pro Ser Phe
            580                 585                 590

Asn Pro Ser Gln Tyr Gln Leu Asp Phe Lys Ile Phe Asp Asp Thr Asn
        595                 600                 605

Leu Ser Asn Ile Asn Pro Leu Phe Ala Gln Gln Ser Gly Cys Thr Phe
    610                 615                 620

Val Ile Ser Asn Met Ala Leu Thr Ala Gln Asn Ser Phe Ile Val Asn
625                 630                 635                 640

Gln His Asn Glu Asn Ala Val Leu Leu His Ser Lys Phe Ile Lys Ser
                645                 650                 655

Asp Lys Arg Lys Trp Phe Gly Glu Val Tyr Glu Ala Tyr Lys Arg Asp
            660                 665                 670

Gly Thr Arg Lys Tyr Glu Val Leu Arg Ser Gly Pro Ile Val Gln Ala
        675                 680                 685

Ser Leu Asn Ile Ser Cys Asp Tyr Met Val Ala Glu Ile Thr Ser Ala
    690                 695                 700

Glu Asp Cys Leu Gln Arg Leu Gly Arg Leu Asp Arg Phe Gly Glu Asn
705                 710                 715                 720

Ser Asp Val Asn Thr Tyr Thr Leu Ala Val Pro Glu Ser Ile Ala Asn
                725                 730                 735

Gly Lys Gly Lys Ser Pro Ala Ala Gly Phe Leu Ser Lys Met Tyr Ala
            740                 745                 750

Leu Ser Ser Val Arg Ala Trp Tyr Gln Phe Leu His Ser Glu Leu Ala
        755                 760                 765

Asp Lys Thr Phe Thr Leu Pro Glu Ile Tyr Gln Leu Tyr Phe Ala Phe
    770                 775                 780

His His Thr Glu Arg Asn Arg Gly Phe Ile Ala Ser Asp Leu Val Ala
785                 790                 795                 800

Ala Leu Lys Asn Ser Val Gln Gln Ile Asn Ala Lys Val Val Asp Pro
                805                 810                 815

Ile Thr Val Pro Pro Lys Asn Asn Lys Asp Lys Lys Arg Thr Lys Ile
            820                 825                 830

Ser Lys Gly Ser Leu Arg Gly Asp Asn Arg Phe Val Gln Leu Ala Val
        835                 840                 845

Val Asp Leu Ser Gln Ala Glu Pro Lys Tyr Ile Glu Ala Tyr Ala Tyr
    850                 855                 860

Ser Ile Pro Leu Asp Asp Asp Ser Asn Ile Asp Asn Leu Thr Tyr Ala
865                 870                 875                 880

Thr Lys Ala Ile Cys Gly Tyr Asp Gln Ser Asp Lys Asp Leu Leu Ser
                885                 890                 895

His Met Phe Lys Lys His His Asn Ile Met Gly Gly Thr Gly Lys Ala
            900                 905                 910

Phe Thr Asp Lys Ile Leu Leu Asn Glu Ala Arg Asp Pro Glu Ser Pro
        915                 920                 925

Ile Tyr Leu Ser Tyr Thr Thr Asn Asp Leu Leu Gln Val Gly Gly Glu
    930                 935                 940

Ser Ala Arg His Pro His Ala Ile Tyr Tyr Ala Val Cys Asp Lys Gln
945                 950                 955                 960

Ala Ile Gly Ala Met Ser Ile Lys Gln Ile Asn Gln Thr Thr Glu Asp
                965                 970                 975

Glu Glu

<210>  42
<211>  315
<212>  PRT
<213>  Shewanella putrefaciens

<220>
<221>  PEPTIDE
<222>  (1)..(315)

<223>  hypothetical protein: WP_011919225.1

<400>  42

Met Gln Lys Val Thr Gly Ile Lys Ser Val Asp Phe Lys Ile Lys Ala
1               5                   10                  15

Leu Gly His Gly Val Val Asn Trp Asn Gly Pro Thr Thr Leu Thr Gly
            20                  25                  30

Asp Asp Gly Lys Thr Val Asp Asn His Thr Leu Pro Lys Leu Arg Gly
        35                  40                  45

Tyr Thr Asn Leu Thr Gly Lys Val Lys Asp Glu Thr Gly Tyr Lys Tyr
    50                  55                  60

Lys Lys Gln Ala Thr Asp Ile Asn Phe Lys Glu Thr Pro Leu Tyr Ile
65                  70                  75                  80

Ser Gln Asn Cys Ile Arg His His Leu Phe Arg Glu Gln Ala Phe Asp
                85                  90                  95

Leu His Tyr Ala Ser Asp Lys Asn Leu Lys Asn Val Leu Ala Ser Ile
            100                 105                 110

Thr Gly Leu Ile Arg Gly Tyr Val Val Pro Ser Ser Gln Cys Lys Arg
        115                 120                 125

Thr Ser Pro Leu Leu Leu Glu Asp Phe Val Asp Gln Leu Gly Asn Gly
    130                 135                 140

Asn Phe Glu Gln Tyr Gly Gln Ala Gly Ala Arg Asp Ser Thr Ser Phe
145                 150                 155                 160

Phe Ser Lys Thr Thr Phe Gly Asp Thr Glu Tyr Ile Ser Tyr Gly Ser
                165                 170                 175

Ile Ser Ile Glu Gln Leu Gln Phe Ile Ser Leu Asp Lys Lys Phe Asp
            180                 185                 190

Arg Ala Ala Met Val Ile Lys Glu Gly Glu Gly Glu Val Ile Ala Ala
        195                 200                 205

Glu Leu Gln Asn Tyr Ile Gln Ser Leu Asn Pro Ser Leu Asn Pro Gln
    210                 215                 220

Ala Ile Phe His Ser Asn Tyr Val Arg Arg Gly Thr Ile Phe Glu Glu
225                 230                 235                 240

Gly Glu Cys Gly Ile Leu Leu Asn Asp Asp Ala Val Lys Ala Leu Val
                245                 250                 255

Ala Glu Thr Leu Glu Arg Leu Ala Asn Leu Ser Ile Arg Gln Ala Lys
            260                 265                 270

Gly Tyr Met Tyr Val Asp Asp Ile Thr Val Asp Tyr Asn Asp Ser His
        275                 280                 285

Lys Met Met Arg Ile Lys Arg Asp Glu Ser Glu Ile Ile Asn Glu Gln
    290                 295                 300

His Ala Pro Phe Ala Gln Tyr Phe Tyr Ala Lys
305                 310                 315

<210>  43
<211>  336
<212>  PRT
<213>  Shewanella putrefaciens

<220>
<221>  PEPTIDE
<222>  (1)..(336)
<223>  hypothetical protein WP_011919226.1

<400>  43

Met Lys Ile Ile Ile Glu Tyr Asp Ser Cys Trp Arg Asn Ala Phe Leu
1               5                   10                  15

Gly Gly Ser Asn Asn Glu Pro Val Pro Lys Lys Gly Arg Glu Phe Leu
            20                  25                  30

Gly Ser Met Thr Ser Leu Lys Lys Glu Gly Asn Phe Lys Val Cys Glu
        35                  40                  45

Asn Thr Leu Asp Thr Val Met Gly Val Leu Asn Arg Leu Ile Gly Asp
    50                  55                  60

Gln Arg Lys Leu Tyr Gln Ala Arg Ser Lys Met Tyr Glu Ser Ala Tyr
65                  70                  75                  80

Tyr Phe Glu Ala Leu Glu Asp Lys Val Ser Phe Ile Asp Lys Pro Gln
                85                  90                  95

Leu Thr Asn Glu Ile Ser Phe Ile Arg Asn Met Asn Gly Ser Thr Asp
            100                 105                 110

Gln Asn Ala Phe Thr Gly Met Ile Lys Val Ser Asp Pro Val Phe Thr

115                 120                 125

Ser Glu Tyr Ser Gln Gln Phe Trp Gly Val Leu Ala Leu Asp Phe Thr
    130                 135                 140

Gln Leu Cys Asp Phe Ile Ile Lys Gln Ser Gln Val Val Gly Ser Ile
145                 150                 155                 160

Glu Leu Asn Pro Leu Ser Ile Ile Asn Arg Leu Glu Ser Leu Asn Gln
                165                 170                 175

Glu Lys Ala Leu Glu Asn Ser Asp Asp Leu Ala Gln Val Leu Lys Val
            180                 185                 190

Leu Asn Glu Tyr Phe Pro Asp Ile Glu Tyr Leu Asn Asn Lys Gly Leu
        195                 200                 205

Ile Thr Pro Ile Ser Ile Tyr Cys Ser Ala Leu Tyr Leu Gln Leu Ala
    210                 215                 220

Arg Leu Glu Thr Ser Phe Asn Met Thr Thr Ala Lys Thr Lys Ala Gly
225                 230                 235                 240

Gly Ile Ser Gly Ile Ser Lys Arg Gly Phe Thr Lys Lys Asp Phe Met
                245                 250                 255

Asp Arg Tyr Thr Thr Gly Pro Lys Lys Thr Ile Trp Gly Asn Pro Phe
            260                 265                 270

Ile Lys Lys Glu Lys Ile Lys Gly Gln Gly Glu Val Thr Ser Met Met
        275                 280                 285

Thr Lys Ala Ser Gly Gln Leu Glu Ile Ser Ile Asp Val Asp Arg Asp
    290                 295                 300

Lys Ala Gln Glu Ile Lys Ile Leu Ile Glu Asn Ala Gly Val Ser Ser
305                 310                 315                 320

Phe Tyr Leu Gly Lys Lys Gly Leu Ala Tyr Val Ser Asn Ile Lys Leu
                325                 330                 335

<210>  44
<211>  183
<212>  PRT
<213>  Shewanella putrefaciens

<220>
<221>  PEPTIDE

<222>  (1)..(183)
<223>  type I-F CRISPR-associated endoribonuclease  Cas6/Csy4:
       WP_011919227.1

<400>  44

Met Asn Ser Tyr Ile Asp Ile Arg Leu Lys Pro Asp Ala Glu Met Arg
1               5                   10                  15

Glu Ala Glu Leu Ser Ser Lys Val Phe Thr Lys Phe His Lys Ala Leu
            20                  25                  30

Val Thr Leu Asn Ser His Lys Ile Gly Ile Ser Phe Pro Gln Met Lys
        35                  40                  45

Leu Ser Leu Gly Gln Leu Phe Arg Ile His Gly Asp Ala Ser Leu Leu
    50                  55                  60

His Asp Leu Gln Gly Leu Asp Trp Leu Gly Pro Leu Ala Gly Tyr Cys
65                  70                  75                  80

Gln Val Thr Ala Val Ser Ala Val Pro Asp His Val Gln Tyr Arg Ile
                85                  90                  95

Val Ser Val Lys Arg Ser Asn Leu Ser Lys Ala Lys Leu Lys Arg Leu
            100                 105                 110

Ile Ala Arg Gly Ser Ile Asp Lys Asp Gly Glu Lys Arg Tyr Lys Val
        115                 120                 125

Lys Met Leu Gly Gln Gly Phe Asp Asn Pro Tyr Leu Asp Leu Phe Ser
    130                 135                 140

Ser Ser Thr Gly Gln Val Tyr Arg Lys Phe Phe Glu Phe Ser Asp Ile
145                 150                 155                 160

Gln Ala His Pro Leu Asp Gly Glu Phe Asp Ser Tyr Gly Leu Ser Lys
                165                 170                 175

Thr Ala Thr Val Pro Trp Phe
            180


<210>  45
<211>  1368
<212>  PRT
<213>  Streptococcus pyogenes


<220>
<221>  PEPTIDE
<222>  (1)..(1368)

<223>  type II CRISPR RNA-guided endonuclease Cas9: WP_020905136.1

<400>  45

Met Asp Lys Lys Tyr Ser Ile Gly Leu Asp Ile Gly Thr Asn Ser Val
1               5                   10                  15

Gly Trp Ala Val Ile Thr Asp Asp Tyr Lys Val Pro Ser Lys Lys Phe
            20                  25                  30

Lys Val Leu Gly Asn Thr Asp Arg His Ser Ile Lys Lys Asn Leu Ile
        35                  40                  45

Gly Ala Leu Leu Phe Asp Ser Gly Glu Thr Ala Glu Ala Thr Arg Leu
    50                  55                  60

Lys Arg Thr Ala Arg Arg Arg Tyr Thr Arg Arg Lys Asn Arg Ile Cys
65                  70                  75                  80

Tyr Leu Gln Glu Ile Phe Ser Asn Glu Met Ala Lys Val Asp Asp Ser
                85                  90                  95

Phe Phe His Arg Leu Glu Glu Ser Phe Leu Val Glu Glu Asp Lys Lys
            100                 105                 110

His Glu Arg His Pro Ile Phe Gly Asn Ile Val Asp Glu Val Ala Tyr
        115                 120                 125

His Glu Lys Tyr Pro Thr Ile Tyr His Leu Arg Lys Lys Leu Val Asp
    130                 135                 140

Ser Thr Asp Lys Ala Asp Leu Arg Leu Ile Tyr Leu Ala Leu Ala His
145                 150                 155                 160

Met Ile Lys Phe Arg Gly His Phe Leu Ile Glu Gly Asp Leu Asn Pro
                165                 170                 175

Asp Asn Ser Asp Val Asp Lys Leu Phe Ile Gln Leu Val Gln Thr Tyr
            180                 185                 190

Asn Gln Leu Phe Glu Glu Asn Pro Ile Asn Ala Ser Gly Val Asp Ala
        195                 200                 205

Lys Ala Ile Leu Ser Ala Arg Leu Ser Lys Ser Arg Arg Leu Glu Asn
    210                 215                 220

Leu Ile Ala Gln Leu Pro Gly Glu Lys Lys Asn Gly Leu Phe Gly Asn
225                 230                 235                 240

Leu Ile Ala Leu Ser Leu Gly Leu Thr Pro Asn Phe Lys Ser Asn Phe
                245                 250                 255

Asp Leu Ala Glu Asp Ala Lys Leu Gln Leu Ser Lys Asp Thr Tyr Asp
            260                 265                 270

Asp Asp Leu Asp Asn Leu Leu Ala Gln Ile Gly Asp Gln Tyr Ala Asp
        275                 280                 285

Leu Phe Leu Ala Ala Lys Asn Leu Ser Asp Ala Ile Leu Leu Ser Asp
    290                 295                 300

Ile Leu Arg Leu Asn Ser Glu Ile Thr Lys Ala Pro Leu Ser Ala Ser
305                 310                 315                 320

Met Ile Lys Arg Tyr Asp Glu His His Gln Asp Leu Thr Leu Leu Lys
                325                 330                 335

Ala Leu Val Arg Gln Gln Leu Pro Glu Lys Tyr Lys Glu Ile Phe Phe
            340                 345                 350

Asp Gln Ser Lys Asn Gly Tyr Ala Gly Tyr Ile Asp Gly Gly Ala Ser
        355                 360                 365

Gln Glu Glu Phe Tyr Lys Phe Ile Lys Pro Ile Leu Glu Lys Met Asp
    370                 375                 380

Gly Thr Glu Glu Leu Leu Ala Lys Leu Asn Arg Glu Asp Leu Leu Arg
385                 390                 395                 400

Lys Gln Arg Thr Phe Asp Asn Gly Ser Ile Pro His Gln Ile His Leu
                405                 410                 415

Gly Glu Leu His Ala Ile Leu Arg Arg Gln Glu Asp Phe Tyr Pro Phe
            420                 425                 430

Leu Lys Asp Asn Arg Glu Lys Ile Glu Lys Ile Leu Thr Phe Arg Ile
        435                 440                 445

Pro Tyr Tyr Val Gly Pro Leu Ala Arg Gly Asn Ser Arg Phe Ala Trp
    450                 455                 460

Met Thr Arg Lys Ser Glu Glu Thr Ile Thr Pro Trp Asn Phe Glu Glu
465                 470                 475                 480

Val Val Asp Lys Gly Ala Ser Ala Gln Ser Phe Ile Glu Arg Met Thr
                485                 490                 495

Asn Phe Asp Lys Asn Leu Pro Asn Glu Lys Val Leu Pro Lys His Ser
        500                 505                 510

Leu Leu Tyr Glu Tyr Phe Thr Val Tyr Asn Glu Leu Thr Lys Val Lys
    515                 520                 525

Tyr Val Thr Glu Gly Met Arg Lys Pro Ala Phe Leu Ser Gly Glu Gln
530                 535                 540

Lys Lys Ala Ile Val Asp Leu Leu Phe Lys Thr Asn Arg Lys Val Thr
545                 550                 555                 560

Val Lys Gln Leu Lys Glu Asp Tyr Phe Lys Lys Ile Glu Cys Phe Asp
                565                 570                 575

Ser Val Glu Ile Ser Gly Val Glu Asp Arg Phe Asn Ala Ser Leu Gly
        580                 585                 590

Thr Tyr His Asp Leu Leu Lys Ile Ile Lys Asp Lys Asp Phe Leu Asp
    595                 600                 605

Asn Glu Glu Asn Glu Asp Ile Leu Glu Asp Ile Val Leu Thr Leu Thr
610                 615                 620

Leu Phe Glu Asp Arg Glu Met Ile Glu Glu Arg Leu Lys Thr Tyr Ala
625                 630                 635                 640

His Leu Phe Asp Asp Lys Val Met Lys Gln Leu Lys Arg Arg Arg Tyr
                645                 650                 655

Thr Gly Trp Gly Arg Leu Ser Arg Lys Leu Ile Asn Gly Ile Arg Asp
        660                 665                 670

Lys Gln Ser Gly Lys Thr Ile Leu Asp Phe Leu Lys Ser Asp Gly Phe
    675                 680                 685

Ala Asn Arg Asn Phe Met Gln Leu Ile His Asp Asp Ser Leu Thr Phe
690                 695                 700

Lys Glu Asp Ile Gln Lys Ala Gln Val Ser Gly Gln Gly Asp Ser Leu
705                 710                 715                 720

His Glu His Ile Ala Asn Leu Ala Gly Ser Pro Ala Ile Lys Lys Gly
                725                 730                 735

Ile Leu Gln Thr Val Lys Val Val Asp Glu Leu Val Lys Val Met Gly

            740                 745                 750

Arg His Lys Pro Glu Asn Ile Val Ile Glu Met Ala Arg Glu Asn Gln
        755                 760                 765

Thr Thr Gln Lys Gly Gln Lys Asn Ser Arg Glu Arg Met Lys Arg Ile
    770                 775                 780

Glu Glu Gly Ile Lys Glu Leu Gly Ser Gln Ile Leu Lys Glu His Pro
785                 790                 795                 800

Val Glu Asn Thr Gln Leu Gln Asn Glu Lys Leu Tyr Leu Tyr Tyr Leu
                805                 810                 815

Gln Asn Gly Arg Asp Met Tyr Val Asp Gln Glu Leu Asp Ile Asn Arg
            820                 825                 830

Leu Ser Asp Tyr Asp Val Asp His Ile Val Pro Gln Ser Phe Ile Lys
        835                 840                 845

Asp Asp Ser Ile Asp Asn Lys Val Leu Thr Arg Ser Asp Lys Asn Arg
    850                 855                 860

Gly Lys Ser Asp Asn Val Pro Ser Glu Glu Val Val Lys Lys Met Lys
865                 870                 875                 880

Asn Tyr Trp Arg Gln Leu Leu Asn Ala Lys Leu Ile Thr Gln Arg Lys
                885                 890                 895

Phe Asp Asn Leu Thr Lys Ala Glu Arg Gly Gly Leu Ser Glu Leu Asp
            900                 905                 910

Lys Ala Gly Phe Ile Lys Arg Gln Leu Val Glu Thr Arg Gln Ile Thr
        915                 920                 925

Lys His Val Ala Gln Ile Leu Asp Ser Arg Met Asn Thr Lys Tyr Asp
    930                 935                 940

Glu Asn Asp Lys Leu Ile Arg Glu Val Lys Val Ile Thr Leu Lys Ser
945                 950                 955                 960

Lys Leu Val Ser Asp Phe Arg Lys Asp Phe Gln Phe Tyr Lys Val Arg
                965                 970                 975

Glu Ile Asn Asn Tyr His His Ala His Asp Ala Tyr Leu Asn Ala Val
            980                 985                 990

Val Gly Thr Ala Leu Ile Lys Lys Tyr Pro Lys Leu Glu Ser Glu Phe
        995                 1000                1005

Val Tyr Gly Asp Tyr Lys Val Tyr Asp Val Arg Lys Met Ile Ala
    1010                1015                1020

Lys Ser Glu Gln Glu Ile Gly Lys Ala Thr Ala Lys Tyr Phe Phe
    1025                1030                1035

Tyr Ser Asn Ile Met Asn Phe Phe Lys Thr Glu Ile Thr Leu Ala
    1040                1045                1050

Asn Gly Glu Ile Arg Lys Arg Pro Leu Ile Glu Thr Asn Gly Glu
    1055                1060                1065

Thr Gly Glu Ile Val Trp Asp Lys Gly Arg Asp Phe Ala Thr Val
    1070                1075                1080

Arg Lys Val Leu Ser Met Pro Gln Val Asn Ile Val Lys Lys Thr
    1085                1090                1095

Glu Val Gln Thr Gly Gly Phe Ser Lys Glu Ser Ile Leu Pro Lys
    1100                1105                1110

Arg Asn Ser Asp Lys Leu Ile Ala Arg Lys Lys Asp Trp Asp Pro
    1115                1120                1125

Lys Lys Tyr Gly Gly Phe Asp Ser Pro Thr Val Ala Tyr Ser Val
    1130                1135                1140

Leu Val Val Ala Lys Val Glu Lys Gly Lys Ser Lys Lys Leu Lys
    1145                1150                1155

Ser Val Lys Glu Leu Leu Gly Leu Thr Ile Met Glu Arg Ser Ser
    1160                1165                1170

Phe Glu Lys Asn Pro Ile Asp Phe Leu Glu Ala Lys Gly Tyr Lys
    1175                1180                1185

Glu Val Lys Lys Asp Leu Ile Ile Lys Leu Pro Lys Tyr Ser Leu
    1190                1195                1200

Phe Glu Leu Glu Asn Gly Arg Lys Arg Met Leu Ala Ser Ala Gly
    1205                1210                1215

Glu Leu Gln Lys Gly Asn Glu Leu Ala Leu Pro Ser Lys Tyr Val
    1220                1225                1230

Asn Phe Leu Tyr Leu Ala Ser His Tyr Glu Lys Leu Lys Gly Ser
    1235                1240                1245

Pro Glu Asp Asn Glu Gln Lys Gln Leu Phe Val Glu Gln His Lys
    1250                1255                1260

His Tyr Leu Asp Glu Ile Ile Glu Gln Ile Ser Glu Phe Ser Lys
    1265                1270                1275

Arg Val Ile Leu Ala Asp Ala Asn Leu Asp Lys Val Leu Ser Ala
    1280                1285                1290

Tyr Asn Lys His Arg Asp Lys Pro Ile Arg Glu Gln Ala Glu Asn
    1295                1300                1305

Ile Ile His Leu Phe Thr Leu Thr Asn Leu Gly Ala Pro Ala Ala
    1310                1315                1320

Phe Lys Tyr Phe Asp Thr Thr Ile Asp Arg Lys Arg Tyr Thr Ser
    1325                1330                1335

Thr Lys Glu Val Leu Asp Ala Thr Leu Ile His Gln Ser Ile Thr
    1340                1345                1350

Gly Leu Tyr Glu Thr Arg Ile Asp Leu Ser Gln Leu Gly Gly Asp
    1355                1360                1365

<210>  46
<211>  1372
<212>  PRT
<213>  Legionella pneumophila

<220>
<221>  PEPTIDE
<222>  (1)..(1372)
<223>  type II-B CRISPR-associated RNA-guided endonuclease Cas9/Csx12:
       WP_011212792.1

<400>  46

Met Glu Ser Ser Gln Ile Leu Ser Pro Ile Gly Ile Asp Leu Gly Gly
1               5                   10                  15

Lys Phe Thr Gly Val Cys Leu Ser His Leu Glu Ala Phe Ala Glu Leu
            20                  25                  30

Pro Asn His Ala Asn Thr Lys Tyr Ser Val Ile Leu Ile Asp His Asn
        35                  40                  45

Asn Phe Gln Leu Ser Gln Ala Gln Arg Arg Ala Thr Arg His Arg Val
    50                  55                  60

Arg Asn Lys Lys Arg Asn Gln Phe Val Lys Arg Val Ala Leu Gln Leu
65                  70                  75                  80

Phe Gln His Ile Leu Ser Arg Asp Leu Asn Ala Lys Glu Glu Thr Ala
                85                  90                  95

Leu Cys His Tyr Leu Asn Asn Arg Gly Tyr Thr Tyr Val Asp Thr Asp
            100                 105                 110

Leu Asp Glu Tyr Ile Lys Asp Glu Thr Thr Ile Asn Leu Leu Lys Glu
        115                 120                 125

Leu Leu Pro Ser Glu Ser Glu His Asn Phe Ile Asp Trp Phe Leu Gln
    130                 135                 140

Lys Met Gln Ser Ser Glu Phe Arg Lys Ile Leu Val Ser Lys Val Glu
145                 150                 155                 160

Glu Lys Lys Asp Asp Lys Glu Leu Lys Asn Ala Val Lys Asn Ile Lys
                165                 170                 175

Asn Phe Ile Thr Gly Phe Glu Lys Asn Ser Val Glu Gly His Arg His
            180                 185                 190

Arg Lys Val Tyr Phe Glu Asn Ile Lys Ser Asp Ile Thr Lys Asp Asn
        195                 200                 205

Gln Leu Asp Ser Ile Lys Lys Lys Ile Pro Ser Val Cys Leu Ser Asn
    210                 215                 220

Leu Leu Gly His Leu Ser Asn Leu Gln Trp Lys Asn Leu His Arg Tyr
225                 230                 235                 240

Leu Ala Lys Asn Pro Lys Gln Phe Asp Glu Gln Thr Phe Gly Asn Glu
                245                 250                 255

Phe Leu Arg Met Leu Lys Asn Phe Arg His Leu Lys Gly Ser Gln Glu
            260                 265                 270

Ser Leu Ala Val Arg Asn Leu Ile Gln Gln Leu Glu Gln Ser Gln Asp
        275                 280                 285

Tyr Ile Ser Ile Leu Glu Lys Thr Pro Pro Glu Ile Thr Ile Pro Pro
    290                 295                 300

Tyr Glu Ala Arg Thr Asn Thr Gly Met Glu Lys Asp Gln Ser Leu Leu
305                 310                 315                 320

Leu Asn Pro Glu Lys Leu Asn Asn Leu Tyr Pro Asn Trp Arg Asn Leu
                325                 330                 335

Ile Pro Gly Ile Ile Asp Ala His Pro Phe Leu Glu Lys Asp Leu Glu
            340                 345                 350

His Thr Lys Leu Arg Asp Arg Lys Arg Ile Ile Ser Pro Ser Lys Gln
        355                 360                 365

Asp Glu Lys Arg Asp Ser Tyr Ile Leu Gln Arg Tyr Leu Asp Leu Asn
    370                 375                 380

Lys Lys Ile Asp Lys Phe Lys Ile Lys Lys Gln Leu Ser Phe Leu Gly
385                 390                 395                 400

Gln Gly Lys Gln Leu Pro Ala Asn Leu Ile Glu Thr Gln Lys Glu Met
                405                 410                 415

Glu Thr His Phe Asn Ser Ser Leu Val Ser Val Leu Ile Gln Ile Ala
            420                 425                 430

Ser Ala Tyr Asn Lys Glu Arg Glu Asp Ala Ala Gln Gly Ile Trp Phe
        435                 440                 445

Asp Asn Ala Phe Ser Leu Cys Glu Leu Ser Asn Ile Asn Pro Pro Arg
    450                 455                 460

Lys Gln Lys Ile Leu Pro Leu Leu Val Gly Ala Ile Leu Ser Glu Asp
465                 470                 475                 480

Phe Ile Asn Asn Lys Asp Lys Trp Ala Lys Phe Lys Ile Phe Trp Asn
                485                 490                 495

Thr His Lys Ile Gly Arg Thr Ser Leu Lys Ser Lys Cys Lys Glu Ile
            500                 505                 510

Glu Glu Ala Arg Lys Asn Ser Gly Asn Ala Phe Lys Ile Asp Tyr Glu
        515                 520                 525

Glu Ala Leu Asn His Pro Glu His Ser Asn Asn Lys Ala Leu Ile Lys
    530                 535                 540

Ile Ile Gln Thr Ile Pro Asp Ile Ile Gln Ala Ile Gln Ser His Leu
545                 550                 555                 560

Gly His Asn Asp Ser Gln Ala Leu Ile Tyr His Asn Pro Phe Ser Leu
                565                 570                 575

Ser Gln Leu Tyr Thr Ile Leu Glu Thr Lys Arg Asp Gly Phe His Lys
            580                 585                 590

Asn Cys Val Ala Val Thr Cys Glu Asn Tyr Trp Arg Ser Gln Lys Thr
        595                 600                 605

Glu Ile Asp Pro Glu Ile Ser Tyr Ala Ser Arg Leu Pro Ala Asp Ser
    610                 615                 620

Val Arg Pro Phe Asp Gly Val Leu Ala Arg Met Met Gln Arg Leu Ala
625                 630                 635                 640

Tyr Glu Ile Ala Met Ala Lys Trp Glu Gln Ile Lys His Ile Pro Asp
                645                 650                 655

Asn Ser Ser Leu Leu Ile Pro Ile Tyr Leu Glu Gln Asn Arg Phe Glu
            660                 665                 670

Phe Glu Glu Ser Phe Lys Lys Ile Lys Gly Ser Ser Ser Asp Lys Thr
        675                 680                 685

Leu Glu Gln Ala Ile Glu Lys Gln Asn Ile Gln Trp Glu Glu Lys Phe
    690                 695                 700

Gln Arg Ile Ile Asn Ala Ser Met Asn Ile Cys Pro Tyr Lys Gly Ala
705                 710                 715                 720

Ser Ile Gly Gly Gln Gly Glu Ile Asp His Ile Tyr Pro Arg Ser Leu
                725                 730                 735

Ser Lys Lys His Phe Gly Val Ile Phe Asn Ser Glu Val Asn Leu Ile
            740                 745                 750

Tyr Cys Ser Ser Gln Gly Asn Arg Glu Lys Lys Glu Glu His Tyr Leu
        755                 760                 765

Leu Glu His Leu Ser Pro Leu Tyr Leu Lys His Gln Phe Gly Thr Asp
    770                 775                 780

Asn Val Ser Asp Ile Lys Asn Phe Ile Ser Gln Asn Val Ala Asn Ile
785                 790                 795                 800

Lys Lys Tyr Ile Ser Phe His Leu Leu Thr Pro Glu Gln Gln Lys Ala

                805                 810                 815

Ala Arg His Ala Leu Phe Leu Asp Tyr Asp Asp Glu Ala Phe Lys Thr
            820                 825                 830

Ile Thr Lys Phe Leu Met Ser Gln Gln Lys Ala Arg Val Asn Gly Thr
        835                 840                 845

Gln Lys Phe Leu Gly Lys Gln Ile Met Glu Phe Leu Ser Thr Leu Ala
    850                 855                 860

Asp Ser Lys Gln Leu Gln Leu Glu Phe Ser Ile Lys Gln Ile Thr Ala
865                 870                 875                 880

Glu Glu Val His Asp His Arg Glu Leu Leu Ser Lys Gln Glu Pro Lys
                885                 890                 895

Leu Val Lys Ser Arg Gln Gln Ser Phe Pro Ser His Ala Ile Asp Ala
            900                 905                 910

Thr Leu Thr Met Ser Ile Gly Leu Lys Glu Phe Pro Gln Phe Ser Gln
        915                 920                 925

Glu Leu Asp Asn Ser Trp Phe Ile Asn His Leu Met Pro Asp Glu Val
    930                 935                 940

His Leu Asn Pro Val Arg Ser Lys Glu Lys Tyr Asn Lys Pro Asn Ile
945                 950                 955                 960

Ser Ser Thr Pro Leu Phe Lys Asp Ser Leu Tyr Ala Glu Arg Phe Ile
                965                 970                 975

Pro Val Trp Val Lys Gly Glu Thr Phe Ala Ile Gly Phe Ser Glu Lys
            980                 985                 990

Asp Leu Phe Glu Ile Lys Pro Ser Asn Lys Glu Lys Leu Phe Thr Leu
        995                 1000                1005

Leu Lys Thr Tyr Ser Thr Lys Asn Pro Gly Glu Ser Leu Gln Glu
    1010                1015                1020

Leu Gln Ala Lys Ser Lys Ala Lys Trp Leu Tyr Phe Pro Ile Asn
    1025                1030                1035

Lys Thr Leu Ala Leu Glu Phe Leu His His Tyr Phe His Lys Glu
    1040                1045                1050

Ile Val Thr Pro Asp Asp Thr Thr Val Cys His Phe Ile Asn Ser
    1055                1060                1065

Leu Arg Tyr Tyr Thr Lys Lys Glu Ser Ile Thr Val Lys Ile Leu
    1070                1075                1080

Lys Glu Pro Met Pro Val Leu Ser Val Lys Phe Glu Ser Ser Lys
    1085                1090                1095

Lys Asn Val Leu Gly Ser Phe Lys His Thr Ile Ala Leu Pro Ala
    1100                1105                1110

Thr Lys Asp Trp Glu Arg Leu Phe Asn His Pro Asn Phe Leu Ala
    1115                1120                1125

Leu Lys Ala Asn Pro Ala Pro Asn Pro Lys Glu Phe Asn Glu Phe
    1130                1135                1140

Ile Arg Lys Tyr Phe Leu Ser Asp Asn Asn Pro Asn Ser Asp Ile
    1145                1150                1155

Pro Asn Asn Gly His Asn Ile Lys Pro Gln Lys His Lys Ala Val
    1160                1165                1170

Arg Lys Val Phe Ser Leu Pro Val Ile Pro Gly Asn Ala Gly Thr
    1175                1180                1185

Met Met Arg Ile Arg Arg Lys Asp Asn Lys Gly Gln Pro Leu Tyr
    1190                1195                1200

Gln Leu Gln Thr Ile Asp Asp Thr Pro Ser Met Gly Ile Gln Ile
    1205                1210                1215

Asn Glu Asp Arg Leu Val Lys Gln Glu Val Leu Met Asp Ala Tyr
    1220                1225                1230

Lys Thr Arg Asn Leu Ser Thr Ile Asp Gly Ile Asn Asn Ser Glu
    1235                1240                1245

Gly Gln Ala Tyr Ala Thr Phe Asp Asn Trp Leu Thr Leu Pro Val
    1250                1255                1260

Ser Thr Phe Lys Pro Glu Ile Ile Lys Leu Glu Met Lys Pro His
    1265                1270                1275

Ser Lys Thr Arg Arg Tyr Ile Arg Ile Thr Gln Ser Leu Ala Asp
    1280                1285                1290

Phe Ile Lys Thr Ile Asp Glu Ala Leu Met Ile Lys Pro Ser Asp
    1295                1300                1305

Ser Ile Asp Asp Pro Leu Asn Met Pro Asn Glu Ile Val Cys Lys
    1310                1315                1320

Asn Lys Leu Phe Gly Asn Glu Leu Lys Pro Arg Asp Gly Lys Met
    1325                1330                1335

Lys Ile Val Ser Thr Gly Lys Ile Val Thr Tyr Glu Phe Glu Ser
    1340                1345                1350

Asp Ser Thr Pro Gln Trp Ile Gln Thr Leu Tyr Val Thr Gln Leu
    1355                1360                1365

Lys Lys Gln Pro
    1370

<210> 47
<211> 1082
<212> PRT
<213> Neisseria meningitidis

<220>
<221> PEPTIDE
<222> (1)..(1082)
<223> type II CRISPR RNA-guided endonuclease Cas9: WP_014574210.1

<400> 47

Met Ala Ala Phe Lys Pro Asn Ser Ile Asn Tyr Ile Leu Gly Leu Asp
1               5                   10                  15

Ile Gly Ile Ala Ser Val Gly Trp Ala Met Val Glu Ile Asp Glu Glu
            20                  25                  30

Glu Asn Pro Ile Arg Leu Ile Asp Leu Gly Val Arg Val Phe Glu Arg
        35                  40                  45

Ala Glu Val Pro Lys Thr Gly Asp Ser Leu Ala Met Ala Arg Arg Leu
    50                  55                  60

Ala Arg Ser Val Arg Arg Leu Thr Arg Arg Arg Ala His Arg Leu Leu
65                  70                  75                  80

Arg Thr Arg Arg Leu Leu Lys Arg Glu Gly Val Leu Gln Ala Ala Asn
                85                  90                  95

Phe Asp Glu Asn Gly Leu Ile Lys Ser Leu Pro Asn Thr Pro Trp Gln

            100                 105                 110

Leu Arg Ala Ala Ala Leu Asp Arg Lys Leu Thr Pro Leu Glu Trp Ser
        115                 120                 125

Ala Val Leu Leu His Leu Ile Lys His Arg Gly Tyr Leu Ser Gln Arg
    130                 135                 140

Lys Asn Glu Gly Glu Thr Ala Asp Lys Glu Leu Gly Ala Leu Leu Lys
145                 150                 155                 160

Gly Val Ala Gly Asn Ala His Ala Leu Gln Thr Gly Asp Phe Arg Thr
                165                 170                 175

Pro Ala Glu Leu Ala Leu Asn Lys Phe Glu Lys Glu Ser Gly His Ile
            180                 185                 190

Arg Asn Gln Arg Ser Asp Tyr Ser His Thr Phe Ser Arg Lys Asp Leu
        195                 200                 205

Gln Ala Glu Leu Ile Leu Leu Phe Glu Lys Gln Lys Glu Phe Gly Asn
    210                 215                 220

Pro His Val Ser Gly Gly Leu Lys Glu Gly Ile Glu Thr Leu Leu Met
225                 230                 235                 240

Thr Gln Arg Pro Ala Leu Ser Gly Asp Ala Val Gln Lys Met Leu Gly
                245                 250                 255

His Cys Thr Phe Glu Pro Ala Glu Pro Lys Ala Ala Lys Asn Thr Tyr
            260                 265                 270

Thr Ala Glu Arg Phe Ile Trp Leu Thr Lys Leu Asn Asn Leu Arg Ile
        275                 280                 285

Leu Glu Gln Gly Ser Glu Arg Pro Leu Thr Asp Thr Glu Arg Ala Thr
    290                 295                 300

Leu Met Asp Glu Pro Tyr Arg Lys Ser Lys Leu Thr Tyr Ala Gln Ala
305                 310                 315                 320

Arg Lys Leu Leu Gly Leu Glu Asp Thr Ala Phe Phe Lys Gly Leu Arg
                325                 330                 335

Tyr Gly Lys Asp Asn Ala Glu Ala Ser Thr Leu Met Glu Met Lys Ala
            340                 345                 350

Tyr His Ala Ile Ser Arg Ala Leu Glu Lys Glu Gly Leu Lys Asp Lys
        355                 360                 365

Lys Ser Pro Leu Asn Leu Ser Pro Glu Leu Gln Asp Glu Ile Gly Thr
    370                 375                 380

Ala Phe Ser Leu Phe Lys Thr Asp Glu Asp Ile Thr Gly Arg Leu Lys
385                 390                 395                 400

Asp Arg Ile Gln Pro Glu Ile Leu Glu Ala Leu Leu Lys His Ile Ser
                405                 410                 415

Phe Asp Lys Phe Val Gln Ile Ser Leu Lys Ala Leu Arg Arg Ile Val
            420                 425                 430

Pro Leu Met Glu Gln Gly Lys Arg Tyr Asp Glu Ala Cys Ala Glu Ile
        435                 440                 445

Tyr Gly Asp His Tyr Gly Lys Lys Asn Thr Glu Glu Lys Ile Tyr Leu
    450                 455                 460

Pro Pro Ile Pro Ala Asp Glu Ile Arg Asn Pro Val Val Leu Arg Ala
465                 470                 475                 480

Leu Ser Gln Ala Arg Lys Val Ile Asn Gly Val Val Arg Arg Tyr Gly
                485                 490                 495

Ser Pro Ala Arg Ile His Ile Glu Thr Ala Arg Glu Val Gly Lys Ser
            500                 505                 510

Phe Lys Asp Arg Lys Glu Ile Glu Lys Arg Gln Glu Glu Asn Arg Lys
        515                 520                 525

Asp Arg Glu Lys Ala Ala Ala Lys Phe Arg Glu Tyr Phe Pro Asn Phe
    530                 535                 540

Val Gly Glu Pro Lys Ser Lys Asp Ile Leu Lys Leu Arg Leu Tyr Glu
545                 550                 555                 560

Gln Gln His Gly Lys Cys Leu Tyr Ser Gly Lys Glu Ile Asn Leu Gly
                565                 570                 575

Arg Leu Asn Glu Lys Gly Tyr Val Glu Ile Asp His Ala Leu Pro Phe
            580                 585                 590

Ser Arg Thr Trp Asp Asp Ser Phe Asn Asn Lys Val Leu Val Leu Gly
        595                 600                 605

Ser Glu Asn Gln Asn Lys Gly Asn Gln Thr Pro Tyr Glu Tyr Phe Asn
    610                 615                 620

Gly Lys Asp Asn Ser Arg Glu Trp Gln Glu Phe Lys Ala Arg Val Glu
625                 630                 635                 640

Thr Ser Arg Phe Pro Arg Ser Lys Lys Gln Arg Ile Leu Leu Gln Lys
                645                 650                 655

Phe Asp Glu Asp Gly Phe Lys Glu Arg Asn Leu Asn Asp Thr Arg Tyr
            660                 665                 670

Val Asn Arg Phe Leu Cys Gln Phe Val Ala Asp Arg Met Arg Leu Thr
        675                 680                 685

Gly Lys Gly Lys Lys Arg Val Phe Ala Ser Asn Gly Gln Ile Thr Asn
    690                 695                 700

Leu Leu Arg Gly Phe Trp Gly Leu Arg Lys Val Arg Ala Glu Asn Asp
705                 710                 715                 720

Arg His His Ala Leu Asp Ala Val Val Val Ala Cys Ser Thr Val Ala
                725                 730                 735

Met Gln Gln Lys Ile Thr Arg Phe Val Arg Tyr Lys Glu Met Asn Ala
            740                 745                 750

Phe Asp Gly Lys Thr Ile Asp Lys Glu Thr Gly Glu Val Leu His Gln
        755                 760                 765

Lys Thr His Phe Pro Gln Pro Trp Glu Phe Phe Ala Gln Glu Val Met
    770                 775                 780

Ile Arg Val Phe Gly Lys Pro Asp Gly Lys Pro Glu Phe Glu Glu Ala
785                 790                 795                 800

Asp Thr Leu Glu Lys Leu Arg Thr Leu Leu Ala Glu Lys Leu Ser Ser
                805                 810                 815

Arg Pro Glu Ala Val His Glu Tyr Val Thr Pro Leu Phe Val Ser Arg
            820                 825                 830

Ala Pro Asn Arg Lys Met Ser Gly Gln Gly His Met Glu Thr Val Lys
        835                 840                 845

Ser Ala Lys Arg Leu Asp Glu Gly Val Ser Val Leu Arg Val Pro Leu
    850                 855                 860

Thr Gln Leu Lys Leu Lys Asp Leu Glu Lys Met Val Asn Arg Glu Arg
865                 870                 875                 880

Glu Pro Lys Leu Tyr Glu Ala Leu Lys Ala Arg Leu Glu Ala His Lys
                885                 890                 895

Asp Asp Pro Ala Lys Ala Phe Ala Glu Pro Phe Tyr Lys Tyr Asp Lys
            900                 905                 910

Ala Gly Asn Arg Thr Gln Gln Val Lys Ala Val Arg Val Glu Gln Val
        915                 920                 925

Gln Lys Thr Gly Val Trp Val Arg Asn His Asn Gly Ile Ala Asp Asn
    930                 935                 940

Ala Thr Met Val Arg Val Asp Val Phe Glu Lys Gly Asp Lys Tyr Tyr
945                 950                 955                 960

Leu Val Pro Ile Tyr Ser Trp Gln Val Ala Lys Gly Ile Leu Pro Asp
                965                 970                 975

Arg Ala Val Val Gln Gly Lys Asp Glu Glu Asp Trp Gln Leu Ile Asp
            980                 985                 990

Asp Ser Phe Asn Phe Lys Phe Ser  Leu His Pro Asn Asp  Leu Val Glu
        995                 1000                1005

Val Ile  Thr Lys Lys Ala Arg  Met Phe Gly Tyr Phe  Ala Ser Cys
    1010                1015                1020

His Arg  Gly Thr Gly Asn Ile  Asn Ile Arg Ile His  Asp Leu Asp
    1025                1030                1035

His Lys  Ile Gly Lys Asn Gly  Ile Leu Glu Gly Ile  Gly Val Lys
    1040                1045                1050

Thr Ala  Leu Ser Phe Gln Lys  Tyr Gln Ile Asp Glu  Leu Gly Lys
    1055                1060                1065

Glu Ile  Arg Pro Cys Arg Leu  Lys Lys Arg Pro Pro  Val Arg
    1070                1075                1080

<210>  48
<211>  244
<212>  PRT
<213>  Staphylococcus epidermidis

<220>
<221>  PEPTIDE
<222>  (1)..(244)
<223>  CRISPR-associated endoribonuclease Cas6: WP_002486027.1

<400>  48

Met Ile Asn Lys Ile Thr Val Glu Leu Asp Leu Pro Glu Ser Ile Arg
1               5                   10                  15

Phe Gln Tyr Leu Gly Ser Val Leu His Gly Val Leu Met Asp Tyr Leu
            20                  25                  30

Ser Asp Asp Ile Ala Asp Gln Leu His His Glu Phe Ala Tyr Ser Pro
        35                  40                  45

Leu Lys Gln Arg Ile Tyr His Lys Asn Lys Lys Ile Ile Trp Glu Ile
    50                  55                  60

Val Cys Met Ser Asp Asn Leu Phe Lys Glu Val Val Lys Leu Phe Ser
65                  70                  75                  80

Ser Lys Asn Ser Leu Leu Leu Lys Tyr Tyr Gln Thr Asn Ile Asp Ile
                85                  90                  95

Gln Ser Phe Gln Ile Glu Lys Ile Asn Val Gln Asn Met Met Asn Gln
            100                 105                 110

Leu Leu Gln Val Glu Asp Leu Ser Arg Tyr Val Arg Leu Asn Ile Gln
        115                 120                 125

Thr Pro Met Ser Phe Lys Tyr Gln Asn Ser Tyr Met Ile Phe Pro Asp
    130                 135                 140

Val Lys Arg Phe Phe Arg Ser Ile Met Ile Gln Phe Asp Ala Phe Phe
145                 150                 155                 160

Glu Glu Tyr Arg Met Tyr Asp Lys Glu Thr Leu Asn Phe Leu Glu Lys
                165                 170                 175

Asn Val Asn Ile Val Asp Tyr Lys Leu Lys Ser Thr Arg Phe Asn Leu
            180                 185                 190

Glu Lys Val Lys Ile Pro Ser Phe Thr Gly Glu Ile Val Phe Lys Ile
        195                 200                 205

Lys Gly Pro Leu Pro Phe Leu Gln Leu Thr His Phe Leu Leu Lys Phe
    210                 215                 220

Gly Glu Phe Ser Gly Ser Gly Ile Lys Thr Ser Leu Gly Met Gly Lys
225                 230                 235                 240

Tyr Ser Ile Ile

<210>  49
<211>  422
<212>  PRT
<213>  Staphylococcus epidermidis

<220>
<221>  PEPTIDE
<222>  (1)..(422)
<223>  type III-A CRISPR-associated protein Csm6: WP_002486034.1

<400>  49

Met Lys Ile Leu Phe Ser Pro Ile Gly Asn Ser Asp Pro Trp Arg Asn
1               5                   10                  15

Asp Arg Asp Gly Ala Met Leu His Ile Val Arg His Tyr Asn Leu Asp
            20                  25                  30

Lys Val Val Leu Tyr Phe Thr Arg Thr Ile Trp Glu Gly Asn Glu Asn
        35                  40                  45

Arg Lys Gly His Lys Ile Tyr Glu Trp Glu Lys Ile Ile Gln Thr Val
    50                  55                  60

Ser Pro Asn Thr Glu Val Glu Ile Ile Ile Glu Asn Val Asp Asn Ala
65                  70                  75                  80

Gln Asp Tyr Asp Val Phe Lys Glu Lys Phe His Lys Tyr Leu Lys Ile
                85                  90                  95

Ile Glu Asp Ser Tyr Glu Asp Cys Glu Ile Ile Leu Asn Val Thr Ser
            100                 105                 110

Gly Thr Pro Gln Met Glu Ser Thr Leu Cys Leu Glu Tyr Ile Val Tyr
        115                 120                 125

Pro Glu Asn Lys Lys Cys Val Gln Val Ser Thr Pro Thr Lys Asp Ser
    130                 135                 140

Asn Ala Gly Ile Glu Tyr Ser Asn Pro Lys Asp Lys Val Glu Glu Phe
145                 150                 155                 160

Glu Ile Val Asn Glu Val Glu Lys Lys Ser Glu Lys Arg Cys Lys Glu

                165                 170                 175

Ile Asn Ile Leu Ser Phe Arg Glu Ala Met Ile Arg Ser Gln Ile Leu
            180                 185                 190

Gly Leu Ile Asp Asn Tyr Asp Tyr Glu Gly Ala Leu Asn Leu Val Ser
        195                 200                 205

Asn Gln Lys Ser Phe Arg Asn Gly Lys Leu Leu Arg Lys Lys Leu Leu
    210                 215                 220

Ser Leu Thr Lys Gln Ile Lys Thr His Glu Val Phe Pro Glu Ile Asn
225                 230                 235                 240

Glu Lys Tyr Arg Asp Asp Ala Leu Lys Lys Ser Leu Phe His Tyr Leu
                245                 250                 255

Leu Leu Asn Met Arg Tyr Asn Arg Leu Asp Val Ala Glu Thr Leu Ile
            260                 265                 270

Arg Val Lys Ser Ile Ala Glu Phe Ile Leu Lys Thr Tyr Ile Glu Ile
        275                 280                 285

His Trp Pro Thr Leu Ile Ile Glu Lys Asp Gly Lys Pro Tyr Leu Asn
    290                 295                 300

Asp Glu Asp Asn Leu Ser Phe Val Tyr Lys Tyr Asn Leu Leu Leu Glu
305                 310                 315                 320

Lys Arg Lys Gln Asn Phe Asp Val Ser Arg Ile Leu Gly Leu Pro Ala
                325                 330                 335

Phe Ile Asp Ile Leu Thr Ile Leu Glu Pro Asn Ser Gln Leu Leu Lys
            340                 345                 350

Glu Val Asn Ala Val Asn Asp Ile Asn Gly Leu Arg Asn Ser Ile Ala
        355                 360                 365

His Asn Leu Asp Thr Leu Asn Leu Asp Lys Asn Lys Asn Tyr Lys Lys
    370                 375                 380

Ile Met Leu Ser Val Glu Ala Ile Lys Asn Met Leu His Ile Ser Phe
385                 390                 395                 400

Pro Glu Ile Glu Glu Glu Asp Tyr Asn Tyr Phe Glu Glu Lys Asn Lys
                405                 410                 415

Glu Phe Lys Glu Leu Leu
            420

<210>  50
<211>  340
<212>  PRT
<213>  Staphylococcus epidermidis

<220>
<221>  PEPTIDE
<222>  (1)..(340)
<223>  type III-A CRISPR-associated RAMP protein Csm5: WP_002486031.1

<400>  50

Met Thr Ile Lys Asn Tyr Glu Val Val Ile Lys Thr Leu Gly Pro Ile
1               5                   10                  15

His Ile Gly Ser Gly Gln Val Met Lys Lys Gln Asp Tyr Ile Tyr Asp
            20                  25                  30

Phe Tyr Asn Ser Lys Val Tyr Met Ile Asn Gly Asn Lys Leu Val Lys
        35                  40                  45

Phe Leu Lys Arg Lys Asn Leu Leu Tyr Thr Tyr Gln Asn Phe Leu Arg
    50                  55                  60

Tyr Pro Pro Lys Asn Pro Arg Glu Asn Gly Leu Lys Asp Tyr Leu Asp
65                  70                  75                  80

Ala Gln Asn Val Lys Gln Ser Glu Trp Glu Ala Phe Val Ser Tyr Ser
                85                  90                  95

Glu Lys Val Asn Gln Gly Lys Lys Tyr Gly Asn Thr Arg Pro Lys Pro
            100                 105                 110

Leu Asn Asp Leu His Leu Met Val Arg Asp Gly Gln Asn Lys Val Tyr
        115                 120                 125

Leu Pro Gly Ser Ser Ile Lys Gly Ala Ile Lys Thr Thr Leu Val Ser
    130                 135                 140

Lys Tyr Asn Asn Glu Lys Asn Lys Asp Ile Tyr Ser Lys Ile Lys Val
145                 150                 155                 160

Ser Asp Ser Lys Pro Ile Asp Glu Ser Asn Leu Ala Ile Tyr Gln Lys
                165                 170                 175

Ile Asp Ile Asn Lys Ser Glu Lys Ser Met Pro Leu Tyr Arg Glu Cys
            180                 185                 190

Ile Asp Val Asn Thr Glu Ile Lys Phe Lys Leu Thr Ile Glu Asp Glu
        195                 200                 205

Ile Tyr Ser Ile Asn Glu Ile Glu Gln Ser Ile Gln Asp Phe Tyr Lys
    210                 215                 220

Asn Tyr Tyr Asp Lys Trp Leu Val Gly Phe Lys Glu Thr Lys Gly Gly
225                 230                 235                 240

Arg Arg Phe Ala Leu Glu Gly Gly Ile Pro Asp Val Leu Asn Gln Asn
                245                 250                 255

Ile Leu Phe Leu Gly Ala Gly Thr Gly Phe Val Ser Lys Thr Thr His
            260                 265                 270

Tyr Gln Leu Lys Asn Arg Lys Gln Ala Lys Gln Asp Ser Phe Glu Ile
        275                 280                 285

Leu Thr Lys Lys Phe Arg Gly Thr Tyr Gly Lys Met Lys Glu Ile Pro
    290                 295                 300

Ser Asn Val Pro Val Ala Leu Lys Gly Thr Thr Asn Gln Ser Arg His
305                 310                 315                 320

Thr Ser Tyr Gln Gln Gly Met Cys Lys Val Ser Phe Gln Glu Leu Asn
                325                 330                 335

Asn Glu Val Leu
            340

<210> 51
<211> 302
<212> PRT
<213> Staphylococcus epidermidis

<220>
<221> PEPTIDE
<222> (1)..(302)
<223> type III-A CRISPR-associated RAMP protein Csm4: WP_002486041.1

<400> 51

Met Ala Thr Lys Val Phe Lys Leu Ser Phe Lys Thr Pro Val His Phe
1               5                   10                  15

Gly Lys Lys Arg Leu Ser Asp Gly Glu Met Thr Ile Thr Ala Asp Thr
            20                  25                  30

Leu Phe Ser Ala Leu Phe Ile Glu Thr Leu Gln Leu Gly Lys Asp Thr
        35                  40                  45

Asp Trp Leu Leu Asn Asp Leu Ile Ile Ser Asp Thr Phe Pro Tyr Glu
    50                  55                  60

Asn Glu Leu Tyr Tyr Leu Pro Lys Pro Leu Ile Lys Ile Asp Ser Lys
65                  70                  75                  80

Glu Glu Asp Asn His Lys Ala Phe Lys Lys Leu Lys Tyr Val Pro Val
                85                  90                  95

His His Tyr Asn Gln Tyr Leu Asn Gly Glu Leu Ser Ala Glu Asp Ala
            100                 105                 110

Thr Asp Leu Asn Asp Ile Phe Asn Ile Gly Tyr Phe Ser Leu Gln Thr
        115                 120                 125

Lys Val Ser Leu Ile Ala Gln Glu Thr Asp Ser Ser Ala Asp Ser Glu
    130                 135                 140

Pro Tyr Ser Val Gly Thr Phe Thr Phe Glu Pro Glu Ala Gly Leu Tyr
145                 150                 155                 160

Phe Ile Ala Lys Gly Ser Glu Glu Thr Leu Asp His Leu Asn Asn Ile
                165                 170                 175

Met Thr Ala Leu Gln Tyr Ser Gly Leu Gly Gly Lys Arg Asn Ala Gly
            180                 185                 190

Tyr Gly Gln Phe Glu Tyr Glu Ile Ile Asn Asn Gln Gln Leu Ser Lys
        195                 200                 205

Leu Leu Asn Gln Asn Gly Lys His Ser Ile Leu Leu Ser Thr Ala Met
    210                 215                 220

Ala Lys Lys Glu Glu Ile Glu Ser Ala Leu Lys Glu Ala Arg Tyr Ile
225                 230                 235                 240

Leu Thr Lys Arg Ser Gly Phe Val Gln Ser Thr Asn Tyr Ser Glu Met
                245                 250                 255

Leu Val Lys Lys Ser Asp Phe Tyr Ser Phe Ser Ser Gly Ser Val Phe
            260                 265                 270

Lys Asn Ile Phe Asn Gly Asp Ile Phe Asn Val Gly His Asn Gly Lys
        275                 280                 285

His Pro Val Tyr Arg Tyr Ala Lys Pro Leu Trp Leu Glu Val
    290                 295                 300

<210>  52
<211>  214
<212>  PRT
<213>  Staphylococcus epidermidis

<220>
<221>  PEPTIDE
<222>  (1)..(214)
<223>  type III-A CRISPR-associated RAMP protein Csm3: WP_002486018.1

<400>  52

Met Tyr Ser Lys Ile Lys Ile Ser Gly Thr Ile Glu Val Val Thr Gly
1               5                   10                  15

Leu His Ile Gly Gly Gly Gly Glu Ser Ser Met Ile Gly Ala Ile Asp
            20                  25                  30

Ser Pro Val Val Arg Asp Leu Gln Thr Lys Leu Pro Ile Ile Pro Gly
        35                  40                  45

Ser Ser Ile Lys Gly Lys Met Arg Asn Leu Leu Ala Lys His Phe Gly
    50                  55                  60

Leu Lys Met Lys Gln Glu Ser His Asn Gln Asp Asp Glu Arg Val Leu
65                  70                  75                  80

Arg Leu Phe Gly Ser Ser Glu Lys Gly Asn Ile Gln Arg Ala Arg Leu
                85                  90                  95

Gln Ile Ser Asp Ala Phe Phe Ser Glu Lys Thr Lys Glu His Phe Ala
            100                 105                 110

Gln Asn Asp Ile Ala Tyr Thr Glu Thr Lys Phe Glu Asn Thr Ile Asn
        115                 120                 125

Arg Leu Thr Ala Val Ala Asn Pro Arg Gln Ile Glu Arg Val Thr Arg
    130                 135                 140

Gly Ser Glu Phe Asp Phe Val Phe Ile Tyr Asn Val Asp Glu Glu Ser
145                 150                 155                 160

Gln Val Glu Asp Asp Phe Glu Asn Ile Glu Lys Ala Ile His Leu Leu
                165                 170                 175

Glu Asn Asp Tyr Leu Gly Gly Gly Gly Thr Arg Gly Asn Gly Arg Ile

180                 185                 190

Gln Phe Lys Asp Thr Asn Ile Glu Thr Val Val Gly Glu Tyr Asp Ser
        195                 200                 205

Thr Asn Leu Lys Ile Lys
    210

<210>  53
<211>  141
<212>  PRT
<213>  Staphylococcus epidermidis

<220>
<221>  PEPTIDE
<222>  (1)..(141)
<223>  type III-A CRISPR-associated protein Csm2
       /protein_id="WP_002486044.1"

<400>  53

Met Ile Leu Ala Lys Thr Lys Ser Gly Lys Thr Ile Asp Leu Thr Phe
1               5                   10                  15

Ala His Glu Val Val Lys Ser Asn Val Lys Asn Val Lys Asp Arg Lys
            20                  25                  30

Gly Lys Glu Lys Gln Val Leu Phe Asn Gly Leu Thr Thr Ser Lys Leu
        35                  40                  45

Arg Asn Leu Met Glu Gln Val Asn Arg Leu Tyr Thr Ile Ala Phe Asn
    50                  55                  60

Ser Asn Glu Asp Gln Leu Asn Glu Glu Phe Ile Asp Glu Leu Glu Tyr
65                  70                  75                  80

Leu Lys Ile Lys Phe Tyr Tyr Glu Ala Gly Arg Glu Lys Ser Val Asp
                85                  90                  95

Glu Phe Leu Lys Lys Thr Leu Met Phe Pro Ile Ile Asp Arg Val Ile
            100                 105                 110

Lys Lys Glu Ser Lys Lys Phe Phe Leu Asp Tyr Cys Lys Tyr Phe Glu
        115                 120                 125

Ala Leu Val Ala Tyr Ala Lys Tyr Tyr Gln Lys Glu Asp
    130                 135                 140

<210>  54
<211>  757

<212>  PRT
<213>  Staphylococcus epidermidis

<220>
<221>  PEPTIDE
<222>  (1)..(757)
<223>  type III-A CRISPR-associated protein Cas10/Csm1
       /protein_id=WP_002486045.1

<400>  54

Met Asn Lys Lys Asn Ile Leu Met Tyr Gly Ser Leu Leu His Asp Ile
1               5                   10                  15

Gly Lys Ile Ile Tyr Arg Ser Gly Asp His Thr Phe Ser Arg Gly Thr
            20                  25                  30

His Ser Lys Leu Gly His Gln Phe Leu Ser Gln Phe Ser Glu Phe Lys
        35                  40                  45

Asp Asn Glu Val Leu Asp Asn Val Ala Tyr His His Tyr Lys Glu Leu
    50                  55                  60

Ala Lys Ala Asn Leu Asp Asn Asp Asn Thr Ala Tyr Ile Thr Tyr Ile
65                  70                  75                  80

Ala Asp Asn Ile Ala Ser Gly Ile Asp Arg Arg Asp Ile Ile Glu Glu
                85                  90                  95

Gly Asp Glu Glu Tyr Glu Lys Gln Leu Phe Asn Phe Asp Lys Tyr Thr
            100                 105                 110

Pro Leu Tyr Ser Val Phe Asn Ile Val Asn Ser Glu Lys Leu Lys Gln
        115                 120                 125

Thr Asn Gly Lys Phe Lys Phe Ser Asn Glu Ser Asn Ile Glu Tyr Pro
    130                 135                 140

Lys Thr Glu Asn Ile Gln Tyr Ser Ser Gly Asn Tyr Thr Thr Leu Met
145                 150                 155                 160

Lys Asp Met Ser His Asp Leu Glu His Lys Leu Ser Ile Lys Glu Gly
                165                 170                 175

Thr Phe Pro Ser Leu Leu Gln Trp Thr Glu Ser Leu Trp Gln Tyr Val
            180                 185                 190

Pro Ser Ser Thr Asn Lys Asn Gln Leu Ile Asp Ile Ser Leu Tyr Asp
        195                 200                 205

His Ser Arg Ile Thr Cys Ala Ile Ala Ser Cys Ile Phe Asp Tyr Leu
    210                 215                 220

Asn Glu Asn Asn Ile His Asn Tyr Lys Asp Glu Leu Phe Ser Lys Tyr
225                 230                 235                 240

Glu Asn Thr Lys Ser Phe Tyr Gln Lys Glu Ala Phe Leu Leu Leu Ser
                245                 250                 255

Met Asp Met Ser Gly Ile Gln Asp Phe Ile Tyr Asn Ile Ser Gly Ser
            260                 265                 270

Lys Ala Leu Lys Ser Leu Arg Ser Arg Ser Phe Tyr Leu Glu Leu Met
        275                 280                 285

Leu Glu Val Ile Val Asp Gln Leu Leu Glu Arg Leu Glu Leu Ala Arg
    290                 295                 300

Ala Asn Leu Leu Tyr Thr Gly Gly Gly His Ala Tyr Leu Leu Val Ser
305                 310                 315                 320

Asn Thr Asp Lys Val Lys Lys Lys Ile Thr Gln Phe Asn Asn Glu Leu
                325                 330                 335

Lys Lys Trp Phe Met Ser Glu Phe Thr Thr Asp Leu Ser Leu Ser Met
            340                 345                 350

Ala Phe Glu Lys Cys Ser Gly Asp Asp Leu Met Asn Thr Ser Gly Asn
        355                 360                 365

Tyr Arg Thr Ile Trp Arg Asn Val Ser Ser Lys Leu Ser Asp Ile Lys
    370                 375                 380

Ala His Lys Tyr Ser Ala Glu Asp Ile Leu Lys Leu Asn His Phe His
385                 390                 395                 400

Ser Tyr Gly Asp Arg Glu Cys Lys Glu Cys Leu Arg Ser Asp Ile Asp
                405                 410                 415

Ile Asn Asp Asp Gly Leu Cys Ser Ile Cys Glu Gly Ile Ile Asn Ile
            420                 425                 430

Ser Asn Asp Leu Arg Asp Lys Ser Phe Phe Val Leu Ser Glu Thr Gly
        435                 440                 445

Lys Leu Lys Met Pro Phe Asn Lys Phe Ile Ser Val Ile Asp Tyr Glu
    450                 455                 460

Glu Ala Glu Met Leu Val Gln Asn Asn Asn Gln Val Arg Ile Tyr Ser
465                 470                 475                 480

Lys Asn Lys Pro Tyr Ile Gly Ile Gly Ile Ser Thr Asn Leu Trp Met
                485                 490                 495

Cys Asp Tyr Asp Tyr Ala Ser Gln Asn Gln Asp Met Arg Glu Lys Gly
            500                 505                 510

Ile Gly Ser Tyr Val Asp Arg Glu Glu Gly Val Lys Arg Leu Gly Val
        515                 520                 525

Val Arg Ala Asp Ile Asp Asn Leu Gly Ala Thr Phe Ile Ser Gly Ile
    530                 535                 540

Pro Glu Lys Tyr Asn Ser Ile Ser Arg Thr Ala Thr Leu Ser Arg Gln
545                 550                 555                 560

Leu Ser Leu Phe Phe Lys Tyr Glu Leu Asn His Leu Leu Glu Asn Tyr
                565                 570                 575

Gln Ile Thr Ala Ile Tyr Ser Gly Gly Asp Asp Leu Phe Leu Ile Gly
            580                 585                 590

Ala Trp Asp Asp Ile Ile Glu Ala Ser Ile Tyr Ile Asn Asp Lys Phe
        595                 600                 605

Lys Glu Phe Thr Leu Asp Lys Leu Thr Leu Ser Ala Gly Val Gly Met
    610                 615                 620

Phe Ser Gly Lys Tyr Pro Val Ser Lys Met Ala Phe Glu Thr Gly Arg
625                 630                 635                 640

Leu Glu Glu Ala Ala Lys Thr Gly Glu Lys Asn Gln Ile Ser Leu Trp
                645                 650                 655

Leu Gln Glu Lys Val Tyr Asn Trp Asp Glu Phe Lys Lys Asn Ile Leu
            660                 665                 670

Glu Glu Lys Leu Leu Val Leu Gln Gln Gly Phe Ser Gln Thr Asp Glu
        675                 680                 685

His Gly Lys Ala Phe Ile Tyr Lys Met Leu Ala Leu Leu Arg Asn Asn
    690                 695                 700

Glu Ala Ile Asn Ile Ala Arg Leu Ala Tyr Leu Leu Ala Arg Ser Lys

705                 710                 715                 720

Met Asn Glu Asp Phe Thr Ser Lys Ile Phe Asn Trp Ala Gln Asn Asp
                725                 730                 735

Lys Asp Lys Asn Gln Leu Ile Thr Ala Leu Glu Tyr Tyr Ile Tyr Gln
            740                 745                 750

Ile Arg Glu Ala Asp
        755

<210>  55
<211>  101
<212>  PRT
<213>  Staphylococcus epidermidis

<220>
<221>  PEPTIDE
<222>  (1)..(101)
<223>  CRISPR-associated endonuclease Cas2 /protein_id=WP_002469399.1

<400>  55

Met Tyr Leu Leu Val Ser Phe Asp Leu Pro Arg Asp Thr Lys Leu Glu
1               5                   10                  15

Arg Arg Ile Ala Ser Lys Tyr Arg Leu Arg Leu Ile Glu Leu Gly Phe
            20                  25                  30

Ser Met Lys Gln Phe Ser Leu Tyr Glu Arg Tyr Val Ser Asn Val Gln
        35                  40                  45

Lys Lys Asp Lys Ile Leu Glu Ile Leu Lys Gln Glu Ile Pro Asp Thr
    50                  55                  60

Gly Ser Ile Thr Leu Tyr Ile Leu Pro Asp Glu Val Asn Ser Asn Gln
65                  70                  75                  80

Ile Thr Ile Leu Gly Lys Glu Val Lys Leu Ala Val Asn Lys Glu Pro
                85                  90                  95

Lys Leu Ile Phe Ile
            100

<210>  56
<211>  301
<212>  PRT
<213>  Staphylococcus epidermidis

<220>

<221>  PEPTIDE
<222>  (1)..(301)
<223>  type II CRISPR-associated endonuclease Cas1:
       /protein_id=WP_002486040.1

<400>  56

Met Lys Asp Val Ile Tyr Val Glu Asn His Tyr Phe Val Thr Ala Lys
1               5                   10                  15

Glu Asn Ser Ile Lys Phe Arg Asn Val Ile Asp Lys Ser Glu Lys Phe
            20                  25                  30

Tyr Leu Phe Glu Glu Ile Glu Ala Ile Ile Phe Asp His Tyr Lys Ser
        35                  40                  45

Tyr Phe Ser His Lys Leu Val Ile Lys Cys Ile Glu Asn Asp Ile Ala
    50                  55                  60

Ile Ile Phe Cys Asp Lys Lys His Ser Pro Val Thr Gln Leu Ile Ser
65                  70                  75                  80

Ser Tyr Gly Met Val Asn Arg Leu Lys Arg Ile Gln Ser Gln Phe Gln
                85                  90                  95

Leu Ser Gly Arg Thr Lys Asp Arg Ile Trp Lys Lys Ile Val Ile Asn
            100                 105                 110

Lys Ile Phe Asn Gln Thr Arg Cys Leu Glu Asn Asn Leu His Asn Glu
        115                 120                 125

Asn Val Lys Leu Met Leu Gly Phe Ala Lys Glu Val Ser Ser Gly Asp
    130                 135                 140

Lys Ser Asn Lys Glu Ala His Ala Thr Arg Ile Tyr Phe Lys Asp Leu
145                 150                 155                 160

Phe Gly Lys Gln Phe Lys Arg Gly Arg Tyr Asn Asp Val Ile Asn Ser
                165                 170                 175

Gly Leu Asn Tyr Gly Tyr Ser Ile Leu Arg Ser Phe Ile Asn Lys Glu
            180                 185                 190

Leu Ala Ile His Gly Phe Glu Met Ser Leu Gly Ile Lys His Gln Ser
        195                 200                 205

Lys Glu Asn Pro Phe Asn Leu Ala Asp Asp Ile Ile Glu Val Phe Arg
    210                 215                 220

Pro Phe Val Asp Asn Ile Val Tyr Glu Ile Val Phe Lys Lys Asn Ile
225                 230                 235                 240

Asp Thr Phe Asp Ile Asn Glu Lys Lys Leu Leu Leu Asn Val Leu Tyr
                245                 250                 255

Glu Arg Cys Ile Ile Asp Lys Lys Val Val Arg Leu Leu Asp Ser Val
            260                 265                 270

Lys Ile Val Ile Gln Ser Leu Ile Arg Cys Tyr Glu Glu Asn Thr Pro
        275                 280                 285

Thr Tyr Leu Leu Leu Pro Lys Met Ile Glu Val Gly Asn
    290                 295                 300

<210>  57
<211>  340
<212>  PRT
<213>  Pyrococcus furiosus

<220>
<221>  PEPTIDE
<222>  (1)..(340)
<223>  type III-B CRISPR module RAMP protein Cmr6:
       /protein_id=WP_011012264.1

<400>  57

Met Lys Glu Val Val Lys Leu Val Leu Leu Gly Glu Arg Gln Asn Ser
1               5                   10                  15

Leu Asn Leu Ser Leu Tyr Phe Asn Lys Tyr Pro Pro Thr Ile Ile Tyr
            20                  25                  30

Pro Glu Val Leu Glu Asp Arg Asn Lys Lys Leu Ala Ser Pro Ser Gly
        35                  40                  45

Ser Gln Arg Lys Ile Ser Leu Leu Val Leu Asn Gln Gly Val Leu Gln
    50                  55                  60

Phe Asn Lys Ile Lys Glu Thr Ile Glu Lys Ser Leu Pro Ile Glu Thr
65                  70                  75                  80

Lys Val Lys Leu Pro Gln Lys Ala Tyr Glu Leu Tyr Lys Lys Tyr Tyr
                85                  90                  95

Gln Asp Tyr Thr Asp Met Leu Asn Ser Leu His Ala Ile Thr Gly Lys
            100                 105                 110

Phe Lys Thr Gln Ser Arg Leu Val Val Gly Leu Gly Asp Glu Ser Val

115                 120                 125

Tyr Glu Thr Ser Ile Arg Leu Leu Arg Asn Tyr Gly Val Pro Tyr Ile
    130                 135                 140

Pro Gly Ser Ala Ile Lys Gly Val Thr Arg His Leu Thr Tyr Tyr Val
145                 150                 155                 160

Leu Ala Glu Phe Ile Asn Glu Gly Asn Asp Phe Tyr Lys Arg Ala Lys
                165                 170                 175

Thr Val Gln Asp Ala Phe Met Lys Gly Asp Pro Lys Glu Ile Leu Ser
            180                 185                 190

Asn Ala Lys Val Pro Glu Arg Cys Ser Arg Leu Cys Lys Glu Phe Leu
        195                 200                 205

Arg Ile Phe Gly Glu Lys Lys Val Pro Glu Ile Ile Asp Glu Leu Ile
    210                 215                 220

Arg Ile Phe Gly Thr Gln Lys Lys Glu Gly Glu Val Val Phe Phe Asp
225                 230                 235                 240

Ala Ile Pro Ile Ala Glu Glu Ile Ala Asp Lys Pro Ile Leu Glu Leu
                245                 250                 255

Asp Ile Met Asn Pro His Tyr Gly Pro Tyr Tyr Gln Ser Gly Glu Lys
            260                 265                 270

Asn Val Pro Pro Pro Gly Asp Trp Tyr Asp Pro Ile Pro Ile Phe Phe
        275                 280                 285

Leu Thr Val Pro Lys Asp Val Pro Phe Leu Val Ala Val Gly Gly Arg
    290                 295                 300

Asp Arg Glu Leu Thr Glu Lys Ala Phe Ser Leu Val Lys Leu Ala Leu
305                 310                 315                 320

Arg Asp Leu Gly Val Gly Ala Lys Thr Ser Leu Gly Tyr Gly Arg Leu
                325                 330                 335

Val Glu Tyr Val
            340

<210>  58
<211>  169
<212>  PRT
<213>  Pyrococcus furiosus

<220>
<221>  PEPTIDE
<222>  (1)..(169)
<223>  type III-B CRISPR module-associated protein  Cmr5:
       /protein_id=WP_011012265.1

<400>  58

Met Glu Val His Met Leu Ser Lys Asp Asn Lys Lys Ser Ile Arg Lys
1               5                   10                  15

Thr Leu Glu Gln Arg Arg Gly Glu Tyr Ala Tyr Tyr Val Ile Lys Glu
            20                  25                  30

Val Ala Asp Leu Asn Asp Lys Gln Leu Glu Glu Lys Tyr Ala Ser Leu
        35                  40                  45

Val Lys Lys Ala Pro Val Met Ile Leu Ser Asn Gly Leu Leu Gln Thr
    50                  55                  60

Leu Ala Phe Leu Leu Ala Lys Ala Glu Thr Ser Pro Glu Lys Ala Asn
65                  70                  75                  80

Gln Ile Leu Ser Arg Val Asn Glu Tyr Pro Pro Arg Phe Ile Glu Lys
                85                  90                  95

Leu Gly Asn Asp Lys Asp Glu His Leu Leu Leu Tyr Leu His Ile Val
            100                 105                 110

Tyr Trp Leu Arg Glu Asn Val Asp Arg Asn Ile Asp Val Lys Thr Leu
        115                 120                 125

Leu Ser Gln Asp Tyr Ser Lys Val Leu Trp Ala Thr Lys Glu Ala Ile
    130                 135                 140

Ala Leu Leu Asn Trp Met Arg Arg Phe Ala Val Ala Met Leu Lys Glu
145                 150                 155                 160

Glu Gly Lys Glu Asn Glu Gly Ser Ser
                165

<210>  59
<211>  295
<212>  PRT
<213>  Pyrococcus furiosus

<220>
<221>  PEPTIDE
<222>  (1)..(295)

<223>  type III-B CRISPR module RAMP protein Cmr4:
       /protein_id=WP_011012266.1

<400>  59

Met Lys Ala Tyr Leu Val Gly Leu Tyr Thr Leu Thr Pro Thr His Pro
1               5                   10                  15

Gly Ser Gly Thr Glu Leu Gly Val Val Asp Gln Pro Ile Gln Arg Glu
            20                  25                  30

Arg His Thr Gly Phe Pro Val Ile Trp Gly Gln Ser Leu Lys Gly Val
        35                  40                  45

Leu Arg Ser Tyr Leu Lys Leu Val Glu Lys Val Asp Glu Glu Lys Ile
    50                  55                  60

Asn Lys Ile Phe Gly Pro Pro Thr Glu Lys Ala His Glu Gln Ala Gly
65                  70                  75                  80

Leu Ile Ser Val Gly Asp Ala Lys Ile Leu Phe Phe Pro Val Arg Ser
                85                  90                  95

Leu Lys Gly Val Tyr Ala Tyr Val Thr Ser Pro Leu Val Leu Asn Arg
            100                 105                 110

Phe Lys Arg Asp Leu Glu Leu Ala Gly Val Lys Asn Phe Gln Thr Glu
        115                 120                 125

Ile Pro Glu Leu Thr Asp Thr Ala Ile Ala Ser Glu Glu Ile Thr Val
    130                 135                 140

Asp Asn Lys Val Ile Leu Glu Glu Phe Ala Ile Leu Ile Gln Lys Asp
145                 150                 155                 160

Asp Lys Gly Ile Leu Glu Ser Val Val Lys Ala Ile Glu Gln Ala Phe
                165                 170                 175

Gly Asn Glu Met Ala Glu Lys Ile Lys Gly Arg Ile Ala Ile Ile Pro
            180                 185                 190

Asp Asp Val Phe Arg Asp Leu Val Glu Leu Ser Thr Glu Ile Val Ala
        195                 200                 205

Arg Ile Arg Ile Asn Ala Glu Thr Gly Thr Val Glu Thr Gly Gly Leu
    210                 215                 220

Trp Tyr Glu Glu Tyr Ile Pro Ser Asp Thr Leu Phe Tyr Ser Leu Ile
225                 230                 235                 240

Leu Val Thr Pro Arg Ala Lys Asp Asn Asp Met Ala Leu Ile Lys Glu
                245                 250                 255

Val Leu Gly Lys Ile Asn Gly Lys Tyr Leu Gln Ile Gly Gly Asn Glu
            260                 265                 270

Thr Val Gly Lys Gly Phe Val Lys Val Thr Leu Lys Glu Val Thr Asn
        275                 280                 285

Asn Gly Gly Thr His Ala Lys
    290                 295

<210>  60
<211>  480
<212>  PRT
<213>  Pyrococcus furiosus

<220>
<221>  PEPTIDE
<222>  (1)..(480)
<223>  TIGR01897 family CRISPR-associated protein:
       /protein_id=WP_011012267.1

<400>  60

Met Gly Met Arg Val Leu Val Thr Thr Trp Gly Asn Pro Phe Gln Trp
1               5                   10                  15

Glu Pro Ile Thr Tyr Glu Tyr Arg Gly Ile Lys Val Lys Ser Arg Asn
            20                  25                  30

Thr Leu Pro Ile Leu Val Lys Thr Leu Glu Pro Glu Arg Ile Leu Ile
        35                  40                  45

Leu Val Ala Asp Thr Met Ala Asn Tyr Tyr Asp Ser Gly Lys Asn Lys
    50                  55                  60

Pro Glu Ile Glu Glu Lys Ser Phe Ser Ser Tyr Ser Glu Val Val Glu
65                  70                  75                  80

Asp Thr Lys Glu Arg Ile Leu Trp His Ile Lys Glu Glu Val Ile Glu
                85                  90                  95

Glu Leu Arg Glu Glu Asp Pro Glu Leu Ala Lys Lys Ile Glu Asn Met
            100                 105                 110

Leu Lys Asp Glu Arg Ile Thr Ile Glu Val Leu Pro Gly Val Gly Val
        115                 120                 125

Phe Gly Asn Ile Thr Val Glu Gly Glu Met Leu Asp Phe Tyr Tyr Tyr
    130                 135                 140

Ala Thr Tyr Lys Leu Ala Glu Trp Leu Pro Val Gln Asn Asn Leu Glu
145                 150                 155                 160

Val Tyr Leu Asp Leu Thr His Gly Ile Asn Phe Met Pro Thr Phe Thr
                165                 170                 175

Tyr Arg Ala Leu Arg Asn Leu Leu Gly Leu Leu Ala Tyr Leu Tyr Asn
            180                 185                 190

Val Lys Phe Glu Ile Val Asn Ser Glu Pro Tyr Pro Leu Gly Val Ser
        195                 200                 205

Gln Glu Ile Arg Glu Asp Thr Ile Leu His Ile Arg Glu Ile Gly Glu
    210                 215                 220

Gly Val Val Arg Pro Arg Pro Gln Tyr Ser Pro Val Glu Gly Lys Leu
225                 230                 235                 240

Tyr Trp Asn Ala Phe Ile Ser Ser Val Ala Asn Gly Phe Pro Leu Val
                245                 250                 255

Phe Ala Ser Phe Tyr Pro Asn Ile Arg Asp Val Glu Asp Tyr Leu Asn
            260                 265                 270

Lys Lys Leu Glu Glu Phe Leu Val Gly Ile Glu Val Gly Glu Arg Glu
        275                 280                 285

Asp Gly Lys Pro Tyr Val Lys Arg Glu Lys Ala Leu Asp Arg Ser Phe
    290                 295                 300

Lys Asn Ala Ser Lys Leu Tyr Tyr Ala Leu Arg Val Phe Asn Thr Lys
305                 310                 315                 320

Phe Gln Asn Tyr Pro Lys Lys Glu Val Pro Ile Glu Glu Ile Met Glu
                325                 330                 335

Ile Ser Lys Ile Phe Glu Ser Leu Pro Arg Ile Gly Ile Ile Leu Glu
            340                 345                 350

Arg Gln Val Glu Trp Leu Arg Asn Leu Val Tyr Gly Arg Leu Trp Tyr
        355                 360                 365

Glu Asn Gly Glu Gln Lys Ile Lys Lys Gly Leu Leu Glu Ile Ile Lys
    370                 375                 380

Asp Lys Lys Asp Lys Arg Lys Glu Ala Glu Ala Leu Lys Lys Gly Lys
385                 390                 395                 400

Thr Ile Ser Leu Ala Glu Ala Ala Lys Leu Thr Arg Ile Phe Ser Pro
                405                 410                 415

Ser Gly Glu Arg Ile Glu Thr Ile Glu Ser Pro Asn Val Val Arg Asn
            420                 425                 430

Phe Ile Ala His Ser Gly Phe Glu Tyr Asn Ile Val Tyr Val Lys Tyr
        435                 440                 445

Asp Arg Leu Ser Asp Arg Leu Tyr Phe Phe Tyr Lys Asp Lys Glu Lys
    450                 455                 460

Ala Ala Asn Leu Ala Tyr Glu Ala Leu Leu Tyr Arg Gly Glu Lys Glu
465                 470                 475                 480

<210>  61
<211>  322
<212>  PRT
<213>  Pyrococcus furiosus

<220>
<221>  PEPTIDE
<222>  (1)..(322)
<223>  type III-B CRISPR module-associated protein Cmr3:
       /protein_id=WP_011012268.1

<400>  61
   Met Ile Glu Val Thr Phe Thr Pro Tyr Asp Val Leu Leu Phe Arg Glu
   1               5                   10                  15
   Ser Arg Pro Phe Asp Ala Gly Ser Glu Ser Val Ala Arg Ser Ile Ile
               20                  25                  30
   Pro Leu Pro Gln Thr Val Ala Gly Ala Ile Arg Thr Leu Leu Phe Tyr
           35                  40                  45
   Lys Gly Leu Lys Asn Cys Val Gly Val Gly Glu Glu Glu Pro Glu Phe
       50                  55                  60
   Thr Leu Val Gly Ile Ala Ile Gly Thr Glu Lys Gly Arg Ile Tyr Pro
   65                  70                  75                  80
   Leu Pro Phe Asn Ile Ile Lys Ser Glu Lys Phe Tyr Lys Val Val Asn
                   85                  90                  95
   Pro Gly Arg Phe Leu Gly Lys Leu Ile Leu Pro Pro Lys Gly Lys Tyr
               100                 105                 110
   Lys Ser Gly Tyr Val Thr Glu Ser Ile Leu Glu Lys Tyr Leu Lys Gly
           115                 120                 125
   Glu Leu Lys Glu Val Glu Glu Asn Lys Val Ile Arg Ile Glu Lys Glu
       130                 135                 140
   Lys Arg Ile Gly Ile Lys Leu Ser Arg Glu Lys Lys Val Val Glu Glu
   145                 150                 155                 160
   Gly Met Leu Tyr Thr Val Glu Phe Leu Arg Ile Glu Lys Ile Tyr Ala
                   165                 170                 175
   Trp Ile Glu Asp Pro Gly Cys Gly Ile Lys Asp Ile Leu Ser Ser Tyr
               180                 185                 190
   Glu Phe Leu Thr Leu Gly Gly Glu Ser Arg Val Ala Phe Val Glu Val
           195                 200                 205
   Asp Asp Lys Thr Pro Asp Ile Phe Asn Arg Glu Leu Gly Ser Thr Lys
       210                 215                 220
   Lys Ala Leu Phe Tyr Phe Ser Thr Pro Thr Ile Gly Lys Val Gly Glu
   225                 230                 235                 240
   Ile Val Gln Glu Leu Glu Lys Arg Leu Asn Ala Lys Ile Asp Asp Tyr
                   245                 250                 255
   Leu Leu Val Ser Ser Arg Pro Thr Ala Ile Ser Gly Trp Asp Met His
               260                 265                 270
   Glu Lys Lys Pro Lys Gly Thr Lys Phe Ala Ile Pro Pro Gly Ser Val
           275                 280                 285
   Leu Phe Val Glu Phe Lys Glu Glu Val Glu Val Pro Pro Tyr Ile Lys
       290                 295                 300
   Leu Gly Lys Leu Lys Lys Leu Gly Tyr Gly Leu Ala Leu Gly Gly Ile
   305                 310                 315                 320
   Trp Glu

<210>  62
<211>  871

<212>  PRT
<213>  Pyrococcus furiosus



<220>
<221>  PEPTIDE
<222>  (1)..(871)
<223>  type III-B CRISPR-associated protein
       Cas10/Cmr2:/protein_id=WP_011012269.1

<400>  62

Met Val Asn Ile Lys Glu Lys Leu Phe Val Tyr Leu His Asp Pro Pro
1               5                   10                  15


Asp Lys Ala Leu Lys Ile Glu Asn His Glu Glu Arg Ser Lys Lys Ile
            20                  25                  30


Leu Ser Ser Gly Asn Ile Gln Tyr Ser Arg Thr Asp Lys Val Lys Gln
        35                  40                  45


Ala Asp Ala Leu Ser Ser Lys Thr Gln Arg Phe Ile Ile Arg Thr Lys
    50                  55                  60


Glu Asn Lys Glu Pro Val Ile Asp Phe Leu Gly Arg Ser Ser Gly Lys
65                  70                  75                  80


Tyr Phe His Val Gly Tyr Pro Val Phe Ile His Pro Ile Ser Thr Glu
                85                  90                  95


Ile Lys Arg Tyr Glu Thr Leu Glu Lys Tyr Ile Asp Leu Gly Arg Ser
            100                 105                 110


Asn Arg Gly Glu Arg Phe Val Asn Glu Phe Leu Glu Arg Val Ser Lys
        115                 120                 125


Leu Glu Gly Asp Val Leu Lys Glu Val Phe Glu Asp Ala Ser Asn Lys
    130                 135                 140


Phe Lys Gly Glu Glu Ser Lys Gln Trp Ala Tyr Ile Trp Gln Phe Tyr
145                 150                 155                 160


Pro Val Lys Leu Lys Glu Gly Val Lys Glu Phe Ala Lys Ser Glu Leu
                165                 170                 175


Lys Leu Lys Glu Glu Glu Ala Glu Lys Phe Ala Glu Glu Phe Val Asn
            180                 185                 190


Leu Pro Ala Asp Thr Arg Phe Pro Asp His Ala Ile Trp Thr His Leu
        195                 200                 205

Asp Leu Thr Ser Ala Leu Ser Val Lys Asp Pro Thr Leu Leu Arg Ile
    210                 215                 220

Lys Ile Val Pro Val Gln Pro Phe Ile Ala Asn Ser Arg Lys Gln Leu
225                 230                 235                 240

Asp Leu Trp Ala Ser Ser His Leu Leu Ser Met Leu Met Tyr Lys Ala
                245                 250                 255

Leu Glu Val Ile Val Asp Lys Phe Gly Pro Glu His Val Ile Tyr Pro
            260                 265                 270

Ser Leu Arg Asp Gln Pro Phe Phe Leu Lys Phe Tyr Leu Gly Glu Asn
        275                 280                 285

Ile Gly Asp Glu Ile Leu Val Ala Asn Leu Pro Asn Lys Ala Leu Ala
    290                 295                 300

Ile Val Ser Gly Lys Glu Ala Glu Lys Ile Glu Glu Glu Ile Lys Lys
305                 310                 315                 320

Arg Ile Arg Asp Phe Leu Leu Gln Leu Tyr Arg Glu Ala Val Asp Trp
                325                 330                 335

Ala Val Glu Asn Gly Val Val Lys Val Asp Arg Ser Glu Lys Asp Ser
            340                 345                 350

Met Leu Lys Glu Ala Tyr Leu Lys Ile Val Arg Glu Tyr Phe Thr Val
        355                 360                 365

Ser Ile Thr Trp Val Ser Leu Ser Glu Lys Glu Asp Ile Tyr Gln Val
    370                 375                 380

Thr Glu Asn Ala Gly Leu Ser Asp Glu Asp Val Lys Lys Trp Leu Lys
385                 390                 395                 400

Phe Ala Glu Lys Lys Glu Asn Ser Arg Val Leu Glu Arg Ile Ala Ile
                405                 410                 415

Tyr Pro Leu Leu Val Lys Ile Leu Asp Ser Leu Gly Glu Arg Lys Val
            420                 425                 430

Thr Glu Glu Arg Phe Glu Lys Ser Glu Gln Leu Lys Gly Trp Lys Cys
        435                 440                 445

His Val Cys Gly Glu Asn Leu Ala Ile Phe Gly Asp Met Tyr Asp His
    450                 455                 460

Asp Asn Leu Lys Ser Leu Trp Leu Asp Glu Glu Pro Leu Cys Pro Met
465                 470                 475                 480

Cys Leu Ile Lys Arg Tyr Tyr Pro Val Trp Ile Arg Ser Lys Thr Gly
                485                 490                 495

Gln Lys Ile Arg Phe Glu Ser Val Val Asp Val Ala Leu Leu Tyr Lys
            500                 505                 510

Asn Trp Arg Lys Ile Phe Asp Glu Lys Tyr Gly Lys Asp Leu Val Ser
        515                 520                 525

Lys Ala Arg Glu Val Ser Glu Asp Phe Val Lys Asp Asn Met Leu Val
    530                 535                 540

Asp Ser Asp Leu Tyr Tyr Ser Ser Thr Trp Glu Ser Gly Leu Ser Lys
545                 550                 555                 560

Lys Leu Lys Asn Lys Lys Glu Ile Asp Glu Glu Lys Val Lys Glu Val
                565                 570                 575

Val Asp Phe Leu Asn Ala Ala Tyr Lys Glu Ile Gly Asn Pro Pro Lys
            580                 585                 590

Tyr Tyr Ala Ile Leu Val Met Asp Gly Asp Asp Met Gly Lys Val Ile
        595                 600                 605

Ser Gly Glu Val Leu Gly Glu Ile Ser Thr Arg Ile His Pro Asn Ile
    610                 615                 620

Arg Asp Tyr Val Glu Ile Pro Glu Ala Lys Tyr Tyr Ser Thr Pro Gln
625                 630                 635                 640

Val His Val Ala Ile Ser Gln Ala Leu Ala Asn Phe Ser Ile Arg Glu
                645                 650                 655

Val Arg Ser Val Val Lys Asp Glu Gly Leu Leu Ile Tyr Ala Gly Gly
            660                 665                 670

Asp Asp Val Leu Ala Ile Leu Pro Val Asp Lys Ala Leu Glu Val Ala
        675                 680                 685

Tyr Lys Ile Arg Lys Glu Phe Gly Lys Ser Phe Glu Asn Gly Ser Leu
    690                 695                 700

Leu Pro Gly Trp Lys Leu Ser Ala Gly Ile Leu Ile Val His Tyr Lys

705                 710                 715                 720

His Pro Leu Tyr Asp Ala Leu Glu Lys Ala Arg Asp Leu Leu Asn Asn
                725                 730                 735

Lys Ala Lys Asn Val Pro Gly Lys Asp Thr Leu Ala Ile Gly Leu Leu
            740                 745                 750

Lys Arg Ser Gly Ser Tyr Tyr Ile Ser Leu Val Gly Trp Glu Leu Ile
        755                 760                 765

Arg Val Phe Tyr Asn Ser Glu Leu Arg Lys Lys Leu Leu Glu Glu Lys
    770                 775                 780

Gly Gly Val Gly Lys Arg Phe Ile Tyr His Val Leu Arg Glu Val Asp
785                 790                 795                 800

Thr Trp Pro Lys Val Gly Ile Asp Glu Met Leu Lys Phe Glu Val Ile
                805                 810                 815

Arg His Ile Arg Gly Arg Asn Lys Glu Glu Thr Lys Glu Leu Arg Glu
            820                 825                 830

Lys Ile Tyr Gly Glu Ile Lys Asp Leu Leu Glu His Val Arg Gly Asn
        835                 840                 845

Asn Glu Val Glu Lys Val Arg Gly Leu Phe Thr Phe Leu Lys Ile Ile
    850                 855                 860

Thr Asp Ala Glu Val Phe Pro
865                 870

<210>  63
<211>  338
<212>  PRT
<213>  Pyrococcus furiosus

<220>
<221>  PEPTIDE
<222>  (1)..(338)
<223>  type III-B CRISPR module RAMP protein Cmr1:
       /protein_id=WP_011012270.1

<400>  63

Met Phe Ile Glu Glu Phe Glu Ile Glu Ser Ile Thr Ser Thr His Leu
1               5                   10                  15

Leu Glu Val Leu Thr Arg Glu Tyr Pro Glu Val Arg Ser Pro Ser Ile
            20                  25                  30

Lys Gly Ala Met Arg Trp Trp Phe Arg Ala Leu Ala Gly Ser Tyr Phe
        35                  40                  45

Gly Asp Asp Ala Gln Lys Leu Lys Glu Ile Glu Asn Gln Val Phe Gly
    50                  55                  60

Ser Thr Lys Glu Arg Ser Arg Val Lys Ile Ser Val Thr Pro Leu Ser
65                  70                  75                  80

Ser Pro Lys Arg Leu Asn Leu Lys Glu Phe Lys Asp Lys Asn Val Gly
                85                  90                  95

Tyr Ile Trp Phe Ser Ile Asn Leu Leu Gly Lys Arg Gly Thr Ile Thr
            100                 105                 110

His Tyr Tyr Pro Pro Gly Ser Arg Phe Arg Val Val Leu Glu Ser Pro
        115                 120                 125

Ser Glu Arg Val Ile Lys Leu Ala Thr Leu Ser Leu Trp Ala Leu Val
    130                 135                 140

Ser Leu Gly Ser Val Gly Phe Arg Ser Arg Arg Gly Thr Gly Ser Met
145                 150                 155                 160

Lys Ile Val Arg Ala Ser Ser Glu Val Leu Glu Asp Leu Gly Leu Thr
                165                 170                 175

Thr Glu Phe Asn Ser Ile Asp Glu Phe Lys Asp Ser Leu Lys Arg Val
            180                 185                 190

Leu Asp Val Thr Gly Glu Ile Leu Gly Val Lys Asn Ser Glu Thr Asn
        195                 200                 205

Lys Ser Leu Pro Ser Tyr Ala Thr Leu Lys Phe Ser Asp Val Glu Val
    210                 215                 220

Phe Gly Pro Gly Lys Asn Thr Trp Glu Val Leu Ala Gln Phe Asn Asn
225                 230                 235                 240

Ser Tyr Lys Glu Tyr Leu Arg Arg Arg Ile Lys Lys Tyr Gln Arg Ile
                245                 250                 255

Ile Phe Gly Leu Pro Arg Phe Lys Leu Arg Gly Val Arg Lys Asp Leu
            260                 265                 270

Arg Arg Ala Ser Pro Leu Trp Phe Gly Val Val Glu Ile Gly Gly Lys

275                 280                 285

Pro Tyr Gly Arg Ile Ile Lys Phe Phe Gln Ser Thr Phe His Pro Glu
    290                 295                 300

Val Arg Ser Lys His Ile Val Asp Trp Asn Val Leu Ser Asn Phe Asp
305                 310                 315                 320

Trp Phe Ile Ser Ser Arg Leu Pro Val Thr Lys Val Trp Gly Gly Trp
                325                 330                 335

Ser Gly

<210>  64
<211>  264
<212>  PRT
<213>  Pyrococcus furiosus

<220>
<221>  PEPTIDE
<222>  (1)..(264)
<223>  CRISPR-associated endoribonuclease Cas6
       /protein_id=WP_011012271.1

<400>  64

Met Arg Phe Leu Ile Arg Leu Val Pro Glu Asp Lys Asp Arg Ala Phe
1               5                   10                  15

Lys Val Pro Tyr Asn His Gln Tyr Tyr Leu Gln Gly Leu Ile Tyr Asn
            20                  25                  30

Ala Ile Lys Ser Ser Asn Pro Lys Leu Ala Thr Tyr Leu His Glu Val
        35                  40                  45

Lys Gly Pro Lys Leu Phe Thr Tyr Ser Leu Phe Met Ala Glu Lys Arg
    50                  55                  60

Glu His Pro Lys Gly Leu Pro Tyr Phe Leu Gly Tyr Lys Lys Gly Phe
65                  70                  75                  80

Phe Tyr Phe Ser Thr Cys Val Pro Glu Ile Ala Glu Ala Leu Val Asn
                85                  90                  95

Gly Leu Leu Met Asn Pro Glu Val Arg Leu Trp Asp Glu Arg Phe Tyr
            100                 105                 110

Leu His Glu Ile Lys Val Leu Arg Glu Pro Lys Lys Phe Asn Gly Ser
        115                 120                 125

Thr Phe Val Thr Leu Ser Pro Ile Ala Val Thr Val Val Arg Lys Gly
    130                 135                 140

Lys Ser Tyr Asp Val Pro Pro Met Glu Lys Glu Phe Tyr Ser Ile Ile
145                 150                 155                 160

Lys Asp Asp Leu Gln Asp Lys Tyr Val Met Ala Tyr Gly Asp Lys Pro
                165                 170                 175

Pro Ser Glu Phe Glu Met Glu Val Leu Ile Ala Lys Pro Lys Arg Phe
            180                 185                 190

Arg Ile Lys Pro Gly Ile Tyr Gln Thr Ala Trp His Leu Val Phe Arg
        195                 200                 205

Ala Tyr Gly Asn Asp Asp Leu Leu Lys Val Gly Tyr Glu Val Gly Phe
    210                 215                 220

Gly Glu Lys Asn Ser Leu Gly Phe Gly Met Val Lys Val Glu Gly Asn
225                 230                 235                 240

Lys Thr Thr Lys Glu Ala Glu Glu Gln Glu Lys Ile Thr Phe Asn Ser
                245                 250                 255

Arg Glu Glu Leu Lys Thr Gly Val
            260

<210>  65
<211>  92
<212>  PRT
<213>  Marinomonas mediterranea

<220>
<221>  misc_feature
<223>  CRISPR-associated endonuclease Cas2:  /protein_id=WP_013659857.1

<400>  65

Met Arg Ile Tyr Leu Ala Cys Phe Asp Ile Glu Asp Asp Lys Lys Arg
1               5                   10                  15

Arg Lys Leu Ser Asn Leu Leu Leu Glu Tyr Gly Asp Arg Val Gln Tyr
            20                  25                  30

Ser Val Phe Glu Ile Ser Leu Lys Asp Glu Asn Glu Leu His Lys Leu
        35                  40                  45

Arg Lys Lys Cys Ser Lys Tyr Thr Glu Glu Ala Asp Ser Leu Arg Phe

50                  55                  60

Tyr Trp Leu Asn Lys Glu Ser Arg Lys His Ser Gln Asp Val Trp Gly
65                  70                  75                  80

Asn Pro Ile Ala Val Phe Pro Ala Ala Val Leu Leu
                85                  90

<210>  66
<211>  957
<212>  PRT
<213>  Marinomonas mediterranea

<220>
<221>  PEPTIDE
<222>  (1)..(957)
<223>  CRISPR-associated endonuclease Cas1: /protein_id=WP_013659858.1

<400>  66

Met Leu Asn Ser Pro Leu Ile Asp Ala Val Leu Pro Leu Arg Ser Val
1               5                   10                  15

Val Ile Thr Leu Arg Trp Leu Ser Pro Ser Lys Thr Gly Phe Leu His
            20                  25                  30

His Ala Gly Leu His Ala Trp Val Arg Phe Leu Ala Gly Ser Pro Glu
        35                  40                  45

Gln Phe Ser Asp Phe Ile Val Val Glu Pro Ile Glu Asn Gly His Ile
    50                  55                  60

Ser Tyr Gln Ala Gly Asp Gly Tyr Arg Phe Arg Ile Thr Val Leu Asn
65                  70                  75                  80

Gly Gly Glu Ser Leu Leu Asp Thr Leu Phe Ser Ser Leu Lys Arg Leu
                85                  90                  95

Pro Glu Ser Ala Ala Asn His Pro Asp Ile Ala Gly Ala Phe Ser Asp
            100                 105                 110

Asn Leu Val Leu Glu Lys Ile Glu Asp Thr Phe Glu His His Gln Val
        115                 120                 125

Thr Gln Ile Glu Asp Leu Ser Val Phe Asp Ile Asn Ala Leu Met Leu
    130                 135                 140

Glu Thr Ala Val Trp Ser Arg Gln Arg Arg Phe Lys Val Ala Phe Asn
145                 150                 155                 160

Thr Pro Ala Arg Leu Val Lys Pro Lys Pro Glu Asp Gly Thr Glu Leu
                165                 170                 175

Lys Gly Gln Asn Arg Tyr Cys Arg Asp Lys Ser Asp Leu Asn Trp Gln
            180                 185                 190

Leu Phe Thr His Arg Leu Thr Asp Thr Phe Ile Asn Leu Phe Gln Ser
        195                 200                 205

Arg Thr Gly Glu Arg Leu Gln Arg Gln Asn Trp Pro Glu Ala Gln Leu
    210                 215                 220

His Ala Gly Leu Ala Val Trp Leu Asn Asn Ser Tyr Thr Asn Lys Lys
225                 230                 235                 240

Glu Lys Lys Val Lys Asp Ala Ser Gly Met Leu Ala Gln Met Gln Ile
                245                 250                 255

Glu Ile Asp Asp Asp Phe Pro Ala Asp Leu Leu Ala Leu Leu Val Leu
            260                 265                 270

Gly Gln Tyr Ile Gly Met Gly Gln Asn Arg Ala Phe Gly Met Gly Gln
        275                 280                 285

Tyr Gln Leu Gln Asp Ala Tyr Gly Tyr Cys Ser Tyr Pro Arg Pro Gln
    290                 295                 300

Ala Ala Lys Ser Leu Leu Glu Lys Ser Leu Ser Asp Ala Ser Leu His
305                 310                 315                 320

Gln Ala Cys Gln Thr Met Tyr Pro Arg Gln Ala Asn Phe Asp Ser Ser
                325                 330                 335

Asp Thr Asp Glu Glu His His Asp Ala Ile Asp Glu Leu Leu Thr Lys
            340                 345                 350

Leu Tyr Val Ser Arg Glu Arg Ile Phe Lys Arg Glu Phe Thr Pro Ser
        355                 360                 365

Gln Leu His Ser Val Glu Ile Glu Lys Pro Glu Gly Gly Thr Arg Leu
    370                 375                 380

Leu Ser Val Pro Asn Trp His Asp Arg Thr Leu Gln Lys Ala Val Thr
385                 390                 395                 400

Glu Cys Leu Gly Asn Thr Leu Glu His Ile Trp Met Lys His Ser Tyr
                405                 410                 415

Gly Tyr Arg Lys Gly His Ser Arg Leu Gln Ala Arg Asp Gln Ile Asn
            420                 425                 430

Gln Tyr Ile Gln Gln Gly Tyr Glu Trp Val Leu Glu Ser Asp Ile Glu
        435                 440                 445

Ser Phe Phe Asp Ser Val Asn Trp Leu Asn Leu Glu Gln Arg Leu Lys
    450                 455                 460

Leu Leu Leu Pro Asn Glu Pro Leu Val Pro Leu Leu Met Gln Trp Val
465                 470                 475                 480

Ser Ala Ala Lys Gln Thr Glu Asp Glu Gln Thr Leu Ala Arg His Asn
                485                 490                 495

Gly Leu Pro Gln Gly Ala Pro Ile Ser Pro Ile Leu Ala Asn Leu Leu
            500                 505                 510

Leu Asp Asp Leu Asp Gln Asp Met Ile Ala Lys Gly His Gln Ile Val
        515                 520                 525

Arg Tyr Ala Asp Asp Phe Val Leu Leu Phe Lys Ser Lys Ala Ala Ala
    530                 535                 540

Glu Ser Ala Leu Asp Asp Ile Ile Thr Ala Leu Lys Glu His His Leu
545                 550                 555                 560

Ala Ile Asn Leu Glu Lys Thr Arg Ile Val Glu Ala Ser Gln Gly Phe
                565                 570                 575

Arg Tyr Leu Gly Tyr Leu Phe Val Asp Gly Tyr Ala Ile Glu Thr Lys
            580                 585                 590

Arg Glu Tyr Arg Lys Glu His Ala Gln Leu Asp Lys Gln Leu Asn Ala
        595                 600                 605

Ser Ser Leu Glu Asn Glu Pro Ser Leu Gln Gln Glu Pro Ala Val Gln
    610                 615                 620

Asn Glu Gln Ser Thr Leu Ile Gly Glu Arg Glu Lys Leu Gly Thr Leu
625                 630                 635                 640

Leu Ile Ile Ala Gly Asp Ile Ala Met Leu Ser Ser Glu Lys Gln Arg
                645                 650                 655

Leu Ile Val Glu Gln Tyr Asp Glu Leu His Thr Tyr Pro Trp Ala Thr

660                 665                 670

Leu Ser Ser Val Leu Leu Val Gly Pro His His Ile Thr Thr Pro Ala
        675                 680                 685

Leu Lys Ser Ala Met Phe His Asn Val Pro Val His Phe Ala Ser Gln
    690                 695                 700

Tyr Gly Arg Tyr Gln Gly Val Ser Ala Gly Ala Ala Pro Ser Val Phe
705                 710                 715                 720

Gly Ala Asp Phe Trp Leu Leu Gln Ala Gln Tyr Leu Gln Gln Glu Thr
                725                 730                 735

Asn Ala Leu Asn Ile Ser Gln Val Leu Ile Gln Ala Arg Ile Glu Gly
            740                 745                 750

Ile Arg Ala Val Ile Ser Arg Arg Glu Lys Asp Ala Pro Glu Leu Asn
        755                 760                 765

Lys Ile Gln Arg Leu Asp Glu Lys Arg Leu Arg Ala Glu Thr Leu Asp
    770                 775                 780

Gln Leu Arg Gly Tyr Glu Gly Gln Ala Ser Lys Gln Leu Trp Ala Phe
785                 790                 795                 800

Phe Gln Arg Ile Leu Glu Glu Asp Trp Gly Phe Thr Gly Arg Asn Arg
                805                 810                 815

Arg Pro Pro Lys Asp Pro Ile Asn Ala Leu Leu Ser Leu Gly Tyr Thr
            820                 825                 830

Tyr Leu Tyr Ser Leu Val Asp Ser Val Asn Arg Thr Val Gly Leu Tyr
        835                 840                 845

Pro Trp Gln Gly Ala Leu His Gln Arg His Gly Tyr His His Thr Leu
    850                 855                 860

Ala Ser Asp Leu Met Glu Pro Trp Arg Tyr Leu Val Glu His Val Val
865                 870                 875                 880

Leu Thr Leu Ile Asn Arg His Gln Ile His Lys Asp Asp Phe Val Ile
                885                 890                 895

Lys Glu Asn Gly Cys Glu Met Ser Ser Gly Ala Arg Lys Thr Leu Leu
            900                 905                 910

Lys Glu Leu Leu Val Gln Leu Thr Lys Val Pro Lys Gly Gly Asn Ser
        915                 920                 925

Leu Leu Thr Glu Met Ser Asn Gln Ser Tyr Arg Leu Ala Leu Ser Cys
    930                 935                 940

Lys Met Gln Gln Arg Phe Ile Ala Trp Ser Pro Lys Arg
945                 950                 955

<210>  67
<211>  256
<212>  PRT
<213>  Marinomonas mediterranea

<220>
<221>  PEPTIDE
<222>  (1)..(256)
<223>  hypothetical protein /protein_id="WP_013659859.1"

<400>  67

Met Asn Asn Ala Lys Thr Lys Ser Asp Thr Asp Gly Glu Gln Tyr Leu
1               5                   10                  15

Lys Asp Ser Phe Gln Arg Glu Gln Glu Leu Leu Glu Ser Lys Leu Leu
            20                  25                  30

Phe Ala Asn Gln Asn Ile Thr His Asn Gly Glu Arg Gly Glu Val Asn
        35                  40                  45

Glu Lys His Phe Ile Glu Tyr Leu Arg Ser His Leu Pro Lys Arg Tyr
    50                  55                  60

Ser Val Asp Ser Ala Ile Val Val Asp Ser Asn Gly Lys Thr Ser Asn
65                  70                  75                  80

Gln Ile Asp Ile Val Val Tyr Asp Asn Gln Tyr Thr Pro Thr Leu Phe
                85                  90                  95

Ala Gln Gln Asp Phe Cys Tyr Val Pro Ala Glu Ala Val Tyr Ala Val
            100                 105                 110

Ile Glu Val Lys Pro Glu Val Asn Arg Asp Tyr Ile Glu Tyr Ala Gln
        115                 120                 125

Lys Gln Ala Tyr Ser Val Glu Ala Leu Glu Arg Thr Ser Ile Glu Ile
    130                 135                 140

Thr His Ala Gly Gly Val Tyr Lys Pro Lys Pro Leu Phe Lys Ile Ile
145                 150                 155                 160

Thr Gly Leu Ile Ala Leu Lys Thr Gly Trp Gln Glu Gly Leu Ala Ser
                165                 170                 175

Asn Ser Phe Lys Asp Ala Ile Gln Met Pro Tyr Thr Asn Gln Thr Arg
            180                 185                 190

Leu Asp Phe Val Thr Ser Leu Gln Gly Gly His Phe Asp Cys Phe Glu
        195                 200                 205

Lys Gly Asn Glu His Tyr Ser Gln Gln Asn His Leu Thr Phe Phe Leu
    210                 215                 220

Phe Arg Leu Leu Ser Gln Leu Gln Ser Leu Gly Thr Val Pro Ala Val
225                 230                 235                 240

Asp Trp Asn Lys Tyr Ala Asn Val Leu Thr Glu Gln Ser Lys Pro Asp
                245                 250                 255

<210>  68
<211>  371
<212>  PRT
<213>  Marinomonas mediterranea

<220>
<221>  PEPTIDE
<222>  (1)..(371)
<223>  type III-B CRISPR module RAMP protein Cmr6
       /protein_id=WP_013659861.1

<400>  68

Met Ser Ser Val Gln Leu Val Pro Gln Arg Leu Phe Asn Ile Tyr Glu
1               5                   10                  15

Met His Leu Lys Ser Arg Thr Ser Asn Gly Leu His Ser Gly Leu Leu
            20                  25                  30

Leu Asp Lys Gly Tyr Pro Ala Ser Ser Thr Gly Val Asn Thr Asp Lys
        35                  40                  45

Glu Ala Lys Ala Arg Phe Ile Lys Ser Ile Cys Gly Ile Lys Ala Ser
    50                  55                  60

Glu Leu Tyr Thr Lys Glu Phe Asn Gln Trp Leu Arg Leu Thr Ser Lys
65                  70                  75                  80

Lys Ser Asn Ser Gly Lys His Thr Asn Asn Phe Glu Gln Leu Pro Leu
                85                  90                  95

Thr Leu Ser Asn Arg Leu Leu Ile Gly Leu Asn Gly Gly Gly Ala Leu
            100                 105                 110

Glu Thr Gly Cys Met Ile Asn His His Asn Gly Met Pro Tyr Ile Pro
        115                 120                 125

Gly Ser Ser Ile Lys Gly Ala Val Arg Asn Phe Ala Ser Arg Asn Leu
    130                 135                 140

Lys Asp His Glu Ser Val Trp Val Gln Phe Phe Gly Asn Lys Pro Glu
145                 150                 155                 160

Asp Asp Leu Glu Asp Pro Phe Ala Gly Leu Ile Thr Phe His Asp Ala
                165                 170                 175

Trp Trp Ile Pro Asn Ser Ala Pro Ser Glu Asn Ala Gly Asn Phe Ser
            180                 185                 190

Asn Phe Pro Phe Ile Gln Asp Ile Val Thr Thr His His Ala Glu Tyr
        195                 200                 205

Tyr Ser Lys Glu Gly Lys Ser Leu Ala Thr Asp Phe Asp Asp Pro Ile
    210                 215                 220

Pro Asn Ser Leu Ile Ser Val His Gly Ser Phe Leu Phe Val Ile Gly
225                 230                 235                 240

Gly Asn Glu Asn Tyr Leu Gly Val Val Lys Gln Met Leu Glu Lys Thr
                245                 250                 255

Leu Lys Glu Asn Gly Ile Gly Ala Lys Ser Thr Ala Gly Tyr Gly Tyr
            260                 265                 270

Phe Asp Glu Ala Asp Glu Ser Ile Thr Lys Gln Phe Ser Ile Leu Leu
        275                 280                 285

Ser Asp Thr Glu Asn Thr Asn Thr Thr Pro His Ser Ser Glu Leu Ile
    290                 295                 300

Arg Glu Val Ser Glu Ile Lys Leu Ser Lys Ile Pro Asp Ile Phe Gly
305                 310                 315                 320

Arg Asn Arg Asn Lys Thr Lys Asn Glu Lys Gly Lys Asp Tyr Arg Ser
                325                 330                 335

Leu Cys Leu Leu Val Cys Lys His Phe Gly Asp Glu Ile Ala Asn Trp
            340                 345                 350

Ala Thr Glu Ser Gly Asn Lys Ala Lys Ala Tyr Lys Glu Leu Lys Glu
        355                 360                 365

Phe Leu Glu
    370

<210>  69
<211>  140
<212>  PRT
<213>  Marinomonas mediterranea

<220>
<221>  PEPTIDE
<222>  (1)..(140)
<223>  type III-B CRISPR module-associated protein Cmr5:
       protein_id=WP_083799838.1

<400>  69

Met Ser Val Arg Gln His Lys Ile Ala Glu Ala Ala Tyr Asp Cys Val
1               5                   10                  15

Asn Lys Arg Ile Glu Glu Gly Ile Asp Gln Lys Lys Tyr Ala Ala Leu
            20                  25                  30

Ala Asn Lys Leu Pro Asn Leu Ile Leu Gln Asn Gly Leu Ala Gln Ala
        35                  40                  45

Thr Gly Phe Leu Met Ala Lys Asn Glu Ser Thr Asn Glu His Lys Lys
    50                  55                  60

Leu Leu Asn Asp Leu Ala Phe Val Val Asn Ser Gly His Leu Glu Asn
65                  70                  75                  80

Gln Asn Thr Pro Asn Ser Asp Asn Gly Lys Glu Leu His Gln Gln Ile
                85                  90                  95

Ile Gln Ser Asp Met Thr Lys Phe Met Gln Leu Thr Gln Gln Ser Leu
            100                 105                 110

Asp Ala Ser Gly Ala Leu Arg Arg Tyr Val Gln Gly Leu Met Asp Thr
        115                 120                 125

Asn Lys Lys Asp Asp Asp Ser Gln Glu Glu Thr Leu
    130                 135                 140

<210>  70
<211>  300
<212>  PRT

<213>  Marinomonas mediterranea

<220>
<221>  PEPTIDE
<222>  (1)..(300)
<223>  type III-B CRISPR module RAMP protein Cmr4:
       /protein_id=WP_013659863.1

<400>  70

Met Asp Leu Asn Ile Tyr His Leu His Thr His Ser Ala Leu His Cys
1               5                   10                  15

Gly Thr Gly Gln Ser Ala Gly Ile Val Asp Leu Pro Ile Ala Arg Asp
            20                  25                  30

Lys Ala Thr His Leu Pro Ile Val Pro Gly Ser Ser Leu Arg Gly Val
        35                  40                  45

Leu Arg Asp Lys Ile Arg Ala Asn Glu Lys Asp Ile Glu Lys Ala Leu
    50                  55                  60

Phe Gly Pro Arg Asp Ile Gln Ser Ser Glu Gln Ser Phe Ala Gly Ala
65                  70                  75                  80

Leu Ser Leu Gly Asp Ala Asn Leu Leu Val Leu Pro Ile Arg Ser Leu
                85                  90                  95

Asn Gly Ile Val Ala Tyr Ala Thr Cys Pro Phe Val Leu Arg Asn Tyr
            100                 105                 110

Ala Asn Asp Thr Lys Ile Ser Val Lys Glu Thr Pro Leu Pro Gln Pro
        115                 120                 125

Ser Ala Gln Gln Ala Leu Val Ser Asn Glu Asn Val Asn Val Ile Gln
    130                 135                 140

His Asp Asn His Thr Glu Leu Leu Val Leu Glu Asp Leu Asp Ile Ser
145                 150                 155                 160

Ala Glu Lys Asp Glu Lys Val Gln Ala Trp Ala Lys Lys Ile Ala Glu
                165                 170                 175

Ala Val Tyr Ser Glu Gln Ser Asp Leu Lys Asp Glu Phe Ile Asn Arg
            180                 185                 190

Phe Val Val Leu Ser Asp Asn Val Phe Ser Tyr Leu Ser Thr Thr Ala
        195                 200                 205

Thr Glu Leu Arg Thr Arg Ile Arg Ile Thr Glu Lys Thr Gly Val Val
    210                 215                 220

Lys Glu Gly Ala Leu Trp Ser Glu Glu Asn Leu Pro Ala Asn Ala Ile
225                 230                 235                 240

Leu Trp Gly Val Tyr Ser Leu Ser Asp Ser Arg Asp Lys Lys Gln Glu
                245                 250                 255

Leu Thr Ala Pro Glu Leu Lys Glu Lys Leu Ser Ala Ser Tyr Thr Leu
            260                 265                 270

Gln Met Gly Gly Asn Ala Gly Ile Gly Arg Gly Leu Val Ser Leu Phe
        275                 280                 285

Thr Lys Lys Glu Glu Lys Glu Thr Gly Gly Gln Glu
    290                 295                 300

<210>  71
<211>  405
<212>  PRT
<213>  Marinomonas mediterranea

<220>
<221>  PEPTIDE
<222>  (1)..(405)
<223>  CRISPR-associated protein, Cmr3: protein_id=WP_013659864.1

<400>  71

Met His Thr His Asp Gln Lys Glu Asn Thr Tyr Leu Leu Asp Pro Lys
1               5                   10                  15

Ala Pro Leu Ile Ile Arg Ser Gly Arg Pro Phe Asp Glu Gln Ala Gly
            20                  25                  30

Ala Asp Asp Ala Arg Phe Pro Pro Pro Ser Thr Met Ala Gly Ala Leu
        35                  40                  45

Arg Ala Ala Tyr Ala Arg Ala Asn Asn Ile Gly Phe Asn Lys Glu Asn
    50                  55                  60

Thr Gln Asp Ile Leu Ser Lys Ala Ile Lys Gly Pro Leu Pro Val Lys
65                  70                  75                  80

Gln Ser Thr Glu Glu Asp Ile Lys Thr Leu Leu Val Pro Lys Pro Glu
                85                  90                  95

Asp Ala Gln Tyr Tyr Tyr Asp Gln Ala Gly Asn Ile Glu Ile Val Arg
            100                 105                 110

Leu Ser Pro Arg Glu Leu Asp Glu Asn Glu Gly Trp Asp Leu Pro Arg
        115                 120                 125

Arg Leu Leu Pro Val Ser Tyr His Asp Asp Gln Glu Lys Arg His Gly
    130                 135                 140

Lys Pro Ala Lys Gly Ala Gln Trp Trp Ser Leu Lys Asp Leu Leu Glu
145                 150                 155                 160

Trp Arg Leu His Lys Pro Ile Asn Tyr Lys Glu Val His Lys Asn Gly
                165                 170                 175

Trp Ile Pro Pro Ala Asp Asp Ile Arg Thr His Val Ala Ile Asn Pro
            180                 185                 190

Gln Ser Phe Ala Ala Glu Asp Gly Gln Leu Phe Gln Thr Ser Gly Leu
        195                 200                 205

Pro Met Trp Gln Val Lys Lys Glu Asn Gln Ser Ala Phe Pro Asp Glu
    210                 215                 220

Lys Ile Leu Leu Ala Gly Cys Ile Asn Gly Asp Leu Phe Pro Glu Ala
225                 230                 235                 240

His Ser Lys Asn Asp Thr Pro Gln Ser Leu Asn Ile Gly Gly Glu Arg
                245                 250                 255

Arg Leu Ala Glu Ile Leu Thr Glu Asp Gln Leu Trp Pro Gln Pro Asp
            260                 265                 270

Glu Gln Ile Leu Arg Asp Ile Leu Glu Gly Glu Ala Leu Ser Leu Thr
        275                 280                 285

Leu Leu Thr Pro Ala Ile Phe Lys Asp Gly Trp Leu Pro Cys Gln Gln
    290                 295                 300

Asp Glu Asn Gly His Phe Ile Tyr Arg Pro Pro Asn Thr Asn Thr Leu
305                 310                 315                 320

Glu Leu Glu Leu Val Ser Ala Ala Ile Gly Arg Trp Thr Ala Gln Ser
                325                 330                 335

Gly Trp Asp Leu Ala Lys His Gln Pro Lys Pro Ser Arg Lys Leu Val
            340                 345                 350

Pro Ala Gly Ala Val Tyr Trp Phe Arg Val Arg Glu Gly Asn Lys His

355                 360                 365

Asp Leu Glu Gln Leu Trp Phe Ser Asn Ile Ser Thr Lys Glu Gln Asp
    370                 375                 380

Arg Lys Asp Gly Phe Gly Leu Val Leu Pro His Ser Thr Thr Leu Ser
385                 390                 395                 400

Ala Ser Gly Ala Lys
                405

<210>  72
<211>  635
<212>  PRT
<213>  Marinomonas mediterranea

<220>
<221>  PEPTIDE
<222>  (1)..(635)
<223>  type III-B CRISPR-associated protein Cas10/Cmr2:
       /protein_id=WP_013659865.1

<400>  72

Met Asn Thr Tyr Leu Ile Thr Ile Ala Ile Gly Pro Val Gln Ser Leu
1               5                   10                  15

Ile Glu Ala Ala Arg Arg Thr Arg Asp Leu Trp Thr Gly Ser Trp Val
            20                  25                  30

Leu Ser Glu Cys Ala Lys Ala Ala Ala Lys Ser Leu His Asp Asn Gln
        35                  40                  45

Ala Asn Leu Pro Asn Thr Gly Ser Ser Cys Leu Ile Phe Pro Ser Pro
    50                  55                  60

Glu Ser Pro Glu Thr Glu Leu Leu Pro Ser Asp Phe Asn Asp Gln Tyr
65                  70                  75                  80

Ala Asn Ile Ser Asn Val Ile Arg Ala Glu Ile Gln Ala Glu Glu Thr
                85                  90                  95

Asp Asp Ile Lys Val Ile Ala Glu Gln Ala Lys Gln Ala Ala Lys Asn
            100                 105                 110

Arg Leu Thr Ala Ile Ala Glu Ser Ala Leu Asn Ser Ala Lys Gly Leu
        115                 120                 125

Pro Ile Arg Arg Asp Leu Trp His Asn Gln Ile Glu His Ile Leu Glu
    130                 135                 140

Met Phe Ala Val Trp Val Lys Val Glu Asp Asn Gln Tyr Gln Thr Ala
145                 150                 155                 160

Ser Lys Lys Leu Asn Ala Leu Leu Leu Ala Arg Lys Ala Ser Arg His
                165                 170                 175

Phe Glu Asn Pro Glu Ile Ser Glu Glu His Gly Ser Leu Leu Lys Ser
            180                 185                 190

Ser Leu Asp Gly Ala Ser Glu Ser Val Val Ala Leu Glu Glu Lys Asp
        195                 200                 205

Lys Thr Thr Ser Glu Glu Ser Asn Ser Ile Val Ser Phe Lys Ser Gly
    210                 215                 220

Ser Ile Lys Arg Tyr Lys Arg Lys Leu Gly Leu Ala Asn Lys Glu Val
225                 230                 235                 240

Leu Asp Ala Leu Gly Val Ile Lys Arg Leu Ser Gly Lys Ala Glu Gln
                245                 250                 255

Phe Thr Pro Tyr Pro Arg Ile Cys Ala Asp Asp Trp Leu Asn Thr Leu
            260                 265                 270

Ser Asn Asp Gln Gln Glu Ser Leu Ser Leu His Phe Asp Lys Leu Lys
        275                 280                 285

Asn Ala Thr Asp Ser Asp Tyr Gly Asp Ala Cys Leu Val Thr Asp Val
    290                 295                 300

Lys Gly Asn Asn Asn Ala Tyr Ser Asp Phe Pro Phe Asp Gly Glu Leu
305                 310                 315                 320

Leu Tyr Asp Phe Arg Leu Gln Asn Ala Leu Gln Asn Thr Glu Arg Asp
                325                 330                 335

Lys Ser Leu Ser Thr Ser Glu Gln Arg Val Leu Gly Glu Leu Gln Asn
            340                 345                 350

Ser Leu Arg Ser Ile Asn Ser Asn Pro Val Pro Tyr Gly Val Ile Leu
        355                 360                 365

Lys Ala Asp Gly Asp Asn Met Gly Glu Leu Phe Ser Gln Ala Gln Ser
    370                 375                 380

Ala Thr Gln Ser Arg Ala Ile Ser Gln Ala Leu His Lys Phe Ala Ser

385                 390                 395                 400

Ser Val Arg Gln Leu Val Gln Lys His Gln Gly His Ala Ile Tyr Ala
                405                 410                 415

Gly Gly Asp Asp Val Leu Ala Met Leu Pro Leu Asp Gln Ala Ile Pro
            420                 425                 430

Cys Ala Glu Gly Leu Ile Asp Glu Phe Ser Asn Cys Met Ser Ser Ile
        435                 440                 445

Ile Gln Glu His His Leu Asp Ile Thr Lys Val Pro Ser Leu Ser Val
    450                 455                 460

Gly Leu Ala Ile Gly His Phe Met Gln Pro Leu Gly His Leu Arg Ala
465                 470                 475                 480

Arg Ala Val Arg Ala Glu Lys Leu Ala Lys Gly Asp His Leu Pro Ser
                485                 490                 495

Asp Lys Lys Arg Asn Ala Leu Ser Ile Cys Leu Gly Leu Arg Ser Gly
            500                 505                 510

Pro Glu Val Ser Trp Arg Cys Arg Trp Asp Asp Glu Glu Thr Ile Asn
        515                 520                 525

Ala Leu Lys Thr Val Met Asn Ala Tyr Ser Gly Asn Ala Gln Glu Leu
    530                 535                 540

Ser Ser Arg Thr Gly Phe Ala Ile Arg Ser Val Ala Leu Gln Phe Ala
545                 550                 555                 560

Trp Ser Lys Thr Ser Ser Glu Ile Ile Ser Pro Ile Gln Gln Ala Glu
                565                 570                 575

Leu Lys Arg Leu Leu Asp Asn Leu Asn Lys Lys Gly Gly Glu His Lys
            580                 585                 590

Ile Ser Lys Glu Met Ile Ala Leu Leu Thr Gln Gln Ala Glu His Ile
        595                 600                 605

Gly Leu Glu Ala Leu Ala Asp Leu Leu Ile Ile Ala Arg Trp Leu Ser
    610                 615                 620

Ala Lys Ala Met Ser Asp Leu Gly Ala Met Glu
625                 630                 635

<210>  73
<211>  423
<212>  PRT
<213>  Marinomonas mediterranea

<220>
<221>  PEPTIDE
<222>  (1)..(423)
<223>  type III-B CRISPR module RAMP protein Cmr1:
       /protein_id=WP_013659866.1

<400>  73

Met Ala Arg Thr Arg Leu Val Asp Ser Ser Gln Leu Asp Ala Leu Gln
1               5                   10                  15

Ser Ala Leu Ser Ser Leu Asp Gln Glu Ser Gln Trp Gln Thr Tyr Glu
            20                  25                  30

Cys Thr Leu Ile Thr Pro Met Tyr Gly Gly Gly Val Glu Ala Gly Glu
        35                  40                  45

Val Asp Lys Gln Met Pro Ile Arg Ala Ser Glu Ile Arg Gly His Leu
    50                  55                  60

Arg Phe Trp Trp Arg Leu Leu Tyr Gly Thr Gln Asp Ser Lys Glu Thr
65                  70                  75                  80

Phe Asp Arg Glu Lys Ala Ile Trp Gly Gly Ile Ala Ala Asn Pro Lys
                85                  90                  95

Asp Ile Lys Lys Ser Leu Val Ser Ile Lys Val Thr Thr His Ala Pro
            100                 105                 110

Ile His Leu Lys Pro Ser Thr Thr Tyr Thr Asp Val Lys Asn Gly Asn
        115                 120                 125

Thr Asn Lys Ile Asn Lys Asn Ser Leu Gly Tyr Ala Phe Gly Ala Ala
    130                 135                 140

Ser Ile Leu Thr Glu Asn Glu Glu Asp Ile Cys Trp Leu Ser Glu Gly
145                 150                 155                 160

Thr Arg Phe Thr Leu Tyr Ile Lys Leu Asp Ile Pro Glu Pro Ser His
                165                 170                 175

Lys Asn Lys Glu Asp Ile Glu Glu Gln Ile Asn Ser Ser Ile Lys Tyr
            180                 185                 190

Trp Ala Asn Phe Gly Gly Leu Gly Ala Arg Thr Arg Arg Gly Phe Gly

195                 200                 205

Ser Ile Tyr Ser Ser Ala Phe Ser Ile Lys Ser Asn Thr Glu Ile Glu
    210                 215                 220

Ser Leu Ile Lys Lys Leu Thr Asn Ser Lys Phe Ala Leu Leu Gln Ser
225                 230                 235                 240

Lys Ser Ala Ser Ala Val Thr Ala Trp Glu Thr Ala Ser Gln Arg Leu
                245                 250                 255

Ser Ser Phe Arg Gln Ile Gly Ile Gly Arg Lys Ser His Lys Asn Arg
            260                 265                 270

Ser Thr Trp Pro Glu Pro Asp Gln Leu Arg Arg Phe Thr Asp Lys Asn
        275                 280                 285

Asp Asn Gly Lys His Lys Pro Glu His Lys Ala Gly Asn Val Phe Pro
    290                 295                 300

Arg Ala Ala Phe Gly Met Pro Ile Leu Phe Lys Phe Lys Ser Ser Ser
305                 310                 315                 320

Asp Pro Gln Thr Leu Arg Leu Leu Pro Gln Gly Ala Glu Arg Phe Ala
                325                 330                 335

Ser Pro Leu Ile Ile Arg Pro Ile Gln Leu Gln Glu Gly Cys Val Ala
            340                 345                 350

Ile Ala Leu Leu Leu Pro Asn Trp Gln Gln Ala Leu Thr Gln Glu Leu
        355                 360                 365

Glu Leu Gln Thr Glu Lys Asp Asp Thr Phe Leu Val Ala Asn Lys Glu
    370                 375                 380

Pro Pro Met Ser Trp Gly Glu Arg Asn Gly Ala Glu Leu Ala Ser Leu
385                 390                 395                 400

Ile Thr Pro Met Gln Gly Arg Ala Asp Asn Pro Leu Glu Ala Phe Leu
                405                 410                 415

Asp Tyr Phe Glu Lys Gly Arg
            420

<210>  74
<211>  300
<212>  PRT
<213>  Methanothermobacter thermautotrophicus

<220>
<221>  PEPTIDE
<222>  (1)..(300)
<223>  hypothetical protein: /protein_id=WP_010875962.1

<400>  74

Met Trp Arg Thr Ile Asn Val Val Phe Lys Leu Lys Thr Pro Leu His
1               5                   10                  15

Ile Gly Tyr Leu Pro Ser Lys Ser Ser Val Val Ser Pro Thr Arg Tyr
            20                  25                  30

Tyr Ile Pro Gly Arg Asn Leu Trp Gly Ala Val Thr Lys Arg Thr Ala
        35                  40                  45

Glu Asn Leu Phe Lys Lys Pro Ala Ala His His Tyr Lys Asn Thr Gly
    50                  55                  60

Lys Asp Ile Met Asn Asn Phe Val Phe Ser Tyr Phe Tyr Ile Tyr Asp
65                  70                  75                  80

Gly Lys Thr Ile Tyr Ser Pro Ser Tyr Thr Asp Glu Gly Ile Lys Tyr
                85                  90                  95

Gly His Asp Ile Lys Ile Thr Arg Ala Glu Phe Glu His Arg Phe Ile
            100                 105                 110

Gly Ser Gln Ile Ser Thr Ala Ile Glu Pro Gly Phe Ser Thr Ala Lys
        115                 120                 125

Asp Gln Ser Leu His Glu Ile Glu Phe Ile Asn Asn Lys Ser Cys Asp
    130                 135                 140

Lys Thr Glu Thr Pro Glu Asp Val Lys Val Thr Gly Trp Ile Trp Ile
145                 150                 155                 160

Lys Glu Asp Ala Lys Ile Asn Asn Arg Glu Val Asp Val Asp Ala Gly
                165                 170                 175

Ile Phe Ile Asp Asn Phe Asn Ile Ile Gln Glu Leu Ile Leu Gly Gly
            180                 185                 190

Glu Ser Lys Tyr Gly Phe Gly His Val Leu Leu Asp Gly Val Asn Lys
        195                 200                 205

Ile Asp Pro Pro Phe Glu Val Lys Val Glu Asp Ser Val Thr Val Ser
    210                 215                 220

Ser Glu Phe Leu Ile Ser His Leu Lys Tyr Asp Lys Asn Leu Asn Phe
225                 230                 235                 240

Met Gly Asp Leu Glu Leu Leu Ala Gly Arg Gly Tyr Phe Asp Pro Glu
                245                 250                 255

Thr Arg Arg Gly Gly Ser Gly Pro Gly Ala Thr Ile Ser Lys Pro Gln
            260                 265                 270

Tyr Tyr Phe Thr Pro Gly Thr Lys Leu Glu Asp Lys Thr Tyr Phe Gln
        275                 280                 285

Met Arg Trp Asp Gly Lys Leu Met Arg Leu Ser Ala
    290                 295                 300

<210>  75
<211>  113
<212>  PRT
<213>  Methanothermobacter thermautotrophicus

<220>
<221>  PEPTIDE
<222>  (1)..(113)
<223>  hypothetical protein: /protein_id=WP_010875963.1

<400>  75

Met Gln Cys Ala Gln Lys Ile Ala Val Lys Ser Asp Arg Asn Leu Ala
1               5                   10                  15

Asn His Leu Arg Lys Ala Leu Gly Val Leu Gln Glu Asp Gly Val Tyr
            20                  25                  30

Ala Met Phe Leu Trp Leu Glu Asp Lys Glu Lys Lys Arg Val Arg Lys
        35                  40                  45

Glu Leu Thr Asp Met Leu Asn Arg Pro Glu Ile Arg Glu Cys Leu Leu
    50                  55                  60

Glu Asp Ser Ser Ser Phe Pro Asp Ser Phe Lys Glu Phe Cys Glu Arg
65                  70                  75                  80

Leu Arg Asp Val Ala Arg Asp Ile Tyr Lys Leu Leu Phe Met Lys Arg
                85                  90                  95

Leu Ile Glu Arg Thr Leu Ile Tyr Ser Leu Tyr His Ala Lys Ala Gly
            100                 105                 110

Glu

<210>  76
<211>  287
<212>  PRT
<213>  Methanothermobacter thermautotrophicus

<220>
<221>  PEPTIDE
<222>  (1)..(287)
<223>  type III-B CRISPR module RAMP protein Cmr4:
       /protein_id=WP_010875964.1

<400>  76

Met Asn Arg Ser Leu Glu Pro Glu Lys Val Tyr Ala Arg Ala Leu Asp
1               5                   10                  15

Pro Val His Ile Gly Ala Gly Gly Tyr Arg Leu Gly Arg Ala Asp Asn
            20                  25                  30

Thr Ile Val Arg Glu Pro Ser Thr Asp Ile Pro Lys Ile Pro Gly Thr
        35                  40                  45

Ser Ile Ala Gly Val Thr Arg Glu Phe Tyr Thr Ala Tyr Leu Ala Glu
    50                  55                  60

Asn Glu Phe Gly Asp Lys Ser Ala Glu Glu Lys Arg Lys Arg Ala Glu
65                  70                  75                  80

Glu Lys Ala Lys Ser Ile Phe Gly Asp Glu Asn Lys Lys Gly Val Leu
                85                  90                  95

Arg Phe Tyr Asp Gly Gln Ile Leu Phe Phe Pro Val Ser Ser Val Arg
            100                 105                 110

Gly Thr Val Trp Ile Thr Thr Glu Gln Leu Phe Asn Tyr Trp Cys Val
        115                 120                 125

Lys Asp Glu Lys Ile Ser Tyr Glu Asp Glu Asn Lys Val Tyr Ala Ile
    130                 135                 140

Arg Gly Glu Leu Asn Gly Arg Leu Asn Leu Gly Trp Leu Leu Phe Glu
145                 150                 155                 160

Val Asn Ser Glu Lys Lys Glu Asp Val Asp Leu Pro Asp Glu Ile Asn
                165                 170                 175

Glu Trp Ile Lys Arg Ala Val Ile Val Pro Glu Asn Leu Phe Ser Pro

180                 185                 190

Ile Val Asn Asp Asn Leu Glu Val Arg Thr Ser Val Arg Ile Asp Pro
        195                 200                 205

Glu Thr Gly Thr Ala Val Glu Gly Ala Leu Phe Thr Tyr Glu Ala Ile
    210                 215                 220

Pro Arg Gly Thr Ile Phe Gly Phe Glu Ile Ala Thr Glu Lys Asp Glu
225                 230                 235                 240

Lys Arg Lys Glu Val Leu Asn Ala Val Ser Pro Tyr Leu Lys Tyr Leu
                245                 250                 255

Gly Ile Gly Gly Met Val Thr Arg Gly Phe Gly Arg Leu Glu Leu Ala
            260                 265                 270

Ala Glu His Ser Glu Asp Glu Gln Val Gly Thr Asp Val Lys Pro
        275                 280                 285

<210>  77
<211>  957
<212>  PRT
<213>  Methanothermobacter thermautotrophicus

<220>
<221>  PEPTIDE
<222>  (1)..(957)
<223>  CRISPR-associated protein Csx11:  /protein_id=WP_010875965.1

<400>  77
   Met Ser Asn Ile Lys Asn Ile Arg Lys Arg Arg Lys Glu Val Leu Leu
   1               5                   10                  15
   Gly Glu Ile Gly Ala Leu Leu His Asp Ile Gly Lys Leu His Pro Asn
               20                  25                  30
   Phe Ile Gly Thr Asn Ser Ile Glu Lys Met Pro Pro Arg Phe Phe His
           35                  40                  45
   Ala Asn Ile Asp Asp Phe Leu Lys Thr Glu Leu Ile Ser Ala Leu Lys
       50                  55                  60
   Ala Phe Glu Asp Leu Glu Ile Asn Val Glu Pro Ile Arg Ile Tyr Asn
   65                  70                  75                  80
   Ile Ile Ser Glu His His Arg Asn Asn Val Leu Gln Gly Cys Asp Arg
                   85                  90                  95
   Lys Asp Ser Ala Asp Asp Lys Gly Ile Val Arg Arg Lys Gln Ser Ile
   100 105 110
   Thr Asp Thr Val Ile Ser Ser Pro Phe Gly Tyr Pro Lys Glu Lys Ile
   115 120 125
   His Leu Asn Cys Leu Gln Lys Glu Phe Asp Asp Leu Gln Thr Ile Leu
   130 135 140
   Val Arg Leu Phe Lys Tyr Tyr Ile Ser Gly Val Val Asn Leu Ser Leu
   145 150 155 160
   Phe Arg Arg Met Leu Met Lys Glu Ala Gln Val Phe Phe Ser His Gly
   165 170 175
   Leu Gly Glu Thr Arg Ile Pro Ser Asn Asp Val Thr Leu Trp Asp His
   180 185 190
   Ser Tyr Ser Thr Ala Ser Arg Phe Lys Ser Ile Leu Val Ala Asn Leu
   195 200 205
   Cys Gly Ala Asp Ile Lys Asp Pro Glu Leu Arg Ile Phe Gly Ile Phe
   210 215 220
   Trp Asn Gly Ile Glu Phe Ile Asn Lys Gly Arg Lys Ile Ala Glu Ile
   225 230 235 240
   Lys Ala Arg Lys Glu Ile Ile Glu Arg Ile Lys Glu Lys Leu Lys Gly
   245 250 255
   Lys Phe Glu Asp Glu Ile Pro Val Gly Asn Ser Ile Tyr Glu Asp Ile
   260 265 270
   Asn Gly Ile Cys Phe Thr Phe Pro Glu Phe Asp Arg Ala Glu Glu Leu
   275 280 285
   Ala Asn Gln Cys Ala Lys Glu Ala Leu Lys Ile Phe Leu Glu Glu Ser
   290 295 300
   Glu Asn Glu Leu Trp Pro Phe Phe Thr Leu Ser Lys Pro Ser Lys Thr
   305 310 315 320
   Leu Thr Val Leu Ala Ser Glu Leu Lys Phe Ala Ser Ser Arg Arg Val
   325 330 335
   Tyr Pro Lys Ile Thr Pro Ala Leu Phe Ile Asp Lys Lys Gln Lys Lys
   340 345 350
   Ile Ile Ala Asp Asn Pro Glu Met Pro Ile Pro Gln Glu Gly Glu Asp
           355                 360                 365
   Ile Cys Pro Leu Cys Arg Leu Arg Ala Lys Pro Ile Glu Lys Glu Arg
       370                 375                 380
   Cys Glu Ile Cys Ser Glu Arg Ile Arg Gly Arg Leu Ala Asn Trp Ser
   385                 390                 395                 400
   Asn Lys Lys Glu Asn Thr Ile Trp Ile Asp Glu Val Ala Asp Lys Asn
                   405                 410                 415
   Asn Arg Ile Ala Leu Ile Ala Leu Asn Phe Asp Leu Asp Lys Trp Phe
               420                 425                 430
   Asp Gly Thr Met Val Gly Thr Ile Tyr Ser Gln Thr Tyr Lys Asp Trp
           435                 440                 445
   Lys His Ser Lys Lys Trp Glu Lys Lys Asn Val Lys Lys Asn Ala Asn
       450                 455                 460
   Ser Val Leu Asn Gly Pro Ile Glu Ala Ile Lys Asp His Val Tyr Lys
   465                 470                 475                 480
   Ile Leu Asn Tyr Ile Leu Glu Asn Glu Asp Ser Asn Glu Glu Lys Ala
                   485                 490                 495
   Ala Lys Leu Leu Asp Thr Phe Phe Glu Glu Glu Ile Gly Leu Arg Asn
               500                 505                 510
   Lys Lys Asp Leu Lys Lys Glu Phe Lys Asn His Leu Gln Asn Ile Arg
           515                 520                 525
   Glu Asn Ile Gly Ala Asp Leu Asn Lys Glu Asn Leu Ala Thr Tyr Leu
       530                 535                 540
   Phe Thr Gln Asn Pro Ser Pro Ala Arg Leu Tyr Arg Ile Trp Lys Glu
   545                 550                 555                 560
   Thr Glu Glu Phe Phe Asp Leu Leu Ile Asn Lys Ile Thr Asp Ile Tyr
                   565                 570                 575
   Ala Tyr Arg Trp Lys Arg Ile Gly Phe Ser Ile Asp Thr His Glu Leu
               580                 585                 590
   Lys Gly Arg Leu Lys Glu Glu Tyr Lys Asn Ile Lys Glu Leu Glu Lys
   595 600 605
   Gly Ser Leu Ile Ile Lys Ile Ser Gly Leu Asp Pro Glu Thr Leu Leu
   610 615 620
   Val Phe His Asp Arg Asn Gly Glu Phe Tyr Thr Ile Glu Ser Leu Gly
   625 630 635 640
   Lys Phe Lys Phe Lys Asn Lys Val Gly Glu Glu Ala Val Lys Glu Ala
   645 650 655
   Leu Lys Gln Gly Ile Lys His Leu Ala Pro Glu Asp Glu Pro Glu Lys
   660 665 670
   Asn Leu Ile Glu Val Gly Lys Thr Ile Lys Thr Glu Asp Asn Leu Tyr
   675 680 685
   Phe Glu Lys Tyr Tyr Pro Leu Ile Glu Ile Asn Arg Ser Pro Leu Ser
   690 695 700
   Leu Arg Phe Ile Val Pro Ala Leu Asp Ser Val Lys Ile Ile Glu Met
   705 710 715 720
   Ile Ala Glu Leu Tyr Asn Lys Arg Phe Glu Lys Val Leu Gly Lys Leu
   725 730 735
   Pro Leu Asn Leu Lys Leu Leu Val Ala Lys Arg Lys Phe Pro Leu Tyr
   740 745 750
   Ile Leu Leu Glu Ala Glu Lys Arg Met Leu Lys Asp Glu Glu Phe Lys
   755 760 765
   Lys Gln Thr Pro Met Asp Pro Trp Trp Ser Val Glu Arg Leu Asp Glu
   770 775 780
   His Tyr Asn Phe Tyr Pro Ile Lys Pro Ala Asn Gly Glu Lys Tyr Thr
   785 790 795 800
   Leu Asp Asp Leu Ser Arg Leu Ser Arg Gly Lys Ile Phe Tyr Leu Tyr
   805 810 815
   Pro Gly Tyr Phe Asp Phe Glu Leu Leu Ser Ala Thr Thr Asp Arg Tyr
   820 825 830
   Asn Ile Cys Tyr Glu Gly Lys Lys Arg Gly His Glu Asp His Arg Leu
   835 840 845
   Phe Ser Gly Arg Pro Leu Tyr Phe His Gln Ile Pro Gln Ile Leu Glu
       850                 855                 860
   Leu Trp Asp Leu Leu Ser Ser Asn Leu Ser Asn Ser Gln Ile Asn Phe
   865                 870                 875                 880
   Ile Glu Lys Ala Leu Thr Ser Lys Leu Arg Glu Trp Lys Asn Val Lys
                   885                 890                 895
   Asp Ala Asn Lys Glu Asn Thr Phe Arg Ile Phe Ala Glu Thr Thr Leu
               900                 905                 910
   Lys Asp Ala Phe Gly His Lys Trp Asp Gly Leu Arg Glu Glu Asn Arg
           915                 920                 925
   Phe Phe Leu Ile Ser Ser Ser Ile Asn Met Leu Leu Leu Asp Thr Ile
       930                 935                 940
   Asn Leu Phe Thr His Val Thr Gly Cys Leu Lys Asp Glu
   945                 950                 955

<210>  78
<211>  261
<212>  PRT
<213>  Methanothermobacter thermautotrophicus

<220>
<221>  PEPTIDE
<222>  (1)..(261)
<223>  hypothetical protein:  /protein_id=WP_010875966.1

<400>  78

Met Asn Leu Asp Gly Val Tyr Asp Phe Tyr Leu His Glu Lys Glu Gly
1               5                   10                  15

Gly Ile Glu Glu Pro Lys Arg Lys Ile Met Asp Ala Val Ala Gly Asp
            20                  25                  30

His Lys Asp Asp Phe Lys Thr Phe Tyr Pro Ser Pro Asp Asp Leu Asn
        35                  40                  45

Tyr Leu Pro Met Asn Ser Val Leu Ile Gly Phe Gln Phe Thr Leu Lys
    50                  55                  60

Lys Pro Tyr Thr Ser Arg Gly Glu Ser Lys Phe Ser Glu Asn Leu Ile
65                  70                  75                  80

Val Arg Asp Gln Phe Thr Gly Leu Cys Met Val Arg Pro Ser Thr Trp
                85                  90                  95

Lys Gly Asn Leu Arg Phe Ala Ala Thr Lys Leu Met Gly Pro Lys Asn
            100                 105                 110

Ser Ile Ile Ile Arg Leu Phe Gly Ser Asn Pro Asp Ser Glu Asp Met
        115                 120                 125

Ile Lys Gly Arg Leu Tyr Phe Phe Pro Thr Phe Phe Lys Glu Gly Ala
    130                 135                 140

Glu Asn Asp Val Ile Thr Pro Leu Lys Arg Glu Thr Arg Thr Pro Ala
145                 150                 155                 160

Arg Gly Pro Ile Pro Leu Glu Val Met Lys Pro Gly Thr Lys Gly Asp
                165                 170                 175

Phe Tyr Leu Leu Tyr Val Pro Tyr Leu Gly Asn Glu Asn Phe Gly Asp
            180                 185                 190

Glu Ile Lys Glu Asp Leu Lys Leu Ile Ala Glu Ala Leu Lys Leu Met
        195                 200                 205

Phe Tyr Thr Tyr Gly Phe Ser Ala Lys Arg Thr Ser Gly Phe Gly Val
    210                 215                 220

Ile Glu Lys Gln Leu Glu Gly Lys Leu Trp Met Arg Gly Gly Val Ser
225                 230                 235                 240

Lys Asn Phe Ser Asn Leu His Glu Leu Ser Glu Ile Leu Asp Gly Tyr
                245                 250                 255

Gly Tyr Glu Gln His
            260

<210> 79
<211> 251
<212> PRT
<213> Methanothermobacter thermautotrophicus

<220>
<221> PEPTIDE
<222> (1)..(251)
<223> type III-B CRISPR module RAMP protein Cmr1:
/protein_id=WP_010875967.1

<400> 79

Met Arg Gly Cys Ser Met Gly Gly Pro Arg Thr Phe Asn Ile Glu Leu
1               5                   10                  15

Gln Ile Leu Asn Glu Ile Phe Thr Gly Gly Pro Glu Arg Arg Ser Glu
            20                  25                  30

Val Ile Arg Asp Thr Gly Leu Ile Gly Ser Leu Arg Trp Trp Tyr Glu
        35                  40                  45

Ala Leu Ile Arg Gly Val Gly Gly Thr Ala Cys Asp Pro Ser Gln Gly
    50                  55                  60

Pro Pro Cys Glu Glu Arg Leu His Cys Asp Ala Cys Glu Leu Phe Gly
65                  70                  75                  80

Cys Ser Gly Trp Ser Arg Lys Phe Ile Phe Arg Ser Arg Glu Glu Ser
                85                  90                  95

Asp Lys Thr Leu Lys Val Gln Ile Thr Glu Leu Arg Arg Met Glu Asp
            100                 105                 110

Val Glu Leu Ala Leu Leu Asn Lys Thr Leu Ser Ile Ile Glu Asn Tyr
        115                 120                 125

Gly Ala Ile Gly Gly Lys Leu Ala His Arg Lys Tyr Gly Ile Ile Lys
    130                 135                 140

Ile Lys Glu Asn Asp Leu Arg Asp Phe Thr Leu Glu Lys Ser Met Leu
145                 150                 155                 160

Gln Gly Tyr Leu Arg Arg Glu Gly Pro His Val Asp Asn Pro Asn Leu
                165                 170                 175

Lys Arg Phe Ile Phe Ile Lys Asn Pro Asn Phe Gln Leu Val Lys Arg
            180                 185                 190

Leu Lys Asn Asp Cys Arg Phe Leu Lys Gly Ser Ser Asn Arg Gly Lys
        195                 200                 205

Arg Tyr Phe Asn Lys Asp Pro Pro Gly Ser Arg Leu Phe Ala Tyr Ala
    210                 215                 220

Asn Glu Asp Glu Tyr Pro Arg Leu Cys Glu Cys Ala Gly Glu Glu Ala
225                 230                 235                 240

Lys Thr Gly Glu Glu Val Leu Gly Gly Leu Ile
                245                 250

<210>  80
<211>  795

<212>  PRT
<213>  Synechocystis spp

<220>
<221>  PEPTIDE
<222>  (1)..(795)
<223>  TIGR03986 family CRISPR-associated RAMP protein
       /protein_id=WP_011153732.1

<400>  80

Met Thr Val Gly Thr Leu Gly Val Val Gly Ser Ala Lys Asn Leu Lys
1               5                   10                  15

Leu Gln Leu Ser Phe Ile Asn Thr Arg Gln Gln Tyr Val Gln Ile Thr
            20                  25                  30

Leu Phe Glu Arg Asn Ser Phe Lys Val Ala Glu Glu Glu Phe Ser Thr
        35                  40                  45

Glu Leu Val Glu Ile Ile Lys Thr Ala Leu Pro Thr Leu Lys Asn Lys
    50                  55                  60

Lys Val Glu Phe Glu Glu Asp Gly Asp Gln Ile Lys Gln Ile Arg Glu
65                  70                  75                  80

Lys Gly Gln Ala Trp Val Gly Ala Ala Glu Gln Ile Ala Pro Tyr Val
                85                  90                  95

Leu Pro Ser Gly Asn Ile Thr Glu Thr Pro Arg Asn Val Asn Ala Ser
            100                 105                 110

Asn Phe His Asn Pro Tyr Asn Phe Val Pro Ala Leu Pro Arg Asp Gly
        115                 120                 125

Ile Thr Gly Asp Leu Gly Asp Cys Ala Pro Ala Gly His Ser Tyr Tyr
    130                 135                 140

His Gly Asp Lys Tyr Ser Gly Arg Ile Ala Val Lys Leu Thr Thr Val
145                 150                 155                 160

Thr Pro Leu Leu Ile Pro Asp Ala Ser Lys Glu Glu Ile Asn Asn Asn
                165                 170                 175

His Lys Thr Tyr Pro Val Arg Ile Gly Lys Asp Gly Lys Pro Tyr Leu
            180                 185                 190

Pro Pro Thr Ser Ile Lys Gly Met Leu Arg Ser Ala Tyr Glu Ala Val
        195                 200                 205

Thr Asn Ser Arg Leu Ala Val Phe Glu Asp His Asp Ser Arg Leu Ala
    210                 215                 220

Tyr Arg Met Pro Ala Thr Met Gly Leu Gln Met Val Pro Ala Arg Ile
225                 230                 235                 240

Glu Gly Asp Asn Ile Val Leu Tyr Pro Gly Thr Ser Arg Ile Gly Asn
                245                 250                 255

Asn Gly Arg Pro Ala Asn Asn Asp Pro Met Tyr Ala Ala Trp Leu Pro
            260                 265                 270

Tyr Tyr Gln Asn Arg Ile Ala Tyr Asp Gly Ser Arg Asp Tyr Gln Met
        275                 280                 285

Ala Glu His Gly Asp His Val Arg Phe Trp Ala Glu Arg Tyr Thr Arg
    290                 295                 300

Gly Asn Phe Cys Tyr Trp Arg Val Arg Gln Ile Ala Arg His Asn Gln
305                 310                 315                 320

Asn Leu Gly Asn Arg Pro Glu Arg Gly Arg Asn Tyr Gly Gln His His
                325                 330                 335

Ser Thr Gly Val Ile Glu Gln Phe Glu Gly Phe Val Tyr Lys Thr Asn
            340                 345                 350

Lys Asn Ile Gly Asn Lys His Asp Glu Arg Val Phe Ile Ile Asp Arg
        355                 360                 365

Glu Ser Ile Glu Ile Pro Leu Ser Arg Asp Leu Arg Arg Lys Trp Arg
    370                 375                 380

Glu Leu Ile Thr Ser Tyr Gln Glu Ile His Lys Lys Glu Val Asp Arg
385                 390                 395                 400

Gly Asp Thr Gly Pro Ser Ala Val Asn Gly Ala Val Trp Ser Arg Gln
                405                 410                 415

Ile Ile Ala Asp Glu Ser Glu Arg Asn Leu Ser Asp Gly Thr Leu Cys
            420                 425                 430

Tyr Ala His Val Lys Lys Glu Asp Gly Gln Tyr Lys Ile Leu Asn Leu
        435                 440                 445

Tyr Pro Val Met Ile Thr Arg Gly Leu Tyr Glu Ile Ala Pro Val Asp
    450                 455                 460

Leu Leu Asp Glu Thr Leu Lys Pro Ala Thr Asp Lys Lys Gln Leu Ser
465                 470                 475                 480

Pro Ala Asp Arg Val Phe Gly Trp Val Asn Gln Arg Gly Asn Gly Cys
                485                 490                 495

Tyr Lys Gly Gln Leu Arg Ile His Ser Val Thr Cys Gln His Asp Asp
            500                 505                 510

Ala Ile Asp Asp Phe Gly Asn Gln Asn Phe Ser Val Pro Leu Ala Ile
        515                 520                 525

Leu Gly Gln Pro Lys Pro Glu Gln Ala Arg Phe Tyr Cys Ala Asp Asp
    530                 535                 540

Arg Lys Gly Ile Pro Leu Glu Asp Gly Tyr Asp Arg Asp Asp Gly Tyr
545                 550                 555                 560

Ser Asp Ser Glu Gln Gly Leu Arg Gly Arg Lys Val Tyr Pro His His
                565                 570                 575

Lys Gly Leu Pro Asn Gly Tyr Trp Ser Asn Pro Thr Glu Asp Arg Ser
            580                 585                 590

Gln Gln Ala Ile Gln Gly His Tyr Gln Glu Tyr Arg Arg Pro Lys Lys
        595                 600                 605

Asp Gly Leu Glu Gln Arg Asp Asp Gln Asn Arg Ser Val Lys Gly Trp
    610                 615                 620

Val Lys Pro Leu Thr Glu Phe Thr Phe Glu Ile Asp Val Thr Asn Leu
625                 630                 635                 640

Ser Glu Val Glu Leu Gly Ala Leu Leu Trp Leu Leu Thr Leu Pro Asp
                645                 650                 655

Leu His Phe His Arg Leu Gly Gly Gly Lys Pro Leu Gly Phe Gly Ser
            660                 665                 670

Val Arg Leu Asp Ile Asp Pro Asp Lys Thr Asp Leu Arg Asn Gly Ala
        675                 680                 685

Gly Trp Arg Asp Tyr Tyr Gly Ser Leu Leu Glu Thr Ser Gln Pro Asp
    690                 695                 700

Phe Thr Thr Leu Ile Ser Gln Trp Ile Asn Ala Phe Gln Thr Ala Val

705                 710                 715                 720

Lys Glu Glu Tyr Gly Ser Ser Ser Phe Asp Gln Val Thr Phe Ile Lys
                725                 730                 735

Ala Ser Gly Gln Ser Leu Gln Gly Phe His Asp Asn Ala Ser Ile His
            740                 745                 750

Tyr Pro Arg Ser Thr Pro Glu Pro Lys Pro Asp Gly Glu Ala Phe Lys
        755                 760                 765

Trp Phe Val Ala Asn Glu Lys Gly Arg Arg Leu Ala Leu Pro Ala Leu
    770                 775                 780

Glu Lys Ser Gln Ser Phe Pro Ile Lys Pro Ser
785                 790                 795

<210>  81
<211>  193
<212>  PRT
<213>  Synechocystis spp

<220>
<221>  PEPTIDE
<222>  (1)..(193)
<223>  TIGR03984 family CRISPR-associated protein
       /protein_id=WP_011153733.1

<400>  81

Met Pro Ala Gly Gly Arg Leu Met Lys Asn Leu Tyr His Tyr His Gln
1               5                   10                  15

Tyr Glu Ile Thr Leu Glu Ser Ala Val Asp Ser Cys Lys Asn His Leu
            20                  25                  30

Gln Ala Ala Ile Gly Leu Leu Tyr Ser Pro Gln Lys Cys Glu Leu Val
        35                  40                  45

Lys Leu Asp Asn Ser Gly Lys Leu Val Asp Ser Tyr Asn Arg Leu Lys
    50                  55                  60

Phe Asn Asn Leu Gly Val Phe Glu Ala Arg Phe Phe Asn Leu Asn Cys
65                  70                  75                  80

Glu Leu Arg Trp Val Asn Glu Ser Asn Gly Asn Gly Thr Ala Val Leu
                85                  90                  95

Leu Ser Glu Ser Asp Ile Thr Leu Thr Gly Phe Glu Lys Gly Leu Gln
            100                 105                 110

Glu Phe Ile Thr Ala Ile Asp Gln Gln Tyr Leu Leu Trp Gly Glu Pro
        115                 120                 125

Ala Lys His Pro Pro Asn Ala Asp Gly Trp Gln Arg Leu Ala Glu Ala
    130                 135                 140

Arg Ile Gly Lys Leu Asp Ile Pro Leu Asp Asn Pro Leu Lys Pro Lys
145                 150                 155                 160

Asp Arg Val Phe Leu Thr Ser Glu Glu Tyr Ile Ala Glu Val Asp Asp
                165                 170                 175

Phe Gly Asn Cys Ala Val Ile Asp Glu Arg Leu Ile Lys Leu Glu Val
            180                 185                 190

Lys

<210>  82
<211>  522
<212>  PRT
<213>  Synechocystis spp

<220>
<221>  PEPTIDE
<222>  (1)..(522)
<223>  hypothetical protein:  /protein_id=WP_011153734.1

<400>  82

Met Ala Arg Lys Val Thr Thr Arg Trp Lys Ile Thr Gly Thr Leu Ile
1               5                   10                  15

Ala Glu Thr Pro Leu His Ile Gly Gly Val Gly Gly Asp Ala Asp Thr
            20                  25                  30

Asp Leu Ala Leu Ala Val Asn Gly Ala Gly Glu Tyr Tyr Val Pro Gly
        35                  40                  45

Thr Ser Leu Ala Gly Ala Leu Arg Gly Trp Met Thr Gln Leu Leu Asn
    50                  55                  60

Asn Asp Glu Ser Gln Ile Lys Asp Leu Trp Gly Asp His Leu Asp Ala
65                  70                  75                  80

Lys Arg Gly Ala Ser Phe Val Ile Val Asp Asp Ala Val Ile His Ile
                85                  90                  95

Pro Asn Asn Ala Asp Val Glu Ile Arg Glu Gly Val Gly Ile Asp Arg
                100                 105                 110

His Phe Gly Thr Ala Ala Asn Gly Phe Lys Tyr Ser Arg Ala Val Ile
            115                 120                 125

Pro Lys Gly Ser Lys Phe Lys Leu Pro Leu Thr Phe Asp Ser Gln Asp
    130                 135                 140

Asp Gly Leu Pro Asn Ala Leu Ile Gln Leu Leu Cys Ala Leu Glu Ala
145                 150                 155                 160

Gly Asp Ile Arg Leu Gly Ala Ala Lys Thr Arg Gly Leu Gly Arg Ile
                165                 170                 175

Lys Leu Asp Asp Leu Lys Leu Lys Ser Phe Ala Leu Asp Lys Pro Glu
                180                 185                 190

Gly Ile Phe Ser Ala Leu Leu Asp Gln Gly Lys Lys Leu Asp Trp Asn
            195                 200                 205

Gln Leu Lys Ala Asn Val Thr Tyr Gln Ser Pro Pro Tyr Leu Gly Ile
    210                 215                 220

Ser Ile Thr Trp Asn Pro Lys Asp Pro Val Met Val Lys Ala Glu Gly
225                 230                 235                 240

Asp Gly Leu Ala Ile Asp Ile Leu Pro Leu Val Ser Gln Val Gly Ser
                245                 250                 255

Asp Val Arg Phe Val Ile Pro Gly Ser Ser Ile Lys Gly Ile Leu Arg
                260                 265                 270

Thr Gln Ala Glu Arg Ile Ile Arg Thr Ile Cys Gln Ser Asn Gly Ser
            275                 280                 285

Glu Lys Asn Phe Leu Glu Gln Leu Arg Ile Asn Leu Val Asn Glu Leu
    290                 295                 300

Phe Gly Ser Ala Ser Leu Ser Gln Lys Gln Asn Gly Lys Asp Ile Asp
305                 310                 315                 320

Leu Gly Lys Ile Gly Ala Leu Ala Val Asn Asp Cys Phe Ser Ser Leu
                325                 330                 335

Ser Met Thr Pro Asp Gln Trp Lys Ala Val Glu Asn Ala Thr Glu Met
                340                 345                 350

Thr Gly Asn Leu Gln Pro Ala Leu Lys Gln Ala Thr Gly Tyr Pro Asn
        355                 360                 365

Asn Ile Ser Gln Ala Tyr Lys Val Leu Gln Pro Ala Met His Val Ala
    370                 375                 380

Val Asp Arg Trp Thr Gly Gly Ala Ala Glu Gly Met Leu Tyr Ser Val
385                 390                 395                 400

Leu Glu Pro Ile Gly Val Thr Trp Glu Pro Ile Gln Val His Leu Asp
                405                 410                 415

Ile Ala Arg Leu Lys Asn Tyr Tyr His Gly Lys Glu Glu Lys Leu Lys
            420                 425                 430

Pro Ala Ile Ala Leu Leu Leu Leu Val Leu Arg Asp Leu Ala Asn Lys
        435                 440                 445

Lys Ile Pro Val Gly Tyr Gly Thr Asn Arg Gly Met Gly Thr Ile Thr
    450                 455                 460

Val Ser Gln Ile Thr Leu Asn Gly Lys Ala Leu Pro Thr Glu Leu Glu
465                 470                 475                 480

Pro Leu Asn Lys Thr Met Thr Cys Pro Asn Leu Thr Asp Leu Asp Glu
                485                 490                 495

Ala Phe Arg Gln Asp Leu Ser Thr Ala Trp Lys Glu Trp Ile Ala Asp
            500                 505                 510

Pro Ile Asp Leu Cys Gln Gln Glu Ala Ala
        515                 520

<210>  83
<211>  791
<212>  PRT
<213>  Synechocystis spp

<220>
<221>  PEPTIDE
<222>  (1)..(791)
<223>  hypothetical protein: /protein_id=WP_011153735.1

<400>  83

Met Arg Gly Ile Glu Ile Thr Ile Thr Met Gln Ser Asp Trp His Val
1               5                   10                  15

Gly Thr Gly Met Gly Arg Gly Glu Leu Asp Ser Val Val Gln Arg Asp

20                  25                  30

Gly Asp Asn Leu Pro Tyr Ile Pro Gly Lys Thr Leu Thr Gly Ile Leu
        35                  40                  45

Arg Asp Ser Cys Glu Gln Val Ala Leu Gly Leu Asp Asn Gly Gln Thr
    50                  55                  60

Arg Gly Leu Trp His Gly Trp Ile Asn Phe Ile Phe Gly Asp Gln Pro
65                  70                  75                  80

Ala Leu Ala Gln Gly Ala Ile Glu Pro Glu Pro Arg Pro Ala Leu Ile
                85                  90                  95

Ala Ile Gly Ser Ala His Leu Asp Pro Lys Leu Lys Ala Ala Phe Gln
            100                 105                 110

Gly Lys Lys Gln Leu Gln Glu Ala Ile Ala Phe Met Lys Pro Gly Val
        115                 120                 125

Ala Ile Asp Ala Ile Thr Gly Thr Ala Lys Lys Asp Phe Leu Arg Phe
    130                 135                 140

Glu Glu Val Val Arg Leu Gly Ala Lys Leu Thr Ala Glu Val Glu Leu
145                 150                 155                 160

Asn Leu Pro Asp Asn Leu Ser Glu Thr Asn Lys Lys Val Ile Ala Gly
                165                 170                 175

Ile Leu Ala Ser Gly Ala Lys Leu Thr Glu Arg Leu Gly Gly Lys Arg
            180                 185                 190

Arg Arg Gly Asn Gly Arg Cys Glu Leu Lys Phe Ser Gly Tyr Ser Asp
        195                 200                 205

Gln Gln Ile Gln Trp Leu Lys Asp Asn Tyr Gln Ser Val Asp Gln Pro
    210                 215                 220

Pro Lys Tyr Gln Gln Asn Lys Leu Gln Ser Ala Gly Asp Asn Pro Glu
225                 230                 235                 240

Gln Gln Pro Pro Trp His Ile Ile Pro Leu Thr Ile Lys Thr Leu Ser
                245                 250                 255

Pro Val Val Leu Pro Ala Arg Thr Val Gly Asn Val Val Glu Cys Leu
            260                 265                 270

Asp Tyr Ile Pro Gly Arg Tyr Leu Leu Gly Tyr Ile His Lys Thr Leu
        275                 280                 285

Gly Glu Tyr Phe Asp Val Ser Gln Ala Ile Ala Ala Gly Asp Leu Ile
    290                 295                 300

Ile Thr Asn Ala Thr Ile Lys Ile Asp Gly Lys Ala Gly Arg Ala Thr
305                 310                 315                 320

Pro Phe Cys Leu Phe Gly Glu Lys Leu Asp Gly Gly Leu Gly Lys Gly
                325                 330                 335

Lys Gly Val Tyr Asn Arg Phe Gln Glu Ser Glu Pro Asp Gly Ile Gln
            340                 345                 350

Leu Lys Gly Glu Arg Gly Gly Tyr Val Gly Gln Phe Glu Gln Glu Gln
        355                 360                 365

Arg Asn Leu Pro Asn Thr Gly Lys Ile Asn Ser Glu Leu Phe Thr His
    370                 375                 380

Asn Thr Ile Gln Asp Asp Val Gln Arg Pro Thr Ser Asp Val Gly Gly
385                 390                 395                 400

Val Tyr Ser Tyr Glu Ala Ile Ile Ala Gly Gln Thr Phe Val Ala Glu
                405                 410                 415

Leu Arg Leu Pro Asp Ser Leu Val Lys Gln Ile Thr Ser Lys Asn Lys
            420                 425                 430

Asn Trp Gln Ala Gln Leu Lys Ala Thr Ile Arg Ile Gly Gln Ser Lys
        435                 440                 445

Lys Asp Gln Tyr Gly Lys Ile Glu Val Thr Ser Gly Asn Ser Ala Asp
    450                 455                 460

Leu Pro Lys Pro Thr Gly Asn Asn Lys Thr Leu Ser Ile Trp Phe Leu
465                 470                 475                 480

Ser Asp Ile Leu Leu Arg Gly Asp Arg Leu Asn Phe Asn Ala Thr Pro
                485                 490                 495

Asp Asp Leu Lys Lys Tyr Leu Glu Asn Ala Leu Asp Ile Lys Leu Lys
            500                 505                 510

Glu Arg Ser Asp Asn Asp Leu Ile Cys Ile Ala Leu Arg Ser Gln Arg
        515                 520                 525

Thr Glu Ser Trp Gln Val Arg Trp Gly Leu Pro Arg Pro Ser Leu Val
    530                 535                 540

Gly Trp Gln Ala Gly Ser Cys Leu Ile Tyr Asp Ile Glu Ser Gly Thr
545                 550                 555                 560

Val Asn Ala Glu Lys Leu Gln Glu Leu Met Ile Thr Gly Ile Gly Asp
                565                 570                 575

Arg Cys Thr Glu Gly Tyr Gly Gln Ile Gly Phe Asn Asp Pro Leu Leu
            580                 585                 590

Ser Ala Ser Leu Gly Lys Leu Thr Ala Lys Pro Lys Ala Ser Asn Asn
        595                 600                 605

Gln Ser Gln Asn Ser Gln Ser Asn Pro Leu Pro Thr Asn His Pro Thr
    610                 615                 620

Gln Asp Tyr Ala Arg Leu Ile Glu Lys Ala Ala Trp Arg Glu Ala Ile
625                 630                 635                 640

Gln Asn Lys Ala Leu Ala Leu Ala Ser Ser Arg Ala Lys Arg Glu Glu
                645                 650                 655

Ile Leu Gly Ile Lys Ile Met Gly Lys Asp Ser Gln Pro Thr Met Thr
            660                 665                 670

Gln Leu Gly Gly Phe Arg Ser Val Leu Lys Arg Leu His Ser Arg Asn
        675                 680                 685

Asn Arg Asp Ile Val Thr Gly Tyr Leu Thr Ala Leu Glu Gln Val Ser
    690                 695                 700

Asn Arg Lys Glu Lys Trp Ser Asn Thr Ser Gln Gly Leu Thr Lys Ile
705                 710                 715                 720

Arg Asn Leu Val Thr Gln Glu Asn Leu Ile Trp Asn His Leu Asp Ile
                725                 730                 735

Asp Phe Ser Pro Leu Thr Ile Thr Gln Asn Gly Val Asn Gln Leu Lys
            740                 745                 750

Ser Glu Leu Trp Ala Glu Ala Val Arg Thr Leu Val Asp Ala Ile Ile
        755                 760                 765

Arg Gly His Lys Arg Asp Leu Glu Lys Ala Gln Glu Asn Glu Ser Asn
    770                 775                 780

Gln Gln Ser Gln Gly Ala Ala
785                 790

<210>  84
<211>  558
<212>  PRT
<213>  Synechocystis spp

<220>
<221>  PEPTIDE
<222>  (1)..(558)
<223>  hypothetical protein/protein_id=WP_011153736.1

<400>  84

Met Phe Leu Val Leu Ile Glu Thr Ser Gly Asn Gln His Phe Ile Phe
1               5                   10                  15

Ser Thr Asn Lys Leu Arg Glu Asn Ile Gly Ala Ser Glu Leu Thr Tyr
            20                  25                  30

Leu Ala Thr Thr Glu Ile Leu Phe Gln Gly Val Asp Arg Val Phe Gln
        35                  40                  45

Thr Asn Tyr Tyr Asp Gln Trp Ser Asp Thr Asn Ser Leu Asn Phe Leu
    50                  55                  60

Ala Asp Ser Lys Leu Asn Pro Ala Ile Asp Asp Pro Lys Asn Asn Ala
65                  70                  75                  80

Asp Ile Glu Ile Leu Leu Ala Thr Ser Gly Lys Ala Ile Ala Leu Val
                85                  90                  95

Lys Glu Glu Gly Lys Ala Lys Gln Leu Ile Lys Glu Val Thr Lys Gln
            100                 105                 110

Ala Leu Ile Asn Ala Pro Gly Leu Glu Ile Gly Gly Ile Tyr Val Asn
        115                 120                 125

Cys Asn Trp Gln Asp Lys Leu Gly Val Ala Lys Ala Val Lys Glu Ala
    130                 135                 140

His Lys Gln Phe Glu Val Asn Arg Ala Lys Arg Ala Gly Ala Asn Gly
145                 150                 155                 160

Arg Phe Leu Arg Leu Pro Ile Ala Ala Gly Cys Ser Val Ser Glu Leu
                165                 170                 175

Pro Ala Ser Asp Phe Asp Tyr Asn Ala Asp Gly Asp Lys Ile Pro Val
            180                 185                 190

Ser Thr Val Ser Lys Val Lys Arg Glu Thr Ala Lys Ser Ala Lys Lys
        195                 200                 205

Arg Leu Arg Ser Val Asp Gly Arg Leu Val Asn Asp Leu Ala Gln Leu
    210                 215                 220

Glu Lys Ser Phe Asp Glu Leu Asp Trp Leu Ala Val Val His Ala Asp
225                 230                 235                 240

Gly Asn Gly Leu Gly Gln Ile Leu Leu Ser Leu Glu Lys Tyr Ile Gly
                245                 250                 255

Glu Gln Thr Asn Arg Asn Tyr Ile Asp Lys Tyr Arg Arg Leu Ser Leu
            260                 265                 270

Ala Leu Asp Asn Cys Thr Ile Asn Ala Phe Lys Met Ala Ile Ala Val
        275                 280                 285

Phe Lys Glu Asp Ser Lys Lys Ile Asp Leu Pro Ile Val Pro Leu Ile
    290                 295                 300

Leu Gly Gly Asp Asp Leu Thr Val Ile Cys Arg Gly Asp Tyr Ala Leu
305                 310                 315                 320

Glu Phe Thr Arg Glu Phe Leu Glu Ala Phe Glu Gly Gln Thr Glu Thr
                325                 330                 335

His Asp Asp Ile Lys Val Ile Ala Gln Lys Ala Phe Gly Val Asp Arg
            340                 345                 350

Leu Ser Ala Cys Ala Gly Ile Ser Ile Ile Lys Pro His Phe Pro Phe
        355                 360                 365

Ser Val Ala Tyr Thr Leu Ala Glu Arg Leu Ile Lys Ser Ala Lys Glu
    370                 375                 380

Val Lys Gln Lys Val Thr Val Thr Asn Ser Ser Pro Ile Thr Pro Phe
385                 390                 395                 400

Pro Cys Ser Ala Ile Asp Phe His Ile Leu Tyr Asp Ser Ser Gly Ile
                405                 410                 415

Asp Phe Asp Arg Ile Arg Glu Lys Leu Arg Pro Glu Asp Asn Thr Glu
            420                 425                 430

Leu Tyr Asn Arg Pro Tyr Val Val Thr Ala Ala Glu Asn Leu Ser Gln
        435                 440                 445

Ala Gln Gly Tyr Glu Trp Ser Gln Ala His Ser Leu Gln Thr Leu Ala
    450                 455                 460

Asp Arg Val Ser Tyr Leu Arg Ser Glu Asp Gly Glu Gly Lys Ser Ala
465                 470                 475                 480

Leu Pro Ser Ser Gln Ser His Ala Leu Arg Thr Ala Leu Tyr Leu Glu
                485                 490                 495

Lys Asn Glu Ala Asp Ala Gln Tyr Ser Leu Ile Ser Gln Arg Tyr Lys
            500                 505                 510

Ile Leu Lys Asn Phe Ala Glu Asp Gly Glu Asn Lys Ser Leu Phe His
        515                 520                 525

Leu Glu Asn Gly Lys Tyr Val Thr Arg Phe Leu Asp Ala Leu Asp Ala
    530                 535                 540

Lys Asp Phe Phe Ala Asn Ala Asn His Lys Asn Gln Gly Glu
545                 550                 555

<210>  85
<211>  367
<212>  PRT
<213>  Synechocystis spp

<220>
<221>  PEPTIDE
<222>  (1)..(367)
<223>  CRISPR-associated endoribonuclease Cas6/protein_id=WP_011153737.1

<400>  85

Met Val Asp Leu Lys Ser Leu Ala Gly Ala Glu Met Val Gly Leu Arg
1               5                   10                  15

Trp Gln Leu Arg Phe Asp Arg Pro Cys Arg Leu Glu Ser His Tyr Val
            20                  25                  30

Lys Gly Leu His Ala Trp Phe Leu His Gln Val Gln Ala Ile Asp Pro
        35                  40                  45

Asp Val Ser Ala Trp Leu His Asp Gly Gln Gly Glu Lys Pro Phe Thr
    50                  55                  60

Ile Ser Arg Leu Ile Gly Pro Thr Leu Trp Gln Glu Gly His Trp His

65                  70                  75                  80

Trp Gln Ile Asn Lys Thr Tyr His Trp Gln Leu Asn Leu Leu Ser Gly
                85                  90                  95

Ala Leu Ile Glu Ala Leu Gln Pro Trp Leu Ala Arg Leu Pro Asn Lys
            100                 105                 110

Ile Val Leu Ala Arg Gln Thr Leu Trp Val Glu Ala Val Asp Cys Tyr
        115                 120                 125

Leu Ala Pro His Asn Tyr Gln Gln Leu Trp Pro Gln Gly Ala Leu Pro
    130                 135                 140

Arg Arg Gln Glu Phe Thr Phe Thr Ser Pro Thr Ser Phe Arg Arg Gln
145                 150                 155                 160

Gly Asn His Tyr Pro Leu Pro Glu Pro Arg Asn Val Leu Gln Ser Tyr
                165                 170                 175

Leu Arg Arg Trp Asn Asp Phe Ser Gly Leu Ala Phe Glu Pro Glu Pro
            180                 185                 190

Phe Leu Asp Tyr Trp Val Pro Gln Asn Val Val Ile Asp Arg His Trp
        195                 200                 205

Leu Glu Ser Val Lys Thr Thr Ala Gly Lys Gln Gly Ser Val Val Gly
    210                 215                 220

Phe Val Gly Ala Val Ser Leu Val Leu Thr Pro Gln Ala Arg Asn Asp
225                 230                 235                 240

Gly Asp Asp Tyr Gly Arg Leu Phe His Ala Leu Cys Arg Tyr Gly Pro
                245                 250                 255

Tyr Cys Gly Thr Gly His Lys Thr Thr Phe Gly Leu Gly Gln Thr Met
            260                 265                 270

Ala Gly Trp Ala Thr Pro Asp Leu Lys Thr Phe Ala Cys Leu Gln Glu
        275                 280                 285

Asp Leu Gln Thr Gln Val Leu Thr Gln Arg Ile Asp Gln Cys Ala Ser
    290                 295                 300

Leu Leu Leu Ala Gln Arg Gln Arg Thr Gly Gly Gln Arg Ala Gln Glu
305                 310                 315                 320

Ile Cys His Thr Leu Ala Thr Ile Phe Val Arg Arg Glu Gln Gly Glu
                325                 330                 335

Ser Leu Gln Glu Ile Ala Leu Asp Leu Gln Leu Pro Tyr Glu Thr Ala
            340                 345                 350

Arg Thr Tyr Ser Lys Arg Ala Lys Arg Ala Leu Ala Asn Val Gln
        355                 360                 365

<210>  86
<211>  766
<212>  PRT
<213>  Thioalkalivibrio sp.

<220>
<221>  PEPTIDE
<222>  (1)..(766)
<223>  Rad3-related DNA helicase-like protein:
       /protein_id=WP_013006550.1

<400>  86

Met Gln Ile Arg Leu Lys Tyr Ser Pro Glu Arg Leu Thr Asp Ile Pro
1               5                   10                  15

Ala Thr Gly Gln Ser Arg Phe Phe Arg Glu Arg Leu His Ala Ala Ala
            20                  25                  30

His Thr Ala Arg Arg Arg Arg Leu Pro Ala Val Gly Leu Asp Ser Ile
        35                  40                  45

Ser Ile Asn Leu Gly Ala Arg Asp Leu Ala Val Leu Ser Gly Leu Arg
    50                  55                  60

Gln Gly Asp Glu Pro Trp Ser Glu Val Leu Ser Glu Leu Leu His Gly
65                  70                  75                  80

His Leu Asn Gln Pro Gly Gly Glu Pro Arg Ser Lys Arg Thr Glu Ala
                85                  90                  95

Ala Asp Arg Arg Ala Glu Gly Pro Asp Trp Met Arg Pro Glu Gln Gly
            100                 105                 110

Arg Phe Tyr Ala Pro Leu Ala Glu Gly Leu Ala Ala Asn Ala Ile Leu
        115                 120                 125

Leu Ala Glu Ala Ala Thr Gly Thr Gly Lys Gly Arg Ala Val Ala Gln
    130                 135                 140

Ala Val Glu Asp Ala Leu Ala Ala Gly Arg Glu Arg Val Val Val Cys

145                 150                 155                 160

Gly Pro Thr Leu Gly Val Leu Ala Ala Leu Ala Arg Glu Phe Gly Ser
                165                 170                 175

Cys Pro Gly Leu Asn Arg Pro Met Gln Phe Phe Leu Gly Ala Ala Gln
            180                 185                 190

Phe Phe His Ala Gly Arg Ala Arg Gln Leu Ala Ser Leu Met Gln Ser
        195                 200                 205

Gly Ser Asp Pro Glu Thr Gln Ala Ser Gly Ser Ala Leu Leu Gln Trp
    210                 215                 220

Leu Asp Ala Gly Ala Pro Ala Gly Arg Thr Asp Ser Thr Ala Ala Phe
225                 230                 235                 240

Ala Ser Val Glu Gly Gln Pro Val Ser His Leu Thr Asp Asp Leu Ala
                245                 250                 255

Thr Leu Leu Pro Asp Gly Tyr Gly Val Glu Glu Leu Glu Leu Ser Ala
            260                 265                 270

Glu Glu Ile Gly Gly Asp Ala Glu Ser Glu Thr Asp Pro Gly Ile Arg
        275                 280                 285

Ala Ala Met Ala Ala Arg Asp Leu Glu Gly Asp Ala Pro Ile Val Phe
    290                 295                 300

Cys Thr His Ala Met Tyr Ala Leu His Ala Arg Met Ile Gly Leu Glu
305                 310                 315                 320

Arg Tyr Leu Leu Pro Pro His Glu Val Leu Val Leu Asp Glu Ala His
                325                 330                 335

Gln Phe Glu Glu Asn Ala Asn Ala Val Leu Ser Pro Thr Ala Ser Leu
            340                 345                 350

Asp Gln Leu Pro Arg Leu Leu Arg Ser Leu Pro Ala Ala Leu Leu Leu
        355                 360                 365

Lys Thr Gly Lys Arg Asp Leu Glu Ala Val Glu Glu Val Leu Lys Asp
    370                 375                 380

Leu His Asn Arg Phe Pro Glu Arg Gly Gly Glu Ile Leu Leu Phe Pro
385                 390                 395                 400

Gly Asp Asp Arg Tyr Ala Ala Val Gln Val Gln Ala Gly Arg Leu His
                405                 410                 415

Gln Ala Val Asp Ala Leu Ile Gly Arg Ala Glu Arg Ala Arg Gln His
            420                 425                 430

Thr Ala Asp Pro Gly Leu Leu Ala Leu Leu Lys Arg Thr Arg Ser Ile
        435                 440                 445

Leu Arg Ala Ser Gln Arg Glu Arg Ala Leu Leu Gln Leu Ser Ser Ser
    450                 455                 460

Pro Val Arg Gly Tyr Leu Ser Leu Ala Val Gly Gly Gln Gly Leu Arg
465                 470                 475                 480

Arg Leu Phe Glu Arg Met Trp Ser Gln Val Asp Ala Ala Val Leu Met
                485                 490                 495

Ser Ala Thr Leu Tyr Val Pro Thr Arg Asp Ser Arg Arg Ala Asp Val
            500                 505                 510

Ser Asp Thr Gln Gly Trp Tyr Ser Ala Arg His Leu Thr Gln Val Leu
        515                 520                 525

Ala Leu Pro Arg Glu Arg Leu Arg Thr Leu Lys Pro Val Arg Pro Asp
    530                 535                 540

Trp Val Arg Gly Asn Val Ile Ala His Ile Glu Arg Asn Pro Glu Arg
545                 550                 555                 560

Arg Pro Pro Ser Thr Ser Glu Thr Asp Pro Ala Ile Tyr Ala Ala Leu
                565                 570                 575

Val Gln Gln Trp Leu Asp Asp Thr Ala Arg Asp Leu Ala Arg Ile Thr
            580                 585                 590

Glu Gly Ala Glu Gln Gly Thr Leu Val Leu Leu Thr Ala Tyr Arg Asp
        595                 600                 605

Ile Glu Gly Leu Ser Glu Arg Leu Ala Asp Thr Leu Gly Glu Arg Leu
    610                 615                 620

Ile Ala Gln Gly Pro Gly Ala Arg Leu Ser Ala Leu Arg Glu Ala Phe
625                 630                 635                 640

Val Ala Ser Ser Arg Asn Gly Glu Arg Pro Val Trp Leu Ala Thr Gly
                645                 650                 655

Ala Ala Trp Thr Gly Ile Asn Leu Thr Ser Glu Gly Leu Ser Gln Val
            660                 665                 670

Ser Asp Leu Val Ile Ala Arg Leu Pro Val Gly Leu Asn Arg Ser Ile
        675                 680                 685

Ser Gln Ala Ala Arg Asn Arg Gly Arg Ala Asn Phe Glu Ser Lys Ala
    690                 695                 700

Ala Glu Thr Tyr Trp Gln Thr Ala Gln Gly Ile Gly Arg Met Val Arg
705                 710                 715                 720

Ser Glu Gln Asp Thr Asp Arg His Leu Trp Val Leu Asp Pro Arg Ala
                725                 730                 735

Val Ile Glu Pro Leu Tyr Thr Pro Val Arg Leu Leu Leu Glu Ser Tyr
            740                 745                 750

Arg Val Ser Asp Gly Pro Arg Asp Ser Asp Cys Ser Pro Pro
        755                 760                 765

<210>  87
<211>  233
<212>  PRT
<213>  Thioalkalivibrio sp.

<220>
<221>  PEPTIDE
<222>  (1)..(233)
<223>  hypothetical protein:   /protein_id=WP_013006551.1

<400>  87

Met Tyr Phe Ala His Thr Val Leu Trp Pro Gly Lys Pro Pro Ala Ala
1               5                   10                  15

Tyr Arg Arg Ile Leu Ala Ser Leu Val Pro Asp Glu Gly Glu Phe Gln
            20                  25                  30

Ile Pro Pro Glu Ile Phe His Tyr Gly Pro Asp Gly Lys Pro Leu Gln
        35                  40                  45

Gly Leu Ser Pro Phe Arg Phe Ser Ser Lys Ser Gly Asp Gly Asp Gln
    50                  55                  60

Thr Gln Leu Val Leu Ser Ala Ile Gly Pro Gln Ala Val Ala Met Leu
65                  70                  75                  80

Arg Arg Glu Thr Trp Lys Ile Thr His Leu Leu Ser Glu Ser Lys Gly

85                 90                  95

His Pro Leu Ala Asp Arg Thr His Thr Gly Tyr Cys Glu Leu Arg Val
            100                 105                 110

Ala Pro Arg Pro Glu Thr Phe Leu Val Asn His Met Ile Ala Glu Arg
        115                 120                 125

His Gln Arg Glu Arg Gly Ala Ser Ser Asp Pro Glu Val Ile Arg Arg
    130                 135                 140

Lys Ile Leu Ala Gly Leu Glu Ala Gln Cys Ala Leu Leu Gly Arg Pro
145                 150                 155                 160

Leu Pro Glu Gly Val Glu Glu Ala Ile Tyr Asp Ile Arg Pro Ser Glu
                165                 170                 175

Lys Thr Phe Ala Thr Pro Ile Ala Gly Gly Met Val Gly Val Gly Pro
            180                 185                 190

His Glu Ile Glu Met Asp Ala Ala Leu Glu Ile Lys Gly Pro Trp His
        195                 200                 205

Val Gly Ser Leu Ala Ala Arg Gly Phe Gly Arg Ile Leu Gln Pro Arg
    210                 215                 220

Arg Ala Gln Asp Arg Lys Val Ala Ala
225                 230

<210>  88
<211>  243
<212>  PRT
<213>  Thioalkalivibrio sp.

<220>
<221>  PEPTIDE
<222>  (1)..(243)
<223>  hypothetical protein /protein_id=WP_018940624.1

<400>  88

Met Leu Ala Glu Ala Thr Gly Leu Glu Pro Ile Gly Gly Pro Ala Pro
1               5                   10                  15

Glu Ala Cys Thr Cys Arg Leu Cys Gly Gly Ala Val Ala Ala Gly Glu
            20                  25                  30

Pro Arg Phe Glu Gln Lys Phe Gly Ala Thr Phe Asn Asp Gly Pro Ala
        35                  40                  45

Thr Leu Asp Arg Val Gly Asn Trp Ile Cys Gly Trp Cys Gly Pro Phe
    50                  55                  60

Leu Glu Ala Ala Gln Met Thr Gln Met Gln Gly Val Leu Val His Gln
65                  70                  75                  80

Ala Glu Gly Glu Arg Gly Gln Ala Trp Arg Val Thr Arg Lys Ala His
                85                  90                  95

Ile Arg Trp Ala Phe Glu Gln Asn Phe Glu Gly Pro Leu Val Ile Ala
            100                 105                 110

Leu Ala Thr Arg Lys Arg Glu His Leu Ile Trp Gln Ala Pro Val Ser
        115                 120                 125

Leu Ser Pro Asp Val Leu Gln Leu Arg Val Gly Arg Asp Thr Leu Leu
    130                 135                 140

Val Arg Arg Pro Val Leu Asp Arg Tyr Arg Glu Ala Ala Thr Asp Glu
145                 150                 155                 160

Arg Leu Glu Ser Leu Phe Pro Arg Thr Ala Ala Asp Lys Arg Leu Tyr
                165                 170                 175

Arg Leu Gly Gln Gln Leu Glu Gln Ala Ser Met Gly Gly Phe Asp Gly
            180                 185                 190

Pro Arg Ala Trp Tyr Ala Asp Leu Pro Glu Asp Leu Val Arg Ala Tyr
        195                 200                 205

Glu Ala Leu Gly Pro Gly Glu Leu Trp Phe Ala Gln Tyr Val Leu Thr
    210                 215                 220

Gln Ala Leu Asp Glu Pro Pro Arg Phe Lys Phe Asp Arg Leu Asn Pro
225                 230                 235                 240

Pro Lys Asp

<210>  89
<211>  349
<212>  PRT
<213>  Thioalkalivibrio sp.

<220>
<221>  PEPTIDE
<222>  (1)..(349)
<223>  type IV CRISPR-associated protein Csf2 /protein_id=WP_013006553.1

<400> 89

Met Ile Arg Leu Glu Gly Thr Leu Tyr Thr Thr Ser Pro Leu His Ile
1               5                   10                  15

Thr Asp Ala Ser Gln Asp Arg Arg Ile Glu Ser Gly Asn Ser Thr Met
            20                  25                  30

Pro Val Thr Gly Thr Gln Thr Leu Thr Val Val Ser Arg Asp Pro Asp
        35                  40                  45

Ser Gly Glu Thr His Thr Arg Arg Val Pro Val Ile Pro Ser Asn Ser
    50                  55                  60

Ile Arg Gly Arg Ile Arg Arg Ala Gly Ala Asp Val Leu Met Glu Ala
65                  70                  75                  80

Leu Lys Ala Lys Gly Glu Asn Leu Thr Leu Pro Ala Tyr His Thr Val
                85                  90                  95

Cys Ala Gly Thr Ala Asp Gly Arg Pro Asp Lys Gly Ser Ser Val Ser
            100                 105                 110

Leu Ser Ala Leu His Gln Arg Arg Glu His Pro Phe Ile Gly Leu Phe
        115                 120                 125

Gly Gly Gly Pro Glu Met Val Glu Ser Arg Phe Leu Thr Asp Thr Ala
    130                 135                 140

Trp Ala Leu Val Pro Glu Thr Val Ala Ser Ile Asp Val Pro Ala Ala
145                 150                 155                 160

Cys Glu Asp Ala Val Val Asp Pro Met Gly Arg Glu Lys Arg Leu Pro
                165                 170                 175

Leu Thr Gln Val Ile Tyr Phe Arg Arg Met Asp Asp Ile Leu Gln Phe
            180                 185                 190

Ser Asp Pro Lys Ala Ala Glu Arg Ile Glu Asp Tyr Glu Thr Ala Ala
        195                 200                 205

Gln Glu Trp Leu Asp Asn Gln Arg Thr Arg Ser Asn Gly Asp Glu Glu
    210                 215                 220

Arg Gly Asp Arg Val Gln Gly Trp Asn Ala Thr Glu Ile Val Leu Ala
225                 230                 235                 240

Gly Met Pro Phe Ser Phe Arg Ile Gly Thr Thr Thr Ser Ser Ser Glu
                245                 250                 255

Val Gln Asp Gly Leu Leu Leu Ala Ala Leu Glu Arg Phe Val Glu Arg
            260                 265                 270

Phe Pro Asn Leu Gly Gly Trp Lys Val Lys Gly Phe Gly Arg Tyr Asp
        275                 280                 285

Leu Ser Ser Ile Arg Met Ser Cys Asp Gly Glu Asp Ala Gly Leu Val
    290                 295                 300

Phe Thr Arg Asp Gly Gly Gln Leu Arg Leu Asn Arg Asp Asn Pro Ala
305                 310                 315                 320

Val Ala Glu Arg Leu Asp Ala Trp Ala Ser Tyr Ala Asn Ser Asp Ala
                325                 330                 335

Leu Thr Ala Ala Glu Ile Gln Glu Trp Ala Lys Ala Ala
            340                 345

<210>  90
<211>  218
<212>  PRT
<213>  Thioalkalivibrio sp.

<220>
<221>  PEPTIDE
<222>  (1)..(218)
<223>  hypothetical protein/protein_id=WP_013006554.1

<400>  90

Met Ala Ser Glu Thr Met Glu Pro Leu Arg Ile Thr Gly Arg Met His
1               5                   10                  15

Gly Leu Met Ile Glu Pro Glu Arg Pro Ile Ala Leu Asp Gly Ile Leu
            20                  25                  30

Ala Trp Ala Ile Val Glu Arg Ala Arg Met Glu Gly Ala Glu Asp Pro
        35                  40                  45

Leu Glu Ala Gln Glu Ser Leu Pro Leu Ala Phe Asp Ala Asp Arg Ser
    50                  55                  60

Val Trp Lys Ala Ser Trp Ile Phe Arg Arg Tyr Glu Ser Pro Leu Gln
65                  70                  75                  80

Thr Arg Gln Gln Thr Lys Lys Thr Asp Val Pro Ala Leu Leu Asp Gly
                85                  90                  95

Arg Asn Ala Gly His Trp Glu Gly Arg Val Asp Ala Ile Ala Thr Gly
                100                 105                 110

Thr Gly Met Tyr Lys Gly Ala Val Leu Asp Arg Thr Tyr Arg Gln Ile
        115                 120                 125

Ser Glu Val Ser Ala Trp Cys Ile Gly Asn Arg Glu Arg Ile Glu Glu
    130                 135                 140

Leu Leu Gly Met Val Asn Gly Leu Gly Ser Leu Arg Arg Gln Gly Phe
145                 150                 155                 160

Gly Arg Ile Ser Gly Trp Ala Val Glu Ser Asp Pro Glu Ala Glu Asn
                165                 170                 175

Gln Trp Ser Gln Arg Ala Leu Pro Asn Asn Pro Gly Gly Phe Leu Arg
                180                 185                 190

Ser Thr Gly Ala Leu Ser Pro Pro Tyr Trp Arg Gln Asp Arg Val Gln
        195                 200                 205

Pro Ile Trp Thr Pro Met Thr Asp Gln Leu
    210                 215

<210>  91
<211>  1300
<212>  PRT
<213>  Francisella cf. novicida Fx1

<220>
<221>  PEPTIDE
<222>  (1)..(1300)
<223>  type V CRISPR-associated protein Cpf1: /protein_id=WP_014550095.1

<400>  91

Met Ser Ile Tyr Gln Glu Phe Val Asn Lys Tyr Ser Leu Ser Lys Thr
1               5                   10                  15

Leu Arg Phe Glu Leu Ile Pro Gln Gly Lys Thr Leu Glu Asn Ile Lys
                20                  25                  30

Ala Arg Gly Leu Ile Leu Asp Asp Glu Lys Arg Ala Lys Asp Tyr Lys
        35                  40                  45

Lys Ala Lys Gln Ile Ile Asp Lys Tyr His Gln Phe Phe Ile Glu Glu
    50                  55                  60

Ile Leu Ser Ser Val Cys Ile Ser Glu Asp Leu Leu Gln Asn Tyr Ser
65                  70                  75                  80

Asp Val Tyr Phe Lys Leu Lys Lys Ser Asp Asp Asp Asn Leu Gln Lys
                85                  90                  95

Asp Phe Lys Ser Ala Lys Asp Thr Ile Lys Lys Gln Ile Ser Glu Tyr
            100                 105                 110

Ile Lys Asp Ser Glu Lys Phe Lys Asn Leu Phe Asn Gln Asn Leu Ile
        115                 120                 125

Asp Ala Lys Lys Gly Gln Glu Ser Asp Leu Ile Leu Trp Leu Lys Gln
    130                 135                 140

Ser Lys Asp Asn Gly Ile Glu Leu Phe Lys Ala Asn Ser Asp Ile Thr
145                 150                 155                 160

Asp Ile Asp Glu Ala Leu Glu Ile Ile Lys Ser Phe Lys Gly Trp Thr
                165                 170                 175

Thr Tyr Phe Lys Gly Phe His Glu Asn Arg Lys Asn Val Tyr Ser Ser
            180                 185                 190

Asn Asp Ile Pro Thr Ser Ile Ile Tyr Arg Ile Val Asp Asp Asn Leu
        195                 200                 205

Pro Lys Phe Leu Glu Asn Lys Ala Lys Tyr Glu Ser Leu Lys Asp Lys
    210                 215                 220

Ala Pro Glu Ala Ile Asn Tyr Glu Gln Ile Lys Lys Asp Leu Ala Glu
225                 230                 235                 240

Glu Leu Thr Phe Asp Ile Asp Tyr Lys Thr Ser Glu Val Asn Gln Arg
                245                 250                 255

Val Phe Ser Leu Asp Glu Val Phe Glu Ile Ala Asn Phe Asn Asn Tyr
            260                 265                 270

Leu Asn Gln Ser Gly Ile Thr Lys Phe Asn Thr Ile Ile Gly Gly Lys
        275                 280                 285

Phe Val Asn Gly Glu Asn Thr Lys Arg Lys Gly Ile Asn Glu Tyr Ile
    290                 295                 300

Asn Leu Tyr Ser Gln Gln Ile Asn Asp Lys Thr Leu Lys Lys Tyr Lys
305                 310                 315                 320

Met Ser Val Leu Phe Lys Gln Ile Leu Ser Asp Thr Glu Ser Lys Ser
                325                 330                 335

Phe Val Ile Asp Lys Leu Glu Asp Asp Ser Asp Val Val Thr Thr Met
            340                 345                 350

Gln Ser Phe Tyr Glu Gln Ile Ala Ala Phe Lys Thr Val Glu Glu Lys
        355                 360                 365

Ser Ile Lys Glu Thr Leu Ser Leu Leu Phe Asp Asp Leu Lys Ala Gln
    370                 375                 380

Lys Leu Asp Leu Ser Lys Ile Tyr Phe Lys Asn Asp Lys Ser Leu Thr
385                 390                 395                 400

Asp Leu Ser Gln Gln Val Phe Asp Asp Tyr Ser Val Ile Gly Thr Ala
                405                 410                 415

Val Leu Glu Tyr Ile Thr Gln Gln Val Ala Pro Lys Asn Leu Asp Asn
            420                 425                 430

Pro Ser Lys Lys Glu Gln Asp Leu Ile Ala Lys Lys Thr Glu Lys Ala
        435                 440                 445

Lys Tyr Leu Ser Leu Glu Thr Ile Lys Leu Ala Leu Glu Glu Phe Asn
    450                 455                 460

Lys His Arg Asp Ile Asp Lys Gln Cys Arg Phe Glu Glu Ile Leu Ala
465                 470                 475                 480

Asn Phe Ala Ala Ile Pro Met Ile Phe Asp Glu Ile Ala Gln Asn Lys
                485                 490                 495

Asp Asn Leu Ala Gln Ile Ser Ile Lys Tyr Gln Asn Gln Gly Lys Lys
            500                 505                 510

Asp Leu Leu Gln Ala Ser Ala Glu Asp Asp Val Lys Ala Ile Lys Asp
        515                 520                 525

Leu Leu Asp Gln Thr Asn Asn Leu Leu His Arg Leu Lys Ile Phe His
    530                 535                 540

Ile Ser Gln Ser Glu Asp Lys Ala Asn Ile Leu Asp Lys Asp Glu His
545                 550                 555                 560

Phe Tyr Leu Val Phe Glu Glu Cys Tyr Phe Glu Leu Ala Asn Ile Val
                565                 570                 575

Pro Leu Tyr Asn Lys Ile Arg Asn Tyr Ile Thr Gln Lys Pro Tyr Ser
            580                 585                 590

Asp Glu Lys Phe Lys Leu Asn Phe Glu Asn Ser Thr Leu Ala Asn Gly
        595                 600                 605

Trp Asp Lys Asn Lys Glu Pro Asp Asn Thr Ala Ile Leu Phe Ile Lys
    610                 615                 620

Asp Asp Lys Tyr Tyr Leu Gly Val Met Asn Lys Lys Asn Asn Lys Ile
625                 630                 635                 640

Phe Asp Asp Lys Ala Ile Lys Glu Asn Lys Gly Glu Gly Tyr Lys Lys
                645                 650                 655

Ile Val Tyr Lys Leu Leu Pro Gly Ala Asn Lys Met Leu Pro Lys Val
            660                 665                 670

Phe Phe Ser Ala Lys Ser Ile Lys Phe Tyr Asn Pro Ser Glu Asp Ile
        675                 680                 685

Leu Arg Ile Arg Asn His Ser Thr His Thr Lys Asn Gly Asn Pro Gln
    690                 695                 700

Lys Gly Tyr Glu Lys Phe Glu Phe Asn Ile Glu Asp Cys Arg Lys Phe
705                 710                 715                 720

Ile Asp Phe Tyr Lys Glu Ser Ile Ser Lys His Pro Glu Trp Lys Asp
                725                 730                 735

Phe Gly Phe Arg Phe Ser Asp Thr Gln Arg Tyr Asn Ser Ile Asp Glu
            740                 745                 750

Phe Tyr Arg Glu Val Glu Asn Gln Gly Tyr Lys Leu Thr Phe Glu Asn
        755                 760                 765

Ile Ser Glu Ser Tyr Ile Asp Ser Val Val Asn Gln Gly Lys Leu Tyr
    770                 775                 780

Leu Phe Gln Ile Tyr Asn Lys Asp Phe Ser Ala Tyr Ser Lys Gly Arg
785                 790                 795                 800

Pro Asn Leu His Thr Leu Tyr Trp Lys Ala Leu Phe Asp Glu Arg Asn
                805                 810                 815

Leu Gln Asp Val Val Tyr Lys Leu Asn Gly Glu Ala Glu Leu Phe Tyr

            820                 825                 830

Arg Lys Lys Ser Ile Pro Lys Lys Ile Thr His Pro Ala Lys Glu Ala
        835                 840                 845

Ile Ala Asn Lys Asn Lys Asp Asn Pro Lys Lys Glu Ser Phe Phe Glu
    850                 855                 860

Tyr Asp Leu Ile Lys Asp Lys Arg Phe Thr Glu Asp Lys Phe Phe Phe
865                 870                 875                 880

His Cys Pro Ile Thr Ile Asn Phe Lys Ser Ser Gly Ala Asn Lys Phe
                885                 890                 895

Asn Asp Glu Ile Asn Leu Leu Leu Lys Glu Lys Ala Asn Asp Val His
            900                 905                 910

Ile Leu Ser Ile Asp Arg Gly Glu Arg His Leu Ala Tyr Tyr Thr Leu
        915                 920                 925

Val Asp Gly Lys Gly Asn Ile Ile Lys Gln Asp Thr Phe Asn Ile Ile
    930                 935                 940

Gly Asn Asp Arg Met Lys Thr Asn Tyr His Asp Lys Leu Ala Ala Ile
945                 950                 955                 960

Glu Lys Asp Arg Asp Ser Ala Arg Lys Asp Trp Lys Lys Ile Asn Asn
                965                 970                 975

Ile Lys Glu Met Lys Glu Gly Tyr Leu Ser Gln Val Val His Glu Ile
            980                 985                 990

Ala Lys Leu Val Ile Glu His Asn Ala Ile Val Val Phe Glu Asp Leu
        995                 1000                1005

Asn Phe Gly Phe Lys Arg Gly Arg Phe Lys Val Glu Lys Gln Val
    1010                1015                1020

Tyr Gln Lys Leu Glu Lys Met Leu Ile Glu Lys Leu Asn Tyr Leu
    1025                1030                1035

Val Phe Lys Asp Asn Glu Phe Asp Lys Thr Gly Gly Val Leu Arg
    1040                1045                1050

Ala Tyr Gln Leu Thr Ala Pro Phe Glu Thr Phe Lys Lys Met Gly
    1055                1060                1065

Lys Gln Thr Gly Ile Ile Tyr Tyr Val Pro Ala Gly Phe Thr Ser
    1070                1075                1080

Lys Ile Cys Pro Val Thr Gly Phe Val Asn Gln Leu Tyr Pro Lys
    1085                1090                1095

Tyr Glu Ser Val Ser Lys Ser Gln Glu Phe Phe Ser Lys Phe Asp
    1100                1105                1110

Lys Ile Cys Tyr Asn Leu Asp Lys Gly Tyr Phe Glu Phe Ser Phe
    1115                1120                1125

Asp Tyr Lys Asn Phe Gly Asp Lys Ala Ala Lys Gly Lys Trp Thr
    1130                1135                1140

Ile Ala Ser Phe Gly Ser Arg Leu Ile Asn Phe Arg Asn Ser Asp
    1145                1150                1155

Lys Asn His Asn Trp Asp Thr Arg Glu Val Tyr Pro Thr Lys Glu
    1160                1165                1170

Leu Glu Lys Leu Leu Lys Asp Tyr Ser Ile Glu Tyr Gly His Gly
    1175                1180                1185

Glu Cys Ile Lys Ala Ala Ile Cys Gly Glu Ser Asp Lys Lys Phe
    1190                1195                1200

Phe Ala Lys Leu Thr Ser Ile Leu Asn Thr Ile Leu Gln Met Arg
    1205                1210                1215

Asn Ser Lys Thr Gly Thr Glu Leu Asp Tyr Leu Ile Ser Pro Val
    1220                1225                1230

Ala Asp Val Asn Gly Asn Phe Phe Asp Ser Arg Gln Ala Pro Lys
    1235                1240                1245

Asn Met Pro Gln Asp Ala Asp Ala Asn Gly Ala Tyr His Ile Gly
    1250                1255                1260

Leu Lys Gly Leu Met Leu Leu Asp Arg Ile Lys Asn Asn Gln Glu
    1265                1270                1275

Gly Lys Lys Leu Asn Leu Val Ile Lys Asn Glu Glu Tyr Phe Glu
    1280                1285                1290

Phe Val Gln Asn Arg Asn Asn
    1295                1300

<210>  92
<211>  1129
<212>  PRT
<213>  Alicyclobacillus acidoterrestris

<220>
<221>  PEPTIDE
<222>  (1)..(1129)
<223>  hypothetical protein /protein_id=WP_021296342.1

<400>  92

Met Ala Val Lys Ser Ile Lys Val Lys Leu Arg Leu Asp Asp Met Pro
1               5                   10                  15

Glu Ile Arg Ala Gly Leu Trp Lys Leu His Lys Glu Val Asn Ala Gly
            20                  25                  30

Val Arg Tyr Tyr Thr Glu Trp Leu Ser Leu Leu Arg Gln Glu Asn Leu
        35                  40                  45

Tyr Arg Arg Ser Pro Asn Gly Asp Gly Glu Gln Glu Cys Asp Lys Thr
    50                  55                  60

Ala Glu Glu Cys Lys Ala Glu Leu Leu Glu Arg Leu Arg Ala Arg Gln
65                  70                  75                  80

Val Glu Asn Gly His Arg Gly Pro Ala Gly Ser Asp Asp Glu Leu Leu
                85                  90                  95

Gln Leu Ala Arg Gln Leu Tyr Glu Leu Leu Val Pro Gln Ala Ile Gly
            100                 105                 110

Ala Lys Gly Asp Ala Gln Gln Ile Ala Arg Lys Phe Leu Ser Pro Leu
        115                 120                 125

Ala Asp Lys Asp Ala Val Gly Gly Leu Gly Ile Ala Lys Ala Gly Asn
    130                 135                 140

Lys Pro Arg Trp Val Arg Met Arg Glu Ala Gly Glu Pro Gly Trp Glu
145                 150                 155                 160

Glu Glu Lys Glu Lys Ala Glu Thr Arg Lys Ser Ala Asp Arg Thr Ala
                165                 170                 175

Asp Val Leu Arg Ala Leu Ala Asp Phe Gly Leu Lys Pro Leu Met Arg
            180                 185                 190

Val Tyr Thr Asp Ser Glu Met Ser Ser Val Glu Trp Lys Pro Leu Arg

195                 200                 205

Lys Gly Gln Ala Val Arg Thr Trp Asp Arg Asp Met Phe Gln Gln Ala
    210                 215                 220

Ile Glu Arg Met Met Ser Trp Glu Ser Trp Asn Gln Arg Val Gly Gln
225                 230                 235                 240

Glu Tyr Ala Lys Leu Val Glu Gln Lys Asn Arg Phe Glu Gln Lys Asn
                245                 250                 255

Phe Val Gly Gln Glu His Leu Val His Leu Val Asn Gln Leu Gln Gln
            260                 265                 270

Asp Met Lys Glu Ala Ser Pro Gly Leu Glu Ser Lys Glu Gln Thr Ala
        275                 280                 285

His Tyr Val Thr Gly Arg Ala Leu Arg Gly Ser Asp Lys Val Phe Glu
    290                 295                 300

Lys Trp Gly Lys Leu Ala Pro Asp Ala Pro Phe Asp Leu Tyr Asp Ala
305                 310                 315                 320

Glu Ile Lys Asn Val Gln Arg Arg Asn Thr Arg Arg Phe Gly Ser His
                325                 330                 335

Asp Leu Phe Ala Lys Leu Ala Glu Pro Glu Tyr Gln Ala Leu Trp Arg
            340                 345                 350

Glu Asp Ala Ser Phe Leu Thr Arg Tyr Ala Val Tyr Asn Ser Ile Leu
        355                 360                 365

Arg Lys Leu Asn His Ala Lys Met Phe Ala Thr Phe Thr Leu Pro Asp
    370                 375                 380

Ala Thr Ala His Pro Ile Trp Thr Arg Phe Asp Lys Leu Gly Gly Asn
385                 390                 395                 400

Leu His Gln Tyr Thr Phe Leu Phe Asn Glu Phe Gly Glu Arg Arg His
                405                 410                 415

Ala Ile Arg Phe His Lys Leu Leu Lys Val Glu Asn Gly Val Ala Arg
            420                 425                 430

Glu Val Asp Asp Val Thr Val Pro Ile Ser Met Ser Glu Gln Leu Asp
        435                 440                 445

Asn Leu Leu Pro Arg Asp Pro Asn Glu Pro Ile Ala Leu Tyr Phe Arg
    450                 455                 460

Asp Tyr Gly Ala Glu Gln His Phe Thr Gly Glu Phe Gly Gly Ala Lys
465                 470                 475                 480

Ile Gln Cys Arg Arg Asp Gln Leu Ala His Met His Arg Arg Arg Gly
                485                 490                 495

Ala Arg Asp Val Tyr Leu Asn Val Ser Val Arg Val Gln Ser Gln Ser
            500                 505                 510

Glu Ala Arg Gly Glu Arg Arg Pro Pro Tyr Ala Ala Val Phe Arg Leu
        515                 520                 525

Val Gly Asp Asn His Arg Ala Phe Val His Phe Asp Lys Leu Ser Asp
    530                 535                 540

Tyr Leu Ala Glu His Pro Asp Asp Gly Lys Leu Gly Ser Glu Gly Leu
545                 550                 555                 560

Leu Ser Gly Leu Arg Val Met Ser Val Asp Leu Gly Leu Arg Thr Ser
                565                 570                 575

Ala Ser Ile Ser Val Phe Arg Val Ala Arg Lys Asp Glu Leu Lys Pro
            580                 585                 590

Asn Ser Lys Gly Arg Val Pro Phe Phe Phe Pro Ile Lys Gly Asn Asp
        595                 600                 605

Asn Leu Val Ala Val His Glu Arg Ser Gln Leu Leu Lys Leu Pro Gly
    610                 615                 620

Glu Thr Glu Ser Lys Asp Leu Arg Ala Ile Arg Glu Glu Arg Gln Arg
625                 630                 635                 640

Thr Leu Arg Gln Leu Arg Thr Gln Leu Ala Tyr Leu Arg Leu Leu Val
                645                 650                 655

Arg Cys Gly Ser Glu Asp Val Gly Arg Arg Glu Arg Ser Trp Ala Lys
            660                 665                 670

Leu Ile Glu Gln Pro Val Asp Ala Ala Asn His Met Thr Pro Asp Trp
        675                 680                 685

Arg Glu Ala Phe Glu Asn Glu Leu Gln Lys Leu Lys Ser Leu His Gly
    690                 695                 700

Ile Cys Ser Asp Lys Glu Trp Met Asp Ala Val Tyr Glu Ser Val Arg
705                 710                 715                 720

Arg Val Trp Arg His Met Gly Lys Gln Val Arg Asp Trp Arg Lys Asp
                725                 730                 735

Val Arg Ser Gly Glu Arg Pro Lys Ile Arg Gly Tyr Ala Lys Asp Val
            740                 745                 750

Val Gly Gly Asn Ser Ile Glu Gln Ile Glu Tyr Leu Glu Arg Gln Tyr
        755                 760                 765

Lys Phe Leu Lys Ser Trp Ser Phe Phe Gly Lys Val Ser Gly Gln Val
    770                 775                 780

Ile Arg Ala Glu Lys Gly Ser Arg Phe Ala Ile Thr Leu Arg Glu His
785                 790                 795                 800

Ile Asp His Ala Lys Glu Asp Arg Leu Lys Lys Leu Ala Asp Arg Ile
                805                 810                 815

Ile Met Glu Ala Leu Gly Tyr Val Tyr Ala Leu Asp Glu Arg Gly Lys
            820                 825                 830

Gly Lys Trp Val Ala Lys Tyr Pro Pro Cys Gln Leu Ile Leu Leu Glu
        835                 840                 845

Glu Leu Ser Glu Tyr Gln Phe Asn Asn Asp Arg Pro Pro Ser Glu Asn
    850                 855                 860

Asn Gln Leu Met Gln Trp Ser His Arg Gly Val Phe Gln Glu Leu Ile
865                 870                 875                 880

Asn Gln Ala Gln Val His Asp Leu Leu Val Gly Thr Met Tyr Ala Ala
                885                 890                 895

Phe Ser Ser Arg Phe Asp Ala Arg Thr Gly Ala Pro Gly Ile Arg Cys
            900                 905                 910

Arg Arg Val Pro Ala Arg Cys Thr Gln Glu His Asn Pro Glu Pro Phe
        915                 920                 925

Pro Trp Trp Leu Asn Lys Phe Val Val Glu His Thr Leu Asp Ala Cys
    930                 935                 940

Pro Leu Arg Ala Asp Asp Leu Ile Pro Thr Gly Glu Gly Glu Ile Phe
945                 950                 955                 960

Val Ser Pro Phe Ser Ala Glu Glu Gly Asp Phe His Gln Ile His Ala
                965                 970                 975

Asp Leu Asn Ala Ala Gln Asn Leu Gln Gln Arg Leu Trp Ser Asp Phe
            980                 985                 990

Asp Ile Ser Gln Ile Arg Leu Arg  Cys Asp Trp Gly Glu  Val Asp Gly
        995                 1000                1005

Glu Leu  Val Leu Ile Pro Arg  Leu Thr Gly Lys Arg  Thr Ala Asp
    1010                1015                1020

Ser Tyr  Ser Asn Lys Val Phe  Tyr Thr Asn Thr Gly  Val Thr Tyr
    1025                1030                1035

Tyr Glu  Arg Glu Arg Gly Lys  Lys Arg Arg Lys Val  Phe Ala Gln
    1040                1045                1050

Glu Lys  Leu Ser Glu Glu Glu  Ala Glu Leu Leu Val  Glu Ala Asp
    1055                1060                1065

Glu Ala  Arg Glu Lys Ser Val  Val Leu Met Arg Asp  Pro Ser Gly
    1070                1075                1080

Ile Ile  Asn Arg Gly Asn Trp  Thr Arg Gln Lys Glu  Phe Trp Ser
    1085                1090                1095

Met Val  Asn Gln Arg Ile Glu  Gly Tyr Leu Val Lys  Gln Ile Arg
    1100                1105                1110

Ser Arg  Val Pro Leu Gln Asp  Ser Ala Cys Glu Asn  Thr Gly Asp
    1115                1120                1125

Ile

<210>  93
<211>  545
<212>  PRT
<213>  Alicyclobacillus acidoterrestris

<220>
<221>  PEPTIDE
<222>  (1)..(545)
<223>  CRISPR-associated endonuclease Cas1:  /protein_id=WP_021296343.1

<400>  93

Met Arg Thr Phe Ile Ser Phe Asp His Val Gln Gly Leu Phe Glu Lys
1               5                   10                  15

Ser Ala Asp Thr Val Glu Gly Ser Gly Gln His Asp Arg Ala Glu Arg
            20                  25                  30

Arg Lys Arg Pro Ser Asp Val Gly His Asp Val Trp Asp Ile Ala Pro
        35                  40                  45

Gln Ser Leu His Leu Gly Asp Glu Asp Leu Gly Ile Val Gly Lys Leu
    50                  55                  60

Asp Ala Ile Arg Tyr Gln Asp Asp Gly Tyr Trp Glu Pro Val Glu Ala
65                  70                  75                  80

Lys His Ser Ser Ala Pro Asn Gly Gly Thr Ser Phe Arg Leu Gly Glu
                85                  90                  95

Trp Glu Leu Asp Gly Lys Ala Trp Pro Asn Asp Gln Ile Gln Leu Cys
            100                 105                 110

Ala Gln Gly Leu Leu Leu Arg Ala Asn Gly Phe Pro Thr Ile Ala Gly
        115                 120                 125

Lys Leu Phe Tyr Arg Gly Asn Arg Gln Thr Val Arg Ile Val Phe Ser
    130                 135                 140

Asp Asp Leu Ile Ala Ala Thr Val Ala Thr Ile Arg Arg Ala His Glu
145                 150                 155                 160

Leu Glu Gln Ala Ser Met Pro Ser Pro Leu Leu Asn Ser Asp Lys Cys
                165                 170                 175

Phe Arg Cys Ser Leu Asn Gly Val Cys Leu Pro Asp Glu Thr Ile Arg
            180                 185                 190

Leu Thr Ser Pro Asp Gly Glu Thr Val Pro Ile Arg Asp Ile Val Pro
        195                 200                 205

Ser Arg Asp Asp Leu Gly Thr Val Tyr Val Ser Glu Pro Gly Thr Arg
    210                 215                 220

Val Gly Lys Arg Gly Tyr Glu Leu Thr Ile Thr Ser Lys Asp Gly Met
225                 230                 235                 240

Glu Ser Thr Val Pro Leu Lys Asp Val Arg His Ile Ser Leu Phe Gly
                245                 250                 255

Asn Val Gln Ile Ser Thr Gln Leu Val His Asp Cys Met Glu Ser Gly
            260                 265                 270

Val Thr Ile Ser Tyr Leu Thr Ala Ala Gly Arg Leu Val Gly Met Asn
        275                 280                 285

His Asn Leu Ile Ser Lys Asn Val Leu Val Arg Arg Glu Gln Phe Lys
    290                 295                 300

Arg Phe Gly Asp Glu Arg Asp Arg Leu Thr Leu Ala Arg Tyr Ile Val
305                 310                 315                 320

Arg Ala Lys Ile Leu Asn Gln Arg Thr Leu Leu Arg Arg Asn Ala His
                325                 330                 335

Gly Leu Asp Lys Val Ile Leu Lys Asp Leu Leu Asp His Ala Arg Arg
            340                 345                 350

Ser Glu Glu Ala Thr Ser Ile Asp Ser Leu Leu Gly Ile Glu Gly Ile
        355                 360                 365

Ala Ala Lys Gln Tyr Met Gln Ala Phe Val Leu Met Leu Lys Val Lys
    370                 375                 380

Gly Val Pro Ala Gly Glu Thr Leu Met Asn Gly Arg Asn Arg Arg Pro
385                 390                 395                 400

Pro Lys Asp Pro Val Asn Ala Leu Leu Ser Leu Gly Tyr Ser Leu Leu
                405                 410                 415

Gln Arg Asp Met Tyr Ala Ala Leu Ala Ala Val Gly Leu Asp Pro Leu
            420                 425                 430

Met Gly Phe Tyr His Arg Ile Glu Thr Gly Arg Pro Ser Leu Val Leu
        435                 440                 445

Asp Met Met Glu Pro Phe Arg Ala Ile Ile Ala Asp Ser Val Val Val
    450                 455                 460

Arg Thr Leu Asn Thr Gly Glu Ile Ala Trp Asp Asp Phe Tyr Ile Gly
465                 470                 475                 480

Gln Glu Ser Cys Ser Leu Lys Leu Pro Gly Arg Lys Arg Phe Phe Gln
                485                 490                 495

Ala Phe Glu Arg Arg Met His Glu Thr Val Thr His Pro Val Phe Gly
            500                 505                 510

Tyr Lys Leu Ser Tyr Arg Arg Met Leu Glu Leu Glu Ala Arg Phe Leu
        515                 520                 525

Ser Arg Tyr Leu Met Gly Glu Leu Pro Glu Tyr Arg Pro Ile Val Thr
    530                 535                 540

Arg
545

<210>  94
<211>  97
<212>  PRT
<213>  Alicyclobacillus acidoterrestris

<220>
<221>  PEPTIDE
<222>  (1)..(97)
<223>  CRISPR-associated endonuclease Cas2:  /protein_id=WP_021296344.1

<400>  94

Met Arg Arg Tyr Val Leu Val Ser Tyr Asp Ile Ser Asp Gln Lys Arg
1               5                   10                  15

Trp Arg Arg Val Tyr Lys Ile Val Arg Gly Tyr Gly Met His Val Gln
            20                  25                  30

Tyr Ser Val Phe Leu Cys Gln Leu Thr Asp Gln Asp Glu Ala Glu Leu
        35                  40                  45

Lys Gln Leu Leu Leu Asp Val Ile His Gln Ser Met Asp Gln Val Ile
    50                  55                  60

Phe Ala Arg Ile Gly Thr Val Gln Ala Lys Ala Ala Glu Arg Asn Met
65                  70                  75                  80

Ser Cys Ile Gly Arg Asp Phe Val Pro Leu Asp Leu Lys Gly Leu Val
                85                  90                  95

Phe

<210>  95
<211>  1252
<212>  PRT
<213>  Oleiphilus sp

<220>
<221>  PEPTIDE

<222>  (1)..(1252)
<223>  hypothetical protein" /protein_id=KZX85786.1

<400>  95

Met Thr Lys Leu Arg His Arg Gln Lys Lys Leu Thr His Asp Trp Ala
1               5                   10                  15

Gly Ser Lys Lys Arg Glu Val Leu Gly Ser Asn Gly Lys Leu Gln Asn
            20                  25                  30

Pro Leu Leu Met Pro Val Lys Lys Gly Gln Val Thr Glu Phe Arg Lys
        35                  40                  45

Ala Phe Ser Ala Tyr Ala Arg Ala Thr Lys Gly Glu Met Thr Asp Gly
    50                  55                  60

Arg Lys Asn Met Phe Thr His Ser Phe Glu Pro Phe Lys Thr Lys Pro
65                  70                  75                  80

Ser Leu His Gln Cys Glu Leu Ala Asp Lys Ala Tyr Gln Ser Leu His
                85                  90                  95

Ser Tyr Leu Pro Gly Ser Leu Ala His Phe Leu Leu Ser Ala His Ala
            100                 105                 110

Leu Gly Phe Arg Ile Phe Ser Lys Ser Gly Glu Ala Thr Ala Phe Gln
        115                 120                 125

Ala Ser Ser Lys Ile Glu Ala Tyr Glu Ser Lys Leu Ala Ser Glu Leu
    130                 135                 140

Ala Cys Val Asp Leu Ser Ile Gln Asn Leu Thr Ile Ser Thr Leu Phe
145                 150                 155                 160

Asn Ala Leu Thr Thr Ser Val Arg Gly Lys Gly Glu Glu Thr Ser Ala
                165                 170                 175

Asp Pro Leu Ile Ala Arg Phe Tyr Thr Leu Leu Thr Gly Lys Pro Leu
            180                 185                 190

Ser Arg Asp Thr Gln Gly Pro Glu Arg Asp Leu Ala Glu Val Ile Ser
        195                 200                 205

Arg Lys Ile Ala Ser Ser Phe Gly Thr Trp Lys Glu Met Thr Ala Asn
    210                 215                 220

Pro Leu Gln Ser Leu Gln Phe Phe Glu Glu Glu Leu His Ala Leu Asp
225                 230                 235                 240

Ala Asn Val Ser Leu Ser Pro Ala Phe Asp Val Leu Ile Lys Met Asn
                245                 250                 255

Asp Leu Gln Gly Asp Leu Lys Asn Arg Thr Ile Val Phe Asp Pro Asp
            260                 265                 270

Ala Pro Val Phe Glu Tyr Asn Ala Glu Asp Pro Ala Asp Ile Ile Ile
        275                 280                 285

Lys Leu Thr Ala Arg Tyr Ala Lys Glu Ala Val Ile Lys Asn Gln Asn
    290                 295                 300

Val Gly Asn Tyr Val Lys Asn Ala Ile Thr Thr Thr Asn Ala Asn Gly
305                 310                 315                 320

Leu Gly Trp Leu Leu Asn Lys Gly Leu Ser Leu Leu Pro Val Ser Thr
                325                 330                 335

Asp Asp Glu Leu Leu Glu Phe Ile Gly Val Glu Arg Ser His Pro Ser
            340                 345                 350

Cys His Ala Leu Ile Glu Leu Ile Ala Gln Leu Glu Ala Pro Glu Leu
        355                 360                 365

Phe Glu Lys Asn Val Phe Ser Asp Thr Arg Ser Glu Val Gln Gly Met
    370                 375                 380

Ile Asp Ser Ala Val Ser Asn His Ile Ala Arg Leu Ser Ser Ser Arg
385                 390                 395                 400

Asn Ser Leu Ser Met Asp Ser Glu Glu Leu Glu Arg Leu Ile Lys Ser
                405                 410                 415

Phe Gln Ile His Thr Pro His Cys Ser Leu Phe Ile Gly Ala Gln Ser
            420                 425                 430

Leu Ser Gln Gln Leu Glu Ser Leu Pro Glu Ala Leu Gln Ser Gly Val
        435                 440                 445

Asn Ser Ala Asp Ile Leu Leu Gly Ser Thr Gln Tyr Met Leu Thr Asn
    450                 455                 460

Ser Leu Val Glu Glu Ser Ile Ala Thr Tyr Gln Arg Thr Leu Asn Arg
465                 470                 475                 480

Ile Asn Tyr Leu Ser Gly Val Ala Gly Gln Ile Asn Gly Ala Ile Lys

                485                 490                 495

Arg Lys Ala Ile Asp Gly Glu Lys Ile His Leu Pro Ala Ala Trp Ser
            500                 505                 510

Glu Leu Ile Ser Leu Pro Phe Ile Gly Gln Pro Val Ile Asp Val Glu
        515                 520                 525

Ser Asp Leu Ala His Leu Lys Asn Gln Tyr Gln Thr Leu Ser Asn Glu
    530                 535                 540

Phe Asp Thr Leu Ile Ser Ala Leu Gln Lys Asn Phe Asp Leu Asn Phe
545                 550                 555                 560

Asn Lys Ala Leu Leu Asn Arg Thr Gln His Phe Glu Ala Met Cys Arg
                565                 570                 575

Ser Thr Lys Lys Asn Ala Leu Ser Lys Pro Glu Ile Val Ser Tyr Arg
            580                 585                 590

Asp Leu Leu Ala Arg Leu Thr Ser Cys Leu Tyr Arg Gly Ser Leu Val
        595                 600                 605

Leu Arg Arg Ala Gly Ile Glu Val Leu Lys Lys His Lys Ile Phe Glu
    610                 615                 620

Ser Asn Ser Glu Leu Arg Glu His Val His Glu Arg Lys His Phe Val
625                 630                 635                 640

Phe Val Ser Pro Leu Asp Arg Lys Ala Lys Lys Leu Leu Arg Leu Thr
                645                 650                 655

Asp Ser Arg Pro Asp Leu Leu His Val Ile Asp Glu Ile Leu Gln His
            660                 665                 670

Asp Asn Leu Glu Asn Lys Asp Arg Glu Ser Leu Trp Leu Val Arg Ser
        675                 680                 685

Gly Tyr Leu Leu Ala Gly Leu Pro Asp Gln Leu Ser Ser Ser Phe Ile
    690                 695                 700

Asn Leu Pro Ile Ile Thr Gln Lys Gly Asp Arg Arg Leu Ile Asp Leu
705                 710                 715                 720

Ile Gln Tyr Asp Gln Ile Asn Arg Asp Ala Phe Val Met Leu Val Thr
                725                 730                 735

Ser Ala Phe Lys Ser Asn Leu Ser Gly Leu Gln Tyr Arg Ala Asn Lys
            740                 745                 750

Gln Ser Phe Val Val Thr Arg Thr Leu Ser Pro Tyr Leu Gly Ser Lys
        755                 760                 765

Leu Val Tyr Val Pro Lys Asp Lys Asp Trp Leu Val Pro Ser Gln Met
    770                 775                 780

Phe Glu Gly Arg Phe Ala Asp Ile Leu Gln Ser Asp Tyr Met Val Trp
785                 790                 795                 800

Lys Asp Ala Gly Arg Leu Cys Val Ile Asp Thr Ala Lys His Leu Ser
                805                 810                 815

Asn Ile Lys Lys Ser Val Phe Ser Ser Glu Glu Val Leu Ala Phe Leu
            820                 825                 830

Arg Glu Leu Pro His Arg Thr Phe Ile Gln Thr Glu Val Arg Gly Leu
        835                 840                 845

Gly Val Asn Val Asp Gly Ile Ala Phe Asn Asn Gly Asp Ile Pro Ser
    850                 855                 860

Leu Lys Thr Phe Ser Asn Cys Val Gln Val Lys Val Ser Arg Thr Asn
865                 870                 875                 880

Thr Ser Leu Val Gln Thr Leu Asn Arg Trp Phe Glu Gly Gly Lys Val
                885                 890                 895

Ser Pro Pro Ser Ile Gln Phe Glu Arg Ala Tyr Tyr Lys Lys Asp Asp
            900                 905                 910

Gln Ile His Glu Asp Ala Ala Lys Arg Lys Ile Arg Phe Gln Met Pro
        915                 920                 925

Ala Thr Glu Leu Val His Ala Ser Asp Asp Ala Gly Trp Thr Pro Ser
    930                 935                 940

Tyr Leu Leu Gly Ile Asp Pro Gly Glu Tyr Gly Met Gly Leu Ser Leu
945                 950                 955                 960

Val Ser Ile Asn Asn Gly Glu Val Leu Asp Ser Gly Phe Ile His Ile
                965                 970                 975

Asn Ser Leu Ile Asn Phe Ala Ser Lys Lys Ser Asn His Gln Thr Lys
            980                 985                 990

Val Val Pro Arg Gln Gln Tyr Lys  Ser Pro Tyr Ala Asn  Tyr Leu Glu
        995                 1000                1005

Gln Ser  Lys Asp Ser Ala Ala  Gly Asp Ile Ala His  Ile Leu Asp
    1010                1015                1020

Arg Leu  Ile Tyr Lys Leu Asn  Ala Leu Pro Val Phe  Glu Ala Leu
    1025                1030                1035

Ser Gly  Asn Ser Gln Ser Ala  Ala Asp Gln Val Trp  Thr Lys Val
    1040                1045                1050

Leu Ser  Phe Tyr Thr Trp Gly  Asp Asn Asp Ala Gln  Asn Ser Ile
    1055                1060                1065

Arg Lys  Gln His Trp Phe Gly  Ala Ser His Trp Asp  Ile Lys Gly
    1070                1075                1080

Met Leu  Arg Gln Pro Pro Thr  Glu Lys Lys Pro Lys  Pro Tyr Ile
    1085                1090                1095

Ala Phe  Pro Gly Ser Gln Val  Ser Ser Tyr Gly Asn  Ser Gln Arg
    1100                1105                1110

Cys Ser  Cys Cys Gly Arg Asn  Pro Ile Glu Gln Leu  Arg Glu Met
    1115                1120                1125

Ala Lys  Asp Thr Ser Ile Lys  Glu Leu Lys Ile Arg  Asn Ser Glu
    1130                1135                1140

Ile Gln  Leu Phe Asp Gly Thr  Ile Lys Leu Phe Asn  Pro Asp Pro
    1145                1150                1155

Ser Thr  Val Ile Glu Arg Arg  Arg His Asn Leu Gly  Pro Ser Arg
    1160                1165                1170

Ile Pro  Val Ala Asp Arg Thr  Phe Lys Asn Ile Ser  Pro Ser Ser
    1175                1180                1185

Leu Glu  Phe Lys Glu Leu Ile  Thr Ile Val Ser Arg  Ser Ile Arg
    1190                1195                1200

His Ser  Pro Glu Phe Ile Ala  Lys Lys Arg Gly Ile  Gly Ser Glu
    1205                1210                1215

Tyr Phe  Cys Ala Tyr Ser Asp  Cys Asn Ser Ser Leu  Asn Ser Glu
    1220                1225                1230

Ala Asn  Ala Ala Ala Asn Val  Ala Gln Lys Phe Gln  Lys Gln Leu
    1235                    1240                    1245

Phe Phe  Glu Leu
    1250

<210>  96
<211>  338
<212>  PRT
<213>  Oleiphilus sp

<220>
<221>  PEPTIDE
<222>  (1)..(338)
<223>  hypothetical protein /protein_id=KZX85787.1

<400>  96

Met Asn Asp Glu Asn Lys Thr Lys Ser His Pro Asn Ser Ile Trp Tyr
1               5                   10                  15

Trp Cys Ser Gly Glu Lys Leu Trp Tyr Leu Pro Tyr Ser Gly Thr Pro
            20                  25                  30

Lys Arg Thr Ser Lys Ser Val Trp Thr Val Ser Gly Ser Phe Gly Gln
        35                  40                  45

Leu Asp Ile Asp Leu Tyr Lys Thr Ser His Ile Leu Phe Tyr Gly Ala
    50                  55                  60

Asn Glu Thr Thr Gly Asp Leu Pro Leu Leu Phe Leu Ser Asp Cys Ala
65                  70                  75                  80

Lys His Ser Val Ser Ile Ser Ile His Arg Asn His Val Ser Glu Pro
                85                  90                  95

Phe Val Phe Tyr Arg Asn Lys Thr Thr Asp Arg Asp Asp Leu Val Thr
            100                 105                 110

Ala Gln Ile Leu His Arg Glu Asn Thr Lys Lys Ser Ala Tyr Ile Ala
        115                 120                 125

Arg Thr Ile Ile Arg Leu Gln Ile Lys Gln Arg Gly Tyr Val Ile Pro
    130                 135                 140

Ser Ser Phe Asp Asp Thr Lys Leu Leu Ser Ser Arg Asn Val Asp Glu
145                 150                 155                 160

Val Arg Thr Ile Glu Ala Thr Ala Ser Arg Glu Tyr Trp Arg Leu Tyr
                165                 170                 175

Phe Lys Lys Leu Gly Leu Glu Gly Leu Gly Arg Arg Asp Asp His Val
            180                 185                 190

Val Val Lys Ala Leu Asp Ala Thr Ser His Phe Leu Ser Gly Ile Met
        195                 200                 205

Leu Arg Trp Val Leu Ser His Glu Leu Ser Pro Ala His Gly Phe Ile
    210                 215                 220

His Lys Ser Ser Ser Tyr Pro Ser Leu Val Tyr Asp Leu Ile Glu Pro
225                 230                 235                 240

Leu Arg Trp Ile Pro Glu Leu Ala Val Phe Gln Ser Tyr Leu Glu Phe
                245                 250                 255

Gly Glu His Arg Leu Leu Glu Arg Ser Ile Glu Arg Phe Lys Gln Met
            260                 265                 270

Leu Asp Glu Pro Ile His Ser Glu Pro Thr Arg Gln Leu Val Tyr Arg
        275                 280                 285

Arg Thr Leu Ile His Gly Gln Val Ile Ala Leu Arg His Tyr Leu Asn
    290                 295                 300

Gly Asn Met Gln Arg Tyr Leu Pro Pro Val Glu Glu Leu Ala Lys Glu
305                 310                 315                 320

Arg Gly Arg Lys Arg Ala Val Ser Tyr Thr Leu Pro Gly Gln Ile Trp
                325                 330                 335

Lys Lys

<210>  97
<211>  347
<212>  PRT
<213>  Bacterium CG09_39_24

<220>
<221>  PEPTIDE
<222>  (1)..(347)
<223>  CRISPR-associated endonuclease Cas1 /protein_id=OJI08768.1

<400>  97

Met Arg Arg Tyr Phe Ala Lys Arg Lys Gln Phe Ile Trp Leu Pro Tyr
1               5                   10                  15

Val Lys Ser Ile Asp Leu Ser Gly Lys Ser Val Lys Phe Ser Tyr Lys
            20                  25                  30

Gly Gly Glu Leu Asp Leu Ser Phe Pro Glu Ile His Ser Ile Met Phe
        35                  40                  45

Tyr Gly Ser Val Cys Pro Leu Glu Gln Glu Phe Leu Glu Lys Cys Ser
    50                  55                  60

Ala Trp Gly Ile Pro Leu Thr Ile His Arg Arg Asn Met Pro Arg Gly
65                  70                  75                  80

Val Trp Ile Val Pro Ser Thr Thr Ser Asn Arg Glu Asp Leu Leu Thr
                85                  90                  95

Lys Gln Ile Leu Phe Arg Gln Asn Glu Lys Lys Arg Ala Tyr Ile Thr
            100                 105                 110

Lys Arg Leu Ile Tyr Ala Lys Phe Lys Ser Met Glu Trp Leu Ile Pro
        115                 120                 125

Ala Pro Thr Gln Glu Leu Gln Gly Leu Ser Ser Val Glu Ser Ile Val
    130                 135                 140

Gly Val Glu Ser Trp His Ala Lys Arg Tyr Trp Glu His Phe Tyr Lys
145                 150                 155                 160

Ser Leu Gly Tyr Pro Lys Asp Arg Arg Arg Ser Lys Gly Asn Phe Ile
                165                 170                 175

Cys Asn Val Leu Asp Ala Ser Ser Lys Phe Val Ser Gly Ile Ile Leu
            180                 185                 190

Arg Trp Val Val Tyr His His Phe Ser Pro Phe His Gly Tyr Thr His
        195                 200                 205

Thr Pro Ser Asp Tyr Pro Ser Leu Ile Tyr Asp Leu Met Glu Pro Tyr
    210                 215                 220

Arg Gly Phe Ile Asp Gly Val Ala Phe Asn Thr Ile Lys Glu Ile Gln
225                 230                 235                 240

Glu Asn Gly Gly Asp Glu Ser Lys Thr Leu Pro Tyr Thr Met Glu Asn
                245                 250                 255

Leu Lys Asp Phe Met Asp Thr Glu Ile Tyr Val Ala Ala Thr Arg Gln

            260                 265                 270

Thr Ala Thr Phe Gln Glu Leu Tyr His Gly Leu Ile Leu Gly Leu Arg
        275                 280                 285

Ser Tyr Leu Leu Gly Asn Lys Arg Phe Ile Val Pro Phe Pro Gly Lys
    290                 295                 300

Pro Ser Gly Gly Arg Pro Val Lys Ala Gly Tyr Lys Leu Tyr Gly Arg
305                 310                 315                 320

Phe Ala Gly Lys Thr Asp Phe Trp Pro Glu Ser Arg Asp Ile Ala Lys
                325                 330                 335

Thr Phe Gln Gln Lys Phe Ser Ile Arg Leu Glu
            340                 345

<210> 98
<211> 1125
<212> PRT
<213> Bacterium CG09_39_24

<220>
<221> PEPTIDE
<222> (1)..(1125)
<223> hypothetical protein: /protein_id=OJI08769.1

<400> 98

Met Arg Lys Lys Leu Phe Lys Gly Tyr Ile Leu His Asn Lys Arg Leu
1               5                   10                  15

Val Tyr Thr Gly Lys Ala Ala Ile Arg Ser Ile Lys Tyr Pro Leu Val
            20                  25                  30

Ala Pro Asn Lys Thr Ala Leu Asn Asn Leu Ser Glu Lys Ile Ile Tyr
        35                  40                  45

Asp Tyr Glu His Leu Phe Gly Pro Leu Asn Val Ala Ser Tyr Ala Arg
    50                  55                  60

Asn Ser Asn Arg Tyr Ser Leu Val Asp Phe Trp Ile Asp Ser Leu Arg
65                  70                  75                  80

Ala Gly Val Ile Trp Gln Ser Lys Ser Thr Ser Leu Ile Asp Leu Ile
                85                  90                  95

Ser Lys Leu Glu Gly Ser Lys Ser Pro Ser Glu Lys Ile Phe Glu Gln
            100                 105                 110

Ile Asp Phe Glu Leu Lys Asn Lys Leu Asp Lys Glu Gln Phe Lys Asp
        115                 120                 125

Ile Ile Leu Leu Asn Thr Gly Ile Arg Ser Ser Ser Asn Val Arg Ser
    130                 135                 140

Leu Arg Gly Arg Phe Leu Lys Cys Phe Lys Glu Glu Phe Arg Asp Thr
145                 150                 155                 160

Glu Glu Val Ile Ala Cys Val Asp Lys Trp Ser Lys Asp Leu Ile Val
                165                 170                 175

Glu Gly Lys Ser Ile Leu Val Ser Lys Gln Phe Leu Tyr Trp Glu Glu
            180                 185                 190

Glu Phe Gly Ile Lys Ile Phe Pro His Phe Lys Asp Asn His Asp Leu
        195                 200                 205

Pro Lys Leu Thr Phe Phe Val Glu Pro Ser Leu Glu Phe Ser Pro His
    210                 215                 220

Leu Pro Leu Ala Asn Cys Leu Glu Arg Leu Lys Lys Phe Asp Ile Ser
225                 230                 235                 240

Arg Glu Ser Leu Leu Gly Leu Asp Asn Asn Phe Ser Ala Phe Ser Asn
                245                 250                 255

Tyr Phe Asn Glu Leu Phe Asn Leu Leu Ser Arg Gly Glu Ile Lys Lys
            260                 265                 270

Ile Val Thr Ala Val Leu Ala Val Ser Lys Ser Trp Glu Asn Glu Pro
        275                 280                 285

Glu Leu Glu Lys Arg Leu His Phe Leu Ser Glu Lys Ala Lys Leu Leu
    290                 295                 300

Gly Tyr Pro Lys Leu Thr Ser Ser Trp Ala Asp Tyr Arg Met Ile Ile
305                 310                 315                 320

Gly Gly Lys Ile Lys Ser Trp His Ser Asn Tyr Thr Glu Gln Leu Ile
                325                 330                 335

Lys Val Arg Glu Asp Leu Lys Lys His Gln Ile Ala Leu Asp Lys Leu
            340                 345                 350

Gln Glu Asp Leu Lys Lys Val Val Asp Ser Ser Leu Arg Glu Gln Ile
        355                 360                 365

Glu Ala Gln Arg Glu Ala Leu Leu Pro Leu Leu Asp Thr Met Leu Lys
    370                 375                 380

Glu Lys Asp Phe Ser Asp Asp Leu Glu Leu Tyr Arg Phe Ile Leu Ser
385                 390                 395                 400

Asp Phe Lys Ser Leu Leu Asn Gly Ser Tyr Gln Arg Tyr Ile Gln Thr
                405                 410                 415

Glu Glu Glu Arg Lys Glu Asp Arg Asp Val Thr Lys Lys Tyr Lys Asp
            420                 425                 430

Leu Tyr Ser Asn Leu Arg Asn Ile Pro Arg Phe Phe Gly Glu Ser Lys
        435                 440                 445

Lys Glu Gln Phe Asn Lys Phe Ile Asn Lys Ser Leu Pro Thr Ile Asp
    450                 455                 460

Val Gly Leu Lys Ile Leu Glu Asp Ile Arg Asn Ala Leu Glu Thr Val
465                 470                 475                 480

Ser Val Arg Lys Pro Pro Ser Ile Thr Glu Glu Tyr Val Thr Lys Gln
                485                 490                 495

Leu Glu Lys Leu Ser Arg Lys Tyr Lys Ile Asn Ala Phe Asn Ser Asn
            500                 505                 510

Arg Phe Lys Gln Ile Thr Glu Gln Val Leu Arg Lys Tyr Asn Asn Gly
        515                 520                 525

Glu Leu Pro Lys Ile Ser Glu Val Phe Tyr Arg Tyr Pro Arg Glu Ser
    530                 535                 540

His Val Ala Ile Arg Ile Leu Pro Val Lys Ile Ser Asn Pro Arg Lys
545                 550                 555                 560

Asp Ile Ser Tyr Leu Leu Asp Lys Tyr Gln Ile Ser Pro Asp Trp Lys
                565                 570                 575

Asn Ser Asn Pro Gly Glu Val Val Asp Leu Ile Glu Ile Tyr Lys Leu
            580                 585                 590

Thr Leu Gly Trp Leu Leu Ser Cys Asn Lys Asp Phe Ser Met Asp Phe
        595                 600                 605

Ser Ser Tyr Asp Leu Lys Leu Phe Pro Glu Ala Ala Ser Leu Ile Lys

610                 615                 620

Asn Phe Gly Ser Cys Leu Ser Gly Tyr Tyr Leu Ser Lys Met Ile Phe
625                 630                 635                 640

Asn Cys Ile Thr Ser Glu Ile Lys Gly Met Ile Thr Leu Tyr Thr Arg
                645                 650                 655

Asp Lys Phe Val Val Arg Tyr Val Thr Gln Met Ile Gly Ser Asn Gln
            660                 665                 670

Lys Phe Pro Leu Leu Cys Leu Val Gly Glu Lys Gln Thr Lys Asn Phe
        675                 680                 685

Ser Arg Asn Trp Gly Val Leu Ile Glu Glu Lys Gly Asp Leu Gly Glu
    690                 695                 700

Glu Lys Asn Gln Glu Lys Cys Leu Ile Phe Lys Asp Lys Thr Asp Phe
705                 710                 715                 720

Ala Lys Ala Lys Glu Val Glu Ile Phe Lys Asn Asn Ile Trp Arg Ile
                725                 730                 735

Arg Thr Ser Lys Tyr Gln Ile Gln Phe Leu Asn Arg Leu Phe Lys Lys
            740                 745                 750

Thr Lys Glu Trp Asp Leu Met Asn Leu Val Leu Ser Glu Pro Ser Leu
        755                 760                 765

Val Leu Glu Glu Glu Trp Gly Val Ser Trp Asp Lys Asp Lys Leu Leu
    770                 775                 780

Pro Leu Leu Lys Lys Glu Lys Ser Cys Glu Glu Arg Leu Tyr Tyr Ser
785                 790                 795                 800

Leu Pro Leu Asn Leu Val Pro Ala Thr Asp Tyr Lys Glu Gln Ser Ala
                805                 810                 815

Glu Ile Glu Gln Arg Asn Thr Tyr Leu Gly Leu Asp Val Gly Glu Phe
            820                 825                 830

Gly Val Ala Tyr Ala Val Val Arg Ile Val Arg Asp Arg Ile Glu Leu
        835                 840                 845

Leu Ser Trp Gly Phe Leu Lys Asp Pro Ala Leu Arg Lys Ile Arg Glu
    850                 855                 860

Arg Val Gln Asp Met Lys Lys Lys Gln Val Met Ala Val Phe Ser Ser
865                 870                 875                 880

Ser Ser Thr Ala Val Ala Arg Val Arg Glu Met Ala Ile His Ser Leu
                885                 890                 895

Arg Asn Gln Ile His Ser Ile Ala Leu Ala Tyr Lys Ala Lys Ile Ile
            900                 905                 910

Tyr Glu Ile Ser Ile Ser Asn Phe Glu Thr Gly Gly Asn Arg Met Ala
        915                 920                 925

Lys Ile Tyr Arg Ser Ile Lys Val Ser Asp Val Tyr Arg Glu Ser Gly
    930                 935                 940

Ala Asp Thr Leu Val Ser Glu Met Ile Trp Gly Lys Lys Asn Lys Gln
945                 950                 955                 960

Met Gly Asn His Ile Ser Ser Tyr Ala Thr Ser Tyr Thr Cys Cys Asn
                965                 970                 975

Cys Ala Arg Thr Pro Phe Glu Leu Val Ile Asp Asn Asp Lys Glu Tyr
            980                 985                 990

Glu Lys Gly Gly Asp Glu Phe Ile  Phe Asn Val Gly Asp  Glu Lys Lys
        995                 1000                 1005

Val Arg  Gly Phe Leu Gln Lys  Ser Leu Leu Gly Lys  Thr Ile Lys
    1010                 1015                 1020

Gly Lys  Glu Val Leu Lys Ser  Ile Lys Glu Tyr Ala  Arg Pro Pro
    1025                 1030                 1035

Ile Arg  Glu Val Leu Leu Glu  Gly Glu Asp Val Glu  Gln Leu Leu
    1040                 1045                 1050

Lys Arg  Arg Gly Asn Ser Tyr  Ile Tyr Arg Cys Pro  Phe Cys Gly
    1055                 1060                 1065

Tyr Lys  Thr Asp Ala Asp Ile  Gln Ala Ala Leu Asn  Ile Ala Cys
    1070                 1075                 1080

Arg Gly  Tyr Ile Ser Asp Asn  Ala Lys Asp Ala Val  Lys Glu Gly
    1085                 1090                 1095

Glu Arg  Lys Leu Asp Tyr Ile  Leu Glu Val Arg Lys  Leu Trp Glu
    1100                 1105                 1110

Lys Asn Gly Ala Val Leu Arg Ser Ala Lys Phe Leu
    1115                1120                1125

<210>  99
<211>  986
<212>  PRT
<213>  Deltaproteobacteria bacterium

<220>
<221>  PEPTIDE
<222>  (1)..(986)
<223>  hypothetical protein  /protein_id=OGP07438.1

<400>  99

Met Glu Lys Arg Ile Asn Lys Ile Arg Lys Lys Leu Ser Ala Asp Asn
1               5                   10                  15

Ala Thr Lys Pro Val Ser Arg Ser Gly Pro Met Lys Thr Leu Leu Val
            20                  25                  30

Arg Val Met Thr Asp Asp Leu Lys Lys Arg Leu Glu Lys Arg Arg Lys
        35                  40                  45

Lys Pro Glu Val Met Pro Gln Val Ile Ser Asn Asn Ala Ala Asn Asn
    50                  55                  60

Leu Arg Met Leu Leu Asp Asp Tyr Thr Lys Met Lys Glu Ala Ile Leu
65                  70                  75                  80

Gln Val Tyr Trp Gln Glu Phe Lys Asp Asp His Val Gly Leu Met Cys
                85                  90                  95

Lys Phe Ala Gln Pro Ala Ser Lys Lys Ile Asp Gln Asn Lys Leu Lys
            100                 105                 110

Pro Glu Met Asp Glu Lys Gly Asn Leu Thr Thr Ala Gly Phe Ala Cys
        115                 120                 125

Ser Gln Cys Gly Gln Pro Leu Phe Val Tyr Lys Leu Glu Gln Val Ser
    130                 135                 140

Glu Lys Gly Lys Ala Tyr Thr Asn Tyr Phe Gly Arg Cys Asn Val Ala
145                 150                 155                 160

Glu His Glu Lys Leu Ile Leu Leu Ala Gln Leu Lys Pro Glu Lys Asp
                165                 170                 175

Ser Asp Glu Ala Val Thr Tyr Ser Leu Gly Lys Phe Gly Gln Arg Ala

180                 185                 190

Leu Asp Phe Tyr Ser Ile His Val Thr Lys Glu Ser Thr His Pro Val
        195                 200                 205

Lys Pro Leu Ala Gln Ile Ala Gly Asn Arg Tyr Ala Ser Gly Pro Val
    210                 215                 220

Gly Lys Ala Leu Ser Asp Ala Cys Met Gly Thr Ile Ala Ser Phe Leu
225                 230                 235                 240

Ser Lys Tyr Gln Asp Ile Ile Ile Glu His Gln Lys Val Val Lys Gly
                245                 250                 255

Asn Gln Lys Arg Leu Glu Ser Leu Arg Glu Leu Ala Gly Lys Glu Asn
            260                 265                 270

Leu Glu Tyr Pro Ser Val Thr Leu Pro Pro Gln Pro His Thr Lys Glu
        275                 280                 285

Gly Val Asp Ala Tyr Asn Glu Val Ile Ala Arg Val Arg Met Trp Val
    290                 295                 300

Asn Leu Asn Leu Trp Gln Lys Leu Lys Leu Ser Arg Asp Asp Ala Lys
305                 310                 315                 320

Pro Leu Leu Arg Leu Lys Gly Phe Pro Ser Phe Pro Val Val Glu Arg
                325                 330                 335

Arg Glu Asn Glu Val Asp Trp Trp Asn Thr Ile Asn Glu Val Lys Lys
            340                 345                 350

Leu Ile Asp Ala Lys Arg Asp Met Gly Arg Val Phe Trp Ser Gly Val
        355                 360                 365

Thr Ala Glu Lys Arg Asn Thr Ile Leu Glu Gly Tyr Asn Tyr Leu Pro
    370                 375                 380

Asn Glu Asn Asp His Lys Lys Arg Glu Gly Ser Leu Glu Asn Pro Lys
385                 390                 395                 400

Lys Pro Ala Lys Arg Gln Phe Gly Asp Leu Leu Leu Tyr Leu Glu Lys
                405                 410                 415

Lys Tyr Ala Gly Asp Trp Gly Lys Val Phe Asp Glu Ala Trp Glu Arg
            420                 425                 430

Ile Asp Lys Lys Ile Ala Gly Leu Thr Ser His Ile Glu Arg Glu Glu
        435                 440                 445

Ala Arg Asn Ala Glu Asp Ala Gln Ser Lys Ala Val Leu Thr Asp Trp
    450                 455                 460

Leu Arg Ala Lys Ala Ser Phe Val Leu Glu Arg Leu Lys Glu Met Asp
465                 470                 475                 480

Glu Lys Glu Phe Tyr Ala Cys Glu Ile Gln Leu Gln Lys Trp Tyr Gly
                485                 490                 495

Asp Leu Arg Gly Asn Pro Phe Ala Val Glu Ala Glu Asn Arg Val Val
            500                 505                 510

Asp Ile Ser Gly Phe Ser Ile Gly Ser Asp Gly His Ser Ile Gln Tyr
        515                 520                 525

Arg Asn Leu Leu Ala Trp Lys Tyr Leu Glu Asn Gly Lys Arg Glu Phe
    530                 535                 540

Tyr Leu Leu Met Asn Tyr Gly Lys Lys Gly Arg Ile Arg Phe Thr Asp
545                 550                 555                 560

Gly Thr Asp Ile Lys Lys Ser Gly Lys Trp Gln Gly Leu Leu Tyr Gly
                565                 570                 575

Gly Gly Lys Ala Lys Val Ile Asp Leu Thr Phe Asp Pro Asp Asp Glu
            580                 585                 590

Gln Leu Ile Ile Leu Pro Leu Ala Phe Gly Thr Arg Gln Gly Arg Glu
        595                 600                 605

Phe Ile Trp Asn Asp Leu Leu Ser Leu Glu Thr Gly Leu Ile Lys Leu
    610                 615                 620

Ala Asn Gly Arg Val Ile Glu Lys Thr Ile Tyr Asn Lys Lys Ile Gly
625                 630                 635                 640

Arg Asp Glu Pro Ala Leu Phe Val Ala Leu Thr Phe Glu Arg Arg Glu
                645                 650                 655

Val Val Asp Pro Ser Asn Ile Lys Pro Val Asn Leu Ile Gly Val Asp
            660                 665                 670

Arg Gly Glu Asn Ile Pro Ala Val Ile Ala Leu Thr Asp Pro Glu Gly
        675                 680                 685

Cys Pro Leu Pro Glu Phe Lys Asp Ser Ser Gly Gly Pro Thr Asp Ile
    690                 695                 700

Leu Arg Ile Gly Glu Gly Tyr Lys Glu Lys Gln Arg Ala Ile Gln Ala
705                 710                 715                 720

Ala Lys Glu Val Glu Gln Arg Arg Ala Gly Gly Tyr Ser Arg Lys Phe
                725                 730                 735

Ala Ser Lys Ser Arg Asn Leu Ala Asp Asp Met Val Arg Asn Ser Ala
            740                 745                 750

Arg Asp Leu Phe Tyr His Ala Val Thr His Asp Ala Val Leu Val Phe
        755                 760                 765

Glu Asn Leu Ser Arg Gly Phe Gly Arg Gln Gly Lys Arg Thr Phe Met
    770                 775                 780

Thr Glu Arg Gln Tyr Thr Lys Met Glu Asp Trp Leu Thr Ala Lys Leu
785                 790                 795                 800

Ala Tyr Glu Gly Leu Thr Ser Lys Thr Tyr Leu Ser Lys Thr Leu Ala
                805                 810                 815

Gln Tyr Thr Ser Lys Thr Cys Ser Asn Cys Gly Phe Thr Ile Thr Thr
            820                 825                 830

Ala Asp Tyr Asp Gly Met Leu Val Arg Leu Lys Lys Thr Ser Asp Gly
        835                 840                 845

Trp Ala Thr Thr Leu Asn Asn Lys Glu Leu Lys Ala Glu Gly Gln Ile
    850                 855                 860

Thr Tyr Tyr Asn Arg Tyr Lys Arg Gln Thr Val Glu Lys Glu Leu Ser
865                 870                 875                 880

Ala Glu Leu Asp Arg Leu Ser Glu Glu Ser Gly Asn Asn Asp Ile Ser
                885                 890                 895

Lys Trp Thr Lys Gly Arg Arg Asp Glu Ala Leu Phe Leu Leu Lys Lys
            900                 905                 910

Arg Phe Ser His Arg Pro Val Gln Glu Gln Phe Val Cys Leu Asp Cys
        915                 920                 925

Gly His Glu Val His Ala Asp Glu Gln Ala Ala Leu Asn Ile Ala Arg
    930                 935                 940

Ser Trp Leu Phe Leu Asn Ser Asn Ser Thr Glu Phe Lys Ser Tyr Lys
945                 950                 955                 960

Ser Gly Lys Gln Pro Phe Val Gly Ala Trp Gln Ala Phe Tyr Lys Arg
                965                 970                 975

Arg Leu Lys Glu Val Trp Lys Pro Asn Ala
            980                 985

<210>  100
<211>  194
<212>  PRT
<213>  Deltaproteobacteria bacterium

<220>
<221>  PEPTIDE
<222>  (1)..(194)
<223>  CRISPR-associated protein Cas4:  /protein_id=OGP07439.1

<400>  100

Met Glu Ala Gln Arg Leu Ile Leu Pro Ile Ser Thr Val Met Glu Ser
1               5                   10                  15

Ile Tyr Cys Pro Arg Asn Ala Trp Tyr Ala Phe Val Gly Glu Arg Arg
            20                  25                  30

Asn Met Ala Lys Ser Val His Phe Thr Glu Ala Val His Ala His Arg
        35                  40                  45

Ala Val Asp Glu Ser Thr Gln Arg Ile Arg Thr Asp Cys Lys Gln Ile
    50                  55                  60

Thr Gly Met Tyr Ile Tyr Ser Asn Lys Leu Gly Leu Thr Gly Arg Ala
65                  70                  75                  80

Asp Thr Val Glu Trp Leu Tyr Gly Ile Pro Ile Pro Val Glu Thr Lys
                85                  90                  95

Thr Gly Ala Ile Arg Asp Phe Glu Asn Phe Arg Val Gln Ile Ala Leu
            100                 105                 110

Gln Ala Leu Cys Leu Glu Glu Met Phe Asn Val Asn Ile Pro Tyr Gly
        115                 120                 125

Glu Ile Phe Phe Cys Glu Thr Met Arg Arg His Glu Ile Ala Val Asp
    130                 135                 140

Glu Asp Leu Arg Thr His Ser Thr Ala Ile Val Val Glu Leu Arg Glu
145                 150                 155                 160

Arg Phe Leu Ser Phe Asp Ile Asn Arg Phe Gln Arg Val Asn Asp His
                165                 170                 175

Arg Cys Pro Lys Cys Gln Tyr Leu Glu Ser Cys Leu Pro Pro Ser Leu
            180                 185                 190

Glu Leu

<210>  101
<211>  328
<212>  PRT
<213>  Deltaproteobacteria bacterium

<220>
<221>  PEPTIDE
<222>  (1)..(328)
<223>  CRISPR-associated endonuclease Cas1:  /protein_id=OGP07440.1

<400>  101

Met Thr Ala Ile Thr Asp Arg Ile Thr Leu Tyr Ile Thr Ala Asp Glu
1               5                   10                  15

Ser Ser Ile Ser Arg Arg Gly Asp Ala Phe Leu Ile Gln Lys Ala Gly
            20                  25                  30

Glu Glu Lys Gly Gln Lys Ile Pro Ala Met Lys Val Lys Asp Ile Val
        35                  40                  45

Val Val Gly His Val Thr Leu Asp Ser Arg Leu Ile Gly Leu Cys Arg
    50                  55                  60

Glu Glu Ser Ile Pro Ile His Phe Leu Ser Gly Arg Trp Glu Tyr Gln
65                  70                  75                  80

Gly Ser Leu Gln Phe Glu Pro Val Lys Asn Leu Phe Ile Arg Arg Ala
                85                  90                  95

Gln Ile Lys Lys His Phe Asp Pro Glu Lys Lys Leu Asp Ile Ser Lys
            100                 105                 110

Lys Ile Val Gly Gly Lys Ile Arg Asn Gln Gln Ala Met Leu Asp Lys
        115                 120                 125

Tyr Arg Lys Asn Leu Lys Leu Ala Cys Pro Gln Ile Asp Ser Val Gly
    130                 135                 140

Asp Met Glu Thr Leu Arg Gly Ile Glu Gly Val Val Ala Lys Glu Tyr
145                 150                 155                 160

Tyr Gly Phe Tyr Pro Ala Ile Ile Lys Asn Ser Glu Phe Thr Phe Thr
                165                 170                 175

Arg Arg Thr Lys Arg Pro Pro Glu Asp Glu Ile Asn Ala Leu Leu Ser
            180                 185                 190

Leu Leu Tyr Thr Leu Ile Phe Asn Glu Ile His Ser Thr Ala Leu Leu
        195                 200                 205

Val Gly Leu Asp Pro Ala Phe Gly Tyr Leu His Asp Val Tyr Tyr Gly
    210                 215                 220

Arg Pro Ser Leu Ile Cys Asp Leu Leu Glu Glu Trp Arg Pro Leu Ala
225                 230                 235                 240

Asp Arg Phe Val Leu Asn Met Ile Asn Arg Lys Glu Val Thr Pro Glu
                245                 250                 255

Asp Phe Arg Lys Glu Thr Asp Gln Lys Gly Val Trp Leu Ser Lys Asp
            260                 265                 270

Gly Tyr Pro Lys Val Ile Lys Lys Trp His Gln Phe Phe Lys Met Asp
        275                 280                 285

Glu Gln Asn Thr Ser Ile Leu Ser Arg Pro Ile Thr Tyr Gln His Ala
    290                 295                 300

Ile Glu Arg Gln Val Arg Thr Phe Ser Gln Tyr Leu Met Asp Asp Lys
305                 310                 315                 320

Asp Asn Tyr Lys Thr Ile Glu Leu
                325

<210>  102
<211>  92
<212>  PRT
<213>  Deltaproteobacteria bacterium

<220>
<221>  PEPTIDE
<222>  (1)..(92)
<223>  CRISPR-associated endonuclease Cas2: /protein_id=OGP07441.1

<400>  102

Met Arg His Leu Ile Cys Tyr Asp Ile Glu Glu Asp Lys Val Arg Ala
1               5                   10                  15

Arg Leu Val Lys Leu Leu Glu Ala Tyr Gly Val Arg Ile Gln Tyr Ser
            20                  25                  30

Val Phe Glu Phe Asn Leu Ser Lys Ala Arg Trp Thr Asp Leu Lys Leu
        35                  40                  45

Asn Leu Lys Glu Lys Gly Phe Leu Asp Gly Ser Ile Ser Ile Val Ile
    50                  55                  60

Tyr Pro Leu Ser Ala Glu Ala Tyr Glu Leu Val Glu Arg Tyr Gly Ala
65                  70                  75                  80

Ala Ser Ile Trp Asp Glu Gly Asp Met Val Phe Asp
                85                  90

<210>  103
<211>  1389
<212>  PRT
<213>  Leptotrichia shahii DSM 19757

<220>
<221>  PEPTIDE
<222>  (1)..(1389)
<223>  type VI-A CRISPR-associated effector C2c2:
       /protein_id=WP_018451595.1

<400>  103

Met Gly Asn Leu Phe Gly His Lys Arg Trp Tyr Glu Val Arg Asp Lys
1               5                   10                  15

Lys Asp Phe Lys Ile Lys Arg Lys Val Lys Val Lys Arg Asn Tyr Asp
            20                  25                  30

Gly Asn Lys Tyr Ile Leu Asn Ile Asn Glu Asn Asn Asn Lys Glu Lys
        35                  40                  45

Ile Asp Asn Asn Lys Phe Ile Arg Lys Tyr Ile Asn Tyr Lys Lys Asn
    50                  55                  60

Asp Asn Ile Leu Lys Glu Phe Thr Arg Lys Phe His Ala Gly Asn Ile
65                  70                  75                  80

Leu Phe Lys Leu Lys Gly Lys Glu Gly Ile Ile Arg Ile Glu Asn Asn
                85                  90                  95

Asp Asp Phe Leu Glu Thr Glu Glu Val Val Leu Tyr Ile Glu Ala Tyr

            100                 105                 110

Gly Lys Ser Glu Lys Leu Lys Ala Leu Gly Ile Thr Lys Lys Lys Ile
        115                 120                 125

Ile Asp Glu Ala Ile Arg Gln Gly Ile Thr Lys Asp Asp Lys Lys Ile
    130                 135                 140

Glu Ile Lys Arg Gln Glu Asn Glu Glu Glu Ile Glu Ile Asp Ile Arg
145                 150                 155                 160

Asp Glu Tyr Thr Asn Lys Thr Leu Asn Asp Cys Ser Ile Ile Leu Arg
                165                 170                 175

Ile Ile Glu Asn Asp Glu Leu Glu Thr Lys Lys Ser Ile Tyr Glu Ile
            180                 185                 190

Phe Lys Asn Ile Asn Met Ser Leu Tyr Lys Ile Ile Glu Lys Ile Ile
        195                 200                 205

Glu Asn Glu Thr Glu Lys Val Phe Glu Asn Arg Tyr Tyr Glu Glu His
    210                 215                 220

Leu Arg Glu Lys Leu Leu Lys Asp Asp Lys Ile Asp Val Ile Leu Thr
225                 230                 235                 240

Asn Phe Met Glu Ile Arg Glu Lys Ile Lys Ser Asn Leu Glu Ile Leu
                245                 250                 255

Gly Phe Val Lys Phe Tyr Leu Asn Val Gly Gly Asp Lys Lys Lys Ser
            260                 265                 270

Lys Asn Lys Lys Met Leu Val Glu Lys Ile Leu Asn Ile Asn Val Asp
        275                 280                 285

Leu Thr Val Glu Asp Ile Ala Asp Phe Val Ile Lys Glu Leu Glu Phe
    290                 295                 300

Trp Asn Ile Thr Lys Arg Ile Glu Lys Val Lys Lys Val Asn Asn Glu
305                 310                 315                 320

Phe Leu Glu Lys Arg Arg Asn Arg Thr Tyr Ile Lys Ser Tyr Val Leu
                325                 330                 335

Leu Asp Lys His Glu Lys Phe Lys Ile Glu Arg Glu Asn Lys Lys Asp
            340                 345                 350

Lys Ile Val Lys Phe Phe Val Glu Asn Ile Lys Asn Asn Ser Ile Lys
        355                 360                 365

Glu Lys Ile Glu Lys Ile Leu Ala Glu Phe Lys Ile Asp Glu Leu Ile
    370                 375                 380

Lys Lys Leu Glu Lys Glu Leu Lys Lys Gly Asn Cys Asp Thr Glu Ile
385                 390                 395                 400

Phe Gly Ile Phe Lys Lys His Tyr Lys Val Asn Phe Asp Ser Lys Lys
                405                 410                 415

Phe Ser Lys Lys Ser Asp Glu Glu Lys Glu Leu Tyr Lys Ile Ile Tyr
            420                 425                 430

Arg Tyr Leu Lys Gly Arg Ile Glu Lys Ile Leu Val Asn Glu Gln Lys
        435                 440                 445

Val Arg Leu Lys Lys Met Glu Lys Ile Glu Ile Glu Lys Ile Leu Asn
    450                 455                 460

Glu Ser Ile Leu Ser Glu Lys Ile Leu Lys Arg Val Lys Gln Tyr Thr
465                 470                 475                 480

Leu Glu His Ile Met Tyr Leu Gly Lys Leu Arg His Asn Asp Ile Asp
                485                 490                 495

Met Thr Thr Val Asn Thr Asp Asp Phe Ser Arg Leu His Ala Lys Glu
            500                 505                 510

Glu Leu Asp Leu Glu Leu Ile Thr Phe Phe Ala Ser Thr Asn Met Glu
        515                 520                 525

Leu Asn Lys Ile Phe Ser Arg Glu Asn Ile Asn Asn Asp Glu Asn Ile
    530                 535                 540

Asp Phe Phe Gly Gly Asp Arg Glu Lys Asn Tyr Val Leu Asp Lys Lys
545                 550                 555                 560

Ile Leu Asn Ser Lys Ile Lys Ile Ile Arg Asp Leu Asp Phe Ile Asp
                565                 570                 575

Asn Lys Asn Asn Ile Thr Asn Asn Phe Ile Arg Lys Phe Thr Lys Ile
            580                 585                 590

Gly Thr Asn Glu Arg Asn Arg Ile Leu His Ala Ile Ser Lys Glu Arg
        595                 600                 605

Asp Leu Gln Gly Thr Gln Asp Asp Tyr Asn Lys Val Ile Asn Ile Ile
    610                 615                 620

Gln Asn Leu Lys Ile Ser Asp Glu Glu Val Ser Lys Ala Leu Asn Leu
625                 630                 635                 640

Asp Val Val Phe Lys Asp Lys Lys Asn Ile Ile Thr Lys Ile Asn Asp
                645                 650                 655

Ile Lys Ile Ser Glu Glu Asn Asn Asn Asp Ile Lys Tyr Leu Pro Ser
            660                 665                 670

Phe Ser Lys Val Leu Pro Glu Ile Leu Asn Leu Tyr Arg Asn Asn Pro
        675                 680                 685

Lys Asn Glu Pro Phe Asp Thr Ile Glu Thr Glu Lys Ile Val Leu Asn
    690                 695                 700

Ala Leu Ile Tyr Val Asn Lys Glu Leu Tyr Lys Lys Leu Ile Leu Glu
705                 710                 715                 720

Asp Asp Leu Glu Glu Asn Glu Ser Lys Asn Ile Phe Leu Gln Glu Leu
                725                 730                 735

Lys Lys Thr Leu Gly Asn Ile Asp Glu Ile Asp Glu Asn Ile Ile Glu
            740                 745                 750

Asn Tyr Tyr Lys Asn Ala Gln Ile Ser Ala Ser Lys Gly Asn Asn Lys
        755                 760                 765

Ala Ile Lys Lys Tyr Gln Lys Lys Val Ile Glu Cys Tyr Ile Gly Tyr
    770                 775                 780

Leu Arg Lys Asn Tyr Glu Glu Leu Phe Asp Phe Ser Asp Phe Lys Met
785                 790                 795                 800

Asn Ile Gln Glu Ile Lys Lys Gln Ile Lys Asp Ile Asn Asp Asn Lys
                805                 810                 815

Thr Tyr Glu Arg Ile Thr Val Lys Thr Ser Asp Lys Thr Ile Val Ile
            820                 825                 830

Asn Asp Asp Phe Glu Tyr Ile Ile Ser Ile Phe Ala Leu Leu Asn Ser
        835                 840                 845

Asn Ala Val Ile Asn Lys Ile Arg Asn Arg Phe Phe Ala Thr Ser Val
    850                 855                 860

Trp Leu Asn Thr Ser Glu Tyr Gln Asn Ile Ile Asp Ile Leu Asp Glu
865                 870                 875                 880

Ile Met Gln Leu Asn Thr Leu Arg Asn Glu Cys Ile Thr Glu Asn Trp
                885                 890                 895

Asn Leu Asn Leu Glu Glu Phe Ile Gln Lys Met Lys Glu Ile Glu Lys
            900                 905                 910

Asp Phe Asp Asp Phe Lys Ile Gln Thr Lys Lys Glu Ile Phe Asn Asn
        915                 920                 925

Tyr Tyr Glu Asp Ile Lys Asn Asn Ile Leu Thr Glu Phe Lys Asp Asp
    930                 935                 940

Ile Asn Gly Cys Asp Val Leu Glu Lys Lys Leu Glu Lys Ile Val Ile
945                 950                 955                 960

Phe Asp Asp Glu Thr Lys Phe Glu Ile Asp Lys Lys Ser Asn Ile Leu
                965                 970                 975

Gln Asp Glu Gln Arg Lys Leu Ser Asn Ile Asn Lys Lys Asp Leu Lys
            980                 985                 990

Lys Lys Val Asp Gln Tyr Ile Lys  Asp Lys Asp Gln Glu  Ile Lys Ser
        995                 1000                1005

Lys Ile  Leu Cys Arg Ile Ile  Phe Asn Ser Asp Phe  Leu Lys Lys
    1010                1015                1020

Tyr Lys  Lys Glu Ile Asp Asn  Leu Ile Glu Asp Met  Glu Ser Glu
    1025                1030                1035

Asn Glu  Asn Lys Phe Gln Glu  Ile Tyr Tyr Pro Lys  Glu Arg Lys
    1040                1045                1050

Asn Glu  Leu Tyr Ile Tyr Lys  Lys Asn Leu Phe Leu  Asn Ile Gly
    1055                1060                1065

Asn Pro  Asn Phe Asp Lys Ile  Tyr Gly Leu Ile Ser  Asn Asp Ile
    1070                1075                1080

Lys Met  Ala Asp Ala Lys Phe  Leu Phe Asn Ile Asp  Gly Lys Asn
    1085                1090                1095

Ile Arg  Lys Asn Lys Ile Ser  Glu Ile Asp Ala Ile  Leu Lys Asn

1100                1105                1110

Leu Asn Asp Lys Leu Asn Gly Tyr Ser Lys Glu Tyr Lys Glu Lys
    1115                1120                1125

Tyr Ile Lys Lys Leu Lys Glu Asn Asp Asp Phe Phe Ala Lys Asn
    1130                1135                1140

Ile Gln Asn Lys Asn Tyr Lys Ser Phe Glu Lys Asp Tyr Asn Arg
    1145                1150                1155

Val Ser Glu Tyr Lys Lys Ile Arg Asp Leu Val Glu Phe Asn Tyr
    1160                1165                1170

Leu Asn Lys Ile Glu Ser Tyr Leu Ile Asp Ile Asn Trp Lys Leu
    1175                1180                1185

Ala Ile Gln Met Ala Arg Phe Glu Arg Asp Met His Tyr Ile Val
    1190                1195                1200

Asn Gly Leu Arg Glu Leu Gly Ile Ile Lys Leu Ser Gly Tyr Asn
    1205                1210                1215

Thr Gly Ile Ser Arg Ala Tyr Pro Lys Arg Asn Gly Ser Asp Gly
    1220                1225                1230

Phe Tyr Thr Thr Thr Ala Tyr Tyr Lys Phe Phe Asp Glu Glu Ser
    1235                1240                1245

Tyr Lys Lys Phe Glu Lys Ile Cys Tyr Gly Phe Gly Ile Asp Leu
    1250                1255                1260

Ser Glu Asn Ser Glu Ile Asn Lys Pro Glu Asn Glu Ser Ile Arg
    1265                1270                1275

Asn Tyr Ile Ser His Phe Tyr Ile Val Arg Asn Pro Phe Ala Asp
    1280                1285                1290

Tyr Ser Ile Ala Glu Gln Ile Asp Arg Val Ser Asn Leu Leu Ser
    1295                1300                1305

Tyr Ser Thr Arg Tyr Asn Asn Ser Thr Tyr Ala Ser Val Phe Glu
    1310                1315                1320

Val Phe Lys Lys Asp Val Asn Leu Asp Tyr Asp Glu Leu Lys Lys
    1325                1330                1335

Lys Phe Lys Leu Ile Gly Asn Asn Asp Ile Leu Glu Arg Leu Met
    1340                1345                1350

Lys Pro Lys Lys Val Ser Val Leu Glu Leu Glu Ser Tyr Asn Ser
    1355                1360                1365

Asp Tyr Ile Lys Asn Leu Ile Ile Glu Leu Leu Thr Lys Ile Glu
    1370                1375                1380

Asn Thr Asn Asp Thr Leu
    1385

<210> 104
<211> 1127
<212> PRT
<213> Prevotella buccae

<220>
<221> PEPTIDE
<222> (1)..(1127)
<223> hypothetical protein: /protein_id=WP_004343973.1

<400> 104

Met Gln Lys Gln Asp Lys Leu Phe Val Asp Arg Lys Lys Asn Ala Ile
1               5                   10                  15

Phe Ala Phe Pro Lys Tyr Ile Thr Ile Met Glu Asn Lys Glu Lys Pro
            20                  25                  30

Glu Pro Ile Tyr Tyr Glu Leu Thr Asp Lys His Phe Trp Ala Ala Phe
        35                  40                  45

Leu Asn Leu Ala Arg His Asn Val Tyr Thr Thr Ile Asn His Ile Asn
    50                  55                  60

Arg Arg Leu Glu Ile Ala Glu Leu Lys Asp Asp Gly Tyr Met Met Gly
65                  70                  75                  80

Ile Lys Gly Ser Trp Asn Glu Gln Ala Lys Lys Leu Asp Lys Lys Val
                85                  90                  95

Arg Leu Arg Asp Leu Ile Met Lys His Phe Pro Phe Leu Glu Ala Ala
            100                 105                 110

Ala Tyr Glu Met Thr Asn Ser Lys Ser Pro Asn Asn Lys Glu Gln Arg
        115                 120                 125

Glu Lys Glu Gln Ser Glu Ala Leu Ser Leu Asn Asn Leu Lys Asn Val
    130                 135                 140

Leu Phe Ile Phe Leu Glu Lys Leu Gln Val Leu Arg Asn Tyr Tyr Ser
145                 150                 155                 160

His Tyr Lys Tyr Ser Glu Glu Ser Pro Lys Pro Ile Phe Glu Thr Ser
                165                 170                 175

Leu Leu Lys Asn Met Tyr Lys Val Phe Asp Ala Asn Val Arg Leu Val
            180                 185                 190

Lys Arg Asp Tyr Met His His Glu Asn Ile Asp Met Gln Arg Asp Phe
        195                 200                 205

Thr His Leu Asn Arg Lys Lys Gln Val Gly Arg Thr Lys Asn Ile Ile
    210                 215                 220

Asp Ser Pro Asn Phe His Tyr His Phe Ala Asp Lys Glu Gly Asn Met
225                 230                 235                 240

Thr Ile Ala Gly Leu Leu Phe Phe Val Ser Leu Phe Leu Asp Lys Lys
                245                 250                 255

Asp Ala Ile Trp Met Gln Lys Lys Leu Lys Gly Phe Lys Asp Gly Arg
            260                 265                 270

Asn Leu Arg Glu Gln Met Thr Asn Glu Val Phe Cys Arg Ser Arg Ile
        275                 280                 285

Ser Leu Pro Lys Leu Lys Leu Glu Asn Val Gln Thr Lys Asp Trp Met
    290                 295                 300

Gln Leu Asp Met Leu Asn Glu Leu Val Arg Cys Pro Lys Ser Leu Tyr
305                 310                 315                 320

Glu Arg Leu Arg Glu Lys Asp Arg Glu Ser Phe Lys Val Pro Phe Asp
                325                 330                 335

Ile Phe Ser Asp Asp Tyr Asn Ala Glu Glu Glu Pro Phe Lys Asn Thr
            340                 345                 350

Leu Val Arg His Gln Asp Arg Phe Pro Tyr Phe Val Leu Arg Tyr Phe
        355                 360                 365

Asp Leu Asn Glu Ile Phe Glu Gln Leu Arg Phe Gln Ile Asp Leu Gly
    370                 375                 380

Thr Tyr His Phe Ser Ile Tyr Asn Lys Arg Ile Gly Asp Glu Asp Glu

385                 390                 395                 400

Val Arg His Leu Thr His His Leu Tyr Gly Phe Ala Arg Ile Gln Asp
                405                 410                 415

Phe Ala Pro Gln Asn Gln Pro Glu Glu Trp Arg Lys Leu Val Lys Asp
            420                 425                 430

Leu Asp His Phe Glu Thr Ser Gln Glu Pro Tyr Ile Ser Lys Thr Ala
        435                 440                 445

Pro His Tyr His Leu Glu Asn Glu Lys Ile Gly Ile Lys Phe Cys Ser
    450                 455                 460

Ala His Asn Asn Leu Phe Pro Ser Leu Gln Thr Asp Lys Thr Cys Asn
465                 470                 475                 480

Gly Arg Ser Lys Phe Asn Leu Gly Thr Gln Phe Thr Ala Glu Ala Phe
                485                 490                 495

Leu Ser Val His Glu Leu Leu Pro Met Met Phe Tyr Tyr Leu Leu Leu
            500                 505                 510

Thr Lys Asp Tyr Ser Arg Lys Glu Ser Ala Asp Lys Val Glu Gly Ile
        515                 520                 525

Ile Arg Lys Glu Ile Ser Asn Ile Tyr Ala Ile Tyr Asp Ala Phe Ala
    530                 535                 540

Asn Asn Glu Ile Asn Ser Ile Ala Asp Leu Thr Arg Arg Leu Gln Asn
545                 550                 555                 560

Thr Asn Ile Leu Gln Gly His Leu Pro Lys Gln Met Ile Ser Ile Leu
                565                 570                 575

Lys Gly Arg Gln Lys Asp Met Gly Lys Glu Ala Glu Arg Lys Ile Gly
            580                 585                 590

Glu Met Ile Asp Asp Thr Gln Arg Arg Leu Asp Leu Leu Cys Lys Gln
        595                 600                 605

Thr Asn Gln Lys Ile Arg Ile Gly Lys Arg Asn Ala Gly Leu Leu Lys
    610                 615                 620

Ser Gly Lys Ile Ala Asp Trp Leu Val Asn Asp Met Met Arg Phe Gln
625                 630                 635                 640

Pro Val Gln Lys Asp Gln Asn Asn Ile Pro Ile Asn Asn Ser Lys Ala
                645                 650                 655

Asn Ser Thr Glu Tyr Arg Met Leu Gln Arg Ala Leu Ala Leu Phe Gly
            660                 665                 670

Ser Glu Asn Phe Arg Leu Lys Ala Tyr Phe Asn Gln Met Asn Leu Val
        675                 680                 685

Gly Asn Asp Asn Pro His Pro Phe Leu Ala Glu Thr Gln Trp Glu His
    690                 695                 700

Gln Thr Asn Ile Leu Ser Phe Tyr Arg Asn Tyr Leu Glu Ala Arg Lys
705                 710                 715                 720

Lys Tyr Leu Lys Gly Leu Lys Pro Gln Asn Trp Lys Gln Tyr Gln His
                725                 730                 735

Phe Leu Ile Leu Lys Val Gln Lys Thr Asn Arg Asn Thr Leu Val Thr
            740                 745                 750

Gly Trp Lys Asn Ser Phe Asn Leu Pro Arg Gly Ile Phe Thr Gln Pro
        755                 760                 765

Ile Arg Glu Trp Phe Glu Lys His Asn Asn Ser Lys Arg Ile Tyr Asp
    770                 775                 780

Gln Ile Leu Ser Phe Asp Arg Val Gly Phe Val Ala Lys Ala Ile Pro
785                 790                 795                 800

Leu Tyr Phe Ala Glu Glu Tyr Lys Asp Asn Val Gln Pro Phe Tyr Asp
                805                 810                 815

Tyr Pro Phe Asn Ile Gly Asn Arg Leu Lys Pro Lys Lys Arg Gln Phe
            820                 825                 830

Leu Asp Lys Lys Glu Arg Val Glu Leu Trp Gln Lys Asn Lys Glu Leu
        835                 840                 845

Phe Lys Asn Tyr Pro Ser Glu Lys Lys Lys Thr Asp Leu Ala Tyr Leu
    850                 855                 860

Asp Phe Leu Ser Trp Lys Lys Phe Glu Arg Glu Leu Arg Leu Ile Lys
865                 870                 875                 880

Asn Gln Asp Ile Val Thr Trp Leu Met Phe Lys Glu Leu Phe Asn Met
                885                 890                 895

Ala Thr Val Glu Gly Leu Lys Ile Gly Glu Ile His Leu Arg Asp Ile
            900                 905                 910

Asp Thr Asn Thr Ala Asn Glu Glu Ser Asn Asn Ile Leu Asn Arg Ile
        915                 920                 925

Met Pro Met Lys Leu Pro Val Lys Thr Tyr Glu Thr Asp Asn Lys Gly
    930                 935                 940

Asn Ile Leu Lys Glu Arg Pro Leu Ala Thr Phe Tyr Ile Glu Glu Thr
945                 950                 955                 960

Glu Thr Lys Val Leu Lys Gln Gly Asn Phe Lys Ala Leu Val Lys Asp
                965                 970                 975

Arg Arg Leu Asn Gly Leu Phe Ser Phe Ala Glu Thr Thr Asp Leu Asn
            980                 985                 990

Leu Glu Glu His Pro Ile Ser Lys  Leu Ser Val Asp Leu  Glu Leu Ile
        995                 1000                 1005

Lys Tyr  Gln Thr Thr Arg Ile  Ser Ile Phe Glu Met  Thr Leu Gly
    1010                 1015                 1020

Leu Glu  Lys Lys Leu Ile Asp  Lys Tyr Ser Thr Leu  Pro Thr Asp
    1025                 1030                 1035

Ser Phe  Arg Asn Met Leu Glu  Arg Trp Leu Gln Cys  Lys Ala Asn
    1040                 1045                 1050

Arg Pro  Glu Leu Lys Asn Tyr  Val Asn Ser Leu Ile  Ala Val Arg
    1055                 1060                 1065

Asn Ala  Phe Ser His Asn Gln  Tyr Pro Met Tyr Asp  Ala Thr Leu
    1070                 1075                 1080

Phe Ala  Glu Val Lys Lys Phe  Thr Leu Phe Pro Ser  Val Asp Thr
    1085                 1090                 1095

Lys Lys  Ile Glu Leu Asn Ile  Ala Pro Gln Leu Leu  Glu Ile Val
    1100                 1105                 1110

Gly Lys  Ala Ile Lys Glu Ile  Glu Lys Ser Glu Asn  Lys Asn
    1115                 1120                 1125

<210>  105
<211>  177

<212>  PRT
<213>  Prevotella buccae

<220>
<221>  PEPTIDE
<222>  (1)..(177)
<223>  hypothetical protein /protein_id=WP_004343974.1

<400>  105

Met Asp Tyr Met Glu Leu Ala Lys Glu Ala Phe Ser Ile Ile Cys Thr
1               5                   10                  15

Phe Ile Ala Ala Tyr Val Ala Tyr Tyr Tyr Ala Ile Lys Gln Leu His
            20                  25                  30

Gln Lys Ser Val Glu Asn Ile Glu Tyr Ala Lys Tyr Gln Ala Val Leu
        35                  40                  45

Gln Ala His Lys Ser Leu Tyr Lys Leu Leu Arg Phe Thr Thr Asn Thr
    50                  55                  60

Glu Asn Glu Asp Ser Ile Leu Ile Trp Glu Lys Thr Lys Asp Gly Lys
65                  70                  75                  80

Gln Glu Ala Thr Tyr Tyr Phe Arg Lys Glu Asn Ile Arg Lys Phe Ile
                85                  90                  95

Lys Glu Leu Ser Lys Glu Ile Tyr Asn Glu Gly Cys Gly Ile Phe Met
            100                 105                 110

Ser Lys Glu Ala Leu Ser Leu Ile Ser Glu Tyr Arg Asn Ile Val Tyr
        115                 120                 125

Gly Phe Met Leu Ser Ala Gln Asn Asn Pro Gln Glu Thr Ile Arg Ile
    130                 135                 140

Thr Asn Arg Glu Ser Val Glu Arg Met Lys Lys Ile His Gln Asn Leu
145                 150                 155                 160

Ser Ile Glu Ile Arg Gln Ala Ile Asn Leu Lys Lys Arg Asp Leu Arg
                165                 170                 175

Phe

<210>  106
<211>  1224
<212>  PRT
<213>  Bergeyella zoohelcum

<220>
<221>  PEPTIDE
<222>  (1)..(1224)
<223>  hypothetical protein  /protein_id=EKB54193.1

<400>  106

Met Glu Asn Lys Thr Ser Leu Gly Asn Asn Ile Tyr Tyr Asn Pro Phe
1               5                   10                  15

Lys Pro Gln Asp Lys Ser Tyr Phe Ala Gly Tyr Phe Asn Ala Ala Met
            20                  25                  30

Glu Asn Thr Asp Ser Val Phe Arg Glu Leu Gly Lys Arg Leu Lys Gly
        35                  40                  45

Lys Glu Tyr Thr Ser Glu Asn Phe Phe Asp Ala Ile Phe Lys Glu Asn
    50                  55                  60

Ile Ser Leu Val Glu Tyr Glu Arg Tyr Val Lys Leu Leu Ser Asp Tyr
65                  70                  75                  80

Phe Pro Met Ala Arg Leu Leu Asp Lys Lys Glu Val Pro Ile Lys Glu
                85                  90                  95

Arg Lys Glu Asn Phe Lys Lys Asn Phe Lys Gly Ile Ile Lys Ala Val
            100                 105                 110

Arg Asp Leu Arg Asn Phe Tyr Thr His Lys Glu His Gly Glu Val Glu
        115                 120                 125

Ile Thr Asp Glu Ile Phe Gly Val Leu Asp Glu Met Leu Lys Ser Thr
    130                 135                 140

Val Leu Thr Val Lys Lys Lys Lys Val Lys Thr Asp Lys Thr Lys Glu
145                 150                 155                 160

Ile Leu Lys Lys Ser Ile Glu Lys Gln Leu Asp Ile Leu Cys Gln Lys
                165                 170                 175

Lys Leu Glu Tyr Leu Arg Asp Thr Ala Arg Lys Ile Glu Glu Lys Arg
            180                 185                 190

Arg Asn Gln Arg Glu Arg Gly Glu Lys Glu Leu Val Ala Pro Phe Lys
        195                 200                 205

Tyr Ser Asp Lys Arg Asp Asp Leu Ile Ala Ala Ile Tyr Asn Asp Ala
    210                 215                 220

Phe Asp Val Tyr Ile Asp Lys Lys Lys Asp Ser Leu Lys Glu Ser Ser
225                 230                 235                 240

Lys Ala Lys Tyr Asn Thr Lys Ser Asp Pro Gln Gln Glu Glu Gly Asp
                245                 250                 255

Leu Lys Ile Pro Ile Ser Lys Asn Gly Val Val Phe Leu Leu Ser Leu
            260                 265                 270

Phe Leu Thr Lys Gln Glu Ile His Ala Phe Lys Ser Lys Ile Ala Gly
        275                 280                 285

Phe Lys Ala Thr Val Ile Asp Glu Ala Thr Val Ser Glu Ala Thr Val
    290                 295                 300

Ser His Gly Lys Asn Ser Ile Cys Phe Met Ala Thr His Glu Ile Phe
305                 310                 315                 320

Ser His Leu Ala Tyr Lys Lys Leu Lys Arg Lys Val Arg Thr Ala Glu
                325                 330                 335

Ile Asn Tyr Gly Glu Ala Glu Asn Ala Glu Gln Leu Ser Val Tyr Ala
            340                 345                 350

Lys Glu Thr Leu Met Met Gln Met Leu Asp Glu Leu Ser Lys Val Pro
        355                 360                 365

Asp Val Val Tyr Gln Asn Leu Ser Glu Asp Val Gln Lys Thr Phe Ile
    370                 375                 380

Glu Asp Trp Asn Glu Tyr Leu Lys Glu Asn Asn Gly Asp Val Gly Thr
385                 390                 395                 400

Met Glu Glu Glu Gln Val Ile His Pro Val Ile Arg Lys Arg Tyr Glu
                405                 410                 415

Asp Lys Phe Asn Tyr Phe Ala Ile Arg Phe Leu Asp Glu Phe Ala Gln
            420                 425                 430

Phe Pro Thr Leu Arg Phe Gln Val His Leu Gly Asn Tyr Leu His Asp
        435                 440                 445

Ser Arg Pro Lys Glu Asn Leu Ile Ser Asp Arg Arg Ile Lys Glu Lys
    450                 455                 460

Ile Thr Val Phe Gly Arg Leu Ser Glu Leu Glu His Lys Lys Ala Leu

465                 470                 475                 480

Phe Ile Lys Asn Thr Glu Thr Asn Glu Asp Arg Glu His Tyr Trp Glu
                485                 490                 495

Ile Phe Pro Asn Pro Asn Tyr Asp Phe Pro Lys Glu Asn Ile Ser Val
            500                 505                 510

Asn Asp Lys Asp Phe Pro Ile Ala Gly Ser Ile Leu Asp Arg Glu Lys
        515                 520                 525

Gln Pro Val Ala Gly Lys Ile Gly Ile Lys Val Lys Leu Leu Asn Gln
    530                 535                 540

Gln Tyr Val Ser Glu Val Asp Lys Ala Val Lys Ala His Gln Leu Lys
545                 550                 555                 560

Gln Arg Lys Ala Ser Lys Pro Ser Ile Gln Asn Ile Ile Glu Glu Ile
                565                 570                 575

Val Pro Ile Asn Glu Ser Asn Pro Lys Glu Ala Ile Val Phe Gly Gly
            580                 585                 590

Gln Pro Thr Ala Tyr Leu Ser Met Asn Asp Ile His Ser Ile Leu Tyr
        595                 600                 605

Glu Phe Phe Asp Lys Trp Glu Lys Lys Lys Glu Lys Leu Glu Lys Lys
    610                 615                 620

Gly Glu Lys Glu Leu Arg Lys Glu Ile Gly Lys Glu Leu Glu Lys Lys
625                 630                 635                 640

Ile Val Gly Lys Ile Gln Ala Gln Ile Gln Gln Ile Ile Asp Lys Asp
                645                 650                 655

Thr Asn Ala Lys Ile Leu Lys Pro Tyr Gln Asp Gly Asn Ser Thr Ala
            660                 665                 670

Ile Asp Lys Glu Lys Leu Ile Lys Asp Leu Lys Gln Glu Gln Asn Ile
        675                 680                 685

Leu Gln Lys Leu Lys Asp Glu Gln Thr Val Arg Glu Lys Glu Tyr Asn
    690                 695                 700

Asp Phe Ile Ala Tyr Gln Asp Lys Asn Arg Glu Ile Asn Lys Val Arg
705                 710                 715                 720

Asp Arg Asn His Lys Gln Tyr Leu Lys Asp Asn Leu Lys Arg Lys Tyr
                725                 730                 735

Pro Glu Ala Pro Ala Arg Lys Glu Val Leu Tyr Tyr Arg Glu Lys Gly
            740                 745                 750

Lys Val Ala Val Trp Leu Ala Asn Asp Ile Lys Arg Phe Met Pro Thr
        755                 760                 765

Asp Phe Lys Asn Glu Trp Lys Gly Glu Gln His Ser Leu Leu Gln Lys
    770                 775                 780

Ser Leu Ala Tyr Tyr Glu Gln Cys Lys Glu Glu Leu Lys Asn Leu Leu
785                 790                 795                 800

Pro Glu Lys Val Phe Gln His Leu Pro Phe Lys Leu Gly Gly Tyr Phe
                805                 810                 815

Gln Gln Lys Tyr Leu Tyr Gln Phe Tyr Thr Cys Tyr Leu Asp Lys Arg
            820                 825                 830

Leu Glu Tyr Ile Ser Gly Leu Val Gln Gln Ala Glu Asn Phe Lys Ser
        835                 840                 845

Glu Asn Lys Val Phe Lys Lys Val Glu Asn Glu Cys Phe Lys Phe Leu
    850                 855                 860

Lys Lys Gln Asn Tyr Thr His Lys Glu Leu Asp Ala Arg Val Gln Ser
865                 870                 875                 880

Ile Leu Gly Tyr Pro Ile Phe Leu Glu Arg Gly Phe Met Asp Glu Lys
                885                 890                 895

Pro Thr Ile Ile Lys Gly Lys Thr Phe Lys Gly Asn Glu Ala Leu Phe
            900                 905                 910

Ala Asp Trp Phe Arg Tyr Tyr Lys Glu Tyr Gln Asn Phe Gln Thr Phe
        915                 920                 925

Tyr Asp Thr Glu Asn Tyr Pro Leu Val Glu Leu Glu Lys Lys Gln Ala
    930                 935                 940

Asp Arg Lys Arg Lys Thr Lys Ile Tyr Gln Gln Lys Lys Asn Asp Val
945                 950                 955                 960

Phe Thr Leu Leu Met Ala Lys His Ile Phe Lys Ser Val Phe Lys Gln
                965                 970                 975

Asp Ser Ile Asp Gln Phe Ser Leu Glu Asp Leu Tyr Gln Ser Arg Glu
            980                 985                 990

Glu Arg Leu Gly Asn Gln Glu Arg  Ala Arg Gln Thr Gly  Glu Arg Asn
        995                 1000                 1005

Thr Asn  Tyr Ile Trp Asn Lys  Thr Val Asp Leu Lys  Leu Cys Asp
    1010                 1015                 1020

Gly Lys  Ile Thr Val Glu Asn  Val Lys Leu Lys Asn  Val Gly Asp
    1025                 1030                 1035

Phe Ile  Lys Tyr Glu Tyr Asp  Gln Arg Val Gln Ala  Phe Leu Lys
    1040                 1045                 1050

Tyr Glu  Glu Asn Ile Glu Trp  Gln Ala Phe Leu Ile  Lys Glu Ser
    1055                 1060                 1065

Lys Glu  Glu Glu Asn Tyr Pro  Tyr Val Val Glu Arg  Glu Ile Glu
    1070                 1075                 1080

Gln Tyr  Glu Lys Val Arg Arg  Glu Glu Leu Leu Lys  Glu Val His
    1085                 1090                 1095

Leu Ile  Glu Glu Tyr Ile Leu  Glu Lys Val Lys Asp  Lys Glu Ile
    1100                 1105                 1110

Leu Lys  Lys Gly Asp Asn Gln  Asn Phe Lys Tyr Tyr  Ile Leu Asn
    1115                 1120                 1125

Gly Leu  Leu Lys Gln Leu Lys  Asn Glu Asp Val Glu  Ser Tyr Lys
    1130                 1135                 1140

Val Phe  Asn Leu Asn Thr Glu  Pro Glu Asp Val Asn  Ile Asn Gln
    1145                 1150                 1155

Leu Lys  Gln Glu Ala Thr Asp  Leu Glu Gln Lys Ala  Phe Val Leu
    1160                 1165                 1170

Thr Tyr  Ile Arg Asn Lys Phe  Ala His Asn Gln Leu  Pro Lys Lys
    1175                 1180                 1185

Glu Phe  Trp Asp Tyr Cys Gln  Glu Lys Tyr Gly Lys  Ile Glu Lys
    1190                 1195                 1200

Glu Lys  Thr Tyr Ala Glu Tyr  Phe Ala Glu Val Phe  Lys Lys Glu
    1205                 1210                 1215

Lys Glu  Ala Leu Ile Lys
    1220

<210>  107
<211>  219
<212>  PRT
<213>  Bergeyella zoohelcum

<220>
<221>  PEPTIDE
<222>  (1)..(219)
<223>  hypothetical protein:  /protein_id=EKB54194.1

<400>  107

Met Asp Arg Leu Ser Ile Tyr Glu Leu Leu Ser Phe Val Val Pro Gly
1               5                   10                  15

Val Ile Met Ile Glu Leu Ile Asn Phe Ser Ala Glu Tyr Val Phe Gly
            20                  25                  30

Lys Ala Pro Leu Ile Thr Ala Gly Asn Leu Ser Asp Gly Leu Leu Phe
        35                  40                  45

Phe Ala Ile Ala Leu Phe Ile Gly Cys Leu Ile His Ile Ile Thr Phe
    50                  55                  60

Trp Leu Ile Lys Arg Lys Trp Tyr Lys Lys Leu Ala Cys Thr Pro Met
65                  70                  75                  80

Asp Lys Met Lys Asn Asp Glu Tyr Ile Thr Glu Ile Leu Pro Asp Leu
                85                  90                  95

Lys Glu Ile Tyr Arg Thr Asn Lys Gly Ile Ser Val Thr Glu Asn Ile
            100                 105                 110

Ser Asn Gly Ser Val Phe Asp Thr Ala Tyr Tyr Tyr Leu Glu Ala Gln
        115                 120                 125

Gly Lys Ile Ser Gln Ala Lys Asn Phe Gln Ser Ile Tyr Phe Leu Phe
    130                 135                 140

Arg Asn Ile Val Thr Leu Ser Leu Phe Val Leu Pro Val Ser Val Ile
145                 150                 155                 160

Phe Leu Leu Ala Ser Phe Phe Met Asn Asp Cys Ser Leu Ser Glu Lys
                165                 170                 175

Ile Ile Thr Ile Ile Ile Gly Thr Phe Val Leu Gly Gly Leu Ser Ser
            180                 185                 190

Val Ile Ala Gln Trp Phe Arg Val Lys Met Thr Asp Arg Ile Phe Gly
        195                 200                 205

Leu Tyr Tyr Ala Glu Leu Thr His Asn Lys Lys
    210                 215

<210>  108
<211>  1121
<212>  PRT
<213>  Fusobacterium perfoetens

<220>
<221>  PEPTIDE
<222>  (1)..(1121)
<223>  hypothetical protein: /protein_id=WP_027128616.1

<400>  108

Met Gly Lys Pro Asn Arg Ser Ser Ile Ile Lys Ile Ile Ile Ser Asn
1               5                   10                  15

Tyr Asp Asn Lys Gly Ile Lys Glu Val Lys Val Arg Tyr Asn Lys Gln
            20                  25                  30

Ala Gln Leu Asp Thr Phe Leu Ile Lys Ser Glu Leu Lys Asp Gly Lys
        35                  40                  45

Phe Ile Leu Tyr Ser Ile Val Asp Lys Ala Arg Glu Lys Tyr Arg Tyr
    50                  55                  60

Ser Phe Glu Ile Asp Lys Thr Asn Ile Asn Lys Asn Glu Ile Leu Ile
65                  70                  75                  80

Ile Lys Lys Asp Ile Tyr Ser Asn Lys Glu Asp Lys Val Ile Arg Lys
                85                  90                  95

Tyr Ile Leu Ser Phe Glu Val Ser Glu Lys Asn Asp Arg Thr Ile Val
            100                 105                 110

Thr Lys Ile Lys Asp Cys Leu Glu Thr Gln Lys Lys Glu Lys Phe Glu
        115                 120                 125

Arg Glu Asn Thr Arg Arg Leu Ile Ser Glu Thr Glu Arg Lys Leu Leu
    130                 135                 140

Ser Glu Glu Thr Gln Lys Thr Tyr Ser Lys Ile Ala Cys Cys Ser Pro
145                 150                 155                 160

Glu Asp Ile Asp Ser Val Lys Ile Tyr Lys Ile Lys Arg Tyr Leu Ala
                165                 170                 175

Tyr Arg Ser Asn Met Leu Leu Phe Phe Ser Leu Ile Asn Asp Ile Phe
            180                 185                 190

Val Lys Gly Val Val Lys Asp Asn Gly Glu Glu Val Gly Glu Ile Trp
        195                 200                 205

Arg Ile Ile Asp Ser Lys Glu Ile Asp Glu Lys Lys Thr Tyr Asp Leu
    210                 215                 220

Leu Val Glu Asn Phe Lys Lys Arg Met Ser Gln Glu Phe Ile Asn Tyr
225                 230                 235                 240

Lys Gln Ser Ile Glu Asn Lys Ile Glu Lys Asn Thr Asn Lys Ile Lys
                245                 250                 255

Glu Ile Glu Gln Lys Leu Lys Lys Glu Lys Tyr Lys Lys Glu Ile Asn
            260                 265                 270

Arg Leu Lys Lys Gln Leu Ile Glu Leu Asn Arg Glu Asn Asp Leu Leu
        275                 280                 285

Glu Lys Asp Lys Ile Glu Leu Ser Asp Glu Glu Ile Arg Glu Asp Ile
    290                 295                 300

Glu Lys Ile Leu Lys Ile Tyr Ser Asp Leu Arg His Lys Leu Met His
305                 310                 315                 320

Tyr Asn Tyr Gln Tyr Phe Glu Asn Leu Phe Glu Asn Lys Lys Ile Ser
                325                 330                 335

Lys Glu Lys Asn Glu Asp Val Asn Leu Thr Glu Leu Leu Asp Leu Asn
            340                 345                 350

Leu Phe Arg Tyr Leu Pro Leu Val Arg Gln Leu Lys Leu Glu Asn Lys
        355                 360                 365

Thr Asn Tyr Leu Glu Lys Glu Asp Lys Ile Thr Val Leu Gly Val Ser
    370                 375                 380

Asp Ser Ala Ile Lys Tyr Tyr Ser Tyr Tyr Asn Phe Leu Cys Glu Gln
385                 390                 395                 400

Lys Asn Gly Phe Asn Asn Phe Ile Asn Ser Phe Phe Ser Asn Asp Gly

405                 410                 415

Glu Glu Asn Lys Ser Phe Lys Glu Lys Ile Asn Leu Ser Leu Glu Lys
            420                 425                 430

Glu Ile Glu Ile Met Glu Lys Glu Thr Asn Glu Lys Ile Lys Glu Ile
        435                 440                 445

Asn Lys Asn Glu Leu Gln Leu Met Lys Glu Gln Lys Glu Leu Gly Thr
    450                 455                 460

Ala Tyr Val Leu Asp Ile His Ser Leu Asn Asp Tyr Lys Ile Ser His
465                 470                 475                 480

Asn Glu Arg Asn Lys Asn Val Lys Leu Gln Asn Asp Ile Met Asn Gly
                485                 490                 495

Asn Arg Asp Lys Asn Ala Leu Asp Lys Ile Asn Lys Lys Leu Val Glu
            500                 505                 510

Leu Lys Ile Lys Met Asp Lys Ile Thr Lys Arg Asn Ser Ile Leu Arg
        515                 520                 525

Leu Lys Tyr Lys Leu Gln Val Ala Tyr Gly Phe Leu Met Glu Glu Tyr
    530                 535                 540

Lys Gly Asn Ile Lys Lys Phe Lys Asp Glu Phe Asp Ile Ser Lys Glu
545                 550                 555                 560

Lys Ile Lys Ser Tyr Lys Ser Lys Gly Glu Lys Tyr Leu Glu Val Lys
                565                 570                 575

Ser Glu Lys Lys Tyr Ile Thr Lys Ile Leu Asn Ser Ile Glu Asp Ile
            580                 585                 590

His Asn Ile Thr Trp Leu Lys Asn Gln Glu Glu Asn Asn Leu Phe Lys
        595                 600                 605

Phe Tyr Val Leu Thr Tyr Ile Leu Leu Pro Phe Glu Phe Arg Gly Asp
    610                 615                 620

Phe Leu Gly Phe Val Lys Lys His Tyr Tyr Asp Ile Lys Asn Val Glu
625                 630                 635                 640

Phe Leu Asp Glu Asn Asn Asp Arg Leu Thr Pro Glu Gln Leu Glu Lys
                645                 650                 655

Met Lys Asn Asp Ser Phe Phe Asn Lys Ile Arg Leu Phe Glu Lys Asn
        660                 665                 670

Ser Lys Lys Tyr Asp Ile Leu Lys Glu Ser Ile Leu Thr Ser Glu Arg
    675                 680                 685

Ile Gly Lys Tyr Phe Ser Leu Leu Asn Thr Gly Ala Lys Tyr Phe Glu
    690                 695                 700

Tyr Gly Gly Glu Glu Asn Arg Gly Ile Phe Asn Lys Asn Ile Ile Ile
705                 710                 715                 720

Pro Ile Phe Lys Tyr Tyr Gln Ile Val Leu Lys Leu Tyr Asn Asp Val
                725                 730                 735

Glu Leu Ala Met Leu Leu Thr Leu Ser Glu Ser Asp Glu Lys Asp Ile
        740                 745                 750

Asn Lys Ile Lys Glu Leu Val Thr Leu Lys Glu Lys Val Ser Pro Lys
    755                 760                 765

Lys Ile Asp Tyr Glu Lys Lys Tyr Lys Phe Ser Val Leu Leu Asp Cys
    770                 775                 780

Phe Asn Arg Ile Ile Asn Leu Gly Lys Lys Asp Phe Leu Ala Ser Glu
785                 790                 795                 800

Glu Val Lys Glu Val Ala Lys Thr Phe Thr Asn Leu Ala Tyr Leu Arg
                805                 810                 815

Asn Lys Ile Cys His Leu Asn Tyr Ser Lys Phe Ile Asp Asp Leu Leu
        820                 825                 830

Thr Ile Asp Thr Asn Lys Ser Thr Thr Asp Ser Glu Gly Lys Leu Leu
    835                 840                 845

Ile Asn Asp Arg Ile Arg Lys Leu Ile Lys Phe Ile Arg Glu Asn Asn
    850                 855                 860

Gln Lys Met Asn Ile Ser Ile Asp Tyr Asn Tyr Ile Asn Asp Tyr Tyr
865                 870                 875                 880

Met Lys Lys Glu Lys Phe Ile Phe Gly Gln Arg Lys Gln Ala Lys Thr
                885                 890                 895

Ile Ile Asp Ser Gly Lys Lys Ala Asn Lys Arg Asn Lys Ala Glu Glu
        900                 905                 910

Leu Leu Lys Met Tyr Arg Val Lys Lys Glu Asn Ile Asn Leu Ile Tyr
        915                 920                 925

Glu Leu Ser Lys Lys Leu Asn Glu Leu Thr Lys Ser Glu Leu Phe Leu
    930                 935                 940

Leu Asp Lys Lys Leu Leu Lys Asp Ile Asp Phe Thr Asp Val Lys Ile
945                 950                 955                 960

Lys Asn Lys Ser Phe Phe Glu Leu Lys Asn Asp Val Lys Glu Val Ala
                965                 970                 975

Asn Ile Lys Gln Ala Leu Gln Lys His Ser Ser Glu Leu Ile Gly Ile
            980                 985                 990

Tyr Lys Lys Glu Val Ile Met Ala  Ile Lys Arg Ser Ile  Val Ser Lys
        995                 1000                 1005

Leu Ile  Tyr Asp Glu Glu Lys  Val Leu Ser Ile Ile  Ile Tyr Asp
    1010                 1015                 1020

Lys Thr  Asn Lys Lys Tyr Glu  Asp Phe Leu Leu Glu  Ile Arg Arg
    1025                 1030                 1035

Glu Arg  Asp Ile Asn Lys Phe  Gln Phe Leu Ile Asp  Glu Lys Lys
    1040                 1045                 1050

Glu Lys  Leu Gly Tyr Glu Lys  Ile Ile Glu Thr Lys  Glu Lys Lys
    1055                 1060                 1065

Lys Val  Val Val Lys Ile Gln  Asn Asn Ser Glu Leu  Val Ser Glu
    1070                 1075                 1080

Pro Arg  Ile Ile Lys Asn Lys  Asp Lys Lys Lys Ala  Lys Thr Pro
    1085                 1090                 1095

Glu Glu  Ile Ser Lys Leu Gly  Ile Leu Asp Leu Thr  Asn His Tyr
    1100                 1105                 1110

Cys Phe  Asn Leu Lys Ile Thr  Leu
    1115                 1120

<210>  109
<211>  30
<212>  DNA
<213>  Archaeoglobus fulgidus

<220>
<221> Unsure
<222> (1)..(30)
<223> DNA coding for Type IA cognate crRNA repeat sequence

<400> 109
gttgaaatca gaccaaaatg ggattgaaag 30


<210> 110
<211> 29
<212> DNA
<213> Clostridium difficile


<220>
<221> Unsure
<222> (1)..(29)
<223> DNA coding for Type IB cognate crRNA repeat sequence

<400> 110
gttttatatt aactaagtgg tatgtaaat 29


<210> 111
<211> 32
<212> DNA
<213> Bacillus halodurans


<220>
<221> Unsure
<222> (1)..(32)
<223> DNA coding for Type IC cognate crRNA repeat sequence

<400> 111
gtcgcactct acacgagtgc gtggattgaa at 32


<210> 112
<211> 37
<212> DNA
<213> Cyanothece sp


<220>
<221> Unsure
<222> (1)..(37)
<223> DNA coding for Type ID cognate crRNA repeat sequence

<400> 112
gtttcaatcc cattactagg attcattaaa aagaaac 37


<210> 113
<211> 29
<212> DNA
<213> Escherichia coli


<220>
<221> Unsure
<222> (1)..(29)

<223>  DNA coding for Type IE cognate crRNA repeat sequence

<400>  113
cggtttatcc ccgctggcgc ggggaactc                                          29


<210>  114
<211>  29
<212>  DNA
<213>  Yersinia pseudotuberculosis


<220>
<221>  Unsure
<222>  (1)..(29)
<223>  DNA coding for Type IF cognate crRNA repeat sequence

<400>  114
cggtttatcc ccgctggcgc ggggaactc                                          29


<210>  115
<211>  28
<212>  DNA
<213>  Shewanella putrefaciens


<220>
<221>  Unsure
<222>  (1)..(28)
<223>  DNA coding for Type IF´ cognate crRNA repeat sequence

<400>  115
gttcaccgcc gcacaggcgg cttagaaa                                           28


<210>  116
<211>  32
<212>  DNA
<213>  Streptococcus pyogenes


<220>
<221>  Unsure
<222>  (1)..(32)
<223>  DNA coding for Type IIA cognate crRNA repeat sequence

<400>  116
atttcaatcc actcacccat gaagggtgag ac                                      32


<210>  117
<211>  37
<212>  DNA
<213>  Legionella pneumophila


<220>
<221>  Unsure
<222>  (1)..(37)
<223>  DNA coding for Type IIB cognate crRNA repeat sequence

<400>  117

ccaataatcc ctcatctaaa aatccaacca ctgaaac 37

<210> 118
<211> 26
<212> DNA
<213> Neisseria meningitidis

<220>
<221> Unsure
<222> (1)..(26)
<223> DNA coding for Type IIC cognate crRNA repeat sequence

<400> 118
gattcccgcg ttcgcgggaa tgacgg 26

<210> 119
<211> 36
<212> DNA
<213> Staphylococcus epidermidis

<220>
<221> Unsure
<222> (1)..(36)
<223> DNA coding for Type IIIA cognate crRNA repeat sequence

<400> 119
gatcgatacc caccccgaag aaaaggggac gagaac 36

<210> 120
<211> 30
<212> DNA
<213> Pyrococcus furiosus

<220>
<221> Unsure
<222> (1)..(30)
<223> DNA coding for Type IIIB cognate crRNA repeat sequence

<400> 120
gttccaataa gactaaaata gaattgaaag 30

<210> 121
<211> 35
<212> DNA
<213> Marinomonas mediterranea

<220>
<221> Unsure
<222> (1)..(35)
<223> DNA coding for Type IIIB* cognate crRNA repeat sequence

<400> 121
gtctcaacgg cctaagcggc cagcgggtct gaaac 35

<210>  122
<211>  30
<212>  DNA
<213>  Methanothermobacter thermautotrophicus

<220>
<221>  Unsure
<222>  (1)..(30)
<223>  DNA coding for Type IIIC cognate crRNA repeat sequence

<400>  122
atttcaatcc cattttggtc tgattttaac                                        30

<210>  123
<211>  37
<212>  DNA
<213>  Synechocystis spp

<220>
<221>  Unsure
<222>  (1)..(37)
<223>  DNA coding for Type IIID cognate crRNA repeat sequence

<400>  123
ctttccttct actaatcccg gcgatcggga ctgaaac                                37

<210>  124
<211>  36
<212>  DNA
<213>  Thioalkalivibrio sp.

<220>
<221>  Unsure
<222>  (1)..(36)
<223>  DNA coding for Type IV cognate crRNA repeat sequence

<400>  124
gtggaaagca cgtccctgtg ggggcgtggt tggaac                                  36

<210>  125
<211>  36
<212>  DNA
<213>  Francisella cf. novicida Fx1

<220>
<221>  Unsure
<222>  (1)..(36)
<223>  DNA coding for TypeV A cognate crRNA repeat sequence

<400>  125
atctacaaca gtagaaatta tttaaagttc ttagac                                 36

<210>  126
<211>  36
<212>  DNA

<213>  Alicyclobacillus acidoterrestris

<220>
<221>  Unsure
<222>  (1)..(36)
<223>  DNA coding for TypeV B cognate crRNA repeat sequence

<400>  126
gtcggatcac tgagcgagcg atctgagaag tggcac                                36

<210>  127
<211>  26
<212>  DNA
<213>  Oleiphilus sp

<220>
<221>  Unsure
<222>  (1)..(26)
<223>  DNA coding for TypeV C cognate crRNA repeat sequence

<400>  127
agaattactg atgttgtgat gaaggc                                           26

<210>  128
<211>  26
<212>  DNA
<213>  Bacterium CG09_39_24

<220>
<221>  Unsure
<222>  (1)..(26)
<223>  DNA coding for TypeV D cognate crRNA repeat sequence

<400>  128
actccgaaag tatcggggat aaaggc                                           26

<210>  129
<211>  37
<212>  DNA
<213>  Deltaproteobacteria bacterium

<220>
<221>  Unsure
<222>  (1)..(37)
<223>  DNA coding for TypeV E cognate crRNA repeat sequence

<400>  129
atttgaaggt atctccgata agtaaaacgc atcaaag                               37

<210>  130
<211>  36
<212>  DNA
<213>  Leptotrichia shahii DSM 19757

<220>
<221>  Unsure
<222>  (1)..(36)
<223>  DNA coding for TypeVI A cognate crRNA repeat sequence

<400>  130
gttttagtcc ccttcgatat tggggtggtc tatatc                                36


<210>  131
<211>  36
<212>  DNA
<213>  Prevotella buccae


<220>
<221>  Unsure
<222>  (1)..(36)
<223>  DNA coding for TypeVI B1 cognate crRNA repeat sequence

<400>  131
gttgcatctg ccttcttttt gaaaggtaaa aacaac                                36


<210>  132
<211>  29
<212>  DNA
<213>  Bergeyella zoohelcum


<220>
<221>  Unsure
<222>  (1)..(29)
<223>  DNA coding for TypeVI B2 cognate crRNA repeat sequence

<400>  132
attctaatcg caccataagg aattgaaac                                        29


<210>  133
<211>  30
<212>  DNA
<213>  Fusobacterium perfoetens


<220>
<221>  Unsure
<222>  (1)..(30)
<223>  DNA coding for TypeVI C cognate crRNA repeat sequence

<400>  133
gactaaaacc aagaaaattg gtatttaaac                                       30


<210>  134
<211>  91
<212>  DNA
<213>  Streptococcus pyogenes


<220>
<221>  gene
<222>  (1)..(91)

<223>  DNA coding for TypeIIA cognate tracRNA

<400>  134
ttgttggaac cattcaaaac agcatagcaa gttaaaataa ggctagtccg ttatcaactt      60

gaaaaagtgg caccgagtcg gtgctttttt t                                     91


<210>  135
<211>  101
<212>  DNA
<213>  Legionella pneumophila


<220>
<221>  gene
<222>  (1)..(101)
<223>  DNA coding for TypeIIB cognate tracRNA

<400>  135
ttaattttgt tgatttcagg gtatcgaaat ttagatgatt tctaatttaa ttattgcctt      60

ctttttcgaa aaatcaagcc taaataatgc aatttgctat t                         101


<210>  136
<211>  109
<212>  DNA
<213>  Neisseria meningitidis


<220>
<221>  gene
<222>  (1)..(109)
<223>  DNA coding for Type II-C cognate tracRNA

<400>  136
acatattgtc gcactgcgaa atgagaaccg ttgctacaat aaggccgtct gaaaagatgt      60

gccgcaacgc tctgcccctt aaagcttctg ctttaagggg catcgttta                 109


<210>  137
<211>  103
<212>  DNA
<213>  Alicyclobacillus acidoterrestris


<220>
<221>  gene
<222>  (1)..(103)
<223>  DNA coding for TypeV-B cognate tracRNA

<400>  137
agaacgctcg ctcagtgttc tgacctttcg agcgcctgtt cagggcgaaa accctgggag      60

gcgctcgaat cataggtggg acaagggatt cgcggcgaaa acg                        103


<210>  138
<211>  91
<212>  DNA
<213>  Deltaproteobacteria bacterium

<220>
<221>  gene
<222>  (1)..(91)
<223>  DNA coding for TypeV-E cognate tracRNA

<400>  138
cttatttatc ggagatagct ccgggtgcaa actcggagct gtttttttac gaaacagcta      60

attttagcca aaagttcttt gaaaacctga t                                     91


<210>  139
<211>  99
<212>  DNA
<213>  Synthetic


<220>
<221>  promoter
<222>  (1)..(99)
<223>  PLtetO-1 (lacking repressor) + RBS

<400>  139
tccctatcag tgatagagat tgacatccct atcagtgata gagatactga gcacatcagc      60

aggacgcact gaccgaattc attaaagagg agaaaggta                             99


<210>  140
<211>  717
<212>  DNA
<213>  Synthetic


<220>
<221>  CDS
<222>  (1)..(717)
<223>  gfp gene encoding Green Fluorescent Protein

<400>  140
atg cgt aaa ggc gaa gag ctg ttc act ggt gtc gtc cct att ctg gtg       48
Met Arg Lys Gly Glu Glu Leu Phe Thr Gly Val Val Pro Ile Leu Val
1               5                   10                  15

gaa ctg gat ggt gat gtc aac ggt cat aag ttt tcc gtg cgt ggc gag       96
Glu Leu Asp Gly Asp Val Asn Gly His Lys Phe Ser Val Arg Gly Glu
            20                  25                  30

ggt gaa ggt gac gca act aat ggt aaa ctg acg ctg aag ttc atc tgt      144
Gly Glu Gly Asp Ala Thr Asn Gly Lys Leu Thr Leu Lys Phe Ile Cys
        35                  40                  45

act act ggt aaa ctg ccg gta cct tgg ccg act ctg gta acg acg ctg      192
Thr Thr Gly Lys Leu Pro Val Pro Trp Pro Thr Leu Val Thr Thr Leu
    50                  55                  60

act tat ggt gtt cag tgc ttt gct cgt tat ccg gac cat atg aag cag      240
Thr Tyr Gly Val Gln Cys Phe Ala Arg Tyr Pro Asp His Met Lys Gln
65                  70                  75                  80

cat gac ttc ttc aag tcc gcc atg ccg gaa ggc tat gtg cag gaa cgc      288

His Asp Phe Phe Lys Ser Ala Met Pro Glu Gly Tyr Val Gln Glu Arg
                85                  90                  95

acg att tcc ttt aag gat gac ggc acg tac aaa acg cgt gcg gaa gtg      336
Thr Ile Ser Phe Lys Asp Asp Gly Thr Tyr Lys Thr Arg Ala Glu Val
            100                 105                 110

aaa ttt gaa ggc gat acc ctg gta aac cgc att gag ctg aaa ggc att      384
Lys Phe Glu Gly Asp Thr Leu Val Asn Arg Ile Glu Leu Lys Gly Ile
        115                 120                 125

gac ttt aaa gaa gac ggc aat atc ctg ggc cat aag ctg gaa tac aat      432
Asp Phe Lys Glu Asp Gly Asn Ile Leu Gly His Lys Leu Glu Tyr Asn
    130                 135                 140

ttt aac agc cac aat gtt tac atc acc gcc gat aaa caa aaa aat ggc      480
Phe Asn Ser His Asn Val Tyr Ile Thr Ala Asp Lys Gln Lys Asn Gly
145                 150                 155                 160

att aaa gcg aat ttt aaa att cgc cac aac gtg gag gat ggc agc gtg      528
Ile Lys Ala Asn Phe Lys Ile Arg His Asn Val Glu Asp Gly Ser Val
                165                 170                 175

cag ctg gct gat cac tac cag caa aac act cca atc ggt gat ggt cct      576
Gln Leu Ala Asp His Tyr Gln Gln Asn Thr Pro Ile Gly Asp Gly Pro
            180                 185                 190

gtt ctg ctg cca gac aat cac tat ctg agc acg caa agc gtt ctg tct      624
Val Leu Leu Pro Asp Asn His Tyr Leu Ser Thr Gln Ser Val Leu Ser
        195                 200                 205

aaa gat ccg aac gag aaa cgc gat cat atg gtt ctg ctg gag ttc gta      672
Lys Asp Pro Asn Glu Lys Arg Asp His Met Val Leu Leu Glu Phe Val
    210                 215                 220

acc gca gcg ggc atc acg cat ggt atg gat gaa ctg tac aaa tga          717
Thr Ala Ala Gly Ile Thr His Gly Met Asp Glu Leu Tyr Lys
225                 230                 235

<210>  141
<211>  238
<212>  PRT
<213>  Synthetic

<400>  141

Met Arg Lys Gly Glu Glu Leu Phe Thr Gly Val Val Pro Ile Leu Val
1               5                   10                  15

Glu Leu Asp Gly Asp Val Asn Gly His Lys Phe Ser Val Arg Gly Glu
            20                  25                  30

Gly Glu Gly Asp Ala Thr Asn Gly Lys Leu Thr Leu Lys Phe Ile Cys
        35                  40                  45

Thr Thr Gly Lys Leu Pro Val Pro Trp Pro Thr Leu Val Thr Thr Leu
    50                  55                  60

Thr Tyr Gly Val Gln Cys Phe Ala Arg Tyr Pro Asp His Met Lys Gln
65                  70                  75                  80

His Asp Phe Phe Lys Ser Ala Met Pro Glu Gly Tyr Val Gln Glu Arg
                85                  90                  95

Thr Ile Ser Phe Lys Asp Asp Gly Thr Tyr Lys Thr Arg Ala Glu Val
            100                 105                 110

Lys Phe Glu Gly Asp Thr Leu Val Asn Arg Ile Glu Leu Lys Gly Ile
        115                 120                 125

Asp Phe Lys Glu Asp Gly Asn Ile Leu Gly His Lys Leu Glu Tyr Asn
    130                 135                 140

Phe Asn Ser His Asn Val Tyr Ile Thr Ala Asp Lys Gln Lys Asn Gly
145                 150                 155                 160

Ile Lys Ala Asn Phe Lys Ile Arg His Asn Val Glu Asp Gly Ser Val
                165                 170                 175

Gln Leu Ala Asp His Tyr Gln Gln Asn Thr Pro Ile Gly Asp Gly Pro
            180                 185                 190

Val Leu Leu Pro Asp Asn His Tyr Leu Ser Thr Gln Ser Val Leu Ser
        195                 200                 205

Lys Asp Pro Asn Glu Lys Arg Asp His Met Val Leu Leu Glu Phe Val
    210                 215                 220

Thr Ala Ala Gly Ile Thr His Gly Met Asp Glu Leu Tyr Lys
225                 230                 235

<210>  142
<211>  142
<212>  DNA
<213>  Synthetic

<220>
<221>  terminator
<222>  (1)..(142)
<223>  rrnB T1 terminator

<400>  142
ctgcagcccg ggggatccca tggtacgcgt gctagaggca tcaaataaaa cgaaaggctc      60

agtcgaaaga ctgggccttt cgttttatct gttgtttgtc ggtgaacgct ctcctgagta     120

ggacaaatcc gccgccctag ac                                              142

<210>  143
<211>  919
<212>  DNA
<213>  Synthetic

<220>
<221>  rep_origin
<222>  (1)..(919)
<223>  ColE1origin of replication + terminator rrnaB

<400>  143
ctaggcgttc ggctgcggcg agcggtatca gctcactcaa aggcggtaat acggttatcc      60

acagaatcag gggataacgc aggaaagaac atgtgagcaa aaggccagca aaaggccagg     120

aaccgtaaaa aggccgcgtt gctggcgttt ttccataggc tccgcccccc tgacgagcat     180

cacaaaaatc gacgctcaag tcagaggtgg cgaaacccga caggactata aagataccag     240

gcgtttcccc ctggaagctc cctcgtgcgc tctcctgttc cgaccctgcc gcttaccgga     300

tacctgtccg cctttctccc ttcgggaagc gtggcgcttt ctcaatgctc acgctgtagg     360

tatctcagtt cggtgtaggt cgttcgctcc aagctgggct gtgtgcacga accccccgtt     420

cagcccgacc gctgcgcctt atccggtaac tatcgtcttg agtccaaccc ggtaagacac     480

gacttatcgc cactggcagc agccactggt aacaggatta gcagagcgag gtatgtaggc     540

ggtgctacag agttcttgaa gtggtggcct aactacggct acactagaag gacagtattt     600

ggtatctgcg ctctgctgaa gccagttacc ttcggaaaaa gagttggtag ctcttgatcc     660

ggcaaacaaa ccaccgctgg tagcggtggt ttttttgttt gcaagcagca gattacgcgc     720

agaaaaaaag gatctcaaga agatcctttg atcttttcta cggggtctga cgctcagtgg     780

aacgaaaact cacgttaagg gattttggtc atgactagtg cttggattct caccaataaa     840

aaacgcccgg cggcaaccga gcgttctgaa caaatccaga tggagttctg aggtcattac     900

tggatctatc aacaggagt                                                  919

<210>  144
<211>  795
<212>  DNA
<213>  Synthetic

<220>
<221>  CDS
<222>  (1)..(795)
<223>  Kanamycin resistance gene

<400>  144
atg att gaa caa gat gga ttg cac gca ggt tct ccg gcc gct tgg gtg      48
gag agg cta ttc ggc tat gac tgg gca caa cag aca atc ggc tgc tct      96
gat gcc gcc gtg ttc cgg ctg tca gcg cag ggg cgc ccg gtt ctt ttt 144
gtc aag acc gac ctg tcc ggt gcc ctg aat gaa ctg cag gac gag gca 192
gcg cgg cta tcg tgg ctg gcc acg acg ggc gtt cct tgc gca gct gtg 240
ctc gac gtt gtc act gaa gcg gga agg gac tgg ctg cta ttg ggc gaa 288
gtg ccg ggg cag gat ctc ctg tca tct cac ctt gct cct gcc gag aaa 336
gta tcc atc atg gct gat gca atg cgg cgg ctg cat acg ctt gat ccg 384
gct acc tgc cca ttc gac cac caa gcg aaa cat cgc atc gag cga gca 432
cgt act cgg atg gaa gcc ggt ctt gtc gat cag gat gat ctg gac gaa 480
gag cat cag ggg ctc gcg cca gcc gaa ctg ttc gcc agg ctc aag gcg 528
cgc atg ccc gac ggc gag gat ctc gtc gtg acc cat ggc gat gcc tgc 576
ttg ccg aat atc atg gtg gaa aat ggc cgc ttt tct gga ttc atc gac 624
tgt ggc cgg ctg ggt gtg gcg gac cgc tat cag gac ata gcg ttg gct 672
acc cgt gat att gct gaa gag ctt ggc ggc gaa tgg gct gac cgc ttc 720
ctc gtg ctt tac ggt atc gcc gct ccc gat tcg cag cgc atc gcc ttc 768
tat cgc ctt ctt gac gag ttc ttc tga 795

<210>  145
<211>  264
<212>  PRT
<213>  Synthetic

<400>  145

<210>  146
<211>  372
<212>  DNA
<213>  Listeria monocytogene prophage

<220>
<221>  CDS
<222>  (1)..(372)
<223>  AcrIIA2 gene encoding an acrIIA2 anti-CRISPR protein

<400>  146
atg aca tta aca aga gca caa aaa aag tat gca gaa gcg atg cac gag        48
Met Thr Leu Thr Arg Ala Gln Lys Lys Tyr Ala Glu Ala Met His Glu
1               5                   10                  15

ttt ata aat atg gtg gat gac ttt gaa gag tct aca ccg gat ttt gca        96
Phe Ile Asn Met Val Asp Asp Phe Glu Glu Ser Thr Pro Asp Phe Ala
            20                  25                  30

aag gaa gtt cta cat gat tct gac tat gta gtt att aca aaa aat gaa        144
Lys Glu Val Leu His Asp Ser Asp Tyr Val Val Ile Thr Lys Asn Glu
        35                  40                  45

aaa tat gca gta gct ctt tgc tct ctt agc act gat gaa tgt gaa tat        192
Lys Tyr Ala Val Ala Leu Cys Ser Leu Ser Thr Asp Glu Cys Glu Tyr
    50                  55                  60

gat act aac tta tac tta gat gaa aaa ttg gtt gat tac tca aca gtt        240
Asp Thr Asn Leu Tyr Leu Asp Glu Lys Leu Val Asp Tyr Ser Thr Val
65                  70                  75                  80

gac gta aac ggt gtg aca tac tac atc aac att gtt gaa act aac gat        288
Asp Val Asn Gly Val Thr Tyr Tyr Ile Asn Ile Val Glu Thr Asn Asp
                85                  90                  95

atc gat gat tta gaa atc gct acg gat gaa gat gag atg aaa agt ggc        336
Ile Asp Asp Leu Glu Ile Ala Thr Asp Glu Asp Glu Met Lys Ser Gly
            100                 105                 110

aac caa gaa att att tta aaa agt gag ttg aag taa                        372
Asn Gln Glu Ile Ile Leu Lys Ser Glu Leu Lys
        115                 120

<210>  147
<211>  123
<212>  PRT
<213>  Listeria monocytogene prophage

<400>  147

Met Thr Leu Thr Arg Ala Gln Lys Lys Tyr Ala Glu Ala Met His Glu
1               5                   10                  15

Phe Ile Asn Met Val Asp Asp Phe Glu Glu Ser Thr Pro Asp Phe Ala
            20                  25                  30

Lys Glu Val Leu His Asp Ser Asp Tyr Val Val Ile Thr Lys Asn Glu
        35                  40                  45

Lys Tyr Ala Val Ala Leu Cys Ser Leu Ser Thr Asp Glu Cys Glu Tyr
    50                  55                  60

Asp Thr Asn Leu Tyr Leu Asp Glu Lys Leu Val Asp Tyr Ser Thr Val
65                  70                  75                  80

Asp Val Asn Gly Val Thr Tyr Tyr Ile Asn Ile Val Glu Thr Asn Asp
                85                  90                  95

Ile Asp Asp Leu Glu Ile Ala Thr Asp Glu Asp Glu Met Lys Ser Gly
            100                 105                 110

Asn Gln Glu Ile Ile Leu Lys Ser Glu Leu Lys
        115                 120

<210>  148
<211>  64
<212>  DNA
<213>  Synthetic

<220>
<221>  promoter
<222>  (1)..(35)
<223>  Constitutive promoter BBaJ23100

<400>  148
ttgacggcta gctcagtcct aggtacagtg ctagctacta gagaagtcta gcgaaccgca      60

ctta                                                                    64

<210>  149
<211>  81
<212>  DNA
<213>  Synthetic

<220>
<221>  misc_structure
<222>  (1)..(81)
<223>  DNA sequence theophyllin riboswitch

<400>  149
atacgactca ctataggtac cggtgatacc agcatcgtct tgatgccctt ggcagcaccc      60

tgctaaggta acaacaagat g                                                81

<210>  150
<211>  4107
<212>  DNA
<213>  Streptococcus pyogenes

<220>
<221>  CDS
<222>  (1)..(4107)
<223>  Cas9 gene

<400>  150
atg gat aag aaa tac tca ata ggc tta gat atc ggc aca aat agc gtc       48
gga tgg gcg gtg atc act gat gaa tat aag gtt ccg tct aaa aag ttc       96
aag gtt ctg gga aat aca gac cgc cac agt atc aaa aaa aat ctt ata      144
ggg gct ctt tta ttt gac agt gga gag aca gcg gaa gcg act cgt ctc      192
aaa cgg aca gct cgt aga agg tat aca cgt cgg aag aat cgt att tgt      240
tat cta cag gag att ttt tca aat gag atg gcg aaa gta gat gat agt      288
ttc ttt cat cga ctt gaa gag tct ttt ttg gtg gaa gaa gac aag aag      336
cat gaa cgt cat cct att ttt gga aat ata gta gat gaa gtt gct tat      384
cat gag aaa tat cca act atc tat cat ctg cga aaa aaa ttg gta gat      432
tct act gat aaa gcg gat ttg cgc tta atc tat ttg gcc tta gcg cat      480
atg att aag ttt cgt ggt cat ttt ttg att gag gga gat tta aat cct      528
gat aat agt gat gtg gac aaa cta ttt atc cag ttg gta caa acc tac      576
aat caa tta ttt gaa gaa aac cct att aac gca agt gga gta gat gct 624
aaa gcg att ctt tct gca cga ttg agt aaa tca aga cga tta gaa aat 672
ctc att gct cag ctc ccc ggt gag aag aaa aat ggc tta ttt ggg aat 720
ctc att gct ttg tca ttg ggt ttg acc cct aat ttt aaa tca aat ttt 768
gat ttg gca gaa gat gct aaa tta cag ctt tca aaa gat act tac gat 816
gat gat tta gat aat tta ttg gcg caa att gga gat caa tat gct gat 864
ttg ttt ttg gca gct aag aat tta tca gat gct att tta ctt tca gat 912
atc cta aga gta aat act gaa ata act aag gct ccc cta tca gct tca 960
atg att aaa cgc tac gat gaa cat cat caa gac ttg act ctt tta aaa 1008
gct tta gtt cga caa caa ctt cca gaa aag tat aaa gaa atc ttt ttt 1056
gat caa tca aaa aac gga tat gca ggt tat att gat ggg gga gct agc 1104
caa gaa gaa ttt tat aaa ttt atc aaa cca att tta gaa aaa atg gat 1152
ggt act gag gaa tta ttg gtg aaa cta aat cgt gaa gat ttg ctg cgc 1200
aag caa cgg acc ttt gac aac ggc tct att ccc cat caa att cac ttg 1248
ggt gag ctg cat gct att ttg aga aga caa gaa gac ttt tat cca ttt 1296
tta aaa gac aat cgt gag aag att gaa aaa atc ttg act ttt cga att 1344
cct tat tat gtt ggt cca ttg gcg cgt ggc aat agt cgt ttt gca tgg         1392
atg act cgg aag tct gaa gaa aca att acc cca tgg aat ttt gaa gaa         1440
gtt gtc gat aaa ggt gct tca gct caa tca ttt att gaa cgc atg aca         1488
aac ttt gat aaa aat ctt cca aat gaa aaa gta cta cca aaa cat agt         1536
ttg ctt tat gag tat ttt acg gtt tat aac gaa ttg aca aag gtc aaa         1584
tat gtt act gaa gga atg cga aaa cca gca ttt ctt tca ggt gaa cag         1632
aag aaa gcc att gtt gat tta ctc ttc aaa aca aat cga aaa gta acc         1680
gtt aag caa tta aaa gaa gat tat ttc aaa aaa ata gaa tgt ttt gat         1728
agt gtt gaa att tca gga gtt gaa gat aga ttt aat gct tca tta ggt         1776
acc tac cat gat ttg cta aaa att att aaa gat aaa gat ttt ttg gat         1824
aat gaa gaa aat gaa gat atc tta gag gat att gtt tta aca ttg acc         1872
tta ttt gaa gat agg gag atg att gag gaa aga ctt aaa aca tat gct         1920
cac ctc ttt gat gat aag gtg atg aaa cag ctt aaa cgt cgc cgt tat         1968
act ggt tgg gga cgt ttg tct cga aaa ttg att aat ggt att agg gat         2016
aag caa tct ggc aaa aca ata tta gat ttt ttg aaa tca gat ggt ttt         2064
gcc aat cgc aat ttt atg cag ctg atc cat gat gat agt ttg aca ttt         2112
aaa gaa gac att caa aaa gca caa gtg tct gga caa ggc gat agt tta 2160
cat gaa cat att gca aat tta gct ggt agc cct gct att aaa aaa ggt 2208
att tta cag act gta aaa gtt gtt gat gaa ttg gtc aaa gta atg ggg 2256
cgg cat aag cca gaa aat atc gtt att gaa atg gca cgt gaa aat cag 2304
aca act caa aag ggc cag aaa aat tcg cga gag cgt atg aaa cga atc 2352
gaa gaa ggt atc aaa gaa tta gga agt cag att ctt aaa gag cat cct 2400
gtt gaa aat act caa ttg caa aat gaa aag ctc tat ctc tat tat ctc 2448
caa aat gga aga gac atg tat gtg gac caa gaa tta gat att aat cgt 2496
tta agt gat tat gat gtc gat cac att gtt cca caa agt ttc ctt aaa 2544
gac gat tca ata gac aat aag gtc tta acg cgt tct gat aaa aat cgt 2592
ggt aaa tcg gat aac gtt cca agt gaa gaa gta gtc aaa aag atg aaa 2640
aac tat tgg aga caa ctt cta aac gcc aag tta atc act caa cgt aag 2688
ttt gat aat tta acg aaa gct gaa cgt gga ggt ttg agt gaa ctt gat 2736
aaa gct ggt ttt atc aaa cgc caa ttg gtt gaa act cgc caa atc act 2784
aag cat gtg gca caa att ttg gat agt cgc atg aat act aaa tac gat 2832
gaa aat gat aaa ctt att cga gag gtt aaa gtg att acc tta aaa tct 2880
aaa tta gtt tct gac ttc cga aaa gat ttc caa ttc tat aaa gta cgt 2928
gag att aac aat tac cat cat gcc cat gat gcg tat cta aat gcc gtc 2976
gtt gga act gct ttg att aag aaa tat cca aaa ctt gaa tcg gag ttt 3024
gtc tat ggt gat tat aaa gtt tat gat gtt cgt aaa atg att gct 3069
aag tct gag caa gaa ata ggc aaa gca acc gca aaa tat ttc ttt 3114
tac tct aat atc atg aac ttc ttc aaa aca gaa att aca ctt gca 3159
aat gga gag att cgc aaa cgc cct cta atc gaa act aat ggg gaa 3204
act gga gaa att gtc tgg gat aaa ggg cga gat ttt gcc aca gtg 3249
cgc aaa gta ttg tcc atg ccc caa gtc aat att gtc aag aaa aca 3294
gaa gta cag aca ggc gga ttc tcc aag gag tca att tta cca aaa 3339
aga aat tcg gac aag ctt att gct cgt aaa aaa gac tgg gat cca 3384
aaa aaa tat ggt ggt ttt gat agt cca acg gta gct tat tca gtc 3429
cta gtg gtt gct aag gtg gaa aaa ggg aaa tcg aag aag tta aaa 3474
tcc gtt aaa gag tta cta ggg atc aca att atg gaa aga agt tcc 3519
ttt gaa aaa aat ccg att gac ttt tta gaa gct aaa gga tat aag 3564
gaa gtt aaa aaa gac tta atc att aaa cta cct aaa tat agt ctt 3609
ttt gag tta gaa aac ggt cgt aaa cgg atg ctg gct agt gcc gga 3654
gaa tta caa aaa gga aat gag ctg gct ctg cca agc aaa tat gtg 3699
aat ttt tta tat tta gct agt cat tat gaa aag ttg aag ggt agt 3744
cca gaa gat aac gaa caa aaa caa ttg ttt gtg gag cag cat aag 3789
cat tat tta gat gag att att gag caa atc agt gaa ttt tct aag 3834
cgt gtt att tta gca gat gcc aat tta gat aaa gtt ctt agt gca 3879
tat aac aaa cat aga gac aaa cca ata cgt gaa caa gca gaa aat 3924
att att cat tta ttt acg ttg acg aat ctt gga gct ccc gct gct 3969
ttt aaa tat ttt gat aca aca att gat cgt aaa cga tat acg tct 4014
aca aaa gaa gtt tta gat gcc act ctt atc cat caa tcc atc act 4059
ggt ctt tat gaa aca cgc att gat ttg agt cag cta gga ggt gac 4104
tga 4107

<210> 151
<211> 1368
<212> PRT
<213> Streptococcus pyogenes

<400> 151

<210>  152
<211>  42
<212>  DNA
<213>  Streptococcus pyogenes

<220>
<221>  terminator
<222>  (1)..(42)
<223>  Native terminator for Cas9 gene

<400>  152
aaaaaaacaa gcgctttcaa aacgcttgtt ttatcatttt ta                          42

<210>  153
<211>  789
<212>  DNA
<213>  Escherichia coli

<220>
<221>  CDS
<222>  (1)..(789)
<223>  aadA spectinomycin resistance gene

<400>  153
atg agg gaa gcg gtg atc gcc gaa gta tcg act caa cta tca gag gta        48
Met Arg Glu Ala Val Ile Ala Glu Val Ser Thr Gln Leu Ser Glu Val
1               5                   10                  15

gtt ggc gtc atc gag cgc cat ctc gaa ccg acg ttg ctg gcc gta cat        96
Val Gly Val Ile Glu Arg His Leu Glu Pro Thr Leu Leu Ala Val His
            20                  25                  30

ttg tac ggc tcc gca gtg gat ggc ggc ctg aag cca cac agt gat att 144
Leu Tyr Gly Ser Ala Val Asp Gly Gly Leu Lys Pro His Ser Asp Ile
        35                  40                  45

gat ttg ctg gtt acg gtg acc gta agg ctt gat gaa aca acg cgg cga 192
Asp Leu Leu Val Thr Val Thr Val Arg Leu Asp Glu Thr Thr Arg Arg
    50                  55                  60

gct ttg atc aac gac ctt ttg gaa act tcg gct tcc cct gga gag agc 240
Ala Leu Ile Asn Asp Leu Leu Glu Thr Ser Ala Ser Pro Gly Glu Ser
65                  70                  75                  80

gag att ctc cgc gct gta gaa gtc acc att gtt gtg cac gac gac atc 288
Glu Ile Leu Arg Ala Val Glu Val Thr Ile Val Val His Asp Asp Ile
                85                  90                  95

att ccg tgg cgt tat cca gct aag cgc gaa ctg caa ttt gga gaa tgg 336
Ile Pro Trp Arg Tyr Pro Ala Lys Arg Glu Leu Gln Phe Gly Glu Trp
            100                 105                 110

cag cgc aat gac att ctt gca ggt atc ttc gag cca gcc acg atc gac 384
Gln Arg Asn Asp Ile Leu Ala Gly Ile Phe Glu Pro Ala Thr Ile Asp
        115                 120                 125

att gat ctg gct atc ttg ctg aca aaa gca aga gaa cat agc gtt gcc 432
Ile Asp Leu Ala Ile Leu Leu Thr Lys Ala Arg Glu His Ser Val Ala
    130                 135                 140

ttg gta ggt cca gcg gcg gag gaa ctc ttt gat ccg gtt cct gaa cag 480
Leu Val Gly Pro Ala Ala Glu Glu Leu Phe Asp Pro Val Pro Glu Gln
145                 150                 155                 160

gat cta ttt gag gcg cta aat gaa acc tta acg cta tgg aac tcg ccg 528
Asp Leu Phe Glu Ala Leu Asn Glu Thr Leu Thr Leu Trp Asn Ser Pro
                165                 170                 175

ccc gac tgg gct ggc gat gag cga aat gta gtg ctt acg ttg tcc cgc 576
Pro Asp Trp Ala Gly Asp Glu Arg Asn Val Val Leu Thr Leu Ser Arg
            180                 185                 190

att tgg tac agc gca gta acc ggc aaa atc gcg ccg aag gat gtc gct 624
Ile Trp Tyr Ser Ala Val Thr Gly Lys Ile Ala Pro Lys Asp Val Ala
        195                 200                 205

gcc gac tgg gca atg gag cgc ctg ccg gcc cag tat cag ccc gtc ata 672
Ala Asp Trp Ala Met Glu Arg Leu Pro Ala Gln Tyr Gln Pro Val Ile
    210                 215                 220

ctt gaa gct aga cag gct tat ctt gga caa gaa gaa gat cgc ttg gcc 720
Leu Glu Ala Arg Gln Ala Tyr Leu Gly Gln Glu Glu Asp Arg Leu Ala
225                 230                 235                 240

tcg cgc gca gat cag ttg gaa gaa ttt gtc cac tac gtg aaa ggc gag 768
Ser Arg Ala Asp Gln Leu Glu Glu Phe Val His Tyr Val Lys Gly Glu
                245                 250                 255

atc acc aag gta gtc ggc aaa 789
Ile Thr Lys Val Val Gly Lys
            260

<210> 154

<211>  263
<212>  PRT
<213>  Escherichia coli

<400>  154

Met Arg Glu Ala Val Ile Ala Glu Val Ser Thr Gln Leu Ser Glu Val
1               5                   10                  15

Val Gly Val Ile Glu Arg His Leu Glu Pro Thr Leu Leu Ala Val His
            20                  25                  30

Leu Tyr Gly Ser Ala Val Asp Gly Gly Leu Lys Pro His Ser Asp Ile
        35                  40                  45

Asp Leu Leu Val Thr Val Thr Val Arg Leu Asp Glu Thr Thr Arg Arg
    50                  55                  60

Ala Leu Ile Asn Asp Leu Leu Glu Thr Ser Ala Ser Pro Gly Glu Ser
65                  70                  75                  80

Glu Ile Leu Arg Ala Val Glu Val Thr Ile Val Val His Asp Asp Ile
                85                  90                  95

Ile Pro Trp Arg Tyr Pro Ala Lys Arg Glu Leu Gln Phe Gly Glu Trp
            100                 105                 110

Gln Arg Asn Asp Ile Leu Ala Gly Ile Phe Glu Pro Ala Thr Ile Asp
        115                 120                 125

Ile Asp Leu Ala Ile Leu Leu Thr Lys Ala Arg Glu His Ser Val Ala
    130                 135                 140

Leu Val Gly Pro Ala Ala Glu Glu Leu Phe Asp Pro Val Pro Glu Gln
145                 150                 155                 160

Asp Leu Phe Glu Ala Leu Asn Glu Thr Leu Thr Leu Trp Asn Ser Pro
                165                 170                 175

Pro Asp Trp Ala Gly Asp Glu Arg Asn Val Val Leu Thr Leu Ser Arg
            180                 185                 190

Ile Trp Tyr Ser Ala Val Thr Gly Lys Ile Ala Pro Lys Asp Val Ala
        195                 200                 205

Ala Asp Trp Ala Met Glu Arg Leu Pro Ala Gln Tyr Gln Pro Val Ile
    210                 215                 220

Leu Glu Ala Arg Gln Ala Tyr Leu Gly Gln Glu Glu Asp Arg Leu Ala

225                 230                 235                 240

Ser Arg Ala Asp Gln Leu Glu Glu Phe Val His Tyr Val Lys Gly Glu
                245                 250                 255

Ile Thr Lys Val Val Gly Lys
            260

<210>  155
<211>  1221
<212>  DNA
<213>  Synthetic

<220>
<221>  rep_origin
<222>  (1)..(1221)
<223>  pSC101 origin of replication

<400>  155
gagttataca cagggctggg atctattctt tttatctttt tttattcttt ctttattcta      60

taaattataa ccacttgaat ataaacaaaa aaaacacaca aaggtctagc ggaatttaca     120

gagggtctag cagaatttac aagttttcca gcaaaggtct agcagaattt acagataccc     180

acaactcaaa ggaaaaggac atgtaattat cattgactag cccatctcaa ttggtatagt     240

gattaaaatc acctagacca attgagatgt atgtctgaat tagttgtttt caaagcaaat     300

gaactagcga ttagtcgcta tgacttaacg gagcatgaaa ccaagctaat tttatgctgt     360

gtggcactac tcaaccccac gattgaaaac cctacaagga aagaacggac ggtatcgttc     420

acttataacc aatacgctca gatgatgaac atcagtaggg aaaatgctta tggtgtatta     480

gctaaagcaa ccagagagct gatgacgaga actgtggaaa tcaggaatcc tttggttaaa     540

ggctttgaga ttttccagtg gacaaactat gccaagttct caagcgaaaa attagaatta     600

gtttttagtg aagagatatt gccttatctt ttccagttaa aaaaattcat aaaatataat     660

ctggaacatg ttaagtcttt tgaaaacaaa tactctatga ggatttatga gtggttatta     720

aaagaactaa cacaaaagaa aactcacaag gcaaatatag agattagcct tgatgaattt     780

aagttcatgt taatgcttga aaataactac catgagttta aaaggcttaa ccaatgggtt     840

ttgaaaccaa taagtaaaga tttaaacact tacagcaata tgaaattggt ggttgataag     900

cgaggccgcc cgactgatac gttgattttc caagttgaac tagatagaca aatggatctc     960

gtaaccgaac ttgagaacaa ccagataaaa atgaatggtg acaaaatacc aacaaccatt    1020

acatcagatt cctacctaca taacggacta agaaaaacac tacacgatgc tttaactgca    1080

aaaattcagc tcaccagttt tgaggcaaaa tttttgagtg acatgcaaag taagtatgat    1140

ctcaatggtt cgttctcatg gctcacgcaa aaacaacgaa ccacactaga gaacatactg    1200

gctaaatacg gaaggatctg a                                              1221

<210>  156
<211>  140
<212>  DNA
<213>  Streptococcus pyogenes

<220>
<221>  primer_bind
<222>  (1)..(140)
<223>  Forward primer for Cas9 gene

<400>  156
aagtctagcg aaccgcactt aatacgactc actataggta ccggtgatac cagcatcgtc      60

ttgatgccct tggcagcacc ctgctaaggt aacaacaaga tgatggataa gaaatactca     120

ataggcttag atatcggcac                                                 140

<210>  157
<211>  69
<212>  DNA
<213>  Streptococcus pyogenes

<220>
<221>  primer_bind
<222>  (1)..(69)
<223>  Reverse primer for Cas9 gene

<400>  157
ctctagtagc tagcactgta cctaggactg agctagccgt caagttagct gtgctctaga      60

agctagcag                                                              69

<210>  158
<211>  311
<212>  DNA
<213>  Synthetic

<220>
<221>  promoter
<222>  (1)..(311)
<223>  pBAD arabinose inducible promoter

<400>  158
acttttcata ctcccgccat tcagagaaga aaccaattgt ccatattgca tcagacattg      60

ccgtcactgc gtcttttact ggctcttctc gctaaccaaa ccggtaaccc cgcttattaa     120

aagcattctg taacaaagcg ggaccaaagc catgacaaaa acgcgtaaca aaagtgtcta     180

taatcacggc agaaaagtcc acattgatta tttgcacggc gtcacacttt gctatgccat     240

agcattttta tccataagat tagcggatcc tacctgacgc tttttatcgc aactctctac     300

tgtttctcca t                                                          311

<210>  159
<211>  879
<212>  DNA
<213>  Escherichia coli

<220>
<221>  CDS
<222>  (1)..(879)
<223>  AraC gene encoding arabinose-inducible transcription factor

<400>  159
atg gct gaa gcg caa aat gat ccc ctg ctg ccg gga tac tcg ttt aat      48
Met Ala Glu Ala Gln Asn Asp Pro Leu Leu Pro Gly Tyr Ser Phe Asn
1               5                   10                  15

gcc cat ctg gtg gcg ggt tta acg ccg att gag gcc aac ggt tat ctc      96
Ala His Leu Val Ala Gly Leu Thr Pro Ile Glu Ala Asn Gly Tyr Leu
            20                  25                  30

gat ttt ttt atc gac cga ccg ctg gga atg aaa ggt tat att ctc aat      144
Asp Phe Phe Ile Asp Arg Pro Leu Gly Met Lys Gly Tyr Ile Leu Asn
        35                  40                  45

ctc acc att cgc ggt cag ggg gtg gtg aaa aat cag gga cga gaa ttt      192
Leu Thr Ile Arg Gly Gln Gly Val Val Lys Asn Gln Gly Arg Glu Phe
    50                  55                  60

gtt tgc cga ccg ggt gat att ttg ctg ttc ccg cca gga gag att cat      240
Val Cys Arg Pro Gly Asp Ile Leu Leu Phe Pro Pro Gly Glu Ile His
65                  70                  75                  80

cac tac ggt cgt cat ccg gag gct cgc gaa tgg tat cac cag tgg gtt      288
His Tyr Gly Arg His Pro Glu Ala Arg Glu Trp Tyr His Gln Trp Val
                85                  90                  95

tac ttt cgt ccg cgc gcc tac tgg cat gaa tgg ctt aac tgg ccg tca      336
Tyr Phe Arg Pro Arg Ala Tyr Trp His Glu Trp Leu Asn Trp Pro Ser
            100                 105                 110

ata ttt gcc aat acg ggg ttc ttt cgc ccg gat gaa gcg cac cag ccg      384
Ile Phe Ala Asn Thr Gly Phe Phe Arg Pro Asp Glu Ala His Gln Pro
        115                 120                 125

cat ttc agc gac ctg ttt ggg caa atc att aac gcc ggg caa ggg gaa      432
His Phe Ser Asp Leu Phe Gly Gln Ile Ile Asn Ala Gly Gln Gly Glu
    130                 135                 140

ggg cgc tat tcg gag ctg ctg gcg ata aat ctg ctt gag caa ttg tta      480
Gly Arg Tyr Ser Glu Leu Leu Ala Ile Asn Leu Leu Glu Gln Leu Leu
145                 150                 155                 160

ctg cgg cgc atg gaa gcg att aac gag tcg ctc cat cca ccg atg gat      528
Leu Arg Arg Met Glu Ala Ile Asn Glu Ser Leu His Pro Pro Met Asp
                165                 170                 175

aat cgg gta cgc gag gct tgt cag tac atc agc gat cac ctg gca gac      576
Asn Arg Val Arg Glu Ala Cys Gln Tyr Ile Ser Asp His Leu Ala Asp
            180                 185                 190

agc aat ttt gat atc gcc agc gtc gca cag cat gtt tgc ttg tcg ccg      624
Ser Asn Phe Asp Ile Ala Ser Val Ala Gln His Val Cys Leu Ser Pro

195                 200                 205

tcg cgt ctg tca cat ctt ttc cgc cag cag tta ggg att agc gtc tta        672
Ser Arg Leu Ser His Leu Phe Arg Gln Gln Leu Gly Ile Ser Val Leu
    210                 215                 220

agc tgg cgc gag gac caa cgt atc agc cag gcg aag ctg ctt ttg agc        720
Ser Trp Arg Glu Asp Gln Arg Ile Ser Gln Ala Lys Leu Leu Leu Ser
225                 230                 235                 240

acc acc cgg atg cct atc gcc acc gtc ggt cgc aat gtt ggt ttt gac        768
Thr Thr Arg Met Pro Ile Ala Thr Val Gly Arg Asn Val Gly Phe Asp
                245                 250                 255

gat caa ctc tat ttc tcg cgg gta ttt aaa aaa tgc acc ggg gcc agc        816
Asp Gln Leu Tyr Phe Ser Arg Val Phe Lys Lys Cys Thr Gly Ala Ser
            260                 265                 270

ccg agc gag ttc cgt gcc ggt tgt gaa gaa aaa gtg aat gat gta gcc        864
Pro Ser Glu Phe Arg Ala Gly Cys Glu Glu Lys Val Asn Asp Val Ala
        275                 280                 285

gtc aag ttg tca taa                                                    879
Val Lys Leu Ser
    290

<210>  160
<211>  292
<212>  PRT
<213>  Escherichia coli

<400>  160

Met Ala Glu Ala Gln Asn Asp Pro Leu Leu Pro Gly Tyr Ser Phe Asn
1               5                   10                  15

Ala His Leu Val Ala Gly Leu Thr Pro Ile Glu Ala Asn Gly Tyr Leu
            20                  25                  30

Asp Phe Phe Ile Asp Arg Pro Leu Gly Met Lys Gly Tyr Ile Leu Asn
        35                  40                  45

Leu Thr Ile Arg Gly Gln Gly Val Val Lys Asn Gln Gly Arg Glu Phe
    50                  55                  60

Val Cys Arg Pro Gly Asp Ile Leu Leu Phe Pro Pro Gly Glu Ile His
65                  70                  75                  80

His Tyr Gly Arg His Pro Glu Ala Arg Glu Trp Tyr His Gln Trp Val
                85                  90                  95

Tyr Phe Arg Pro Arg Ala Tyr Trp His Glu Trp Leu Asn Trp Pro Ser
            100                 105                 110

Ile Phe Ala Asn Thr Gly Phe Phe Arg Pro Asp Glu Ala His Gln Pro

115                 120                 125

His Phe Ser Asp Leu Phe Gly Gln Ile Ile Asn Ala Gly Gln Gly Glu
    130                 135                 140

Gly Arg Tyr Ser Glu Leu Leu Ala Ile Asn Leu Leu Glu Gln Leu Leu
145                 150                 155                 160

Leu Arg Arg Met Glu Ala Ile Asn Glu Ser Leu His Pro Pro Met Asp
                165                 170                 175

Asn Arg Val Arg Glu Ala Cys Gln Tyr Ile Ser Asp His Leu Ala Asp
            180                 185                 190

Ser Asn Phe Asp Ile Ala Ser Val Ala Gln His Val Cys Leu Ser Pro
        195                 200                 205

Ser Arg Leu Ser His Leu Phe Arg Gln Gln Leu Gly Ile Ser Val Leu
    210                 215                 220

Ser Trp Arg Glu Asp Gln Arg Ile Ser Gln Ala Lys Leu Leu Leu Ser
225                 230                 235                 240

Thr Thr Arg Met Pro Ile Ala Thr Val Gly Arg Asn Val Gly Phe Asp
                245                 250                 255

Asp Gln Leu Tyr Phe Ser Arg Val Phe Lys Lys Cys Thr Gly Ala Ser
            260                 265                 270

Pro Ser Glu Phe Arg Ala Gly Cys Glu Glu Lys Val Asn Asp Val Ala
        275                 280                 285

Val Lys Leu Ser
    290

<210>  161
<211>  103
<212>  DNA
<213>  Synthetic

<220>
<221>  Unsure
<222>  (1)..(103)
<223>  DNA for gRNA_Cas9 transcript targeting camR gene

<400>  161
ctataaccag accgttcagc gttttagagc tagaaatagc aagttaaaat aaggctagtc      60

cgttatcaac ttgaaaaagt ggcaccgagt cggtgctttt ttt                       103

<210>  162
<211>  365
<212>  DNA
<213>  Synthetic

<220>
<221>  terminator
<222>  (1)..(365)
<223>  TrrnB terminator for DNA coding gRNA_Cas9 transcript

<400>  162
gaagcttggg cccgaacaaa aactcatctc agaagaggat ctgaatagcg ccgtcgacca      60

tcatcatcat cattgagttt aaacggtctc cagcttggct gttttggcgg atgagagaag     120

attttcagcc tgatacagat taaatcagaa cgcagaagcg gtctgataaa acagaatttg     180

cctggcggca gtagcgcggt ggtcccacct gaccccatgc cgaactcaga agtgaaacgc     240

cgtagcgccg atggtagtgt ggggtctccc catgcgagag tagggaactg ccaggcatca     300

aataaaacga aaggctcagt cgaaagactg ggcctttcgt tttatctgtt gtttgtcggt     360

gaact                                                                 365

<210>  163
<211>  718
<212>  DNA
<213>  Synthetic

<220>
<221>  rep_origin
<222>  (1)..(718)
<223>  p15A origin of replication

<400>  163
ttaataagat gatcttcttg agatcgtttt ggtctgcgcg taatctcttg ctctgaaaac      60

gaaaaaaccg ccttgcaggg cggtttttcg aaggttctct gagctaccaa ctctttgaac     120

cgaggtaact ggcttggagg agcgcagtca ccaaaacttg tcctttcagt ttagccttaa     180

ccggcgcatg acttcaagac taactcctct aaatcaatta ccagtggctg ctgccagtgg     240

tgcttttgca tgtctttccg ggttggactc aagacgatag ttaccggata aggcgcagcg     300

gtcggactga acggggggtt cgtgcataca gtccagcttg gagcgaactg cctacccgga     360

actgagtgtc aggcgtggaa tgagacaaac gcggccataa cagcggaatg acaccggtaa     420

accgaaaggc aggaacagga gagcgcacga gggagccgcc agggggaaac gcctggtatc     480

tttatagtcc tgtcgggttt cgccaccact gatttgagcg tcagatttcg tgatgcttgt     540

caggggggcg gagcctatgg aaaaacggct ttgccgcggc cctctcactt ccctgttaag     600

tatcttcctg gcatcttcca ggaaatctcc gccccgttcg taagccattt ccgctcgccg     660

cagtcgaacg accgagcgta gcgagtcagt gagcgaggaa gcggaatata tccctagg       718

<210>  164
<211>  657
<212>  DNA
<213>  Escherichia coli

<220>
<221>  CDS
<222>  (1)..(657)
<223>  Chloramphenicol resistance gene

<400>  164
atg gag aaa aaa atc act gga tat acc acc gtt gat ata tcc caa tgg      48
Met Glu Lys Lys Ile Thr Gly Tyr Thr Thr Val Asp Ile Ser Gln Trp
1               5                   10                  15

cat cgt aaa gaa cat ttt gag gca ttt cag tca gtt gct caa tgt acc      96
His Arg Lys Glu His Phe Glu Ala Phe Gln Ser Val Ala Gln Cys Thr
            20                  25                  30

tat aac cag acc gtt cag ctg gat att acg gcc ttt tta aag acc gta      144
Tyr Asn Gln Thr Val Gln Leu Asp Ile Thr Ala Phe Leu Lys Thr Val
        35                  40                  45

aag aaa aat aag cac aag ttt tat ccg gcc ttt att cac att ctt gcc      192
Lys Lys Asn Lys His Lys Phe Tyr Pro Ala Phe Ile His Ile Leu Ala
    50                  55                  60

cgc ctg atg aat gct cat ccg gaa ttt cgt atg gca atg aaa gac ggt      240
Arg Leu Met Asn Ala His Pro Glu Phe Arg Met Ala Met Lys Asp Gly
65                  70                  75                  80

gag ctg gtg ata tgg gat agt gtt cac cct tgt tac acc gtt ttc cat      288
Glu Leu Val Ile Trp Asp Ser Val His Pro Cys Tyr Thr Val Phe His
                85                  90                  95

gag caa act gaa acg ttt tca tcg ctc tgg agt gaa tac cac gac gat      336
Glu Gln Thr Glu Thr Phe Ser Ser Leu Trp Ser Glu Tyr His Asp Asp
            100                 105                 110

ttc cgg cag ttt cta cac ata tat tcg caa gat gtg gcg tgt tac ggt      384
Phe Arg Gln Phe Leu His Ile Tyr Ser Gln Asp Val Ala Cys Tyr Gly
        115                 120                 125

gaa aac ctg gcc tat ttc cct aaa ggg ttt att gag aat atg ttt ttc      432
Glu Asn Leu Ala Tyr Phe Pro Lys Gly Phe Ile Glu Asn Met Phe Phe
    130                 135                 140

gtc tca gcc aat ccc tgg gtg agt ttc acc agt ttt gat tta aac gtg      480
Val Ser Ala Asn Pro Trp Val Ser Phe Thr Ser Phe Asp Leu Asn Val
145                 150                 155                 160

gcc aat atg gac aac ttc ttc gcc ccc gtt ttc acc atg ggc aaa tat      528
Ala Asn Met Asp Asn Phe Phe Ala Pro Val Phe Thr Met Gly Lys Tyr
                165                 170                 175

tat acg caa ggc gac aag gtg ctg atg ccg ctg gcg att cag gtt cat      576
Tyr Thr Gln Gly Asp Lys Val Leu Met Pro Leu Ala Ile Gln Val His
            180                 185                 190

cat gcc gtt tgt gat ggc ttc cat gtc ggc aga atg ctt aat gaa tta      624

His Ala Val Cys Asp Gly Phe His Val Gly Arg Met Leu Asn Glu Leu
        195                 200                 205

caa cag tac tgc gat gag tgg cag ggc ggg gcg                         657
Gln Gln Tyr Cys Asp Glu Trp Gln Gly Gly Ala
    210                 215

<210>  165
<211>  219
<212>  PRT
<213>  Escherichia coli

<400>  165

Met Glu Lys Lys Ile Thr Gly Tyr Thr Thr Val Asp Ile Ser Gln Trp
1               5                   10                  15

His Arg Lys Glu His Phe Glu Ala Phe Gln Ser Val Ala Gln Cys Thr
            20                  25                  30

Tyr Asn Gln Thr Val Gln Leu Asp Ile Thr Ala Phe Leu Lys Thr Val
        35                  40                  45

Lys Lys Asn Lys His Lys Phe Tyr Pro Ala Phe Ile His Ile Leu Ala
    50                  55                  60

Arg Leu Met Asn Ala His Pro Glu Phe Arg Met Ala Met Lys Asp Gly
65                  70                  75                  80

Glu Leu Val Ile Trp Asp Ser Val His Pro Cys Tyr Thr Val Phe His
                85                  90                  95

Glu Gln Thr Glu Thr Phe Ser Ser Leu Trp Ser Glu Tyr His Asp Asp
            100                 105                 110

Phe Arg Gln Phe Leu His Ile Tyr Ser Gln Asp Val Ala Cys Tyr Gly
        115                 120                 125

Glu Asn Leu Ala Tyr Phe Pro Lys Gly Phe Ile Glu Asn Met Phe Phe
    130                 135                 140

Val Ser Ala Asn Pro Trp Val Ser Phe Thr Ser Phe Asp Leu Asn Val
145                 150                 155                 160

Ala Asn Met Asp Asn Phe Phe Ala Pro Val Phe Thr Met Gly Lys Tyr
                165                 170                 175

Tyr Thr Gln Gly Asp Lys Val Leu Met Pro Leu Ala Ile Gln Val His
            180                 185                 190

His Ala Val Cys Asp Gly Phe His Val Gly Arg Met Leu Asn Glu Leu
        195                 200                 205

Gln Gln Tyr Cys Asp Glu Trp Gln Gly Gly Ala
    210                 215

<210>  166
<211>  20
<212>  DNA
<213>  Synthetic

<220>
<221>  misc_binding
<222>  (1)..(20)
<223>  CmR + strand binding site for gRNA spacer sequence

<400>  166
ctataaccag accgttcagc                                                   20

<210>  167
<211>  160
<212>  DNA
<213>  Synthetic

<220>
<221>  promoter
<222>  (1)..(160)
<223>  ProC synthetic promoter

<400>  167
ttctagagca cagctaacac cacgtcgtcc ctatctgctg ccctaggtct atgagtggtt       60

gctggataac tttacgggca tgcataaggc tcgtatgata tattcaggga gaccacaacg      120

gtttccctct acaaataatt ttgtttaact tttactagag                             160

<210>  168
<211>  1368
<212>  PRT
<213>  Streptococcus pyogenes

<220>
<221>  mat_peptide
<222>  (1)..(1368)
<223>  Mutant dCas9 protein

<400>  168

Met Asp Lys Lys Tyr Ser Ile Gly Leu Ala Ile Gly Thr Asn Ser Val
1               5                   10                  15

Gly Trp Ala Val Ile Thr Asp Asp Tyr Lys Val Pro Ser Lys Lys Phe
            20                  25                  30

Lys Val Leu Gly Asn Thr Asp Arg His Ser Ile Lys Lys Asn Leu Ile
        35                  40                  45

Gly Ala Leu Leu Phe Asp Ser Gly Glu Thr Ala Glu Ala Thr Arg Leu
    50                  55                  60

Lys Arg Thr Ala Arg Arg Arg Tyr Thr Arg Arg Lys Asn Arg Ile Cys
65                  70                  75                  80

Tyr Leu Gln Glu Ile Phe Ser Asn Glu Met Ala Lys Val Asp Asp Ser
                85                  90                  95

Phe Phe His Arg Leu Glu Glu Ser Phe Leu Val Glu Glu Asp Lys Lys
            100                 105                 110

His Glu Arg His Pro Ile Phe Gly Asn Ile Val Asp Glu Val Ala Tyr
        115                 120                 125

His Glu Lys Tyr Pro Thr Ile Tyr His Leu Arg Lys Lys Leu Val Asp
    130                 135                 140

Ser Thr Asp Lys Ala Asp Leu Arg Leu Ile Tyr Leu Ala Leu Ala His
145                 150                 155                 160

Met Ile Lys Phe Arg Gly His Phe Leu Ile Glu Gly Asp Leu Asn Pro
                165                 170                 175

Asp Asn Ser Asp Val Asp Lys Leu Phe Ile Gln Leu Val Gln Thr Tyr
            180                 185                 190

Asn Gln Leu Phe Glu Glu Asn Pro Ile Asn Ala Ser Gly Val Asp Ala
        195                 200                 205

Lys Ala Ile Leu Ser Ala Arg Leu Ser Lys Ser Arg Arg Leu Glu Asn
    210                 215                 220

Leu Ile Ala Gln Leu Pro Gly Glu Lys Lys Asn Gly Leu Phe Gly Asn
225                 230                 235                 240

Leu Ile Ala Leu Ser Leu Gly Leu Thr Pro Asn Phe Lys Ser Asn Phe
                245                 250                 255

Asp Leu Ala Glu Asp Ala Lys Leu Gln Leu Ser Lys Asp Thr Tyr Asp
            260                 265                 270

Asp Asp Leu Asp Asn Leu Leu Ala Gln Ile Gly Asp Gln Tyr Ala Asp
        275                 280                 285

Leu Phe Leu Ala Ala Lys Asn Leu Ser Asp Ala Ile Leu Leu Ser Asp
    290                 295                 300

Ile Leu Arg Leu Asn Ser Glu Ile Thr Lys Ala Pro Leu Ser Ala Ser
305                 310                 315                 320

Met Ile Lys Arg Tyr Asp Glu His His Gln Asp Leu Thr Leu Leu Lys
                325                 330                 335

Ala Leu Val Arg Gln Gln Leu Pro Glu Lys Tyr Lys Glu Ile Phe Phe
            340                 345                 350

Asp Gln Ser Lys Asn Gly Tyr Ala Gly Tyr Ile Asp Gly Gly Ala Ser
        355                 360                 365

Gln Glu Glu Phe Tyr Lys Phe Ile Lys Pro Ile Leu Glu Lys Met Asp
    370                 375                 380

Gly Thr Glu Glu Leu Leu Ala Lys Leu Asn Arg Glu Asp Leu Leu Arg
385                 390                 395                 400

Lys Gln Arg Thr Phe Asp Asn Gly Ser Ile Pro His Gln Ile His Leu
                405                 410                 415

Gly Glu Leu His Ala Ile Leu Arg Arg Gln Glu Asp Phe Tyr Pro Phe
            420                 425                 430

Leu Lys Asp Asn Arg Glu Lys Ile Glu Lys Ile Leu Thr Phe Arg Ile
        435                 440                 445

Pro Tyr Tyr Val Gly Pro Leu Ala Arg Gly Asn Ser Arg Phe Ala Trp
    450                 455                 460

Met Thr Arg Lys Ser Glu Glu Thr Ile Thr Pro Trp Asn Phe Glu Glu
465                 470                 475                 480

Val Val Asp Lys Gly Ala Ser Ala Gln Ser Phe Ile Glu Arg Met Thr
                485                 490                 495

Asn Phe Asp Lys Asn Leu Pro Asn Glu Lys Val Leu Pro Lys His Ser
            500                 505                 510

Leu Leu Tyr Glu Tyr Phe Thr Val Tyr Asn Glu Leu Thr Lys Val Lys
        515                 520                 525

Tyr Val Thr Glu Gly Met Arg Lys Pro Ala Phe Leu Ser Gly Glu Gln
    530                 535                 540

Lys Lys Ala Ile Val Asp Leu Leu Phe Lys Thr Asn Arg Lys Val Thr
545                 550                 555                 560

Val Lys Gln Leu Lys Glu Asp Tyr Phe Lys Lys Ile Glu Cys Phe Asp
                565                 570                 575

Ser Val Glu Ile Ser Gly Val Glu Asp Arg Phe Asn Ala Ser Leu Gly
            580                 585                 590

Thr Tyr His Asp Leu Leu Lys Ile Ile Lys Asp Lys Asp Phe Leu Asp
        595                 600                 605

Asn Glu Glu Asn Glu Asp Ile Leu Glu Asp Ile Val Leu Thr Leu Thr
    610                 615                 620

Leu Phe Glu Asp Arg Glu Met Ile Glu Glu Arg Leu Lys Thr Tyr Ala
625                 630                 635                 640

His Leu Phe Asp Asp Lys Val Met Lys Gln Leu Lys Arg Arg Arg Tyr
                645                 650                 655

Thr Gly Trp Gly Arg Leu Ser Arg Lys Leu Ile Asn Gly Ile Arg Asp
            660                 665                 670

Lys Gln Ser Gly Lys Thr Ile Leu Asp Phe Leu Lys Ser Asp Gly Phe
        675                 680                 685

Ala Asn Arg Asn Phe Met Gln Leu Ile His Asp Asp Ser Leu Thr Phe
    690                 695                 700

Lys Glu Asp Ile Gln Lys Ala Gln Val Ser Gly Gln Gly Asp Ser Leu
705                 710                 715                 720

His Glu His Ile Ala Asn Leu Ala Gly Ser Pro Ala Ile Lys Lys Gly
                725                 730                 735

Ile Leu Gln Thr Val Lys Val Val Asp Glu Leu Val Lys Val Met Gly
            740                 745                 750

Arg His Lys Pro Glu Asn Ile Val Ile Glu Met Ala Arg Glu Asn Gln
        755                 760                 765

Thr Thr Gln Lys Gly Gln Lys Asn Ser Arg Glu Arg Met Lys Arg Ile
    770                 775                 780

Glu Glu Gly Ile Lys Glu Leu Gly Ser Gln Ile Leu Lys Glu His Pro

785                 790                 795                 800

Val Glu Asn Thr Gln Leu Gln Asn Glu Lys Leu Tyr Leu Tyr Tyr Leu
                805                 810                 815

Gln Asn Gly Arg Asp Met Tyr Val Asp Gln Glu Leu Asp Ile Asn Arg
            820                 825                 830

Leu Ser Asp Tyr Asp Val Asp Ala Ile Val Pro Gln Ser Phe Ile Lys
        835                 840                 845

Asp Asp Ser Ile Asp Asn Lys Val Leu Thr Arg Ser Asp Lys Asn Arg
    850                 855                 860

Gly Lys Ser Asp Asn Val Pro Ser Glu Glu Val Val Lys Lys Met Lys
865                 870                 875                 880

Asn Tyr Trp Arg Gln Leu Leu Asn Ala Lys Leu Ile Thr Gln Arg Lys
                885                 890                 895

Phe Asp Asn Leu Thr Lys Ala Glu Arg Gly Gly Leu Ser Glu Leu Asp
            900                 905                 910

Lys Ala Gly Phe Ile Lys Arg Gln Leu Val Glu Thr Arg Gln Ile Thr
        915                 920                 925

Lys His Val Ala Gln Ile Leu Asp Ser Arg Met Asn Thr Lys Tyr Asp
    930                 935                 940

Glu Asn Asp Lys Leu Ile Arg Glu Val Lys Val Ile Thr Leu Lys Ser
945                 950                 955                 960

Lys Leu Val Ser Asp Phe Arg Lys Asp Phe Gln Phe Tyr Lys Val Arg
                965                 970                 975

Glu Ile Asn Asn Tyr His His Ala His Asp Ala Tyr Leu Asn Ala Val
            980                 985                 990

Val Gly Thr Ala Leu Ile Lys Lys  Tyr Pro Lys Leu Glu  Ser Glu Phe
        995                 1000                1005

Val Tyr  Gly Asp Tyr Lys Val  Tyr Asp Val Arg Lys  Met Ile Ala
    1010                1015                1020

Lys Ser  Glu Gln Glu Ile Gly  Lys Ala Thr Ala Lys  Tyr Phe Phe
    1025                1030                1035

Tyr Ser Asn Ile Met Asn Phe Phe Lys Thr Glu Ile Thr Leu Ala
    1040                1045                1050

Asn Gly Glu Ile Arg Lys Arg Pro Leu Ile Glu Thr Asn Gly Glu
    1055                1060                1065

Thr Gly Glu Ile Val Trp Asp Lys Gly Arg Asp Phe Ala Thr Val
    1070                1075                1080

Arg Lys Val Leu Ser Met Pro Gln Val Asn Ile Val Lys Lys Thr
    1085                1090                1095

Glu Val Gln Thr Gly Gly Phe Ser Lys Glu Ser Ile Leu Pro Lys
    1100                1105                1110

Arg Asn Ser Asp Lys Leu Ile Ala Arg Lys Lys Asp Trp Asp Pro
    1115                1120                1125

Lys Lys Tyr Gly Gly Phe Asp Ser Pro Thr Val Ala Tyr Ser Val
    1130                1135                1140

Leu Val Val Ala Lys Val Glu Lys Gly Lys Ser Lys Lys Leu Lys
    1145                1150                1155

Ser Val Lys Glu Leu Leu Gly Leu Thr Ile Met Glu Arg Ser Ser
    1160                1165                1170

Phe Glu Lys Asn Pro Ile Asp Phe Leu Glu Ala Lys Gly Tyr Lys
    1175                1180                1185

Glu Val Lys Lys Asp Leu Ile Ile Lys Leu Pro Lys Tyr Ser Leu
    1190                1195                1200

Phe Glu Leu Glu Asn Gly Arg Lys Arg Met Leu Ala Ser Ala Gly
    1205                1210                1215

Glu Leu Gln Lys Gly Asn Glu Leu Ala Leu Pro Ser Lys Tyr Val
    1220                1225                1230

Asn Phe Leu Tyr Leu Ala Ser His Tyr Glu Lys Leu Lys Gly Ser
    1235                1240                1245

Pro Glu Asp Asn Glu Gln Lys Gln Leu Phe Val Glu Gln His Lys
    1250                1255                1260

His Tyr Leu Asp Glu Ile Ile Glu Gln Ile Ser Glu Phe Ser Lys
    1265                1270                1275

Arg Val Ile Leu Ala Asp Ala Asn Leu Asp Lys Val Leu Ser Ala
    1280                1285                1290

Tyr Asn Lys His Arg Asp Lys Pro Ile Arg Glu Gln Ala Glu Asn
    1295                1300                1305

Ile Ile His Leu Phe Thr Leu Thr Asn Leu Gly Ala Pro Ala Ala
    1310                1315                1320

Phe Lys Tyr Phe Asp Thr Thr Ile Asp Arg Lys Arg Tyr Thr Ser
    1325                1330                1335

Thr Lys Glu Val Leu Asp Ala Thr Leu Ile His Gln Ser Ile Thr
    1340                1345                1350

Gly Leu Tyr Glu Thr Arg Ile Asp Leu Ser Gln Leu Gly Gly Asp
    1355                1360                1365